# EUROPEAN PATENT APPLICATION

(11) **EP 2 281 891 A2**
(43) Date of publication of application: **09.02.2011**
(21) Application number: 10179497.2
(22) Date of filing: 19.06.2001
(51) Int. Cl.: C12N 15/63, C07K 14/315, A61K 39/09

(54) **Streptococcus antigens**

(30) Priority: 20.06.2000 US 212683 P
(62) Divisional of application: 01949136.4
(71) Applicant: ID Biomedical Corporation, Laval, QC H7V 3S8 (CA)
(72) Inventor: Hamel, Josée, Sillery Québec G1T 1N6 (CA); Ouellet, Catherine, St-Jean-Chrysostome Québec G6Z 2Z8 (CA); Charland, Nathalie, Breakeyville Québec G0S 1E3 (CA); Martin, Denis, St-Augustin Québec G3A 1E9 (CA); Brodeur, Bernard, Sillery Québec G1T 1N6 (CA)
(74) Representative: Johnston, Caroline Louise

(57) **Abstract**

Streptococcus polypeptides and polynucleotides encoding them are disclosed. Said polypeptides may be useful vaccine components for the prophylaxis or therapy of streptococcus infection in animals. Also disclosed are recombinant methods of producing the protein antigens as well as diagnostic assays for detecting streptococcus bacterial infection.

SEQUENCE LISTING

<110> SHIRE BIOCHEM INC.
      HAMEL, Josee
      OUELLET, Catherine
      CHARLAND, Nathalie
      MARTIN, Denis
      BRODEUR, Bernard

<120> STREPTOCOCCUS ANTIGENS


<130> 12806-19PCT

<140> PCT/CA01/00908
<141> 2001-06-19

<150> US 60/212,683
<151> 2000-06-20

<160> 384

<170> FastSEQ for Windows Version 4.0

<210> 1
<211> 3120
<212> DNA
<213> BVH-3

<400> 1
atgaaattta gtaaaaaata tatagcagct ggatcagctg ttatcgtatc cttgagtcta      60
tgtgcctatg cactaaacca gcatcgttcg caggaaaata aggacaataa tcgtgtctct     120
tatgtggatg gcagccagtc aagtcagaaa agtgaaaact tgacaccaga ccaggttagc     180
cagaaagaag gaattcaggc tgagcaaatt gtaatcaaaa ttacagatca gggctatgta     240
acgtcacacg gtgaccacta tcattactat aatgggaaag ttccttatga tgccctcttt     300
agtgaagaac tcttgatgaa ggatccaaac tatcaactta aagacgctga tattgtcaat     360
gaagtcaagg gtggttatat catcaaggtc gatggaaaat attatgtcta cctgaaagat     420
gcagctcatg ctgataatgt tcgaactaaa gatgaaatca atcgtcaaaa acaagaacat     480
gtcaaagata atgagaaggt taactctaat gttgctgtag caaggtctca gggacgatat     540
acgacaaatg atggttatgt ctttaatcca gctgatatta tcgaagatac gggtaatgct     600
tatatcgttc ctcatggagg tcactatcac tacattccca aaagcgattt atctgctagt     660
gaattagcag cagctaaagc acatctggct ggaaaaaata tgcaaccgag tcagttaagc     720
tattcttcaa cagctagtga caataacacg caatctgtag caaaaggatc aactagcaag     780
ccagcaaata aatctgaaaa tctccagagt cttttgaagg aactctatga ttcacctagc     840
gcccaacgtt acagtgaatc agatggcctg gtctttgacc ctgctaagat tatcagtcgt     900
acaccaaatg gagttgcgat tccgcatggc gaccattacc actttattcc ttacagcaag     960
ctttctgctt tagaagaaaa gattgccaga atggtgccta tcagtggaac tggttctaca    1020
gtttctacaa atgcaaaacc taatgaagta gtgtctagtc taggcagtct ttcaagcaat    1080
ccttcttctt taacgacaag taaggagctc tcttcagcat ctgatggtta tatttttaat    1140
ccaaaagata tcgttgaaga aacggctaca gcttatattg taagacatgg tgatcatttc    1200
cattacattc caaaatcaaa tcaaattggg caaccgactc ttccaaacaa tagtctagca    1260
acaccttctc catctcttcc aatcaatcca ggaacttcac atgagaaaca tgaagaagat    1320
ggatacggat ttgatgctaa tcgtattatc gctgaagatg aatcaggttt tgtcatgagt    1380
cacggagacc acaatcatta tttcttcaag aaggacttga cagaagagca aattaaggct    1440
gcgcaaaaac atttagagga agttaaaact agtcataatg gattagattc tttgtcatct    1500
catgaacagg attatccagg taatgccaaa gaaatgaaag atttagataa aaaaatcgaa    1560
gaaaaaattg ctggcattat gaaacaatat ggtgtcaaac gtgaaagtat tgtcgtgaat    1620
aaagaaaaaa atgcgattat ttatccgcat ggagatcacc atcatgcaga tccgattgat    1680
gaacataaac cggttggaat tggtcattct cacagtaact atgaactgtt taaacccgaa    1740
gaaggagttg ctaaaaaaga agggaataaa gtttatactg gagaagaatt aacgaatgtt    1800
gttaatttgt taaaaaatag tacgtttaat aatcaaaact ttactctagc caatggtcaa    1860
aaacgcgttt cttttagttt tccgcctgaa ttggagaaaa aattaggtat caatatgcta    1920
gtaaaattaa taacaccaga tggaaaagta ttggagaaag tatctggtaa agtatttgga    1980
gaaggagtag ggaatattgc aaactttgaa ttagatcaac cttatttacc aggacaaaca    2040
tttaagtata ctatcgcttc aaaagattat ccagaagtaa gttatgatgg tacatttaca    2100
gttccaacct ctttagctta caaaatggcc agtcaaacga ttttctatcc tttccatgca    2160
ggggatactt atttaagagt gaaccctcaa tttgcagtgc ctaaaggaac tgatgcttta    2220

gtcagagtgt ttgatgaatt tcatggaaat gcttatttag aaaataacta taaagttggt 2280
gaaatcaaat taccgattcc gaaattaaac caaggaacaa ccagaacggc cggaaataaa 2340
attcctgtaa ccttcatggc aaatgcttat ttggacaatc aatcgactta tattgtggaa 2400
gtacctatct tggaaaaaga aaatcaaact gataaaccaa gtattctacc acaatttaaa 2460
aggaataaag cacaagaaaa ctcaaaactt gatgaaaagg tagaagaacc aaagactagt 2520
gagaaggtag aaaaagaaaa actttctgaa actgggaata gtactagtaa ttcaacgtta 2580
gaagaagttc ctacagtgga tcctgtacaa gaaaaagtag caaaatttgc tgaaagttat 2640
gggatgaagc tagaaaatgt cttgtttaat atggacggaa caattgaatt atatttacca 2700
tcaggagaag tcattaaaaa gaatatggca gattttacag gagaagcacc tcaaggaaat 2760
ggtgaaaata aaccatctga aatggaaaaa gtatctactg gaacagttga gaaccaacca 2820
acagaaaata aaccagcaga ttctttacca gaggcaccaa acgaaaaacc tgtaaaacca 2880
gaaaactcaa cggataatgg aatgttgaat ccagaaggga atgtggggag tgaccctatg 2940
ttagatccag cattagagga agctccagca gtagatcctg tacaagaaaa attagaaaaa 3000
tttacagcta gttacggatt aggcttagat agtgttatat tcaatatgga tggaacgatt 3060
gaattaagat tgccaagtgg agaagtgata aaaaagaatt tatctgattt catagcgtaa 3120

<210> 2
<211> 5048
<212> DNA
<213> BVH-3

<400> 2
   aattccttgt cgggtaagtt ccgacccgca cgaaaggcgt aatgatttgg gcactgtctc 60
   aacgagagac tcggtgaaat tttagtacct gtgaagatgc aggttacccg cgacaggacg 120
   gaaagacccc atggagcttt actgcagttt gatattgagt gtctgtacca catgtacagg 180
   ataggtagga gtctaagaga tcgggacgcc agtttcgaag gagacgctgt tgggatacta 240
   cccttgtgtt atggccactc taacccagat aggtgatccc tatcggagac agtgtctgac 300
   gggcagtttg actggggcgg tcgcctccta aaggtaacgg aggcgcccaa aaggttccct 360
   cagaatggtt ggaaatcatt cgcagagtgt aaaggtataa gggagcttga ctgcgagagc 420
   tacaactcga gcagggacga aagtcgggct tagtgatccg gtggttccgt atggaagggc 480
   catcgctcaa cggataaaag ctaccctggg gataacaggc ttatctcccc caagagttca 540
   catcgacggg gaggtttggc acctcgatgt cggctcgtcg catcctgggg ctgtagtcgg 600
   tcccaagggt tgggctgttc gcccattaaa gcggcacgcg agctgggttc agaacgtcgt 660
   gagacagttc ggtccctatc cgtcgcgggc gtaggaaatt tgagaggatc tgctcctagt 720
   acgagaggac cagagtggac ttaccgctgg tgtaccagtt gtcttgccaa aggcatcgct 780
   gggtagctat gtagggaagg gataaacgct gaaagcatct aagtgtgaaa cccacctcaa 840
   gatgagattt cccatgatta tatatcagta agagccctga gagatgatca ggtagatagg 900
   ttagaagtgg aagtgtggcg acacatgtag cggactaata ctaatagctc gaggacttat 960
   ccaaagtaac tgagaatatg aaagcgaacg gttttcttaa attgaataga tattcaattt 1020
   tgagtaggta ttactcagag ttaagtgacg atagcctagg agatacacct gtacccatgc 1080
   cgaacacaga agttaagccc tagaacgccg gaagtagttg ggggttgccc cctgtgagat 1140
   agggaagtcg cttagctcta gggagtttag ctcagctggg agagcatctg ccttacaagc 1200
   agagggtcag cggttcgatc ccgttaactc ccaaaggtcc cgtagtgtag cggttatcac 1260
   gtcgccctgt cacggcgaag atcgcgggtt cgattcccgt cgggaccgtt taaggtaacg 1320
   caagttattt tagactcgtt agctcagttg gtagagcaat tgacttttaa tcaatgggtc 1380
   actggttcga gcccagtacg ggtcatatat gcgggtttgg cggaattcta atctctttga 1440
   aatcatcttc tctcactttc caaaactcta ttacctctta ttataccaca tttcaatctt 1500
   caacttccca gtaatataag cacctctggc gaaagaagtt tcaatgtcct aaagtaataa 1560
   gtgaatccaa ttcaggaact ccaagaacaa aagaaacatc tggtgtcaca agtattggat 1620
   ggcacagagt cacgtggtag tctgacccta gcagaaattt taaatagtaa actatttact 1680
   ggttaattaa atggttaaat aaccggttta gaaaactatt taataaagta aaagaagttg 1740
   agaaaaaact tcatcattta ttgaaatgag ggatttatga aatttagtaa aaaatatata 1800
   gcagctggat cagctgttat cgtatccttg agtctatgtg cctatgcact aaaccagcat 1860
   cgttcgcagg aaaataagga caataatcgt gtctcttatg tggatggcag ccagtcaagt 1920
   cagaaaagtg aaaacttgac accagaccag gttagccaga aagaaggaat tcaggctgag 1980
   caaattgtaa tcaaaattac agatcagggc tatgtaacgt cacacggtga ccactatcat 2040
   tactataatg ggaaagttcc ttatgatgcc ctctttagtg aagaactctt gatgaaggat 2100
   ccaaactatc aacttaaaga cgctgatatt gtcaatgaag tcaagggtgg ttatatcatc 2160
   aaggtcgatg gaaaatatta tgtctacctg aaagatgcag ctcatgctga taatgttcga 2220
   actaaagatg aaatcaatcg tcaaaaacaa gaacatgtca aagataatga gaaggttaac 2280
   tctaatgttg ctgtagcaag gtctcaggga cgatatacga caaatgatgg ttatgtcttt 2340
   aatccagctg atattatcga agatacgggt aatgcttata tcgttcctca tggaggtcac 2400
   tatcactaca ttcccaaaag cgatttatct gctagtgaat tagcagcagc taaagcacat 2460
   ctggctggaa aaaatatgca accgagtcag ttaagctatt cttcaacagc tagtgacaat 2520
   aacacgcaat ctgtagcaaa aggatcaact agcaagccag caaataaatc tgaaaatctc 2580
   cagagtcttt tgaaggaact ctatgattca cctagcgccc aacgttacag tgaatcagat 2640
   ggcctggtct ttgaccctgc taagattatc agtcgtacac caaatggagt tgcgattccg 2700
   catggcgacc attaccactt tattccttac agcaagcttt ctgctttaga agaaaagatt 2760
   gccagaatgg tgcctatcag tggaactggt tctacagttt ctacaaatgc aaaacctaat 2820
   gaagtagtgt ctagtctagg cagtctttca agcaatcctt cttctttaac gacaagtaag 2880
   gagctctctt cagcatctga tggttatatt tttaatccaa aagatatcgt tgaagaaacg 2940
   gctacagctt atattgtaag acatggtgat catttccatt acattccaaa atcaaatcaa 3000
   attgggcaac cgactcttcc aaacaatagt ctagcaacac cttctccatc tcttccaatc 3060
   aatccaggaa cttcacatga gaaacatgaa gaagatggat acggatttga tgctaatcgt 3120
   attatcgctg aagatgaatc aggttttgtc atgagtcacg gagaccacaa tcattatttc 3180
   ttcaagaagg acttgacaga agagcaaatt aaggctgcgc aaaaacattt agaggaagtt 3240
   aaaactagtc ataatggatt agattctttg tcatctcatg aacaggatta tccaggtaat 3300
   gccaaagaaa tgaaagattt agataaaaaa atcgaagaaa aaattgctgg cattatgaaa 3360
   caatatggtg tcaaacgtga aagtattgtc gtgaataaag aaaaaaatgc gattatttat 3420
   ccgcatggag atcaccatca tgcagatccg attgatgaac ataaaccggt tggaattggt 3480
   cattctcaca gtaactatga actgtttaaa cccgaagaag gagttgctaa aaaagaaggg 3540
   aataaagttt atactggaga agaattaacg aatgttgtta atttgttaaa aaatagtacg 3600
   tttaataatc aaaactttac tctagccaat ggtcaaaaac gcgtttcttt tagttttccg 3660
   cctgaattgg agaaaaaatt aggtatcaat atgctagtaa aattaataac accagatgga 3720
   aaagtattgg agaaagtatc tggtaaagta tttggagaag gagtagggaa tattgcaaac 3780
   tttgaattag atcaacctta tttaccagga caaacattta agtatactat cgcttcaaaa 3840
   gattatccag aagtaagtta tgatggtaca tttacagttc caacctcttt agcttacaaa 3900
   atggccagtc aaacgatttt ctatcctttc catgcagggg atacttattt aagagtgaac 3960
   cctcaatttg cagtgcctaa aggaactgat gctttagtca gagtgtttga tgaatttcat 4020
   ggaaatgctt atttagaaaa taactataaa gttggtgaaa tcaaattacc gattccgaaa 4080
   ttaaaccaag gaacaaccag aacggccgga aataaaattc ctgtaacctt catggcaaat 4140
   gcttatttgg acaatcaatc gacttatatt gtggaagtac ctatcttgga aaaagaaaat 4200
   caaactgata aaccaagtat tctaccacaa tttaaaagga ataaagcaca agaaaactca 4260
   aaacttgatg aaaaggtaga agaaccaaag actagtgaga aggtagaaaa agaaaaactt 4320
   tctgaaactg ggaatagtac tagtaattca acgttagaag aagttcctac agtggatcct 4380
   gtacaagaaa aagtagcaaa atttgctgaa agttatggga tgaagctaga aaatgtcttg 4440
   tttaatatgg acggaacaat tgaattatat ttaccatcag gagaagtcat taaaaagaat 4500
   atggcagatt ttacaggaga agcacctcaa ggaaatggtg aaaataaacc atctgaaaat 4560
   ggaaaagtat ctactggaac agttgagaac caaccaacag aaaataaacc agcagattct 4620
   ttaccagagg caccaaacga aaaacctgta aaaccagaaa actcaacgga taatggaatg 4680
   ttgaatccag aagggaatgt ggggagtgac cctatgttag atccagcatt agaggaagct 4740
   ccagcagtag atcctgtaca agaaaaatta gaaaaattta cagctagtta cggattaggc 4800
   ttagatagtg ttatattcaa tatggatgga acgattgaat taagattgcc aagtggagaa 4860
   gtgataaaaa agaatttatc tgatttcata gcgtaaggaa tagcagtaga aaaagtctga 4920
   atcaaaaatg aagttctctc aaaagttaga aataaaactc tgactttggg agaatttcat 4980
   tttattatta atatataaaa tttcttgaca tacaacttaa aaagaggtgg aatatttact 5040
   agttaatt 5048

<210> 3
<211> 2523
<212> DNA
<213> BVH-11

<400> 3
atgaaaatca ataaaaaata tctagctggg tcagtagcta cacttgtttt aagtgtctgt 60
gcttatgaac taggtttgca tcaagctcaa actgtaaaag aaaataatcg tgtttcctat 120
atagatggaa aacaagcgac gcaaaaaacg gagaatttga ctcctgatga ggttagcaag 180
cgtgaaggaa tcaacgccga acaaatcgtc atcaagatta cggatcaagg ttatgtgacc 240
tctcatggag accattatca ttactataat ggcaaggtcc cttatgatgc catcatcagt 300
gaagagctcc tcatgaaaga tccgaattat cagttgaagg attcagacat tgtcaatgaa 360
atcaagggtg gttatgtcat taaggtaaac ggtaaatact atgtttacct taaggatgca 420
gctcatgcgg ataatgtccg tacaaaagaa gaaatcaatc ggcaaaaaca agaacatagt 480
cagcatcgtg aaggagggac ttcagcaaac gatggtgcgg tagcctttgc acgttcacag 540
ggacgctaca ccacagatga tggttatatc ttcaatgcat ctgatatcat cgaagatacg 600
ggcgatgcct atatcgttcc tcatggagat cattaccatt acattcctaa gaatgagtta 660
tcagctagcg agttggctgc tgcagaagcc ttcctatctg gtcgggaaaa tctgtcaaat 720
ttaagaacct atcgccgaca aaatagcgat aacactccaa gaacaaactg ggtaccttct 780
gtaagcaatc caggaactac aaatactaac acaagcaaca acagcaacac taacagtcaa 840
gcaagtcaaa gtaatgacat tgatagtctc ttgaaacagc tctacaaact gcctttgagt 900
caacgccatg tagaatctga tggccttatt ttcgacccag cgcaaatcac aagtcgaacc 960
gccagaggtg tagctgtccc tcatggtaac cattaccact ttatccctta tgaacaaatg 1020
tctgaattgg aaaaacgaat tgctcgtatt attccccttc gttatcgttc aaaccattgg 1080
gtaccagatt caagaccaga agaaccaagt ccacaaccga ctccagaacc tagtccaagt 1140
ccgcaacctg caccaaatcc tcaaccagct ccaagcaatc caattgatga gaaattggtc 1200

aaagaagctg ttcgaaaagt aggcgatggt tatgtctttg aggagaatgg agtttctcgt 1260
tatatcccag ccaagaatct ttcagcagaa acagcagcag gcattgatag caaactggcc 1320
aagcaggaaa gtttatctca taagctagga gctaagaaaa ctgacctccc atctagtgat 1380
cgagaatttt acaataaggc ttatgactta ctagcaagaa ttcaccaaga tttacttgat 1440
aataaaggtc gacaagttga ttttgaggct ttggataacc tgttggaacg actcaaggat 1500
gtctcaagtg ataaagtcaa gttagtggat gatattcttg ccttcttagc tccgattcgt 1560
catccagaac gtttaggaaa accaaatgcg caaattacct acactgatga tgagattcaa 1620
gtagccaagt tggcaggcaa gtacacaaca gaagacggtt atatctttga tcctcgtgat 1680
ataaccagtg atgaggggga tgcctatgta actccacata tgacccatag ccactggatt 1740
aaaaaagata gtttgtctga agctgagaga gcggcagccc aggcttatgc taaagagaaa 1800
ggtttgaccc ctccttcgac agaccatcag gattcaggaa atactgaggc aaaaggagca 1860
gaagctatct acaaccgcgt gaaagcagct aagaaggtgc cacttgatcg tatgccttac 1920
aatcttcaat atactgtaga agtcaaaaac ggtagtttaa tcatacctca ttatgaccat 1980
taccataaca tcaaatttga gtggtttgac gaaggccttt atgaggcacc taaggggtat 2040
actcttgagg atcttttggc gactgtcaag tactatgtcg aacatccaaa cgaacgtccg 2100
cattcagata atggttttgg taacgctagc gaccatgttc aaagaaacaa aaatggtcaa 2160
gctgatacca atcaaacgga aaaaccaagc gaggagaaac ctcagacaga aaaacctgag 2220
gaagaaaccc ctcgagaaga gaaaccacaa agcgagaaac cagagtctcc aaaaccaaca 2280
gaggaaccag aagaagaatc accagaggaa tcagaagaac ctcaggtcga gactgaaaag 2340
gttgaagaaa aactgagaga ggctgaagat ttacttggaa aaatccagga tccaattatc 2400
aagtccaatg ccaaagagac tctcacagga ttaaaaaata atttactatt tggcacccag 2460
gacaacaata ctattatggc agaagctgaa aaactattgg ctttattaaa ggagagtaag 2520
taa 2523

<210> 4
<211> 2647
<212> DNA
<213> BVH-11

<400> 4
cagagatctt agtgaatcaa atatacttaa gaaaagagga aagaatgaaa atcaataaaa 60
aatatctagc tgggtcagta gctacacttg ttttaagtgt ctgtgcttat gaactaggtt 120
tgcatcaagc tcaaactgta aaagaaaata atcgtgtttc ctatatagat ggaaaacaag 180
cgacgcaaaa aacggagaat ttgactcctg atgaggttag caagcgtgaa ggaatcaacg 240
ccgaacaaat cgtcatcaag attacggatc aaggttatgt gacctctcat ggagaccatt 300
atcattacta taatggcaag gtcccttatg atgccatcat cagtgaagag ctcctcatga 360
aagatccgaa ttatcagttg aaggattcag acattgtcaa tgaaatcaag ggtggttatg 420
tcattaaggt aaacggtaaa tactatgttt accttaagga tgcagctcat gcggataatg 480
tccgtacaaa agaagaaatc aatcggcaaa aacaagaaca tagtcagcat cgtgaaggag 540
ggacttcagc aaacgatggt gcggtagcct ttgcacgttc acagggacgc tacaccacag 600
atgatggtta tatcttcaat gcatctgata tcatcgaaga tacgggcgat gcctatatcg 660
ttcctcatgg agatcattac cattacattc ctaagaatga gttatcagct agcgagttgg 720
ctgctgcaga agccttccta tctggtcggg aaaatctgtc aaatttaaga acctatcgcc 780
gacaaaatag cgataacact ccaagaacaa actgggtacc ttctgtaagc aatccaggaa 840
ctacaaatac taacacaagc aacaacagca acactaacag tcaagcaagt caaagtaatg 900
acattgatag tctcttgaaa cagctctaca aactgccttt gagtcaacgc catgtagaat 960
ctgatggcct tattttcgac ccagcgcaaa tcacaagtcg aaccgccaga ggtgtagctg 1020
tccctcatgg taaccattac cactttatcc cttatgaaca aatgtctgaa ttggaaaaac 1080
gaattgctcg tattattccc cttcgttatc gttcaaacca ttgggtacca gattcaagac 1140
cagaagaacc aagtccacaa ccgactccag aacctagtcc aagtccgcaa cctgcaccaa 1200
atcctcaacc agctccaagc aatccaattg atgagaaatt ggtcaaagaa gctgttcgaa 1260
aagtaggcga tggttatgtc tttgaggaga atggagtttc tcgttatatc ccagccaaga 1320
atctttcagc agaaacagca gcaggcattg atagcaaact ggccaagcag gaaagtttat 1380
ctcataagct aggagctaag aaaactgacc tcccatctag tgatcgagaa ttttacaata 1440
aggcttatga cttactagca agaattcacc aagatttact tgataataaa ggtcgacaag 1500
ttgattttga ggctttggat aacctgttgg aacgactcaa ggatgtctca agtgataaag 1560
tcaagttagt ggatgatatt cttgccttct tagctccgat tcgtcatcca gaacgtttag 1620
gaaaaccaaa tgcgcaaatt acctacactg atgatgagat tcaagtagcc aagttggcag 1680
gcaagtacac aacagaagac ggttatatct ttgatcctcg tgatataacc agtgatgagg 1740
gggatgccta tgtaactcca catatgaccc atagccactg gattaaaaaa gatagtttgt 1800
ctgaagctga gagagcggca gcccaggctt atgctaaaga gaaaggtttg acccctcctt 1860
cgacagacca tcaggattca ggaaatactg aggcaaaagg agcagaagct atctacaacc 1920
gcgtgaaagc agctaagaag gtgccacttg atcgtatgcc ttacaatctt caatatactg 1980
tagaagtcaa aaacggtagt ttaatcatac ctcattatga ccattaccat aacatcaaat 2040
ttgagtggtt tgacgaaggc ctttatgagg cacctaaggg gtatactctt gaggatcttt 2100
tggcgactgt caagtactat gtcgaacatc caaacgaacg tccgcattca gataatggtt 2160
ttggtaacgc tagcgaccat gttcaaagaa acaaaaatgg tcaagctgat accaatcaaa 2220
cggaaaaacc aagcgaggag aaacctcaga cagaaaaacc tgaggaagaa acccctcgag 2280

aagagaaacc acaaagcgag aaaccagagt ctccaaaacc aacagaggaa ccagaagaag 2340
aatcaccaga ggaatcagaa gaacctcagg tcgagactga aaaggttgaa gaaaaactga 2400
gagaggctga agatttactt ggaaaaatcc aggatccaat tatcaagtcc aatgccaaag 2460
agactctcac aggattaaaa aataatttac tatttggcac ccaggacaac aatactatta 2520
tggcagaagc tgaaaaacta ttggctttat taaaggagag taagtaaagg tagcagcatt 2580
ttctaactcc taaaaacagg ataggagaac gggaaaacga aaaatgagag cagaatgtga 2640
gttctag 2647

<210> 5
<211> 2639
<212> DNA
<213> BVH-11-2

<400> 5
gggtcttaaa actctgaatc ctttagaggc agacccacaa aatgacaaga cctatttaga 60
aaatctggaa gaaaatatga gtgttctagc agaagaatta aagtgaggaa agaatgaaaa 120
tcaataaaaa atatctagca ggttcagtgg cagtccttgc cctaagtgtt tgttcctatg 180
aacttggtcg tcaccaagct ggtcaggtta agaaagagtc taatcgagtt tcttatatag 240
atggtgatca ggctggtcaa aaggcagaaa atttgacacc agatgaagtc agtaagagag 300
aggggatcaa cgccgaacaa attgttatca agattacgga tcaaggttat gtgacctctc 360
atggagacca ttatcattac tataatggca aggttcctta tgatgccatc atcagtgaag 420
aacttctcat gaaagatccg aattatcagt tgaaggattc agacattgtc aatgaaatca 480
agggtggcta tgtgattaag gtagacggaa aatactatgt ttaccttaaa gatgcggccc 540
atgcggacaa tattcggaca aaagaagaga ttaaacgtca gaagcaggaa cacagtcata 600
atcataactc aagagcagat aatgctgttg ctgcagccag agcccaagga cgttatacaa 660
cggatgatgg gtatatcttc aatgcatctg atatcattga ggacacgggt gatgcttata 720
tcgttcctca cggcgaccat taccattaca ttcctaagaa tgagttatca gctagcgagt 780
tagctgctgc agaagcctat tggaatggga agcagggatc tcgtccttct tcaagttcta 840
gttataatgc aaatccagtt caaccaagat tgtcagagaa ccacaatctg actgtcactc 900
caacttatca tcaaaatcaa ggggaaaaca tttcaagcct tttacgtgaa ttgtatgcta 960
aacccttatc agaacgccat gtagaatctg atggccttat tttcgaccca gcgcaaatca 1020
caagtcgaac cgccagaggt gtagctgtcc ctcatggtaa ccattaccac tttatccctt 1080
atgaacaaat gtctgaattg gaaaaacgaa ttgctcgtat tattcccctt cgttatcgtt 1140
caaaccattg ggtaccagat tcaagaccag aacaaccaag tccacaatcg actccggaac 1200
ctagtccaag tctgcaacct gcaccaaatc ctcaaccagc tccaagcaat ccaattgatg 1260
agaaattggt caaagaagct gttcgaaaag taggcgatgg ttatgtcttt gaggagaatg 1320
gagtttctcg ttatatccca gccaaggatc tttcagcaga aacagcagca ggcattgata 1380
gcaaactggc caagcaggaa agtttatctc ataagctagg agctaagaaa actgacctcc 1440
catctagtga tcgagaattt tacaataagg cttatgactt actagcaaga attcaccaag 1500
atttacttga taataaaggt cgacaagttg attttgaggt tttggataac ctgttggaac 1560
gactcaagga tgtctcaagt gataaagtca agttagtgga tgatattctt gccttcttag 1620
ctccgattcg tcatccagaa cgtttaggaa aaccaaatgc gcaaattacc tacactgatg 1680
atgagattca agtagccaag ttggcaggca agtacacaac agaagacggt tatatctttg 1740
atcctcgtga tataaccagt gatgaggggg atgcctatgt aactccacat atgacccata 1800
gccactggat taaaaaagat agtttgtctg aagctgagag agcggcagcc caggcttatg 1860
ctaaagagaa aggtttgacc cctccttcga cagaccacca ggattcagga aatactgagg 1920
caaaaggagc agaagctatc tacaaccgcg tgaaagcagc taagaaggtg ccacttgatc 1980
gtatgcctta caatcttcaa tatactgtag aagtcaaaaa cggtagttta atcatacctc 2040
attatgacca ttaccataac atcaaatttg agtggtttga cgaaggcctt tatgaggcac 2100
ctaaggggta tagtcttgag gatcttttgg cgactgtcaa gtactatgtc gaacatccaa 2160
acgaacgtcc gcattcagat aatggttttg gtaacgctag tgaccatgtt cgtaaaaata 2220
aggcagacca agatagtaaa cctgatgaag ataaggaaca tgatgaagta agtgagccaa 2280
ctcaccctga atctgatgaa aaagagaatc acgctggttt aaatccttca gcagataatc 2340
tttataaacc aagcactgat acggaagaga cagaggaaga agctgaagat accacagatg 2400
aggctgaaat tcctcaagta gagaattctg ttattaacgc taagatagca gatgcggagg 2460
ccttgctaga aaaagtaaca gatcctagta ttagacaaaa tgctatggag acattgactg 2520
gtctaaaaag tagtcttctt ctcggaacga aagataataa cactatttca gcagaagtag 2580
atagtctctt ggctttgtta aaagaaagtc aaccggctcc tatacagtag taaaatgaa 2639

<210> 6
<211> 1039
<212> PRT
<213> BVH-3

<400> 6
Met Lys Phe Ser Lys Lys Tyr Ile Ala Ala Gly Ser Ala Val Ile Val
1               5                   10                  15
Ser Leu Ser Leu Cys Ala Tyr Ala Leu Asn Gln His Arg Ser Gln Glu

            20                  25                  30
Asn Lys Asp Asn Asn Arg Val Ser Tyr Val Asp Gly Ser Gln Ser Ser
        35                  40                  45
Gln Lys Ser Glu Asn Leu Thr Pro Asp Gln Val Ser Gln Lys Glu Gly
    50                  55                  60
Ile Gln Ala Glu Gln Ile Val Ile Lys Ile Thr Asp Gln Gly Tyr Val
65                  70                  75                  80
Thr Ser His Gly Asp His Tyr His Tyr Tyr Asn Gly Lys Val Pro Tyr
                85                  90                  95
Asp Ala Leu Phe Ser Glu Glu Leu Leu Met Lys Asp Pro Asn Tyr Gln
            100                 105                 110
Leu Lys Asp Ala Asp Ile Val Asn Glu Val Lys Gly Gly Tyr Ile Ile
        115                 120                 125
Lys Val Asp Gly Lys Tyr Tyr Val Tyr Leu Lys Asp Ala Ala His Ala
    130                 135                 140
Asp Asn Val Arg Thr Lys Asp Glu Ile Asn Arg Gln Lys Gln Glu His
145                 150                 155                 160
Val Lys Asp Asn Glu Lys Val Asn Ser Asn Val Ala Val Ala Arg Ser
                165                 170                 175
Gln Gly Arg Tyr Thr Thr Asn Asp Gly Tyr Val Phe Asn Pro Ala Asp
            180                 185                 190
Ile Ile Glu Asp Thr Gly Asn Ala Tyr Ile Val Pro His Gly Gly His
        195                 200                 205
Tyr His Tyr Ile Pro Lys Ser Asp Leu Ser Ala Ser Glu Leu Ala Ala
    210                 215                 220
Ala Lys Ala His Leu Ala Gly Lys Asn Met Gln Pro Ser Gln Leu Ser
225                 230                 235                 240
Tyr Ser Ser Thr Ala Ser Asp Asn Asn Thr Gln Ser Val Ala Lys Gly
                245                 250                 255
Ser Thr Ser Lys Pro Ala Asn Lys Ser Glu Asn Leu Gln Ser Leu Leu
            260                 265                 270
Lys Glu Leu Tyr Asp Ser Pro Ser Ala Gln Arg Tyr Ser Glu Ser Asp
        275                 280                 285
Gly Leu Val Phe Asp Pro Ala Lys Ile Ile Ser Arg Thr Pro Asn Gly
    290                 295                 300
Val Ala Ile Pro His Gly Asp His Tyr His Phe Ile Pro Tyr Ser Lys
305                 310                 315                 320
Leu Ser Ala Leu Glu Glu Lys Ile Ala Arg Met Val Pro Ile Ser Gly
                325                 330                 335
Thr Gly Ser Thr Val Ser Thr Asn Ala Lys Pro Asn Glu Val Val Ser
            340                 345                 350
Ser Leu Gly Ser Leu Ser Ser Asn Pro Ser Ser Leu Thr Thr Ser Lys
        355                 360                 365
Glu Leu Ser Ser Ala Ser Asp Gly Tyr Ile Phe Asn Pro Lys Asp Ile
    370                 375                 380
Val Glu Glu Thr Ala Thr Ala Tyr Ile Val Arg His Gly Asp His Phe
385                 390                 395                 400
His Tyr Ile Pro Lys Ser Asn Gln Ile Gly Gln Pro Thr Leu Pro Asn
                405                 410                 415
Asn Ser Leu Ala Thr Pro Ser Pro Ser Leu Pro Ile Asn Pro Gly Thr
            420                 425                 430
Ser His Glu Lys His Glu Glu Asp Gly Tyr Gly Phe Asp Ala Asn Arg
        435                 440                 445
Ile Ile Ala Glu Asp Glu Ser Gly Phe Val Met Ser His Gly Asp His
    450                 455                 460
Asn His Tyr Phe Phe Lys Lys Asp Leu Thr Glu Glu Gln Ile Lys Ala
465                 470                 475                 480
Ala Gln Lys His Leu Glu Glu Val Lys Thr Ser His Asn Gly Leu Asp
                485                 490                 495
Ser Leu Ser Ser His Glu Gln Asp Tyr Pro Gly Asn Ala Lys Glu Met
            500                 505                 510
Lys Asp Leu Asp Lys Lys Ile Glu Glu Lys Ile Ala Gly Ile Met Lys
        515                 520                 525
Gln Tyr Gly Val Lys Arg Glu Ser Ile Val Val Asn Lys Glu Lys Asn
    530                 535                 540
Ala Ile Ile Tyr Pro His Gly Asp His His His Ala Asp Pro Ile Asp
545                 550                 555                 560
Glu His Lys Pro Val Gly Ile Gly His Ser His Ser Asn Tyr Glu Leu

565                 570                 575
Phe Lys Pro Glu Glu Gly Val Ala Lys Lys Glu Gly Asn Lys Val Tyr
            580                 585                 590
Thr Gly Glu Glu Leu Thr Asn Val Val Asn Leu Leu Lys Asn Ser Thr
        595                 600                 605
Phe Asn Asn Gln Asn Phe Thr Leu Ala Asn Gly Gln Lys Arg Val Ser
    610                 615                 620
Phe Ser Phe Pro Pro Glu Leu Glu Lys Lys Leu Gly Ile Asn Met Leu
625                 630                 635                 640
Val Lys Leu Ile Thr Pro Asp Gly Lys Val Leu Glu Lys Val Ser Gly
                645                 650                 655
Lys Val Phe Gly Glu Gly Val Gly Asn Ile Ala Asn Phe Glu Leu Asp
            660                 665                 670
Gln Pro Tyr Leu Pro Gly Gln Thr Phe Lys Tyr Thr Ile Ala Ser Lys
        675                 680                 685
Asp Tyr Pro Glu Val Ser Tyr Asp Gly Thr Phe Thr Val Pro Thr Ser
    690                 695                 700
Leu Ala Tyr Lys Met Ala Ser Gln Thr Ile Phe Tyr Pro Phe His Ala
705                 710                 715                 720
Gly Asp Thr Tyr Leu Arg Val Asn Pro Gln Phe Ala Val Pro Lys Gly
                725                 730                 735
Thr Asp Ala Leu Val Arg Val Phe Asp Glu Phe His Gly Asn Ala Tyr
            740                 745                 750
Leu Glu Asn Asn Tyr Lys Val Gly Glu Ile Lys Leu Pro Ile Pro Lys
        755                 760                 765
Leu Asn Gln Gly Thr Thr Arg Thr Ala Gly Asn Lys Ile Pro Val Thr
    770                 775                 780
Phe Met Ala Asn Ala Tyr Leu Asp Asn Gln Ser Thr Tyr Ile Val Glu
785                 790                 795                 800
Val Pro Ile Leu Glu Lys Glu Asn Gln Thr Asp Lys Pro Ser Ile Leu
                805                 810                 815
Pro Gln Phe Lys Arg Asn Lys Ala Gln Glu Asn Ser Lys Leu Asp Glu
            820                 825                 830
Lys Val Glu Glu Pro Lys Thr Ser Glu Lys Val Glu Lys Glu Lys Leu
        835                 840                 845
Ser Glu Thr Gly Asn Ser Thr Ser Asn Ser Thr Leu Glu Glu Val Pro
    850                 855                 860
Thr Val Asp Pro Val Gln Glu Lys Val Ala Lys Phe Ala Glu Ser Tyr
865                 870                 875                 880
Gly Met Lys Leu Glu Asn Val Leu Phe Asn Met Asp Gly Thr Ile Glu
                885                 890                 895
Leu Tyr Leu Pro Ser Gly Glu Val Ile Lys Lys Asn Met Ala Asp Phe
            900                 905                 910
Thr Gly Glu Ala Pro Gln Gly Asn Gly Glu Asn Lys Pro Ser Glu Asn
        915                 920                 925
Gly Lys Val Ser Thr Gly Thr Val Glu Asn Gln Pro Thr Glu Asn Lys
    930                 935                 940
Pro Ala Asp Ser Leu Pro Glu Ala Pro Asn Glu Lys Pro Val Lys Pro
945                 950                 955                 960
Glu Asn Ser Thr Asp Asn Gly Met Leu Asn Pro Glu Gly Asn Val Gly
                965                 970                 975
Ser Asp Pro Met Leu Asp Pro Ala Leu Glu Glu Ala Pro Ala Val Asp
            980                 985                 990
Pro Val Gln Glu Lys Leu Glu Lys Phe Thr Ala Ser Tyr Gly Leu Gly
        995                 1000                1005
Leu Asp Ser Val Ile Phe Asn Met Asp Gly Thr Ile Glu Leu Arg Leu
    1010                1015                1020
Pro Ser Gly Glu Val Ile Lys Lys Asn Leu Ser Asp Phe Ile Ala
1025                1030                1035

<210> 7
<211> 840
<212> PRT
<213> BVH-11

<400> 7
Met Lys Ile Asn Lys Lys Tyr Leu Ala Gly Ser Val Ala Thr Leu Val
1               5                   10                  15

Leu Ser Val Cys Ala Tyr Glu Leu Gly Leu His Gln Ala Gln Thr Val
            20                  25                  30
Lys Glu Asn Asn Arg Val Ser Tyr Ile Asp Gly Lys Gln Ala Thr Gln
        35                  40                  45
Lys Thr Glu Asn Leu Thr Pro Asp Glu Val Ser Lys Arg Glu Gly Ile
    50                  55                  60
Asn Ala Glu Gln Ile Val Ile Lys Ile Thr Asp Gln Gly Tyr Val Thr
65                  70                  75                  80
Ser His Gly Asp His Tyr His Tyr Tyr Asn Gly Lys Val Pro Tyr Asp
                85                  90                  95
Ala Ile Ile Ser Glu Glu Leu Leu Met Lys Asp Pro Asn Tyr Gln Leu
            100                 105                 110
Lys Asp Ser Asp Ile Val Asn Glu Ile Lys Gly Gly Tyr Val Ile Lys
        115                 120                 125
Val Asn Gly Lys Tyr Tyr Val Tyr Leu Lys Asp Ala Ala His Ala Asp
    130                 135                 140
Asn Val Arg Thr Lys Glu Glu Ile Asn Arg Gln Lys Gln Glu His Ser
145                 150                 155                 160
Gln His Arg Glu Gly Gly Thr Ser Ala Asn Asp Gly Ala Val Ala Phe
                165                 170                 175
Ala Arg Ser Gln Gly Arg Tyr Thr Thr Asp Asp Gly Tyr Ile Phe Asn
            180                 185                 190
Ala Ser Asp Ile Ile Glu Asp Thr Gly Asp Ala Tyr Ile Val Pro His
        195                 200                 205
Gly Asp His Tyr His Tyr Ile Pro Lys Asn Glu Leu Ser Ala Ser Glu
    210                 215                 220
Leu Ala Ala Ala Glu Ala Phe Leu Ser Gly Arg Glu Asn Leu Ser Asn
225                 230                 235                 240
Leu Arg Thr Tyr Arg Arg Gln Asn Ser Asp Asn Thr Pro Arg Thr Asn
                245                 250                 255
Trp Val Pro Ser Val Ser Asn Pro Gly Thr Thr Asn Thr Asn Thr Ser
            260                 265                 270
Asn Asn Ser Asn Thr Asn Ser Gln Ala Ser Gln Ser Asn Asp Ile Asp
        275                 280                 285
Ser Leu Leu Lys Gln Leu Tyr Lys Leu Pro Leu Ser Gln Arg His Val
    290                 295                 300
Glu Ser Asp Gly Leu Ile Phe Asp Pro Ala Gln Ile Thr Ser Arg Thr
305                 310                 315                 320
Ala Arg Gly Val Ala Val Pro His Gly Asn His Tyr His Phe Ile Pro
                325                 330                 335
Tyr Glu Gln Met Ser Glu Leu Glu Lys Arg Ile Ala Arg Ile Ile Pro
            340                 345                 350
Leu Arg Tyr Arg Ser Asn His Trp Val Pro Asp Ser Arg Pro Glu Glu
        355                 360                 365
Pro Ser Pro Gln Pro Thr Pro Glu Pro Ser Pro Ser Pro Gln Pro Ala
    370                 375                 380
Pro Asn Pro Gln Pro Ala Pro Ser Asn Pro Ile Asp Glu Lys Leu Val
385                 390                 395                 400
Lys Glu Ala Val Arg Lys Val Gly Asp Gly Tyr Val Phe Glu Glu Asn
                405                 410                 415
Gly Val Ser Arg Tyr Ile Pro Ala Lys Asn Leu Ser Ala Glu Thr Ala
            420                 425                 430
Ala Gly Ile Asp Ser Lys Leu Ala Lys Gln Glu Ser Leu Ser His Lys
        435                 440                 445
Leu Gly Ala Lys Lys Thr Asp Leu Pro Ser Ser Asp Arg Glu Phe Tyr
    450                 455                 460
Asn Lys Ala Tyr Asp Leu Leu Ala Arg Ile His Gln Asp Leu Leu Asp
465                 470                 475                 480
Asn Lys Gly Arg Gln Val Asp Phe Glu Ala Leu Asp Asn Leu Leu Glu
                485                 490                 495
Arg Leu Lys Asp Val Ser Ser Asp Lys Val Lys Leu Val Asp Asp Ile
            500                 505                 510
Leu Ala Phe Leu Ala Pro Ile Arg His Pro Glu Arg Leu Gly Lys Pro
        515                 520                 525
Asn Ala Gln Ile Thr Tyr Thr Asp Asp Glu Ile Gln Val Ala Lys Leu
    530                 535                 540
Ala Gly Lys Tyr Thr Thr Glu Asp Gly Tyr Ile Phe Asp Pro Arg Asp
545                 550                 555                 560

Ile Thr Ser Asp Glu Gly Asp Ala Tyr Val Thr Pro His Met Thr His
                565                 570                 575
Ser His Trp Ile Lys Lys Asp Ser Leu Ser Glu Ala Glu Arg Ala Ala
            580                 585                 590
Ala Gln Ala Tyr Ala Lys Glu Lys Gly Leu Thr Pro Pro Ser Thr Asp
        595                 600                 605
His Gln Asp Ser Gly Asn Thr Glu Ala Lys Gly Ala Glu Ala Ile Tyr
    610                 615                 620
Asn Arg Val Lys Ala Ala Lys Lys Val Pro Leu Asp Arg Met Pro Tyr
625                 630                 635                 640
Asn Leu Gln Tyr Thr Val Glu Val Lys Asn Gly Ser Leu Ile Ile Pro
                645                 650                 655
His Tyr Asp His Tyr His Asn Ile Lys Phe Glu Trp Phe Asp Glu Gly
            660                 665                 670
Leu Tyr Glu Ala Pro Lys Gly Tyr Thr Leu Glu Asp Leu Leu Ala Thr
        675                 680                 685
Val Lys Tyr Tyr Val Glu His Pro Asn Glu Arg Pro His Ser Asp Asn
    690                 695                 700
Gly Phe Gly Asn Ala Ser Asp His Val Gln Arg Asn Lys Asn Gly Gln
705                 710                 715                 720
Ala Asp Thr Asn Gln Thr Glu Lys Pro Ser Glu Glu Lys Pro Gln Thr
                725                 730                 735
Glu Lys Pro Glu Glu Glu Thr Pro Arg Glu Glu Lys Pro Gln Ser Glu
            740                 745                 750
Lys Pro Glu Ser Pro Lys Pro Thr Glu Glu Pro Glu Glu Glu Ser Pro
        755                 760                 765
Glu Glu Ser Glu Glu Pro Gln Val Glu Thr Glu Lys Val Glu Glu Lys
    770                 775                 780
Leu Arg Glu Ala Glu Asp Leu Leu Gly Lys Ile Gln Asp Pro Ile Ile
785                 790                 795                 800
Lys Ser Asn Ala Lys Glu Thr Leu Thr Gly Leu Lys Asn Asn Leu Leu
                805                 810                 815
Phe Gly Thr Gln Asp Asn Asn Thr Ile Met Ala Glu Ala Glu Lys Leu
            820                 825                 830
Leu Ala Leu Leu Lys Glu Ser Lys
        835                 840

<210> 8
<211> 838

<212> PRT
<213> BVH-11-2

<400> 8
Met Lys Ile Asn Lys Lys Tyr Leu Ala Gly Ser Val Ala Val Leu Ala
 1               5                  10                  15
Leu Ser Val Cys Ser Tyr Glu Leu Gly Arg His Gln Ala Gly Gln Val
            20                  25                  30
Lys Lys Glu Ser Asn Arg Val Ser Tyr Ile Asp Gly Asp Gln Ala Gly
        35                  40                  45
Gln Lys Ala Glu Asn Leu Thr Pro Asp Glu Val Ser Lys Arg Glu Gly
    50                  55                  60
Ile Asn Ala Glu Gln Ile Val Ile Lys Ile Thr Asp Gln Gly Tyr Val
65                  70                  75                  80
Thr Ser His Gly Asp His Tyr His Tyr Tyr Asn Gly Lys Val Pro Tyr
                85                  90                  95

Asp Ala Ile Ile Ser Glu Glu Leu Leu Met Lys Asp Pro Asn Tyr Gln
            100                 105                 110
Leu Lys Asp Ser Asp Ile Val Asn Glu Ile Lys Gly Gly Tyr Val Ile
        115                 120                 125
Lys Val Asp Gly Lys Tyr Tyr Val Tyr Leu Lys Asp Ala Ala His Ala
    130                 135                 140
Asp Asn Ile Arg Thr Lys Glu Glu Ile Lys Arg Gln Lys Gln Glu His
145                 150                 155                 160
Ser His Asn His Asn Ser Arg Ala Asp Asn Ala Val Ala Ala Ala Arg
                165                 170                 175
Ala Gln Gly Arg Tyr Thr Thr Asp Asp Gly Tyr Ile Phe Asn Ala Ser

            180                 185                 190
Asp Ile Ile Glu Asp Thr Gly Asp Ala Tyr Ile Val Pro His Gly Asp
        195                 200                 205
His Tyr His Tyr Ile Pro Lys Asn Glu Leu Ser Ala Ser Glu Leu Ala
    210                 215                 220
Ala Ala Glu Ala Tyr Trp Asn Gly Lys Gln Gly Ser Arg Pro Ser Ser
225                 230                 235                 240
Ser Ser Ser Tyr Asn Ala Asn Pro Val Gln Pro Arg Leu Ser Glu Asn
                245                 250                 255
His Asn Leu Thr Val Thr Pro Thr Tyr His Gln Asn Gln Gly Glu Asn
            260                 265                 270
Ile Ser Ser Leu Leu Arg Glu Leu Tyr Ala Lys Pro Leu Ser Glu Arg
        275                 280                 285
His Val Glu Ser Asp Gly Leu Ile Phe Asp Pro Ala Gln Ile Thr Ser
    290                 295                 300
Arg Thr Ala Arg Gly Val Ala Val Pro His Gly Asn His Tyr His Phe
305                 310                 315                 320
Ile Pro Tyr Glu Gln Met Ser Glu Leu Glu Lys Arg Ile Ala Arg Ile
                325                 330                 335
Ile Pro Leu Arg Tyr Arg Ser Asn His Trp Val Pro Asp Ser Arg Pro
            340                 345                 350
Glu Gln Pro Ser Pro Gln Ser Thr Pro Glu Pro Ser Pro Ser Leu Gln
        355                 360                 365
Pro Ala Pro Asn Pro Gln Pro Ala Pro Ser Asn Pro Ile Asp Glu Lys
    370                 375                 380
Leu Val Lys Glu Ala Val Arg Lys Val Gly Asp Gly Tyr Val Phe Glu
385                 390                 395                 400
Glu Asn Gly Val Ser Arg Tyr Ile Pro Ala Lys Asp Leu Ser Ala Glu
                405                 410                 415
Thr Ala Ala Gly Ile Asp Ser Lys Leu Ala Lys Gln Glu Ser Leu Ser
            420                 425                 430
His Lys Leu Gly Ala Lys Lys Thr Asp Leu Pro Ser Ser Asp Arg Glu
        435                 440                 445
Phe Tyr Asn Lys Ala Tyr Asp Leu Leu Ala Arg Ile His Gln Asp Leu
    450                 455                 460
Leu Asp Asn Lys Gly Arg Gln Val Asp Phe Glu Val Leu Asp Asn Leu
465                 470                 475                 480
Leu Glu Arg Leu Lys Asp Val Ser Ser Asp Lys Val Lys Leu Val Asp
                485                 490                 495
Asp Ile Leu Ala Phe Leu Ala Pro Ile Arg His Pro Glu Arg Leu Gly
            500                 505                 510
Lys Pro Asn Ala Gln Ile Thr Tyr Thr Asp Asp Glu Ile Gln Val Ala
        515                 520                 525
Lys Leu Ala Gly Lys Tyr Thr Thr Glu Asp Gly Tyr Ile Phe Asp Pro
    530                 535                 540
Arg Asp Ile Thr Ser Asp Glu Gly Asp Ala Tyr Val Thr Pro His Met
545                 550                 555                 560
Thr His Ser His Trp Ile Lys Lys Asp Ser Leu Ser Glu Ala Glu Arg
                565                 570                 575
Ala Ala Ala Gln Ala Tyr Ala Lys Glu Lys Gly Leu Thr Pro Pro Ser
            580                 585                 590
Thr Asp His Gln Asp Ser Gly Asn Thr Glu Ala Lys Gly Ala Glu Ala
        595                 600                 605
Ile Tyr Asn Arg Val Lys Ala Ala Lys Lys Val Pro Leu Asp Arg Met
    610                 615                 620
Pro Tyr Asn Leu Gln Tyr Thr Val Glu Val Lys Asn Gly Ser Leu Ile
625                 630                 635                 640
Ile Pro His Tyr Asp His Tyr His Asn Ile Lys Phe Glu Trp Phe Asp
                645                 650                 655
Glu Gly Leu Tyr Glu Ala Pro Lys Gly Tyr Ser Leu Glu Asp Leu Leu
            660                 665                 670
Ala Thr Val Lys Tyr Tyr Val Glu His Pro Asn Glu Arg Pro His Ser
        675                 680                 685
Asp Asn Gly Phe Gly Asn Ala Ser Asp His Val Arg Lys Asn Lys Ala
    690                 695                 700
Asp Gln Asp Ser Lys Pro Asp Glu Asp Lys Glu His Asp Glu Val Ser
705                 710                 715                 720
Glu Pro Thr His Pro Glu Ser Asp Glu Lys Glu Asn His Ala Gly Leu

725                 730                 735
Asn Pro Ser Ala Asp Asn Leu Tyr Lys Pro Ser Thr Asp Thr Glu Glu
            740                 745                 750
Thr Glu Glu Glu Ala Glu Asp Thr Thr Asp Glu Ala Glu Ile Pro Gln
        755                 760                 765

Val Glu Asn Ser Val Ile Asn Ala Lys Ile Ala Asp Ala Glu Ala Leu
    770                 775                 780
Leu Glu Lys Val Thr Asp Pro Ser Ile Arg Gln Asn Ala Met Glu Thr
785                 790                 795                 800
Leu Thr Gly Leu Lys Ser Ser Leu Leu Leu Gly Thr Lys Asp Asn Asn
                805                 810                 815
Thr Ile Ser Ala Glu Val Asp Ser Leu Leu Ala Leu Leu Lys Glu Ser
            820                 825                 830
Gln Pro Ala Pro Ile Gln
        835

<210> 9
<211> 2528
<212> DNA
<213> BVH-3

<400> 9
tgtgcctatg cactaaacca gcatcgttcg caggaaaata aggacaataa tcgtgtctct      60
tatgtggatg gcagccagtc aagtcagaaa agtgaaaact tgacaccaga ccaggttagc     120
cagaaagaag gaattcaggc tgagcaaatt gtaatcaaaa ttacagatca gggctatgta     180
acgtcacacg gtgatcacta tcattactat aatgggaaag ttccttatga tgccctcttt     240
agtgaagaac tcttgatgaa ggatccaaac tatcaactta aagacgctga tattgtcaat     300
gaagtcaagg gtggttatat catcaaggtc gatggaaaat attatgtcta cctgaaagat     360
gcagctcatg ctgataatgt tcgaactaaa gatgaaatca atcgtcaaaa acaagaacat     420
gtcaaagata atgagaaggt taactctaat gttgctgtag caaggtctca gggacgatat     480
acgacaaatg atggttatgt ctttaatcca gctgatatta tcgaagatac gggtaatgct     540
tatatcgttc ctcatggagg tcactatcac tacattccca aaagcgattt atctgctagt     600
gaattagcag cagctaaagc acatctggct ggaaaaaata tgcaaccgag tcagttaagc     660
tattcttcaa caccttctcc atctcttcca atcaatccag gaacttcaca tgagaaacat     720
gaagaagatg gatacggatt tgatgctaat cgtattatcg ctgaagatga atcaggtttt     780
gtcatgagtc acggagacca caatcattat ttcttcaaga aggacttgac agaagagcaa     840
attaaggctg cgcaaaaaca tttagaggaa gttaaaacta gtcataatgg attagattct     900
ttgtcatctc atgaacagga ttatccaagt aatgccaaag aaatgaaaga tttagataaa     960
aaaatcgaag aaaaaattgc tggcattatg aaacaatatg gtgtcaaacg tgaaagtatt    1020
gtcgtgaata aagaaaaaaa tgcgattatt tatccgcatg gagatcacca tcatgcagat    1080
ccgattgatg aacataaacc ggttggaatt ggtcattctc acagtaacta tgaactgttt    1140
aaacccgaag aaggagttgc taaaaaagaa gggaataaag tttatactgg agaagaatta    1200
acgaatgttg ttaatttgtt aaaaaatagt acgtttaata atcaaaactt tactctagcc    1260
aatggtcaaa aacgcgtttc ttttagtttt ccgcctgaat tggagaaaaa attaggtatc    1320
aatatgctag taaaattaat aacaccagat ggaaaagtat tggagaaagt atctggtaaa    1380
gtatttggag aaggagtagg gaatattgca aactttgaat tagatcaacc ttatttacca    1440
ggacaaacat ttaagtatac tatcgcttca aaagattatc cagaagtaag ttatgatggt    1500
acatttacag ttccaacctc tttagcttac aaaatggcca gtcaaacgat tttctatcct    1560
ttccatgcag ggatacttta tttaagagtg aaccctcaat ttgcagtgcc taaaggaact    1620
gatgctttag tcagagtgtt tgatgaattt catggaaatg cttatttaga aaataactat    1680
aaagttggtg aaatcaaatt accgattccg aaattaaacc aaggaacaac cagaacggcc    1740
ggaaataaaa ttcctgtaac cttcatggca aatgcttatt tggacaatca atcgacttat    1800
attgtggaag tacctatctt ggaaaaagaa aatcaaactg ataaaccaag tattctacca    1860
caatttaaaa ggaataaagc acaagaaaac tcaaaacttg atgaaaaggt agaagaacca    1920
aagactagtg agaaggtaga aaaagaaaaa ctttctgaaa ctgggaatag tactagtaat    1980
tcaacgttag aagaagttcc tacagtggat cctgtacaag aaaaagtagc aaaatttgct    2040
gaaagttatg ggatgaagct agaaaatgtc ttgtttaata tggacggaac aattgaatta    2100
tatttaccat cgggagaagt cattaaaaag aatatggcag attttacagg agaagcacct    2160
caaggaaatg gtgaaaataa accatctgaa aatggaaaag tatctactgg aacagttgag    2220
aaccaaccaa cagaaaataa accagcagat tctttaccag aggcaccaaa cgaaaaacct    2280
gtaaaaccag aaaactcaac ggataatgga atgttgaatc cagaagggaa tgtggggagt    2340
gaccctatgt tagattcagc attagaggaa gctccagcag tagatcctgt acaagaaaaa    2400
ttagaaaaat ttacagctag ttacggatta ggcttagata gtgttatatt caatatggat    2460
ggaacgattg aattaagatt gccaagtgga gaagtgataa aaaagaattt attgatctca    2520
tagcgtaa                                                             2528

<210> 10

<211> 840
<212> PRT
<213> BVH-3

<400> 10
Cys Ala Tyr Ala Leu Asn Gln His Arg Ser Gln Glu Asn Lys Asp Asn
1               5                   10                  15
Asn Arg Val Ser Tyr Val Asp Gly Ser Gln Ser Ser Gln Lys Ser Glu
            20                  25                  30
Asn Leu Thr Pro Asp Gln Val Ser Gln Lys Glu Gly Ile Gln Ala Glu
        35                  40                  45
Gln Ile Val Ile Lys Ile Thr Asp Gln Gly Tyr Val Thr Ser His Gly
    50                  55                  60
Asp His Tyr His Tyr Tyr Asn Gly Lys Val Pro Tyr Asp Ala Leu Phe
65                  70                  75                  80
Ser Glu Glu Leu Leu Met Lys Asp Pro Asn Tyr Gln Leu Lys Asp Ala
                85                  90                  95
Asp Ile Val Asn Glu Val Lys Gly Gly Tyr Ile Ile Lys Val Asp Gly
            100                 105                 110
Lys Tyr Tyr Val Tyr Leu Lys Asp Ala Ala His Ala Asp Asn Val Arg
        115                 120                 125
Thr Lys Asp Glu Ile Asn Arg Gln Lys Gln Glu His Val Lys Asp Asn
    130                 135                 140
Glu Lys Val Asn Ser Asn Val Ala Val Ala Arg Ser Gln Gly Arg Tyr
145                 150                 155                 160
Thr Thr Asn Asp Gly Tyr Val Phe Asn Pro Ala Asp Ile Ile Glu Asp
                165                 170                 175
Thr Gly Asn Ala Tyr Ile Val Pro His Gly Gly His Tyr His Tyr Ile
            180                 185                 190
Pro Lys Ser Asp Leu Ser Ala Ser Glu Leu Ala Ala Ala Lys Ala His
        195                 200                 205
Leu Ala Gly Lys Asn Met Gln Pro Ser Gln Leu Ser Tyr Ser Ser Thr
    210                 215                 220
Pro Ser Pro Ser Leu Pro Ile Asn Pro Gly Thr Ser His Glu Lys His
225                 230                 235                 240
Glu Glu Asp Gly Tyr Gly Phe Asp Ala Asn Arg Ile Ile Ala Glu Asp
                245                 250                 255
Glu Ser Gly Phe Val Met Ser His Gly Asp His Asn His Tyr Phe Phe
            260                 265                 270
Lys Lys Asp Leu Thr Glu Glu Gln Ile Lys Ala Ala Gln Lys His Leu
        275                 280                 285
Glu Glu Val Lys Thr Ser His Asn Gly Leu Asp Ser Leu Ser Ser His
    290                 295                 300
Glu Gln Asp Tyr Pro Ser Asn Ala Lys Glu Met Lys Asp Leu Asp Lys
305                 310                 315                 320
Lys Ile Glu Glu Lys Ile Ala Gly Ile Met Lys Gln Tyr Gly Val Lys
                325                 330                 335
Arg Glu Ser Ile Val Val Asn Lys Glu Lys Asn Ala Ile Ile Tyr Pro
            340                 345                 350
His Gly Asp His His His Ala Asp Pro Ile Asp Glu His Lys Pro Val
        355                 360                 365
Gly Ile Gly His Ser His Ser Asn Tyr Glu Leu Phe Lys Pro Glu Glu
    370                 375                 380
Gly Val Ala Lys Lys Glu Gly Asn Lys Val Tyr Thr Gly Glu Glu Leu
385                 390                 395                 400
Thr Asn Val Val Asn Leu Leu Lys Asn Ser Thr Phe Asn Asn Gln Asn
                405                 410                 415
Phe Thr Leu Ala Asn Gly Gln Lys Arg Val Ser Phe Ser Phe Pro Pro
            420                 425                 430
Glu Leu Glu Lys Lys Leu Gly Ile Asn Met Leu Val Lys Leu Ile Thr
        435                 440                 445
Pro Asp Gly Lys Val Leu Glu Lys Val Ser Gly Lys Val Phe Gly Glu
    450                 455                 460
Gly Val Gly Asn Ile Ala Asn Phe Glu Leu Asp Gln Pro Tyr Leu Pro
465                 470                 475                 480
Gly Gln Thr Phe Lys Tyr Thr Ile Ala Ser Lys Asp Tyr Pro Glu Val
                485                 490                 495
Ser Tyr Asp Gly Thr Phe Thr Val Pro Thr Ser Leu Ala Tyr Lys Met

500                 505                 510
Ala Ser Gln Thr Ile Phe Tyr Pro Phe His Ala Gly Asp Thr Tyr Leu
        515                 520                 525
Arg Val Asn Pro Gln Phe Ala Val Pro Lys Gly Thr Asp Ala Leu Val
    530                 535                 540
Arg Val Phe Asp Glu Phe His Gly Asn Ala Tyr Leu Glu Asn Asn Tyr
545                 550                 555                 560
Lys Val Gly Glu Ile Lys Leu Pro Ile Pro Lys Leu Asn Gln Gly Thr
                565                 570                 575
Thr Arg Thr Ala Gly Asn Lys Ile Pro Val Thr Phe Met Ala Asn Ala
            580                 585                 590
Tyr Leu Asp Asn Gln Ser Thr Tyr Ile Val Glu Val Pro Ile Leu Glu
        595                 600                 605
Lys Glu Asn Gln Thr Asp Lys Pro Ser Ile Leu Pro Gln Phe Lys Arg
    610                 615                 620
Asn Lys Ala Gln Glu Asn Ser Lys Leu Asp Glu Lys Val Glu Glu Pro
625                 630                 635                 640
Lys Thr Ser Glu Lys Val Glu Lys Glu Lys Leu Ser Glu Thr Gly Asn
                645                 650                 655
Ser Thr Ser Asn Ser Thr Leu Glu Glu Val Pro Thr Val Asp Pro Val
            660                 665                 670
Gln Glu Lys Val Ala Lys Phe Ala Glu Ser Tyr Gly Met Lys Leu Glu
        675                 680                 685
Asn Val Leu Phe Asn Met Asp Gly Thr Ile Glu Leu Tyr Leu Pro Ser
    690                 695                 700
Gly Glu Val Ile Lys Lys Asn Met Ala Asp Phe Thr Gly Glu Ala Pro
705                 710                 715                 720
Gln Gly Asn Gly Glu Asn Lys Pro Ser Glu Asn Gly Lys Val Ser Thr
                725                 730                 735
Gly Thr Val Glu Asn Gln Pro Thr Glu Asn Lys Pro Ala Asp Ser Leu
            740                 745                 750
Pro Glu Ala Pro Asn Glu Lys Pro Val Lys Pro Glu Asn Ser Thr Asp
        755                 760                 765
Asn Gly Met Leu Asn Pro Glu Gly Asn Val Gly Ser Asp Pro Met Leu
    770                 775                 780
Asp Ser Ala Leu Glu Glu Ala Pro Ala Val Asp Pro Val Gln Glu Lys
785                 790                 795                 800
Leu Glu Lys Phe Thr Ala Ser Tyr Gly Leu Gly Leu Asp Ser Val Ile
                805                 810                 815
Phe Asn Met Asp Gly Thr Ile Glu Leu Arg Leu Pro Ser Gly Glu Val
            820                 825                 830
Ile Lys Lys Asn Leu Leu Ile Ser
        835                 840

<210> 11
<211> 94
<212> PRT
<213> Unknown

<220>
<223> 4D4.9

<400> 11
Asp Gln Gly Tyr Val Thr Ser His Gly Asp His Tyr His Tyr Tyr Asn
1               5                   10                  15
Gly Lys Val Pro Tyr Asp Ala Leu Phe Ser Glu Glu Leu Leu Met Lys
            20                  25                  30
Asp Pro Asn Tyr Gln Leu Lys Asp Ala Asp Ile Val Asn Glu Val Lys
        35                  40                  45
Gly Gly Tyr Ile Ile Lys Val Asp Gly Lys Tyr Tyr Val Tyr Leu Lys
    50                  55                  60
Asp Ala Ala His Ala Asp Asn Val Arg Thr Lys Asp Glu Ile Asn Arg
65                  70                  75                  80
Gln Lys Gln Glu His Val Lys Asp Asn Glu Lys Val Asn Ser
                85                  90

<210> 12
<211> 42

<212> PRT
<213> Unknown

<220>
<223> 7G11.7

<400> 12
Gly Ile Gln Ala Glu Gln Ile Val Ile Lys Ile Thr Asp Gln Gly Tyr
1               5                   10                  15
Val Thr Ser His Gly Asp His Tyr His Tyr Tyr Asn Gly Lys Val Pro
            20                  25                  30
Tyr Asp Ala Leu Phe Ser Glu Glu Leu Leu
        35                  40

<210> 13
<211> 39
<212> PRT
<213> Unknown

<220>
<223> 7G11.9

<400> 13
Thr Ala Tyr Ile Val Arg His Gly Asp His Phe His Tyr Ile Pro Lys
1               5                   10                  15
Ser Asn Gln Ile Gly Gln Pro Thr Leu Pro Asn Asn Ser Leu Ala Thr
            20                  25                  30
Pro Ser Pro Ser Leu Pro Ile
        35

<210> 14
<211> 36
<212> PRT
<213> Unknown

<220>
<223> 4D3.4

<400> 14
Thr Ser Asn Ser Thr Leu Glu Glu Val Pro Thr Val Asp Pro Val Gln
1               5                   10                  15
Glu Lys Val Ala Lys Phe Ala Glu Ser Tyr Gly Met Lys Leu Glu Asn
            20                  25                  30
Val Leu Phe Asn
        35

<210> 15
<211> 24
<212> PRT
<213> Unknown

<220>
<223> 8E3.1

<400> 15
Met Asp Gly Thr Ile Glu Leu Arg Leu Pro Ser Gly Glu Val Ile Lys
1               5                   10                  15
Lys Asn Leu Ser Asp Phe Ile Ala
            20

<210> 16
<211> 35
<212> PRT
<213> Unknown

<220>
<223> 1G2.2

<400> 16
Tyr Gly Leu Gly Leu Asp Ser Val Ile Phe Asn Met Asp Gly Thr Ile
1               5                   10                  15
Glu Leu Arg Leu Pro Ser Gly Glu Val Ile Lys Lys Asn Leu Ser Asp
            20                  25                  30
Phe Ile Ala
        35

<210> 17
<211> 57
<212> PRT
<213> Unknown

<220>
<223> 10C12.7

<400> 17
Pro Ala Leu Glu Glu Ala Pro Ala Val Asp Pro Val Gln Glu Lys Leu
1               5                   10                  15
Glu Lys Phe Thr Ala Ser Tyr Gly Leu Gly Leu Asp Ser Val Ile Phe
            20                  25                  30
Asn Met Asp Gly Thr Ile Glu Leu Arg Leu Pro Ser Gly Glu Val Ile
        35                  40                  45
Lys Lys Asn Leu Ser Asp Phe Ile Ala
    50                  55

<210> 18
<211> 40
<212> PRT
<213> Unknown

<220>
<223> 14F6.3

<400> 18
Lys Val Glu Glu Pro Lys Thr Ser Glu Lys Val Glu Lys Glu Lys Leu
1               5                   10                  15
Ser Glu Thr Gly Asn Ser Thr Ser Asn Ser Thr Leu Glu Glu Val Pro
            20                  25                  30
Thr Val Asp Pro Val Gln Glu Lys
        35                  40

<210> 19
<211> 78
<212> PRT
<213> Unknown

<220>
<223> B12D8.2

<400> 19
Met Lys Asp Leu Asp Lys Lys Ile Glu Glu Lys Ile Ala Gly Ile Met
1               5                   10                  15
Lys Gln Tyr Gly Val Lys Arg Glu Ser Ile Val Val Asn Lys Glu Lys
            20                  25                  30
Asn Ala Ile Ile Tyr Pro His Gly Asp His His His Ala Asp Pro Ile
        35                  40                  45
Asp Glu His Lys Pro Val Gly Ile Gly His Ser His Ser Asn Tyr Glu
    50                  55                  60
Leu Phe Lys Pro Glu Glu Gly Val Ala Lys Lys Glu Gly Asn
65                  70                  75

<210> 20
<211> 51
<212> PRT
<213> Unknown

<220>

<223> 7F4.1

<400> 20
Ala Ile Ile Tyr Pro His Gly Asp His His His Ala Asp Pro Ile Asp
1               5                   10                  15
Glu His Lys Pro Val Gly Ile Gly His Ser His Ser Asn Tyr Glu Leu
            20                  25                  30
Phe Lys Pro Glu Glu Gly Val Ala Lys Lys Glu Gly Asn Lys Val Tyr
        35                  40                  45
Thr Gly Glu
    50

<210> 21
<211> 29
<212> PRT
<213> Unknown

<220>
<223> 10D7.5

<400> 21
Ile Gln Val Ala Lys Leu Ala Gly Lys Tyr Thr Thr Glu Asp Gly Tyr
1               5                   10                  15
Ile Phe Asp Pro Arg Asp Ile Thr Ser Asp Glu Gly Asp
            20                  25

<210> 22
<211> 86
<212> PRT
<213> Unknown

<220>
<223> 10G9.3 and 10A2.2 and B11B8.1

<400> 22
Asp His Gln Asp Ser Gly Asn Thr Glu Ala Lys Gly Ala Glu Ala Ile
1               5                   10                  15
Tyr Asn Arg Val Lys Ala Ala Lys Lys Val Pro Leu Asp Arg Met Pro
            20                  25                  30
Tyr Asn Leu Gln Tyr Thr Val Glu Val Lys Asn Gly Ser Leu Ile Ile
        35                  40                  45
Pro His Tyr Asp His Tyr His Asn Ile Lys Phe Glu Trp Phe Asp Glu
    50                  55                  60
Gly Leu Tyr Glu Ala Pro Lys Gly Tyr Ser Leu Glu Asp Leu Leu Ala
65                  70                  75                  80
Thr Val Lys Tyr Tyr Val
                85

<210> 23
<211> 41
<212> PRT
<213> Unknown

<220>
<223> 11B8.4

<400> 23
Gly Leu Tyr Glu Ala Pro Lys Gly Tyr Ser Leu Glu Asp Leu Leu Ala
1               5                   10                  15
Thr Val Lys Tyr Tyr Val Glu His Pro Asn Glu Arg Pro His Ser Asp
            20                  25                  30
Asn Gly Phe Gly Asn Ala Ser Asp His
        35                  40

<210> 24
<211> 69
<212> PRT
<213> Unknown

<220>
<223> 3A4.1

<400> 24
Val Glu Asn Ser Val Ile Asn Ala Lys Ile Ala Asp Ala Glu Ala Leu
1               5                   10                  15
Leu Glu Lys Val Thr Asp Pro Ser Ile Arg Gln Asn Ala Met Glu Thr
            20                  25                  30
Leu Thr Gly Leu Lys Ser Ser Leu Leu Leu Gly Thr Lys Asp Asn Asn
        35                  40                  45
Thr Ile Ser Ala Glu Val Asp Ser Leu Leu Ala Leu Leu Lys Glu Ser
    50                  55                  60
Gln Pro Ala Pro Ile
65

<210> 25
<211> 27
<212> DNA
<213> Unknown

<220>
<223> OCRR 479

<400> 25
cagtagatct gtgcctatgc actaaac                                          27

<210> 26
<211> 33
<212> DNA
<213> Unknown

<220>
<223> OCRR 480

<400> 26
gatctctaga ctactgctat tccttacgct atg                                   33

<210> 27
<211> 30
<212> DNA
<213> Unknown

<220>
<223> OCRR 497

<400> 27
atcactcgag cattacctgg ataatcctgt                                       30

<210> 28
<211> 26
<212> DNA
<213> Unknown

<220>
<223> OCRR 498

<400> 28
ctgctaagct tatgaaagat ttagat                                           26

<210> 29
<211> 24
<212> DNA
<213> Unknown

<220>
<223> OCRR 499

<400> 29
gatactcgag ctgctattcc ttac                                              24

<210> 30
<211> 28
<212> DNA
<213> Unknown

<220>
<223> HAMJ 172

<400> 30
gaatctcgag ttaagctgct gctaattc                                          28

<210> 31
<211> 32
<212> DNA
<213> Unknown

<220>
<223> HAMJ 247

<400> 31
gacgctcgag cgctatgaaa tcagataaat tc                                     32

<210> 32
<211> 37
<212> DNA
<213> Unknown

<220>
<223> HAMJ 248

<400> 32
gacgctcgag ggcattacct ggataatcct gttcatg                                37

<210> 33
<211> 33
<212> DNA
<213> Unknown

<220>
<223> HAMJ 249

<400> 33
cagtagatct cttcatcatt tattgaaaag agg                                    33

<210> 34
<211> 40
<212> DNA
<213> Unknown

<220>
<223> HAMJ 278

<400> 34
ttatttcttc catatggact tgacagaaga gcaaattaag                             40

<210> 35
<211> 32
<212> DNA
<213> Unknown

<220>
<223> HAMJ 279

<400> 35
cgccaagctt cgctatgaaa tcagataaat tc                                     32

<210> 36
<211> 34
<212> DNA
<213> Unknown

<220>
<223> HAMJ 280

<400> 36
cgccaagctt ttccacaata taagtcgatt gatt                                    34

<210> 37
<211> 40
<212> DNA
<213> Unknown

<220>
<223> HAMJ 281

<400> 37
ttatttcttc catatggaag tacctatctt ggaaaaagaa                              40

<210> 38
<211> 37
<212> DNA
<213> Unknown

<220>
<223> HAMJ 300

<400> 38
ttatttcttc catatggtgc ctatgcacta aaccagc                                 37

<210> 39
<211> 40
<212> DNA
<213> Unknown

<220>
<223> HAMJ 313

<400> 39
ataagaatgc ggccgcttcc acaatataag tcgattgatt                              40

<210> 40
<211> 31
<212> DNA
<213> Unknown

<220>
<223> OCRR 487

<400> 40
cagtagatct gtgcttatga actaggtttg c                                       31

<210> 41
<211> 32
<212> DNA
<213> Unknown

<220>
<223> OCRR 488

<400> 41
gatcaagctt gctgctacct ttacttactc tc                                      32

<210> 42

<211> 26
<212> DNA
<213> Unknown

<220>
<223> HAMJ 171

<400> 42
ctgagatatc cgttatcgtt caaacc                                            26

<210> 43
<211> 28
<212> DNA
<213> Unknown

<220>
<223> HAMJ 251

<400> 43
ctgcaagctt ttaaagggga ataatacg                                          28

<210> 44
<211> 29
<212> DNA
<213> Unknown

<220>
<223> HAMJ 264

<400> 44
cagtagatct gcagaagcct tcctatctg                                         29

<210> 45
<211> 33
<212> DNA
<213> Unknown

<220>
<223> HAMJ 282

<400> 45
tcgccaagct tcgttatcgt tcaaaccatt ggg                                    33

<210> 46
<211> 45
<212> DNA
<213> Unknown

<220>
<223> HAMJ 283

<400> 46
ataagaatgc ggccgcctta ctctccttta ataaagccaa tagtt                       45

<210> 47
<211> 34
<212> DNA
<213> Unknown

<220>
<223> HAMJ 284

<400> 47
catgccatgg acattgatag tctcttgaaa cagc                                   34

<210> 48
<211> 37
<212> DNA

<213> Unknown

<220>
<223> HAMJ 285

<400> 48
cgccaagctt cttactctcc tttaataaag ccaatag                                37

<210> 49
<211> 31
<212> DNA
<213> Unknown

<220>
<223> HAMJ 286

<400> 49
cgacaagctt aacatggtcg ctagcgttac c                                      31

<210> 50
<211> 30
<212> DNA
<213> Unknown

<220>
<223> HAMJ 287

<400> 50
cataccatgg gcctttatga ggcacctaag                                        30

<210> 51
<211> 34
<212> DNA
<213> Unknown

<220>
<223> HAMJ 288

<400> 51
cgacaagctt aagtaaatct tcagcctctc tcag                                   34

<210> 52
<211> 31
<212> DNA
<213> Unknown

<220>
<223> HAMJ 289

<400> 52
gataccatgg ctagcgacca tgttcaaaga a                                      31

<210> 53
<211> 36
<212> DNA
<213> Unknown

<220>
<223> HAMJ 290

<400> 53
cgccaagctt atcatccact aacttgactt tatcac                                 36

<210> 54
<211> 33
<212> DNA
<213> Unknown

<220>
<223> HAMJ 291

<400> 54
cataccatgg atattcttgc cttcttagct ccg                                    33

<210> 55
<211> 30
<212> DNA
<213> Unknown

<220>
<223> HAMJ 301

<400> 55
catgccatgg tgcttatgaa ctaggtttgc                                        30

<210> 56
<211> 32
<212> DNA
<213> Unknown

<220>
<223> HAMJ 302

<400> 56
cgccaagctt tagcgttacc aaaaccatta tc                                     32

<210> 57
<211> 36
<212> DNA
<213> Unknown

<220>
<223> HAMJ 160

<400> 57
gtattagatc tgttcctatg aacttggtcg tcacca                                 36

<210> 58
<211> 28
<212> DNA
<213> Unknown

<220>
<223> HAMJ 186

<400> 58
cgcctctaga ctactgtata ggagccgg                                          28

<210> 59
<211> 34
<212> DNA
<213> Unknown

<220>
<223> HAMJ 292

<400> 59
catgccatgg aaaacatttc aagcctttta cgtg                                   34

<210> 60
<211> 34
<212> DNA
<213> Unknown

<220>
<223> HAMJ 293

<400> 60
cgacaagctt ctgtatagga gccggttgac tttc                                34

<210> 61
<211> 34
<212> DNA
<213> Unknown

<220>
<223> HAMJ 294

<400> 61
catgccatgg ttcgtaaaaa taaggcagac caag                                34

<210> 62
<211> 30
<212> DNA
<213> Unknown

<220>
<223> HAMJ 297

<400> 62
catgccatgg aagcctattg gaatgggaag                                     30

<210> 63
<211> 31
<212> DNA
<213> Unknown

<220>
<223> HAMJ 352

<400> 63
catgccatgg aagcctattg gaatgggaag c                                   31

<210> 64
<211> 31
<212> DNA
<213> Unknown

<220>
<223> HAMJ 353

<400> 64
cgccaagctt gtaggtaatt tgcgcatttg g                                   31

<210> 65
<211> 30
<212> DNA
<213> Unknown

<220>
<223> HAMJ 354

<400> 65
cgccaagctt ctgtatagga gccggttgac                                     30

<210> 66
<211> 32
<212> DNA
<213> Unknown

<220>
<223> HAMJ 355

<400> 66

catgccatgg atattcttgc cttcttagct cc                                    32

<210> 67
<211> 38
<212> DNA
<213> Unknown

<220>
<223> HAMJ 404

<400> 67
ttatttcttc catatgcatg gtgatcattt ccattaca                              38

<210> 68
<211> 34
<212> DNA
<213> Unknown

<220>
<223> HAMJ 464

<400> 68
gatgcatatg aatatgcaac cgagtcagtt aagc                                  34

<210> 69
<211> 31
<212> DNA
<213> Unknown

<220>
<223> HAMJ 465

<400> 69
gatgctcgag agcatcaaat ccgtatccat c                                     31

<210> 70
<211> 31
<212> DNA
<213> Unknown

<220>
<223> HAMJ 466

<400> 70
gatgcatatg gatcatttcc attacattcc a                                     31

<210> 71
<211> 30
<212> DNA
<213> Unknown

<220>
<223> HAMJ 467

<400> 71
gacaagcttg gcattacctg gataatcctg                                       30

<210> 72
<211> 31
<212> DNA
<213> Unknown

<220>
<223> HAMJ 352

<400> 72
catgccatgg aagcctattg gaatgggaag c                                     31

<210> 73
<211> 38
<212> DNA
<213> Unknown

<220>
<223> HAMJ 470

<400> 73
ataagaatgc ggccgccgct atgaaatcag ataaattc                                38

<210> 74

<400> 74
000

<210> 75

<400> 75
000

<210> 76

<400> 76
000

<210> 77

<400> 77
000

<210> 78

<400> 78
000

<210> 79

<400> 79
000

<210> 80

<400> 80
000

<210> 81

<400> 81
000

<210> 82

<400> 82
000

<210> 83

<400> 83
000

<210> 84

<400> 84
000

<210> 85

<400>  85
000

<210>  86

<400>  86
000

<210>  87

<400>  87
000

<210>  88

<400>  88
000

<210>  89

<400>  89
000

<210>  90

<400>  90
000

<210>  91

<400>  91
000

<210>  92

<400>  92
000

<210>  93

<400>  93
000

<210>  94

<400>  94
000

<210>  95

<400>  95
000

<210>  96

<400>  96
000

<210>  97

<400>  97
000

<210>  98

<400>  98
000

<210> 99

<400> 99
000

<210> 100

<400> 100
000

<210> 101

<400> 101
000

<210> 102

<400> 102
000

<210> 103

<400> 103
000

<210> 104

<400> 104
000

<210> 105

<400> 105
000

<210> 106

<400> 106
000

<210> 107

<400> 107
000

<210> 108

<400> 108
000

<210> 109

<400> 109
000

<210> 110

<400> 110
000

<210> 111

<400> 111
000

<210> 112

<400> 112

000

<210> 113

<400> 113
000

<210> 114

<400> 114
000

<210> 115

<400> 115
000

<210> 116

<400> 116
000

<210> 117

<400> 117
000

<210> 118

<400> 118
000

<210> 119

<400> 119
000

<210> 120

<400> 120
000

<210> 121

<400> 121
000

<210> 122

<400> 122
000

<210> 123

<400> 123
000

<210> 124

<400> 124
000

<210> 125

<400> 125
000

<210> 126

<400> 126
000

<210> 127

<400> 127
000

<210> 128

<400> 128
000

<210> 129

<400> 129
000

<210> 130

<400> 130
000

<210> 131

<400> 131
000

<210> 132

<400> 132
000

<210> 133

<400> 133
000

<210> 134

<400> 134
000

<210> 135

<400> 135
000

<210> 136

<400> 136
000

<210> 137

<400> 137
000

<210> 138

<400> 138
000

<210> 139

<400> 139
000

<210>  140

<400>  140
000

<210>  141

<400>  141
000

<210>  142

<400>  142
000

<210>  143

<400>  143
000

<210>  144

<400>  144
000

<210>  145

<400>  145
000

<210>  146

<400>  146
000

<210>  147

<400>  147
000

<210>  148

<400>  148
000

<210>  149

<400>  149
000

<210>  150

<400>  150
000

<210>  151

<400>  151
000

<210>  152

<400>  152
000

<210>  153

<400> 153
000

<210> 154

<400> 154
000

<210> 155

<400> 155
000

<210> 156

<400> 156
000

<210> 157

<400> 157
000

<210> 158

<400> 158
000

<210> 159

<400> 159
000

<210> 160

<400> 160
000

<210> 161

<400> 161
000

<210> 162

<400> 162
000

<210> 163
<211> 22
<212> PRT
<213> Unknown

<220>
<223> Mab H11-B-11B8 target epitope

<400> 163
Gly Leu Tyr Glu Ala Pro Lys Gly Tyr Ser Leu Glu Asp Leu Leu Ala
1               5                   10                  15
Thr Val Lys Tyr Tyr Val
            20

<210> 164

<400> 164
000

<210> 165

<400> 165
000

<210> 166

<400> 166
000

<210> 167

<400> 167
000

<210> 168
<211> 34
<212> DNA
<213> Unknown

<220>
<223> HAMJ 471

<400> 168
atatgggccc ctgtatagga gccggttgac tttc                                34

<210> 169
<211> 34
<212> DNA
<213> Unknown

<220>
<223> HAMJ 472

<400> 169
atatgggccc aatatgcaac cgagtcagtt aagc                                34

<210> 170
<211> 31
<212> DNA
<213> Unknown

<220>
<223> HAMJ 350

<400> 170
atatgggccc aacatggtcg ctagcgttac c                                   31

<210> 171
<211> 34
<212> DNA
<213> Unknown

<220>
<223> HAMJ 351

<400> 171
tcccgggccc gacttgacag aagagcaaat taag                                34

<210> 172
<211> 34
<212> DNA
<213> Unknown

<220>
<223> HAMJ 358

<400> 172
catgccatgg gacttgacag aagagcaaat taag                                34

<210> 173
<211> 32
<212> DNA
<213> Unknown

<220>
<223> HAMJ 359

<400> 173
tcccgggccc cgctatgaaa tcagataaat tc                                32

<210> 174
<211> 35
<212> DNA
<213> Unknown

<220>
<223> HAMJ 403

<400> 174
atatgggccc gacattgata gtctcttgaa acagc                             35

<210> 175
<211> 31
<212> DNA
<213> Unknown

<220>
<223> HAMJ 361

<400> 175
cgccaagctt aacatggtcg ctagcgttac c                                 31

<210> 176
<211> 37
<212> DNA
<213> Unknown

<220>
<223> HAMJ 483

<400> 176
atatgggccc cttactctcc tttaataaag ccaatag                           37

<210> 177
<211> 45
<212> DNA
<213> Unknown

<220>
<223> HAMJ 513

<400> 177
gaatcaggtt ttgtcatgag ttccggagac cacaatcatt atttc                  45

<210> 178
<211> 45
<212> DNA
<213> Unknown

<220>
<223> HAMJ 514

<400> 178
gaaataatga ttgtggtctc cggaactcat gacaaaacct gattc                  45

<210> 179

<211> 43
<212> DNA
<213> Unknown

<220>
<223> HAMJ 515

<400> 179
gtcatgagtt ccggagactc caatcattat ttcttcaaga agg                43

<210> 180
<211> 43
<212> DNA
<213> Unknown

<220>
<223> HAMJ 516

<400> 180
ccttcttgaa gaaataatga ttggagtctc cggaactcat gac                43

<210> 181
<211> 44
<212> DNA
<213> Unknown

<220>
<223> HAMJ 517

<400> 181
atgagttcgg agactccaat tcttatttct tcaagaagga cttg               44

<210> 182
<211> 45
<212> DNA
<213> Unknown

<220>
<223> HAMJ 518

<400> 182
caagtccttc ttgaagaaat aagaattgga gtctccggaa ctcat              45

<210> 183
<211> 35
<212> DNA
<213> Unknown

<220>
<223> CHAN 51

<400> 183
gcgattattt atccgtctgg agatcaccat catgc                         35

<210> 184
<211> 35
<212> DNA
<213> Unknown

<220>
<223> CHAN 52

<400> 184
gcatgatggt gatctccaga cggataaata atcgc                         35

<210> 185
<211> 30
<212> DNA

<213> Unknown

<220>
<223> CHAN 53

<400> 185
ccgtctggag atggccatca tgcagatccg                                        30

<210> 186
<211> 30
<212> DNA
<213> Unknown

<220>
<223> CHAN 54

<400> 186
cggatctgca tgatggccat ctccagacgg                                        30

<210> 187
<211> 34
<212> DNA
<213> Unknown

<220>
<223> CHAN 47

<400> 187
ccgcagggag ataagcgtca tgcagatccg attg                                   34

<210> 188
<211> 34
<212> DNA
<213> Unknown

<220>
<223> CHAN 48

<400> 188
caatcggatc tgcatgacgc ttatctccct gcgg                                   34

<210> 189
<211> 34
<212> DNA
<213> Unknown

<220>
<223> CHAN 55

<400> 189
ccgtctggag atggcactca tgcagatccg attg                                   34

<210> 190
<211> 34
<212> DNA
<213> Unknown

<220>
<223> CHAN 56

<400> 190
caatcggatc tgcatgagtg ccatctccag acgg                                   34

<210> 191
<211> 37
<212> DNA
<213> Unknown

<220>
<223> CHAN 57

<400> 191
ccgtctggag atggcacttc tgcagatccg attgatg                                37

<210> 192
<211> 37
<212> DNA
<213> Unknown

<220>
<223> CHAN 58

<400> 192
catcaatcgg atctgcagaa gtgccatctc cagacgg                                37

<210> 193
<211> 30
<212> DNA
<213> Unknown

<220>
<223> HAMJ 523

<400> 193
ccgcatggag atggccatca tgcagatccg                                        30

<210> 194
<211> 30
<212> DNA
<213> Unknown

<220>
<223> HAMJ 524

<400> 194
cggatctgca tgatggccat ctccatgcgg                                        30

<210> 195
<211> 43
<212> DNA
<213> Unknown

<220>
<223> HAMJ 526

<400> 195
gtcatgagtc acggagactc caatcattat ttcttcaaga agg                         43

<210> 196
<211> 43
<212> DNA
<213> Unknown

<220>
<223> HAMJ 527

<400> 196
ccttcttgaa gaaataatga ttggagtctc cgtgactcat gac                         43

<210> 197
<211> 45
<212> DNA
<213> Unknown

<220>
<223> HAMJ 528

<400> 197
atgagtcacg gagaccacaa ttcttatttc ttcaagaagg acttg                45

<210> 198
<211> 45
<212> DNA
<213> Unknown

<220>
<223> HAMJ 529

<400> 198
caagtccttc ttgaagaaat aagaattgtg gtctccgtga ctcat                45

<210> 199
<211> 45
<212> DNA
<213> Unknown

<220>
<223> HAMJ 569

<400> 199
tacctcatta tgactcttac tctaacatca aatttgagtg gtttg                45

<210> 200
<211> 45
<212> DNA
<213> Unknown

<220>
<223> HAMJ 570

<400> 200
caaaccactc aaatttgatg ttagagtaag agtcataatg aggta                45

<210> 201
<211> 45
<212> DNA
<213> Unknown

<220>
<223> HAMJ 571

<400> 201
taccttctta tgaccattac tctaacatca aatttgagtg gtttg                45

<210> 202
<211> 44
<212> DNA
<213> Unknown

<220>
<223> HAMJ 572

<400> 202
aaaccactca aatttgatgt tagagtaatg gtcataagaa ggta                 44

<210> 203
<211> 45
<212> DNA
<213> Unknown

<220>
<223> HAMJ 573

<400> 203

aacggtagtt taatcatacc ttctaaagac cattaccata acatc 45

<210> 204
<211> 45
<212> DNA
<213> Unknown

<220>
<223> HAMJ 574

<400> 204
gatgttatgg taatggtctt tagaaggtat gattaaacta ccgtt 45

<210> 205
<211> 46
<212> DNA
<213> Unknown

<220>
<223> HAMJ 575

<400> 205
cggtagttta atcatacctc ataaggactc ttaccataac atcaaa 46

<210> 206
<211> 46
<212> DNA
<213> Unknown

<220>
<223> HAMJ 576

<400> 206
tttgatgtta tggtaagagt ccttatgagg tatgattaaa ctaccg 46

<210> 207
<211> 46
<212> DNA
<213> Unknown

<220>
<223> HAMJ 577

<400> 207
aacggtagtt taatcatacc tgaccattac cataacatca aatttg 46

<210> 208
<211> 46
<212> DNA
<213> Unknown

<220>
<223> HAMJ 578

<400> 208
caaatttgat gttatggtaa tggtcaggta tgattaaact accgtt 46

<210> 209
<211> 45
<212> DNA
<213> Unknown

<220>
<223> HAMJ 579

<400> 209
aacggtagtt taatcatacc ttaccataac atcaaatttg agtgg 45

<210> 210
<211> 45
<212> DNA
<213> Unknown

<220>
<223> HAMJ 580

<400> 210
ccactcaaat ttgatgttat ggtaaggtat gattaaacta ccgtt                45

<210> 211
<211> 45
<212> DNA
<213> Unknown

<220>
<223> HAMJ 581

<400> 211
accggtagtt taatcatacc taacatcaaa tttgagtggt ttgac                45

<210> 212
<211> 45
<212> DNA
<213> Unknown

<220>
<223> HAMJ 582

<400> 212
gtcaaaccac tcaaatttga tgttaggtat gattaaacta ccgtt                45

<210> 213
<211> 35
<212> DNA
<213> Unknown

<220>
<223> HAMJ 536

<400> 213
cctatgtaac tccacatata acccatagcc actgg                35

<210> 214
<211> 35
<212> DNA
<213> Unknown

<220>
<223> HAMJ 537

<400> 214
ccagtggcta tgggttatat gtggagttac atagg                35

<210> 215
<211> 36
<212> DNA
<213> Unknown

<220>
<223> HAMJ 550

<400> 215
cgtgaaagta ttgtcgtaaa taaagaaaaa aatgcg                36

<210> 216
<211> 36

<212> DNA
<213> Unknown

<220>
<223> HAMJ 551

<400> 216
cgcatttttt tctttattta cgacaatact ttcacg                                36

<210> 217
<211> 49
<212> DNA
<213> Unknown

<220>
<223> HAMJ 586

<400> 217
catgaagaag atggttacgg tttcgatgct aaccgtatta tcgctgaag                  49

<210> 218
<211> 48
<212> DNA
<213> Unknown

<220>
<223> HAMJ 587

<400> 218
cttcagcgat aatacggtta gcatcgaaac cgtaaccatc ttcttctg                   48

<210> 219
<211> 42
<212> DNA
<213> Unknown

<220>
<223> HAMJ 588

<400> 219
gaatcaggtt ttgtcatgag tgaccacaat cattatttct tc                         42

<210> 220
<211> 42
<212> DNA
<213> Unknown

<220>
<223> HAMJ 589

<400> 220
gaagaaataa tgattgtggt cactcatgac aaaacctgat tc                         42

<210> 221
<211> 45
<212> DNA
<213> Unknown

<220>
<223> HAMJ 590

<400> 221
gaagatgaat caggttttgt catgagtaat cattatttct tcaag                      45

<210> 222
<211> 45
<212> DNA
<213> Unknown

<220>
<223> HAMJ 591

<400> 222
cttgaagaaa taatgattac tcatgacaaa acctgattca tcttc                45

<210> 223
<211> 45
<212> DNA
<213> Unknown

<220>
<223> HAMJ 592

<400> 223
gaagatgaat caggttttgt catgagttat ttcttcaaga aggac                45

<210> 224
<211> 45
<212> DNA
<213> Unknown

<220>
<223> HAMJ 593

<400> 224
gtccttcttg aagaaataac tcatgacaaa acctgattca tcttc                45

<210> 225
<211> 42
<212> DNA
<213> Unknown

<220>
<223> HAMJ 594

<400> 225
aaaatgcgat tatttatccg caccatcatg cagatccgat tg                   42

<210> 226
<211> 42
<212> DNA
<213> Unknown

<220>
<223> HAMJ 595

<400> 226
caatcggatc tgcatgatgg tgcggataaa taatcgcatt tt                   42

<210> 227
<211> 45
<212> DNA
<213> Unknown

<220>
<223> HAMJ 600

<400> 227
aaaatgcgat tatttatccg gcagatccga ttgatgaaca taaac                45

<210> 228
<211> 45
<212> DNA
<213> Unknown

<220>

<223> HAMJ 601

<400> 228
gtttatgttc atcaatcgga tctgccggat aaataatcgc atttt                45

<210> 229
<211> 45
<212> DNA
<213> Unknown

<220>
<223> HAMJ 604

<400> 229
gatgctaacc gtataatcgc cgaagacgaa tcaggttttg tcatg                45

<210> 230
<211> 45
<212> DNA
<213> Unknown

<220>
<223> HAMJ 605

<400> 230
catgacaaaa cctgattcgt cttcggcgat tatacggtta gcatc                45

<210> 231
<211> 43
<212> DNA
<213> Unknown

<220>
<223> HAMJ 606

<400> 231
cgccgaagac gaatccggct ttgtaatgag tcacggagac tcc                  43

<210> 232
<211> 43
<212> DNA
<213> Unknown

<220>
<223> HAMJ 607

<400> 232
ggagtctccg tgactcatta caaagccgga ttcgtcttcg gcg                  43

<210> 233
<211> 45
<212> DNA
<213> Unknown

<220>
<223> HAMJ 608

<400> 233
catctcatga acaggattat cccggcaacg ccaaagaaat gaaag                45

<210> 234
<211> 45
<212> DNA
<213> Unknown

<220>
<223> HAMJ 609

<400> 234
ctttcatttc tttggcgttg ccgggataat cctgttcatg agatg                45

<210> 235
<211> 569
<212> PRT
<213> Unknown

<220>
<223> NEW 1

<400> 235
Met Asp Leu Thr Glu Glu Gln Ile Lys Ala Ala Gln Lys His Leu Glu
1               5                   10                  15
Glu Val Lys Thr Ser His Asn Gly Leu Asp Ser Leu Ser Ser His Glu
            20                  25                  30
Gln Asp Tyr Pro Gly Asn Ala Lys Glu Met Lys Asp Leu Asp Lys Lys
        35                  40                  45
Ile Glu Glu Lys Ile Ala Gly Ile Met Lys Gln Tyr Gly Val Lys Arg
    50                  55                  60
Glu Ser Ile Val Val Asn Lys Glu Lys Asn Ala Ile Ile Tyr Pro His
65                  70                  75                  80
Gly Asp His His His Ala Asp Pro Ile Asp Glu His Lys Pro Val Gly
                85                  90                  95
Ile Gly His Ser His Ser Asn Tyr Glu Leu Phe Lys Pro Glu Glu Gly
            100                 105                 110
Val Ala Lys Lys Glu Gly Asn Lys Val Tyr Thr Gly Glu Glu Leu Thr
        115                 120                 125
Asn Val Val Asn Leu Leu Lys Asn Ser Thr Phe Asn Asn Gln Asn Phe
    130                 135                 140
Thr Leu Ala Asn Gly Gln Lys Arg Val Ser Phe Ser Phe Pro Pro Glu
145                 150                 155                 160
Leu Glu Lys Lys Leu Gly Ile Asn Met Leu Val Lys Leu Ile Thr Pro
                165                 170                 175
Asp Gly Lys Val Leu Glu Lys Val Ser Gly Lys Val Phe Gly Glu Gly
            180                 185                 190
Val Gly Asn Ile Ala Asn Phe Glu Leu Asp Gln Pro Tyr Leu Pro Gly
        195                 200                 205
Gln Thr Phe Lys Tyr Thr Ile Ala Ser Lys Asp Tyr Pro Glu Val Ser
    210                 215                 220
Tyr Asp Gly Thr Phe Thr Val Pro Thr Ser Leu Ala Tyr Lys Met Ala
225                 230                 235                 240
Ser Gln Thr Ile Phe Tyr Pro Phe His Ala Gly Asp Thr Tyr Leu Arg
                245                 250                 255
Val Asn Pro Gln Phe Ala Val Pro Lys Gly Thr Asp Ala Leu Val Arg
            260                 265                 270
Val Phe Asp Glu Phe His Gly Asn Ala Tyr Leu Glu Asn Asn Tyr Lys
        275                 280                 285
Val Gly Glu Ile Lys Leu Pro Ile Pro Lys Leu Asn Gln Gly Thr Thr
    290                 295                 300
Arg Thr Ala Gly Asn Lys Ile Pro Val Thr Phe Met Ala Asn Ala Tyr
305                 310                 315                 320
Leu Asp Asn Gln Ser Thr Tyr Ile Val Glu Val Pro Ile Leu Glu Lys
                325                 330                 335
Glu Asn Gln Thr Asp Lys Pro Ser Ile Leu Pro Gln Phe Lys Arg Asn
            340                 345                 350
Lys Ala Gln Glu Asn Ser Lys Leu Asp Glu Lys Val Glu Glu Pro Lys
        355                 360                 365
Thr Ser Glu Lys Val Glu Lys Glu Lys Leu Ser Glu Thr Gly Asn Ser
    370                 375                 380
Thr Ser Asn Ser Thr Leu Glu Glu Val Pro Thr Val Asp Pro Val Gln
385                 390                 395                 400
Glu Lys Val Ala Lys Phe Ala Glu Ser Tyr Gly Met Lys Leu Glu Asn
                405                 410                 415
Val Leu Phe Asn Met Asp Gly Thr Ile Glu Leu Tyr Leu Pro Ser Gly
            420                 425                 430
Glu Val Ile Lys Lys Asn Met Ala Asp Phe Thr Gly Glu Ala Pro Gln
        435                 440                 445

Gly Asn Gly Glu Asn Lys Pro Ser Glu Asn Gly Lys Val Ser Thr Gly
    450                 455                 460
Thr Val Glu Asn Gln Pro Thr Glu Asn Lys Pro Ala Asp Ser Leu Pro
465                 470                 475                 480
Glu Ala Pro Asn Glu Lys Pro Val Lys Pro Glu Asn Ser Thr Asp Asn
                485                 490                 495
Gly Met Leu Asn Pro Glu Gly Asn Val Gly Ser Asp Pro Met Leu Asp
            500                 505                 510
Pro Ala Leu Glu Glu Ala Pro Ala Val Asp Pro Val Gln Glu Lys Leu
        515                 520                 525
Glu Lys Phe Thr Ala Ser Tyr Gly Leu Gly Leu Asp Ser Val Ile Phe
    530                 535                 540
Asn Met Asp Gly Thr Ile Glu Leu Arg Leu Pro Ser Gly Glu Val Ile
545                 550                 555                 560
Lys Lys Asn Leu Ser Asp Phe Ile Ala
                565

<210> 236

<400> 236
000

<210> 237

<400> 237
000

<210> 238

<400> 238
000

<210> 239

<400> 239
000

<210> 240

<400> 240
000

<210> 241

<400> 241
000

<210> 242

<400> 242
000

<210> 243

<400> 243
000

<210> 244

<400> 244
000

<210> 245

<400> 245
000

<210> 246

<400> 246
000

<210> 247

<400> 247
000

<210> 248

<400> 248
000

<210> 249

<400> 249
000

<210> 250

<400> 250
000

<210> 251

<400> 251
000

<210> 252

<400> 252
000

<210> 253

<400> 253
000

<210> 254

<400> 254
000

<210> 255
<211> 569
<212> PRT
<213> Unknown

<220>
<223> NEW 1

<400> 255
Met Asp Leu Thr Glu Glu Gln Ile Lys Ala Ala Gln Lys His Leu Glu
1               5                   10                  15
Glu Val Lys Thr Ser His Asn Gly Leu Asp Ser Leu Ser Ser His Glu
            20                  25                  30
Gln Asp Tyr Pro Gly Asn Ala Lys Glu Met Lys Asp Leu Asp Lys Lys
        35                  40                  45
Ile Glu Glu Lys Ile Ala Gly Ile Met Lys Gln Tyr Gly Val Lys Arg
    50                  55                  60
Glu Ser Ile Val Val Asn Lys Glu Lys Asn Ala Ile Ile Tyr Pro His
65                  70                  75                  80
Gly Asp His His His Ala Asp Pro Ile Asp Glu His Lys Pro Val Gly
                85                  90                  95
Ile Gly His Ser His Ser Asn Tyr Glu Leu Phe Lys Pro Glu Glu Gly
            100                 105                 110
Val Ala Lys Lys Glu Gly Asn Lys Val Tyr Thr Gly Glu Glu Leu Thr

115                 120                 125
Asn Val Val Asn Leu Leu Lys Asn Ser Thr Phe Asn Asn Gln Asn Phe
    130                 135                 140
Thr Leu Ala Asn Gly Gln Lys Arg Val Ser Phe Ser Phe Pro Pro Glu
145                 150                 155                 160
Leu Glu Lys Lys Leu Gly Ile Asn Met Leu Val Lys Leu Ile Thr Pro
                165                 170                 175
Asp Gly Lys Val Leu Glu Lys Val Ser Gly Lys Val Phe Gly Glu Gly
            180                 185                 190
Val Gly Asn Ile Ala Asn Phe Glu Leu Asp Gln Pro Tyr Leu Pro Gly
        195                 200                 205
Gln Thr Phe Lys Tyr Thr Ile Ala Ser Lys Asp Tyr Pro Glu Val Ser
    210                 215                 220
Tyr Asp Gly Thr Phe Thr Val Pro Thr Ser Leu Ala Tyr Lys Met Ala
225                 230                 235                 240
Ser Gln Thr Ile Phe Tyr Pro Phe His Ala Gly Asp Thr Tyr Leu Arg
                245                 250                 255
Val Asn Pro Gln Phe Ala Val Pro Lys Gly Thr Asp Ala Leu Val Arg
            260                 265                 270
Val Phe Asp Glu Phe His Gly Asn Ala Tyr Leu Glu Asn Asn Tyr Lys
        275                 280                 285
Val Gly Glu Ile Lys Leu Pro Ile Pro Lys Leu Asn Gln Gly Thr Thr
    290                 295                 300
Arg Thr Ala Gly Asn Lys Ile Pro Val Thr Phe Met Ala Asn Ala Tyr
305                 310                 315                 320
Leu Asp Asn Gln Ser Thr Tyr Ile Val Glu Val Pro Ile Leu Glu Lys
                325                 330                 335
Glu Asn Gln Thr Asp Lys Pro Ser Ile Leu Pro Gln Phe Lys Arg Asn
            340                 345                 350
Lys Ala Gln Glu Asn Ser Lys Leu Asp Glu Lys Val Glu Glu Pro Lys
        355                 360                 365
Thr Ser Glu Lys Val Glu Lys Glu Lys Leu Ser Glu Thr Gly Asn Ser
    370                 375                 380
Thr Ser Asn Ser Thr Leu Glu Glu Val Pro Thr Val Asp Pro Val Gln
385                 390                 395                 400
Glu Lys Val Ala Lys Phe Ala Glu Ser Tyr Gly Met Lys Leu Glu Asn
                405                 410                 415
Val Leu Phe Asn Met Asp Gly Thr Ile Glu Leu Tyr Leu Pro Ser Gly
            420                 425                 430
Glu Val Ile Lys Lys Asn Met Ala Asp Phe Thr Gly Glu Ala Pro Gln
        435                 440                 445
Gly Asn Gly Glu Asn Lys Pro Ser Glu Asn Gly Lys Val Ser Thr Gly
    450                 455                 460
Thr Val Glu Asn Gln Pro Thr Glu Asn Lys Pro Ala Asp Ser Leu Pro
465                 470                 475                 480
Glu Ala Pro Asn Glu Lys Pro Val Lys Pro Glu Asn Ser Thr Asp Asn
                485                 490                 495
Gly Met Leu Asn Pro Glu Gly Asn Val Gly Ser Asp Pro Met Leu Asp
            500                 505                 510
Pro Ala Leu Glu Glu Ala Pro Ala Val Asp Pro Val Gln Glu Lys Leu
        515                 520                 525
Glu Lys Phe Thr Ala Ser Tyr Gly Leu Gly Leu Asp Ser Val Ile Phe
    530                 535                 540
Asn Met Asp Gly Thr Ile Glu Leu Arg Leu Pro Ser Gly Glu Val Ile
545                 550                 555                 560
Lys Lys Asn Leu Ser Asp Phe Ile Ala
                565

<210> 256
<211> 569
<212> PRT
<213> Unknown

<220>
<223> NEW 35A

<400> 256
Met Asp Leu Thr Glu Glu Gln Ile Lys Ala Ala Gln Lys His Leu Glu

1                   5                   10                  15
Glu Val Lys Thr Ser His Asn Gly Leu Asp Ser Leu Ser Ser His Glu
                20                  25                  30
Gln Asp Tyr Pro Gly Asn Ala Lys Glu Met Lys Asp Leu Asp Lys Lys
            35                  40                  45
Ile Glu Glu Lys Ile Ala Gly Ile Met Lys Gln Tyr Gly Val Lys Arg
    50                  55                  60
Glu Ser Ile Val Val Asn Lys Glu Lys Asn Ala Ile Ile Tyr Pro Ser
65                  70                  75                  80
Gly Asp Gly Thr Ser Ala Asp Pro Ile Asp Glu His Lys Pro Val Gly
                85                  90                  95
Ile Gly His Ser His Ser Asn Tyr Glu Leu Phe Lys Pro Glu Glu Gly
            100                 105                 110
Val Ala Lys Lys Glu Gly Asn Lys Val Tyr Thr Gly Glu Glu Leu Thr
        115                 120                 125
Asn Val Val Asn Leu Leu Lys Asn Ser Thr Phe Asn Asn Gln Asn Phe
    130                 135                 140
Thr Leu Ala Asn Gly Gln Lys Arg Val Ser Phe Ser Phe Pro Pro Glu
145                 150                 155                 160
Leu Glu Lys Lys Leu Gly Ile Asn Met Leu Val Lys Leu Ile Thr Pro
                165                 170                 175
Asp Gly Lys Val Leu Glu Lys Val Ser Gly Lys Val Phe Gly Glu Gly
            180                 185                 190
Val Gly Asn Ile Ala Asn Phe Glu Leu Asp Gln Pro Tyr Leu Pro Gly
        195                 200                 205
Gln Thr Phe Lys Tyr Thr Ile Ala Ser Lys Asp Tyr Pro Glu Val Ser
    210                 215                 220
Tyr Asp Gly Thr Phe Thr Val Pro Thr Ser Leu Ala Tyr Lys Met Ala
225                 230                 235                 240
Ser Gln Thr Ile Phe Tyr Pro Phe His Ala Gly Asp Thr Tyr Leu Arg
                245                 250                 255
Val Asn Pro Gln Phe Ala Val Pro Lys Gly Thr Asp Ala Leu Val Arg
            260                 265                 270
Val Phe Asp Glu Phe His Gly Asn Ala Tyr Leu Glu Asn Asn Tyr Lys
        275                 280                 285
Val Gly Glu Ile Lys Leu Pro Ile Pro Lys Leu Asn Gln Gly Thr Thr
    290                 295                 300
Arg Thr Ala Gly Asn Lys Ile Pro Val Thr Phe Met Ala Asn Ala Tyr
305                 310                 315                 320
Leu Asp Asn Gln Ser Thr Tyr Ile Val Glu Val Pro Ile Leu Glu Lys
                325                 330                 335
Glu Asn Gln Thr Asp Lys Pro Ser Ile Leu Pro Gln Phe Lys Arg Asn
            340                 345                 350
Lys Ala Gln Glu Asn Ser Lys Leu Asp Glu Lys Val Glu Glu Pro Lys
        355                 360                 365
Thr Ser Glu Lys Val Glu Lys Glu Lys Leu Ser Glu Thr Gly Asn Ser
    370                 375                 380
Thr Ser Asn Ser Thr Leu Glu Glu Val Pro Thr Val Asp Pro Val Gln
385                 390                 395                 400
Glu Lys Val Ala Lys Phe Ala Glu Ser Tyr Gly Met Lys Leu Glu Asn
                405                 410                 415
Val Leu Phe Asn Met Asp Gly Thr Ile Glu Leu Tyr Leu Pro Ser Gly
            420                 425                 430
Glu Val Ile Lys Lys Asn Met Ala Asp Phe Thr Gly Glu Ala Pro Gln
        435                 440                 445
Gly Asn Gly Glu Asn Lys Pro Ser Glu Asn Gly Lys Val Ser Thr Gly
    450                 455                 460
Thr Val Glu Asn Gln Pro Thr Glu Asn Lys Pro Ala Asp Ser Leu Pro
465                 470                 475                 480
Glu Ala Pro Asn Glu Lys Pro Val Lys Pro Glu Asn Ser Thr Asp Asn
                485                 490                 495
Gly Met Leu Asn Pro Glu Gly Asn Val Gly Ser Asp Pro Met Leu Asp
            500                 505                 510
Pro Ala Leu Glu Glu Ala Pro Ala Val Asp Pro Val Gln Glu Lys Leu
        515                 520                 525
Glu Lys Phe Thr Ala Ser Tyr Gly Leu Gly Leu Asp Ser Val Ile Phe
    530                 535                 540
Asn Met Asp Gly Thr Ile Glu Leu Arg Leu Pro Ser Gly Glu Val Ile

545                 550                 555                 560
Lys Lys Asn Leu Ser Asp Phe Ile Ala
                565

<210> 257
<211> 819
<212> DNA
<213> Unknown

<220>
<223> NEW43 gene

<400> 257
atgcaaatta cctacactga tgatgagatt caggtagcca agttggcagg caagtacaca      60
acagaagacg gttatatctt tgatactagt tggattaaaa aagatagttt gtctgaagct     120
gagagagcgg cagcccaggc ttatgctaaa gagaaaggtt tgacccctcc ttcgacagac     180
caccaggatt caggaaatac tgaggcaaaa ggagcagaag ctatctacaa ccgcgtgaaa     240
gcagctaaga aggtgccact tgatcgtatg ccttacaatc ttcagtatac tgtagaagtc     300
aaaaacggta gtttaatcat acctcattat gaccattacc ataacatcaa atttgagtgg     360
tttgacgaag gcctttatga ggcacctaag gggtatagtc ttgaggatct tttggcgact     420
gtcaagtact atgtcgaacc gcggaacgct agtgaccatg ttcgtaaaaa taaggcagac     480
caagatagta aacctgatga agataaggaa catgatgaag taagtgagcc aactcaccct     540
gaatctgatg aaaaagagaa tcacgctggt ttaaatcctt cagcagataa tctttataaa     600
ccaagcactg atacggaaga gacagaggaa gaagctgaag ataccacaga tgaggctgaa     660
attcctggta cccctagtat tagacaaaat gctatggaga cattgactgg tctaaaaagt     720
agtcttcttc tcggaacgaa agataataac actatttcag cagaagtaga tagtctcttg     780
gctttgttaa aagaaagtca accggctcct atacagtag                            819

<210> 258
<211> 272
<212> PRT
<213> Unknown

<220>
<223> NEW43 polypeptide

<400> 258
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro His Tyr Asp His
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val

245                 250                 255
Asp Ser Leu Leu Ala Leu Leu Lys Glu Ser Gln Pro Ala Pro Ile Gln
            260                 265                 270

<210> 259
<211> 36
<212> DNA
<213> Unknown

<220>
<223> HAMJ 490

<400> 259
ccgaattcca tatgcaaatt acctacactg atgatg                                36

<210> 260
<211> 29
<212> DNA
<213> Unknown

<220>
<223> HAMJ 491

<400> 260
ggactagtat caaagatata accgtcttc                                        29

<210> 261
<211> 33
<212> DNA
<213> Unknown

<220>
<223> HAMJ 492

<400> 261
ggactagttg gattaaaaaa gatagtttgt ctg                                   33

<210> 262
<211> 32
<212> DNA
<213> Unknown

<220>
<223> HAMJ 493

<400> 262
ttcccgcggt tcgacatagt acttgacagt cg                                    32

<210> 263
<211> 29
<212> DNA
<213> Unknown

<220>
<223> HAMJ 494

<400> 263
ttcccgcgga acgctagtga ccatgttcg                                        29

<210> 264
<211> 31
<212> DNA
<213> Unknown

<220>
<223> HAMJ 495

<400> 264

cggggtacca ggaatttcag cctcatctgt g 31

<210> 265
<211> 36
<212> DNA
<213> Unknown

<220>
<223> HAMJ 496

<400> 265
cccggtaccc ctagtattag acaaaatgct atggag 36

<210> 266
<211> 29
<212> DNA
<213> Unknown

<220>
<223> HAMJ 583

<400> 266
ggatcccggg aggtatgatt aaactaccg 29

<210> 267
<211> 34
<212> DNA
<213> Unknown

<220>
<223> HAMJ 584

<400> 267
catgcccggg aacatcaaat ttgagtggtt tgac 34

<210> 268
<211> 37
<212> DNA
<213> Unknown

<220>
<223> HAMJ 610

<400> 268
cttgatcgac atatgttggc aggcaagtac acaacag 37

<210> 269
<211> 45
<212> DNA
<213> Unknown

<220>
<223> HAMJ 497

<400> 269
aacggtagtt taatcatacc ttcttatgac cattaccata acatc 45

<210> 270
<211> 45
<212> DNA
<213> Unknown

<220>
<223> HAMJ 498

<400> 270
gatgttatgg taatggtcat aagaaggtat gattaaacta ccgtt 45

<210> 271
<211> 45
<212> DNA
<213> Unknown

<220>
<223> HAMJ 499

<400> 271
aatcatacct tcttatgact cttaccataa catcaaattt gagtg                45

<210> 272
<211> 45
<212> DNA
<213> Unknown

<220>
<223> HAMJ 500

<400> 272
cactcaaatt tgatgttatg gtaagagtca taagaaggta tgatt                45

<210> 273
<211> 45
<212> DNA
<213> Unknown

<220>
<223> HAMJ 501

<400> 273
taccttctta tgactcttac tctaacatca aatttgagtg gtttg                45

<210> 274
<211> 45
<212> DNA
<213> Unknown

<220>
<223> HAMJ 502

<400> 274
caaaccactc aaatttgatg ttagagtaag agtcataaga aggta                45

<210> 275
<211> 45
<212> DNA
<213> Unknown

<220>
<223> HAMJ 530

<400> 275
aatcatacct cattatgact cttaccataa catcaaattt gagtg                45

<210> 276
<211> 45
<212> DNA
<213> Unknown

<220>
<223> HAMJ 531

<400> 276
cactcaaatt tgatgttatg gtaagagtca taatgaggta tgatt                45

<210> 277
<211> 45

<212> DNA
<213> Unknown

<220>
<223> HAMJ 532

<400> 277
tacctcatta tgaccattac tctaacatca aatttgagtg gtttg                45

<210> 278
<211> 45
<212> DNA
<213> Unknown

<220>
<223> HAMJ 533

<400> 278
caaaccactc aaatttgatg ttagagtaat ggtcataatg aggta                45

<210> 279
<211> 45
<212> DNA
<213> Unknown

<220>
<223> HAMJ 569

<400> 279
tacctcatta tgactcttac tctaacatca aatttgagtg gtttg                45

<210> 280
<211> 45
<212> DNA
<213> Unknown

<220>
<223> HAMJ 570

<400> 280
caaaccactc aaatttgatg ttagagtaag agtcataatg aggta                45

<210> 281
<211> 45
<212> DNA
<213> Unknown

<220>
<223> HAMJ 571

<400> 281
taccttctta tgaccattac tctaacatca aatttgagtg gtttg                45

<210> 282
<211> 44
<212> DNA
<213> Unknown

<220>
<223> HAMJ 572

<400> 282
aaaccactca aatttgatgt tagagtaatg gtcataagaa ggta                 44

<210> 283
<211> 45
<212> DNA
<213> Unknown

<220>
<223> HAMJ 573

<400> 283
aacggtagtt taatcatacc ttctaaagac cattaccata acatc                45

<210> 284
<211> 45
<212> DNA
<213> Unknown

<220>
<223> HAMJ 574

<400> 284
gatgttatgg taatggtctt tagaaggtat gattaaacta ccgtt                45

<210> 285
<211> 46
<212> DNA
<213> Unknown

<220>
<223> HAMJ 575

<400> 285
cggtagttta atcatacctc ataaggactc ttaccataac atcaaa               46

<210> 286
<211> 46
<212> DNA
<213> Unknown

<220>
<223> HAMJ 576

<400> 286
tttgatgtta tggtaagagt ccttatgagg tatgattaaa ctaccg               46

<210> 287
<211> 46
<212> DNA
<213> Unknown

<220>
<223> HAMJ 577

<400> 287
aacggtagtt taatcatacc tgaccattac cataacatca aatttg               46

<210> 288
<211> 46
<212> DNA
<213> Unknown

<220>
<223> HAMJ 578

<400> 288
caaatttgat gttatggtaa tggtcaggta tgattaaact accgtt               46

<210> 289
<211> 45
<212> DNA
<213> Unknown

<220>

<223> HAMJ 579

<400> 289
aacggtagtt taatcatacc ttaccataac atcaaatttg agtgg                45

<210> 290
<211> 45
<212> DNA
<213> Unknown

<220>
<223> HAMJ 580

<400> 290
ccactcaaat ttgatgttat ggtaaggtat gattaaacta ccgtt                45

<210> 291
<211> 45
<212> DNA
<213> Unknown

<220>
<223> HAMJ 581

<400> 291
accggtagtt taatcatacc taacatcaaa tttgagtggt ttgac                45

<210> 292
<211> 45
<212> DNA
<213> Unknown

<220>
<223> HAMJ 582

<400> 292
gtcaaaccac tcaaatttga tgttaggtat gattaaacta ccgtt                45

<210> 293

<211> 272
<212> PRT
<213> Unknown

<220>
<223> NEW 60

<400> 293
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp His
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160

Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270

<210> 294

<211> 272
<212> PRT
<213> Unknown

<220>
<223> NEW 61

<400> 294
Met Gln Ile Thr Tyr Thr Asp Asp Glu Ile Gln Val Ala Lys Leu Ala
 1               5                  10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro His Tyr Asp Ser
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270

<210> 295

<211> 272
<212> PRT
<213> Unknown

<220>
<223> NEW 62

<400> 295
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro His Tyr Asp His
            100                 105                 110
Tyr Ser Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270

<210> 296

<211> 272
<212> PRT
<213> Unknown

<220>
<223> NEW 80

<400> 296
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp Ser
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu

165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270

<210> 297

<211> 272
<212> PRT
<213> Unknown

<220>
<223> NEW 81

<400> 297
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp Ser
            100                 105                 110
Tyr Ser Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270

<210> 298

<211> 272
<212> PRT
<213> Unknown

<220>
<223> NEW 82

<400> 298
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro His Tyr Asp Ser
            100                 105                 110
Tyr Ser Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270

<210> 299

<211> 272
<212> PRT
<213> Unknown

<220>
<223> NEW 83

<400> 299
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp His
            100                 105                 110
Tyr Ser Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175

Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270

<210> 300

<211> 272
<212> PRT
<213> Unknown

<220>
<223> NEW 84

<400> 300
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Lys Asp His
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270

<210> 301

<211> 272
<212> PRT
<213> Unknown

<220>
<223> NEW 85

<400> 301

Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro His Lys Asp Ser
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270

<210> 302

<211> 270
<212> PRT
<213> Unknown

<220>
<223> NEW 88D1

<400> 302
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Asp His Tyr His
            100                 105                 110
Asn Ile Lys Phe Glu Trp Phe Asp Glu Gly Leu Tyr Glu Ala Pro Lys
        115                 120                 125
Gly Tyr Ser Leu Glu Asp Leu Leu Ala Thr Val Lys Tyr Tyr Val Glu
    130                 135                 140
Pro Arg Asn Ala Ser Asp His Val Arg Lys Asn Lys Ala Asp Gln Asp
145                 150                 155                 160
Ser Lys Pro Asp Glu Asp Lys Glu His Asp Glu Val Ser Glu Pro Thr
                165                 170                 175
His Pro Glu Ser Asp Glu Lys Glu Asn His Ala Gly Leu Asn Pro Ser

180                 185                 190
Ala Asp Asn Leu Tyr Lys Pro Ser Thr Asp Thr Glu Glu Thr Glu Glu
        195                 200                 205
Glu Ala Glu Asp Thr Thr Asp Glu Ala Glu Ile Pro Gly Thr Pro Ser
    210                 215                 220
Ile Arg Gln Asn Ala Met Glu Thr Leu Thr Gly Leu Lys Ser Ser Leu
225                 230                 235                 240
Leu Leu Gly Thr Lys Asp Asn Asn Thr Ile Ser Ala Glu Val Asp Ser
                245                 250                 255
Leu Leu Ala Leu Leu Lys Glu Ser Gln Pro Ala Pro Ile Gln
            260                 265                 270

<210> 303

<211> 268
<212> PRT
<213> Unknown

<220>
<223> NEW 88D2

<400> 303
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Tyr His Asn Ile
            100                 105                 110
Lys Phe Glu Trp Phe Asp Glu Gly Leu Tyr Glu Ala Pro Lys Gly Tyr
        115                 120                 125
Ser Leu Glu Asp Leu Leu Ala Thr Val Lys Tyr Tyr Val Glu Pro Arg
    130                 135                 140
Asn Ala Ser Asp His Val Arg Lys Asn Lys Ala Asp Gln Asp Ser Lys
145                 150                 155                 160
Pro Asp Glu Asp Lys Glu His Asp Glu Val Ser Glu Pro Thr His Pro
                165                 170                 175
Glu Ser Asp Glu Lys Glu Asn His Ala Gly Leu Asn Pro Ser Ala Asp
            180                 185                 190
Asn Leu Tyr Lys Pro Ser Thr Asp Thr Glu Glu Thr Glu Glu Glu Ala
        195                 200                 205
Glu Asp Thr Thr Asp Glu Ala Glu Ile Pro Gly Thr Pro Ser Ile Arg
    210                 215                 220
Gln Asn Ala Met Glu Thr Leu Thr Gly Leu Lys Ser Ser Leu Leu Leu
225                 230                 235                 240
Gly Thr Lys Asp Asn Asn Thr Ile Ser Ala Glu Val Asp Ser Leu Leu
                245                 250                 255
Ala Leu Leu Lys Glu Ser Gln Pro Ala Pro Ile Gln
            260                 265

<210> 304

<211> 266
<212> PRT
<213> Unknown

<220>
<223> NEW 88

<400> 304

Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Asn Ile Lys Phe
            100                 105                 110
Glu Trp Phe Asp Glu Gly Leu Tyr Glu Ala Pro Lys Gly Tyr Ser Leu
        115                 120                 125
Glu Asp Leu Leu Ala Thr Val Lys Tyr Tyr Val Glu Pro Arg Asn Ala
    130                 135                 140
Ser Asp His Val Arg Lys Asn Lys Ala Asp Gln Asp Ser Lys Pro Asp
145                 150                 155                 160
Glu Asp Lys Glu His Asp Glu Val Ser Glu Pro Thr His Pro Glu Ser
                165                 170                 175
Asp Glu Lys Glu Asn His Ala Gly Leu Asn Pro Ser Ala Asp Asn Leu
            180                 185                 190
Tyr Lys Pro Ser Thr Asp Thr Glu Glu Thr Glu Glu Glu Ala Glu Asp
        195                 200                 205
Thr Thr Asp Glu Ala Glu Ile Pro Gly Thr Pro Ser Ile Arg Gln Asn
    210                 215                 220
Ala Met Glu Thr Leu Thr Gly Leu Lys Ser Ser Leu Leu Leu Gly Thr
225                 230                 235                 240
Lys Asp Asn Asn Thr Ile Ser Ala Glu Val Asp Ser Leu Leu Ala Leu
                245                 250                 255
Leu Lys Glu Ser Gln Pro Ala Pro Ile Gln
            260                 265

<210> 305

<400> 305
000

<210> 306

<400> 306
000

<210> 307

<400> 307
000

<210> 308

<400> 308
000

<210> 309

<400> 309
000

<210> 310

<400> 310
000

<210> 311

<400> 311

000

<210> 312

<400> 312
000

<210> 313

<400> 313
000

<210> 314

<400> 314
000

<210> 315

<400> 315
000

<210> 316

<400> 316
000

<210> 317

<400> 317
000

<210> 318

<400> 318
000

<210> 319

<400> 319
000

<210> 320

<400> 320
000

<210> 321

<400> 321
000

<210> 322

<400> 322
000

<210> 323

<400> 323
000

<210> 324

<400> 324
000

<210> 325

<400> 325
000

<210> 326

<400> 326
000

<210> 327
<211> 41
<212> DNA
<213> Unknown

<220>
<223> HAMJ 564

<400> 327
atatgggccc caaattacct acactgatga tgagattcag g                41

<210> 328
<211> 43
<212> DNA
<213> Unknown

<220>
<223> HAMJ 556

<400> 328
ataagaatgc ggccgcctac tgtataggag ccggttgact ttc              43

<210> 329
<211> 33
<212> DNA
<213> Unknown

<220>
<223> HAMJ 489

<400> 329
ccgaattcca tatgcaaatt gggcaaccga ctc                         33

<210> 330
<211> 29
<212> DNA
<213> Unknown

<220>
<223> HAMJ 559

<400> 330
atatgggccc caaattgggc aaccgactc                              29

<210> 331
<211> 41
<212> DNA
<213> Unknown

<220>
<223> HAMJ 557

<400> 331
ataagaatgc ggccgcttac gctatgaaat cagataaatt c                41

<210> 332

<211> 906
<212> PRT

<213> Unknown

<220>
<223> VP 94

<400> 332
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp His
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Gly Pro Gln Ile Gly Gln Pro Thr Leu Pro Asn Asn Ser Leu Ala Thr
        275                 280                 285
Pro Ser Pro Ser Leu Pro Ile Asn Pro Gly Thr Ser His Glu Lys His
    290                 295                 300
Glu Glu Asp Gly Tyr Gly Phe Asp Ala Asn Arg Ile Ile Ala Glu Asp
305                 310                 315                 320
Glu Ser Gly Phe Val Met Ser His Gly Asp Ser Asn His Tyr Phe Phe
                325                 330                 335
Lys Lys Asp Leu Thr Glu Glu Gln Ile Lys Ala Ala Gln Lys His Leu
            340                 345                 350
Glu Glu Val Lys Thr Ser His Asn Gly Leu Asp Ser Leu Ser Ser His
        355                 360                 365
Glu Gln Asp Tyr Pro Gly Asn Ala Lys Glu Met Lys Asp Leu Asp Lys
    370                 375                 380
Lys Ile Glu Glu Lys Ile Ala Gly Ile Met Lys Gln Tyr Gly Val Lys
385                 390                 395                 400
Arg Glu Ser Ile Val Val Asn Lys Glu Lys Asn Ala Ile Ile Tyr Pro
                405                 410                 415
Ser Gly Asp His His His Ala Asp Pro Ile Asp Glu His Lys Pro Val
            420                 425                 430
Gly Ile Gly His Ser His Ser Asn Tyr Glu Leu Phe Lys Pro Glu Glu
        435                 440                 445
Gly Val Ala Lys Lys Glu Gly Asn Lys Val Tyr Thr Gly Glu Glu Leu
    450                 455                 460
Thr Asn Val Val Asn Leu Leu Lys Asn Ser Thr Phe Asn Asn Gln Asn
465                 470                 475                 480
Phe Thr Leu Ala Asn Gly Gln Lys Arg Val Ser Phe Ser Phe Pro Pro
                485                 490                 495

Glu Leu Glu Lys Lys Leu Gly Ile Asn Met Leu Val Lys Leu Ile Thr
            500                 505                 510
Pro Asp Gly Lys Val Leu Glu Lys Val Ser Gly Lys Val Phe Gly Glu
        515                 520                 525
Gly Val Gly Asn Ile Ala Asn Phe Glu Leu Asp Gln Pro Tyr Leu Pro
    530                 535                 540
Gly Gln Thr Phe Lys Tyr Thr Ile Ala Ser Lys Asp Tyr Pro Glu Val
545                 550                 555                 560
Ser Tyr Asp Gly Thr Phe Thr Val Pro Thr Ser Leu Ala Tyr Lys Met
                565                 570                 575
Ala Ser Gln Thr Ile Phe Tyr Pro Phe His Ala Gly Asp Thr Tyr Leu
            580                 585                 590
Arg Val Asn Pro Gln Phe Ala Val Pro Lys Gly Thr Asp Ala Leu Val
        595                 600                 605
Arg Val Phe Asp Glu Phe His Gly Asn Ala Tyr Leu Glu Asn Asn Tyr
    610                 615                 620
Lys Val Gly Glu Ile Lys Leu Pro Ile Pro Lys Leu Asn Gln Gly Thr
625                 630                 635                 640
Thr Arg Thr Ala Gly Asn Lys Ile Pro Val Thr Phe Met Ala Asn Ala
                645                 650                 655
Tyr Leu Asp Asn Gln Ser Thr Tyr Ile Val Glu Val Pro Ile Leu Glu
            660                 665                 670
Lys Glu Asn Gln Thr Asp Lys Pro Ser Ile Leu Pro Gln Phe Lys Arg
        675                 680                 685
Asn Lys Ala Gln Glu Asn Ser Lys Leu Asp Glu Lys Val Glu Glu Pro
    690                 695                 700
Lys Thr Ser Glu Lys Val Glu Lys Glu Lys Leu Ser Glu Thr Gly Asn
705                 710                 715                 720
Ser Thr Ser Asn Ser Thr Leu Glu Glu Val Pro Thr Val Asp Pro Val
                725                 730                 735
Gln Glu Lys Val Ala Lys Phe Ala Glu Ser Tyr Gly Met Lys Leu Glu
            740                 745                 750
Asn Val Leu Phe Asn Met Asp Gly Thr Ile Glu Leu Tyr Leu Pro Ser
        755                 760                 765
Gly Glu Val Ile Lys Lys Asn Met Ala Asp Phe Thr Gly Glu Ala Pro
    770                 775                 780
Gln Gly Asn Gly Glu Asn Lys Pro Ser Glu Asn Gly Lys Val Ser Thr
785                 790                 795                 800
Gly Thr Val Glu Asn Gln Pro Thr Glu Asn Lys Pro Ala Asp Ser Leu
                805                 810                 815
Pro Glu Ala Pro Asn Glu Lys Pro Val Lys Pro Glu Asn Ser Thr Asp
            820                 825                 830
Asn Gly Met Leu Asn Pro Glu Gly Asn Val Gly Ser Asp Pro Met Leu
        835                 840                 845
Asp Pro Ala Leu Glu Glu Ala Pro Ala Val Asp Pro Val Gln Glu Lys
    850                 855                 860
Leu Glu Lys Phe Thr Ala Ser Tyr Gly Leu Gly Leu Asp Ser Val Ile
865                 870                 875                 880
Phe Asn Met Asp Gly Thr Ile Glu Leu Arg Leu Pro Ser Gly Glu Val
                885                 890                 895
Ile Lys Lys Asn Leu Ser Asp Phe Ile Ala
            900                 905

<210> 333

<211> 900
<212> PRT
<213> Unknown

<220>
<223> VP 108

<400> 333
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu

35                  40                  45
Ser Gly Phe Val Met Ser His Gly Asp Ser Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser
    130                 135                 140
Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu

580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala Gly Pro Gln Ile Thr Tyr Thr
625                 630                 635                 640
Asp Asp Glu Ile Gln Val Ala Lys Leu Ala Gly Lys Tyr Thr Thr Glu
                645                 650                 655
Asp Gly Tyr Ile Phe Asp Thr Ser Trp Ile Lys Lys Asp Ser Leu Ser
            660                 665                 670
Glu Ala Glu Arg Ala Ala Ala Gln Ala Tyr Ala Lys Glu Lys Gly Leu
        675                 680                 685
Thr Pro Pro Ser Thr Asp His Gln Asp Ser Gly Asn Thr Glu Ala Lys
    690                 695                 700
Gly Ala Glu Ala Ile Tyr Asn Arg Val Lys Ala Ala Lys Lys Val Pro
705                 710                 715                 720
Leu Asp Arg Met Pro Tyr Asn Leu Gln Tyr Thr Val Glu Val Lys Asn
                725                 730                 735
Gly Ser Leu Ile Ile Pro Asn Ile Lys Phe Glu Trp Phe Asp Glu Gly
            740                 745                 750
Leu Tyr Glu Ala Pro Lys Gly Tyr Ser Leu Glu Asp Leu Leu Ala Thr
        755                 760                 765
Val Lys Tyr Tyr Val Glu Pro Arg Asn Ala Ser Asp His Val Arg Lys
    770                 775                 780
Asn Lys Ala Asp Gln Asp Ser Lys Pro Asp Glu Asp Lys Glu His Asp
785                 790                 795                 800
Glu Val Ser Glu Pro Thr His Pro Glu Ser Asp Glu Lys Glu Asn His
                805                 810                 815
Ala Gly Leu Asn Pro Ser Ala Asp Asn Leu Tyr Lys Pro Ser Thr Asp
            820                 825                 830
Thr Glu Glu Thr Glu Glu Glu Ala Glu Asp Thr Thr Asp Glu Ala Glu
        835                 840                 845
Ile Pro Gly Thr Pro Ser Ile Arg Gln Asn Ala Met Glu Thr Leu Thr
    850                 855                 860
Gly Leu Lys Ser Ser Leu Leu Leu Gly Thr Lys Asp Asn Asn Thr Ile
865                 870                 875                 880
Ser Ala Glu Val Asp Ser Leu Leu Ala Leu Leu Lys Glu Ser Gln Pro
                885                 890                 895
Ala Pro Ile Gln
            900

<210> 334

<211> 900
<212> PRT
<213> Unknown

<220>
<223> VP 109

<400> 334
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Asn Ile Lys Phe
            100                 105                 110
Glu Trp Phe Asp Glu Gly Leu Tyr Glu Ala Pro Lys Gly Tyr Ser Leu
        115                 120                 125

Glu Asp Leu Leu Ala Thr Val Lys Tyr Tyr Val Glu Pro Arg Asn Ala
    130                 135                 140
Ser Asp His Val Arg Lys Asn Lys Ala Asp Gln Asp Ser Lys Pro Asp
145                 150                 155                 160
Glu Asp Lys Glu His Asp Glu Val Ser Glu Pro Thr His Pro Glu Ser
                165                 170                 175
Asp Glu Lys Glu Asn His Ala Gly Leu Asn Pro Ser Ala Asp Asn Leu
            180                 185                 190
Tyr Lys Pro Ser Thr Asp Thr Glu Glu Thr Glu Glu Glu Ala Glu Asp
        195                 200                 205
Thr Thr Asp Glu Ala Glu Ile Pro Gly Thr Pro Ser Ile Arg Gln Asn
    210                 215                 220
Ala Met Glu Thr Leu Thr Gly Leu Lys Ser Ser Leu Leu Leu Gly Thr
225                 230                 235                 240
Lys Asp Asn Asn Thr Ile Ser Ala Glu Val Asp Ser Leu Leu Ala Leu
                245                 250                 255
Leu Lys Glu Ser Gln Pro Ala Pro Ile Gln Gly Pro Gln Ile Gly Gln
            260                 265                 270
Pro Thr Leu Pro Asn Asn Ser Leu Ala Thr Pro Ser Pro Ser Leu Pro
        275                 280                 285
Ile Asn Pro Gly Thr Ser His Glu Lys His Glu Glu Asp Gly Tyr Gly
    290                 295                 300
Phe Asp Ala Asn Arg Ile Ile Ala Glu Asp Glu Ser Gly Phe Val Met
305                 310                 315                 320
Ser His Gly Asp Ser Asn His Tyr Phe Phe Lys Lys Asp Leu Thr Glu
                325                 330                 335
Glu Gln Ile Lys Ala Ala Gln Lys His Leu Glu Glu Val Lys Thr Ser
            340                 345                 350
His Asn Gly Leu Asp Ser Leu Ser Ser His Glu Gln Asp Tyr Pro Gly
        355                 360                 365
Asn Ala Lys Glu Met Lys Asp Leu Asp Lys Lys Ile Glu Glu Lys Ile
    370                 375                 380
Ala Gly Ile Met Lys Gln Tyr Gly Val Lys Arg Glu Ser Ile Val Val
385                 390                 395                 400
Asn Lys Glu Lys Asn Ala Ile Ile Tyr Pro Ser Gly Asp His His His
                405                 410                 415
Ala Asp Pro Ile Asp Glu His Lys Pro Val Gly Ile Gly His Ser His
            420                 425                 430
Ser Asn Tyr Glu Leu Phe Lys Pro Glu Glu Gly Val Ala Lys Lys Glu
        435                 440                 445
Gly Asn Lys Val Tyr Thr Gly Glu Glu Leu Thr Asn Val Val Asn Leu
    450                 455                 460
Leu Lys Asn Ser Thr Phe Asn Asn Gln Asn Phe Thr Leu Ala Asn Gly
465                 470                 475                 480
Gln Lys Arg Val Ser Phe Ser Phe Pro Pro Glu Leu Glu Lys Lys Leu
                485                 490                 495
Gly Ile Asn Met Leu Val Lys Leu Ile Thr Pro Asp Gly Lys Val Leu
            500                 505                 510
Glu Lys Val Ser Gly Lys Val Phe Gly Glu Gly Val Gly Asn Ile Ala
        515                 520                 525
Asn Phe Glu Leu Asp Gln Pro Tyr Leu Pro Gly Gln Thr Phe Lys Tyr
    530                 535                 540
Thr Ile Ala Ser Lys Asp Tyr Pro Glu Val Ser Tyr Asp Gly Thr Phe
545                 550                 555                 560
Thr Val Pro Thr Ser Leu Ala Tyr Lys Met Ala Ser Gln Thr Ile Phe
                565                 570                 575
Tyr Pro Phe His Ala Gly Asp Thr Tyr Leu Arg Val Asn Pro Gln Phe
            580                 585                 590
Ala Val Pro Lys Gly Thr Asp Ala Leu Val Arg Val Phe Asp Glu Phe
        595                 600                 605
His Gly Asn Ala Tyr Leu Glu Asn Asn Tyr Lys Val Gly Glu Ile Lys
    610                 615                 620
Leu Pro Ile Pro Lys Leu Asn Gln Gly Thr Thr Arg Thr Ala Gly Asn
625                 630                 635                 640
Lys Ile Pro Val Thr Phe Met Ala Asn Ala Tyr Leu Asp Asn Gln Ser
                645                 650                 655
Thr Tyr Ile Val Glu Val Pro Ile Leu Glu Lys Glu Asn Gln Thr Asp
            660                 665                 670

Lys Pro Ser Ile Leu Pro Gln Phe Lys Arg Asn Lys Ala Gln Glu Asn
        675                 680                 685
Ser Lys Leu Asp Glu Lys Val Glu Glu Pro Lys Thr Ser Glu Lys Val
    690                 695                 700
Glu Lys Glu Lys Leu Ser Glu Thr Gly Asn Ser Thr Ser Asn Ser Thr
705                 710                 715                 720
Leu Glu Glu Val Pro Thr Val Asp Pro Val Gln Glu Lys Val Ala Lys
                725                 730                 735
Phe Ala Glu Ser Tyr Gly Met Lys Leu Glu Asn Val Leu Phe Asn Met
            740                 745                 750
Asp Gly Thr Ile Glu Leu Tyr Leu Pro Ser Gly Glu Val Ile Lys Lys
        755                 760                 765
Asn Met Ala Asp Phe Thr Gly Glu Ala Pro Gln Gly Asn Gly Glu Asn
    770                 775                 780
Lys Pro Ser Glu Asn Gly Lys Val Ser Thr Gly Thr Val Glu Asn Gln
785                 790                 795                 800
Pro Thr Glu Asn Lys Pro Ala Asp Ser Leu Pro Glu Ala Pro Asn Glu
                805                 810                 815
Lys Pro Val Lys Pro Glu Asn Ser Thr Asp Asn Gly Met Leu Asn Pro
            820                 825                 830
Glu Gly Asn Val Gly Ser Asp Pro Met Leu Asp Pro Ala Leu Glu Glu
        835                 840                 845
Ala Pro Ala Val Asp Pro Val Gln Glu Lys Leu Glu Lys Phe Thr Ala
    850                 855                 860
Ser Tyr Gly Leu Gly Leu Asp Ser Val Ile Phe Asn Met Asp Gly Thr
865                 870                 875                 880
Ile Glu Leu Arg Leu Pro Ser Gly Glu Val Ile Lys Lys Asn Leu Ser
                885                 890                 895
Asp Phe Ile Ala
            900

<210> 335

<211> 900
<212> PRT
<213> Unknown

<220>
<223> VP 110


<400> 335
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp His
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205

Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Gly Pro Gln Ile Gly Gln Pro Thr Leu Pro Asn Asn Ser Leu Ala Thr
        275                 280                 285
Pro Ser Pro Ser Leu Pro Ile Asn Pro Gly Thr Ser His Glu Lys His
    290                 295                 300
Glu Glu Asp Gly Tyr Gly Phe Asp Ala Asn Arg Ile Ile Ala Glu Asp
305                 310                 315                 320
Glu Ser Gly Phe Val Met Ser Tyr Phe Phe Lys Lys Asp Leu Thr Glu
                325                 330                 335
Glu Gln Ile Lys Ala Ala Gln Lys His Leu Glu Glu Val Lys Thr Ser
            340                 345                 350
His Asn Gly Leu Asp Ser Leu Ser Ser His Glu Gln Asp Tyr Pro Gly
        355                 360                 365
Asn Ala Lys Glu Met Lys Asp Leu Asp Lys Lys Ile Glu Glu Lys Ile
    370                 375                 380
Ala Gly Ile Met Lys Gln Tyr Gly Val Lys Arg Glu Ser Ile Val Val
385                 390                 395                 400
Asn Lys Glu Lys Asn Ala Ile Ile Tyr Pro His Gly Asp His His His
                405                 410                 415
Ala Asp Pro Ile Asp Glu His Lys Pro Val Gly Ile Gly His Ser His
            420                 425                 430
Ser Asn Tyr Glu Leu Phe Lys Pro Glu Glu Gly Val Ala Lys Lys Glu
        435                 440                 445
Gly Asn Lys Val Tyr Thr Gly Glu Glu Leu Thr Asn Val Val Asn Leu
    450                 455                 460
Leu Lys Asn Ser Thr Phe Asn Asn Gln Asn Phe Thr Leu Ala Asn Gly
465                 470                 475                 480
Gln Lys Arg Val Ser Phe Ser Phe Pro Pro Glu Leu Glu Lys Lys Leu
                485                 490                 495
Gly Ile Asn Met Leu Val Lys Leu Ile Thr Pro Asp Gly Lys Val Leu
            500                 505                 510
Glu Lys Val Ser Gly Lys Val Phe Gly Glu Gly Val Gly Asn Ile Ala
        515                 520                 525
Asn Phe Glu Leu Asp Gln Pro Tyr Leu Pro Gly Gln Thr Phe Lys Tyr
    530                 535                 540
Thr Ile Ala Ser Lys Asp Tyr Pro Glu Val Ser Tyr Asp Gly Thr Phe
545                 550                 555                 560
Thr Val Pro Thr Ser Leu Ala Tyr Lys Met Ala Ser Gln Thr Ile Phe
                565                 570                 575
Tyr Pro Phe His Ala Gly Asp Thr Tyr Leu Arg Val Asn Pro Gln Phe
            580                 585                 590
Ala Val Pro Lys Gly Thr Asp Ala Leu Val Arg Val Phe Asp Glu Phe
        595                 600                 605
His Gly Asn Ala Tyr Leu Glu Asn Asn Tyr Lys Val Gly Glu Ile Lys
    610                 615                 620
Leu Pro Ile Pro Lys Leu Asn Gln Gly Thr Thr Arg Thr Ala Gly Asn
625                 630                 635                 640
Lys Ile Pro Val Thr Phe Met Ala Asn Ala Tyr Leu Asp Asn Gln Ser
                645                 650                 655
Thr Tyr Ile Val Glu Val Pro Ile Leu Glu Lys Glu Asn Gln Thr Asp
            660                 665                 670
Lys Pro Ser Ile Leu Pro Gln Phe Lys Arg Asn Lys Ala Gln Glu Asn
        675                 680                 685
Ser Lys Leu Asp Glu Lys Val Glu Glu Pro Lys Thr Ser Glu Lys Val
    690                 695                 700
Glu Lys Glu Lys Leu Ser Glu Thr Gly Asn Ser Thr Ser Asn Ser Thr
705                 710                 715                 720
Leu Glu Glu Val Pro Thr Val Asp Pro Val Gln Glu Lys Val Ala Lys
                725                 730                 735
Phe Ala Glu Ser Tyr Gly Met Lys Leu Glu Asn Val Leu Phe Asn Met
            740                 745                 750

Asp Gly Thr Ile Glu Leu Tyr Leu Pro Ser Gly Glu Val Ile Lys Lys
            755                 760                 765
Asn Met Ala Asp Phe Thr Gly Glu Ala Pro Gln Gly Asn Gly Glu Asn
    770                 775                 780
Lys Pro Ser Glu Asn Gly Lys Val Ser Thr Gly Thr Val Glu Asn Gln
785                 790                 795                 800
Pro Thr Glu Asn Lys Pro Ala Asp Ser Leu Pro Glu Ala Pro Asn Glu
                805                 810                 815
Lys Pro Val Lys Pro Glu Asn Ser Thr Asp Asn Gly Met Leu Asn Pro
            820                 825                 830
Glu Gly Asn Val Gly Ser Asp Pro Met Leu Asp Pro Ala Leu Glu Glu
        835                 840                 845
Ala Pro Ala Val Asp Pro Val Gln Glu Lys Leu Glu Lys Phe Thr Ala
    850                 855                 860
Ser Tyr Gly Leu Gly Leu Asp Ser Val Ile Phe Asn Met Asp Gly Thr
865                 870                 875                 880
Ile Glu Leu Arg Leu Pro Ser Gly Glu Val Ile Lys Lys Asn Leu Ser
                885                 890                 895
Asp Phe Ile Ala
            900

<210> 336

<211> 894
<212> PRT
<213> Unknown

<220>
<223> VP 111

<400> 336
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp His
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Gly Pro Gln Ile Gly Gln Pro Thr Leu Pro Asn Asn Ser Leu Ala Thr
        275                 280                 285
Pro Ser Pro Ser Leu Pro Ile Asn Pro Gly Thr Ser His Glu Lys His

290                 295                 300
Glu Glu Asp Gly Tyr Gly Phe Asp Ala Asn Arg Ile Ile Ala Glu Asp
305                 310                 315                 320
Glu Ser Gly Phe Val Met Ser Tyr Phe Phe Lys Lys Asp Leu Thr Glu
                325                 330                 335
Glu Gln Ile Lys Ala Ala Gln Lys His Leu Glu Glu Val Lys Thr Ser
            340                 345                 350
His Asn Gly Leu Asp Ser Leu Ser Ser His Glu Gln Asp Tyr Pro Gly
        355                 360                 365
Asn Ala Lys Glu Met Lys Asp Leu Asp Lys Lys Ile Glu Glu Lys Ile
    370                 375                 380
Ala Gly Ile Met Lys Gln Tyr Gly Val Lys Arg Glu Ser Ile Val Val
385                 390                 395                 400
Asn Lys Glu Lys Asn Ala Ile Ile Tyr Pro Ala Asp Pro Ile Asp Glu
                405                 410                 415
His Lys Pro Val Gly Ile Gly His Ser His Ser Asn Tyr Glu Leu Phe
            420                 425                 430
Lys Pro Glu Glu Gly Val Ala Lys Lys Glu Gly Asn Lys Val Tyr Thr
        435                 440                 445
Gly Glu Glu Leu Thr Asn Val Val Asn Leu Leu Lys Asn Ser Thr Phe
    450                 455                 460
Asn Asn Gln Asn Phe Thr Leu Ala Asn Gly Gln Lys Arg Val Ser Phe
465                 470                 475                 480
Ser Phe Pro Pro Glu Leu Glu Lys Lys Leu Gly Ile Asn Met Leu Val
                485                 490                 495
Lys Leu Ile Thr Pro Asp Gly Lys Val Leu Glu Lys Val Ser Gly Lys
            500                 505                 510
Val Phe Gly Glu Gly Val Gly Asn Ile Ala Asn Phe Glu Leu Asp Gln
        515                 520                 525
Pro Tyr Leu Pro Gly Gln Thr Phe Lys Tyr Thr Ile Ala Ser Lys Asp
    530                 535                 540
Tyr Pro Glu Val Ser Tyr Asp Gly Thr Phe Thr Val Pro Thr Ser Leu
545                 550                 555                 560
Ala Tyr Lys Met Ala Ser Gln Thr Ile Phe Tyr Pro Phe His Ala Gly
                565                 570                 575
Asp Thr Tyr Leu Arg Val Asn Pro Gln Phe Ala Val Pro Lys Gly Thr
            580                 585                 590
Asp Ala Leu Val Arg Val Phe Asp Glu Phe His Gly Asn Ala Tyr Leu
        595                 600                 605
Glu Asn Asn Tyr Lys Val Gly Glu Ile Lys Leu Pro Ile Pro Lys Leu
    610                 615                 620
Asn Gln Gly Thr Thr Arg Thr Ala Gly Asn Lys Ile Pro Val Thr Phe
625                 630                 635                 640
Met Ala Asn Ala Tyr Leu Asp Asn Gln Ser Thr Tyr Ile Val Glu Val
                645                 650                 655
Pro Ile Leu Glu Lys Glu Asn Gln Thr Asp Lys Pro Ser Ile Leu Pro
            660                 665                 670
Gln Phe Lys Arg Asn Lys Ala Gln Glu Asn Ser Lys Leu Asp Glu Lys
        675                 680                 685
Val Glu Glu Pro Lys Thr Ser Glu Lys Val Glu Lys Glu Lys Leu Ser
    690                 695                 700
Glu Thr Gly Asn Ser Thr Ser Asn Ser Thr Leu Glu Glu Val Pro Thr
705                 710                 715                 720
Val Asp Pro Val Gln Glu Lys Val Ala Lys Phe Ala Glu Ser Tyr Gly
                725                 730                 735
Met Lys Leu Glu Asn Val Leu Phe Asn Met Asp Gly Thr Ile Glu Leu
            740                 745                 750
Tyr Leu Pro Ser Gly Glu Val Ile Lys Lys Asn Met Ala Asp Phe Thr
        755                 760                 765
Gly Glu Ala Pro Gln Gly Asn Gly Glu Asn Lys Pro Ser Glu Asn Gly

    770                 775                 780
Lys Val Ser Thr Gly Thr Val Glu Asn Gln Pro Thr Glu Asn Lys Pro
785                 790                 795                 800
Ala Asp Ser Leu Pro Glu Ala Pro Asn Glu Lys Pro Val Lys Pro Glu
                805                 810                 815
Asn Ser Thr Asp Asn Gly Met Leu Asn Pro Glu Gly Asn Val Gly Ser
            820                 825                 830

Asp Pro Met Leu Asp Pro Ala Leu Glu Glu Ala Pro Ala Val Asp Pro
        835                 840                 845
Val Gln Glu Lys Leu Glu Lys Phe Thr Ala Ser Tyr Gly Leu Gly Leu
    850                 855                 860
Asp Ser Val Ile Phe Asn Met Asp Gly Thr Ile Glu Leu Arg Leu Pro
865                 870                 875                 880
Ser Gly Glu Val Ile Lys Lys Asn Leu Ser Asp Phe Ile Ala
                885                 890

<210> 337

<211> 894
<212> PRT
<213> Unknown

<220>
<223> VP 112

<400> 337
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Asn Ile Lys Phe
            100                 105                 110
Glu Trp Phe Asp Glu Gly Leu Tyr Glu Ala Pro Lys Gly Tyr Ser Leu
        115                 120                 125
Glu Asp Leu Leu Ala Thr Val Lys Tyr Tyr Val Glu Pro Arg Asn Ala
    130                 135                 140
Ser Asp His Val Arg Lys Asn Lys Ala Asp Gln Asp Ser Lys Pro Asp
145                 150                 155                 160
Glu Asp Lys Glu His Asp Glu Val Ser Glu Pro Thr His Pro Glu Ser
                165                 170                 175
Asp Glu Lys Glu Asn His Ala Gly Leu Asn Pro Ser Ala Asp Asn Leu
            180                 185                 190
Tyr Lys Pro Ser Thr Asp Thr Glu Glu Thr Glu Glu Glu Ala Glu Asp
        195                 200                 205
Thr Thr Asp Glu Ala Glu Ile Pro Gly Thr Pro Ser Ile Arg Gln Asn
    210                 215                 220
Ala Met Glu Thr Leu Thr Gly Leu Lys Ser Ser Leu Leu Leu Gly Thr
225                 230                 235                 240
Lys Asp Asn Asn Thr Ile Ser Ala Glu Val Asp Ser Leu Leu Ala Leu
                245                 250                 255
Leu Lys Glu Ser Gln Pro Ala Pro Ile Gln Gly Pro Gln Ile Gly Gln
            260                 265                 270
Pro Thr Leu Pro Asn Asn Ser Leu Ala Thr Pro Ser Pro Ser Leu Pro
        275                 280                 285
Ile Asn Pro Gly Thr Ser His Glu Lys His Glu Glu Asp Gly Tyr Gly
    290                 295                 300
Phe Asp Ala Asn Arg Ile Ile Ala Glu Asp Glu Ser Gly Phe Val Met
305                 310                 315                 320
Ser Tyr Phe Phe Lys Lys Asp Leu Thr Glu Glu Gln Ile Lys Ala Ala
                325                 330                 335
Gln Lys His Leu Glu Glu Val Lys Thr Ser His Asn Gly Leu Asp Ser
            340                 345                 350
Leu Ser Ser His Glu Gln Asp Tyr Pro Gly Asn Ala Lys Glu Met Lys
        355                 360                 365
Asp Leu Asp Lys Lys Ile Glu Glu Lys Ile Ala Gly Ile Met Lys Gln
    370                 375                 380
Tyr Gly Val Lys Arg Glu Ser Ile Val Val Asn Lys Glu Lys Asn Ala

385                 390                 395                 400
Ile Ile Tyr Pro His Gly Asp His His His Ala Asp Pro Ile Asp Glu
                405                 410                 415
His Lys Pro Val Gly Ile Gly His Ser His Ser Asn Tyr Glu Leu Phe
            420                 425                 430
Lys Pro Glu Glu Gly Val Ala Lys Lys Glu Gly Asn Lys Val Tyr Thr
        435                 440                 445
Gly Glu Glu Leu Thr Asn Val Val Asn Leu Leu Lys Asn Ser Thr Phe
    450                 455                 460
Asn Asn Gln Asn Phe Thr Leu Ala Asn Gly Gln Lys Arg Val Ser Phe
465                 470                 475                 480
Ser Phe Pro Pro Glu Leu Glu Lys Lys Leu Gly Ile Asn Met Leu Val
                485                 490                 495
Lys Leu Ile Thr Pro Asp Gly Lys Val Leu Glu Lys Val Ser Gly Lys
            500                 505                 510
Val Phe Gly Glu Gly Val Gly Asn Ile Ala Asn Phe Glu Leu Asp Gln
        515                 520                 525
Pro Tyr Leu Pro Gly Gln Thr Phe Lys Tyr Thr Ile Ala Ser Lys Asp
    530                 535                 540
Tyr Pro Glu Val Ser Tyr Asp Gly Thr Phe Thr Val Pro Thr Ser Leu
545                 550                 555                 560
Ala Tyr Lys Met Ala Ser Gln Thr Ile Phe Tyr Pro Phe His Ala Gly
                565                 570                 575
Asp Thr Tyr Leu Arg Val Asn Pro Gln Phe Ala Val Pro Lys Gly Thr
            580                 585                 590
Asp Ala Leu Val Arg Val Phe Asp Glu Phe His Gly Asn Ala Tyr Leu
        595                 600                 605
Glu Asn Asn Tyr Lys Val Gly Glu Ile Lys Leu Pro Ile Pro Lys Leu
    610                 615                 620
Asn Gln Gly Thr Thr Arg Thr Ala Gly Asn Lys Ile Pro Val Thr Phe
625                 630                 635                 640
Met Ala Asn Ala Tyr Leu Asp Asn Gln Ser Thr Tyr Ile Val Glu Val
                645                 650                 655
Pro Ile Leu Glu Lys Glu Asn Gln Thr Asp Lys Pro Ser Ile Leu Pro
            660                 665                 670
Gln Phe Lys Arg Asn Lys Ala Gln Glu Asn Ser Lys Leu Asp Glu Lys
        675                 680                 685
Val Glu Glu Pro Lys Thr Ser Glu Lys Val Glu Lys Glu Lys Leu Ser
    690                 695                 700
Glu Thr Gly Asn Ser Thr Ser Asn Ser Thr Leu Glu Glu Val Pro Thr
705                 710                 715                 720
Val Asp Pro Val Gln Glu Lys Val Ala Lys Phe Ala Glu Ser Tyr Gly
                725                 730                 735
Met Lys Leu Glu Asn Val Leu Phe Asn Met Asp Gly Thr Ile Glu Leu
            740                 745                 750
Tyr Leu Pro Ser Gly Glu Val Ile Lys Lys Asn Met Ala Asp Phe Thr
        755                 760                 765
Gly Glu Ala Pro Gln Gly Asn Gly Glu Asn Lys Pro Ser Glu Asn Gly
    770                 775                 780
Lys Val Ser Thr Gly Thr Val Glu Asn Gln Pro Thr Glu Asn Lys Pro
785                 790                 795                 800
Ala Asp Ser Leu Pro Glu Ala Pro Asn Glu Lys Pro Val Lys Pro Glu
                805                 810                 815
Asn Ser Thr Asp Asn Gly Met Leu Asn Pro Glu Gly Asn Val Gly Ser
            820                 825                 830
Asp Pro Met Leu Asp Pro Ala Leu Glu Glu Ala Pro Ala Val Asp Pro
        835                 840                 845
Val Gln Glu Lys Leu Glu Lys Phe Thr Ala Ser Tyr Gly Leu Gly Leu
    850                 855                 860
Asp Ser Val Ile Phe Asn Met Asp Gly Thr Ile Glu Leu Arg Leu Pro
865                 870                 875                 880
Ser Gly Glu Val Ile Lys Lys Asn Leu Ser Asp Phe Ile Ala
                885                 890

<210> 338

<211> 888
<212> PRT

<213> Unknown

<220>
<223> VP 113

<400> 338
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Asn Ile Lys Phe
            100                 105                 110
Glu Trp Phe Asp Glu Gly Leu Tyr Glu Ala Pro Lys Gly Tyr Ser Leu
        115                 120                 125
Glu Asp Leu Leu Ala Thr Val Lys Tyr Tyr Val Glu Pro Arg Asn Ala
    130                 135                 140
Ser Asp His Val Arg Lys Asn Lys Ala Asp Gln Asp Ser Lys Pro Asp
145                 150                 155                 160
Glu Asp Lys Glu His Asp Glu Val Ser Glu Pro Thr His Pro Glu Ser
                165                 170                 175
Asp Glu Lys Glu Asn His Ala Gly Leu Asn Pro Ser Ala Asp Asn Leu
            180                 185                 190
Tyr Lys Pro Ser Thr Asp Thr Glu Glu Thr Glu Glu Glu Ala Glu Asp
        195                 200                 205
Thr Thr Asp Glu Ala Glu Ile Pro Gly Thr Pro Ser Ile Arg Gln Asn
    210                 215                 220
Ala Met Glu Thr Leu Thr Gly Leu Lys Ser Ser Leu Leu Leu Gly Thr
225                 230                 235                 240
Lys Asp Asn Asn Thr Ile Ser Ala Glu Val Asp Ser Leu Leu Ala Leu
                245                 250                 255
Leu Lys Glu Ser Gln Pro Ala Pro Ile Gln Gly Pro Gln Ile Gly Gln
            260                 265                 270
Pro Thr Leu Pro Asn Asn Ser Leu Ala Thr Pro Ser Pro Ser Leu Pro
        275                 280                 285
Ile Asn Pro Gly Thr Ser His Glu Lys His Glu Glu Asp Gly Tyr Gly
    290                 295                 300
Phe Asp Ala Asn Arg Ile Ile Ala Glu Asp Glu Ser Gly Phe Val Met
305                 310                 315                 320
Ser Tyr Phe Phe Lys Lys Asp Leu Thr Glu Glu Gln Ile Lys Ala Ala
                325                 330                 335
Gln Lys His Leu Glu Glu Val Lys Thr Ser His Asn Gly Leu Asp Ser
            340                 345                 350
Leu Ser Ser His Glu Gln Asp Tyr Pro Gly Asn Ala Lys Glu Met Lys
        355                 360                 365
Asp Leu Asp Lys Lys Ile Glu Glu Lys Ile Ala Gly Ile Met Lys Gln
    370                 375                 380
Tyr Gly Val Lys Arg Glu Ser Ile Val Val Asn Lys Glu Lys Asn Ala
385                 390                 395                 400
Ile Ile Tyr Pro Ala Asp Pro Ile Asp Glu His Lys Pro Val Gly Ile
                405                 410                 415
Gly His Ser His Ser Asn Tyr Glu Leu Phe Lys Pro Glu Glu Gly Val
            420                 425                 430
Ala Lys Lys Glu Gly Asn Lys Val Tyr Thr Gly Glu Glu Leu Thr Asn
        435                 440                 445
Val Val Asn Leu Leu Lys Asn Ser Thr Phe Asn Asn Gln Asn Phe Thr
    450                 455                 460
Leu Ala Asn Gly Gln Lys Arg Val Ser Phe Ser Phe Pro Pro Glu Leu
465                 470                 475                 480
Glu Lys Lys Leu Gly Ile Asn Met Leu Val Lys Leu Ile Thr Pro Asp
                485                 490                 495

Gly Lys Val Leu Glu Lys Val Ser Gly Lys Val Phe Gly Glu Gly Val
                500                 505                 510
Gly Asn Ile Ala Asn Phe Glu Leu Asp Gln Pro Tyr Leu Pro Gly Gln
        515                 520                 525
Thr Phe Lys Tyr Thr Ile Ala Ser Lys Asp Tyr Pro Glu Val Ser Tyr
    530                 535                 540
Asp Gly Thr Phe Thr Val Pro Thr Ser Leu Ala Tyr Lys Met Ala Ser
545                 550                 555                 560
Gln Thr Ile Phe Tyr Pro Phe His Ala Gly Asp Thr Tyr Leu Arg Val
                565                 570                 575
Asn Pro Gln Phe Ala Val Pro Lys Gly Thr Asp Ala Leu Val Arg Val
            580                 585                 590
Phe Asp Glu Phe His Gly Asn Ala Tyr Leu Glu Asn Asn Tyr Lys Val
        595                 600                 605
Gly Glu Ile Lys Leu Pro Ile Pro Lys Leu Asn Gln Gly Thr Thr Arg
    610                 615                 620
Thr Ala Gly Asn Lys Ile Pro Val Thr Phe Met Ala Asn Ala Tyr Leu
625                 630                 635                 640
Asp Asn Gln Ser Thr Tyr Ile Val Glu Val Pro Ile Leu Glu Lys Glu
                645                 650                 655
Asn Gln Thr Asp Lys Pro Ser Ile Leu Pro Gln Phe Lys Arg Asn Lys
            660                 665                 670
Ala Gln Glu Asn Ser Lys Leu Asp Glu Lys Val Glu Glu Pro Lys Thr
        675                 680                 685
Ser Glu Lys Val Glu Lys Glu Lys Leu Ser Glu Thr Gly Asn Ser Thr
    690                 695                 700
Ser Asn Ser Thr Leu Glu Glu Val Pro Thr Val Asp Pro Val Gln Glu
705                 710                 715                 720
Lys Val Ala Lys Phe Ala Glu Ser Tyr Gly Met Lys Leu Glu Asn Val
                725                 730                 735
Leu Phe Asn Met Asp Gly Thr Ile Glu Leu Tyr Leu Pro Ser Gly Glu
            740                 745                 750
Val Ile Lys Lys Asn Met Ala Asp Phe Thr Gly Glu Ala Pro Gln Gly
        755                 760                 765
Asn Gly Glu Asn Lys Pro Ser Glu Asn Gly Lys Val Ser Thr Gly Thr
    770                 775                 780
Val Glu Asn Gln Pro Thr Glu Asn Lys Pro Ala Asp Ser Leu Pro Glu
785                 790                 795                 800
Ala Pro Asn Glu Lys Pro Val Lys Pro Glu Asn Ser Thr Asp Asn Gly
                805                 810                 815
Met Leu Asn Pro Glu Gly Asn Val Gly Ser Asp Pro Met Leu Asp Pro
            820                 825                 830
Ala Leu Glu Glu Ala Pro Ala Val Asp Pro Val Gln Glu Lys Leu Glu
        835                 840                 845
Lys Phe Thr Ala Ser Tyr Gly Leu Gly Leu Asp Ser Val Ile Phe Asn
    850                 855                 860
Met Asp Gly Thr Ile Glu Leu Arg Leu Pro Ser Gly Glu Val Ile Lys
865                 870                 875                 880
Lys Asn Leu Ser Asp Phe Ile Ala
                885

<210> 339

<211> 900
<212> PRT
<213> Unknown

<220>
<223> VP 114

<400> 339
Met Gln Ile Thr Tyr Thr Asp Asp Glu Ile Gln Val Ala Lys Leu Ala
 1               5                  10                  15
Gly Lys Tyr Thr Thr Glu Asp Gly Tyr Ile Phe Asp Thr Ser Trp Ile
                20                  25                  30
Lys Lys Asp Ser Leu Ser Glu Ala Glu Arg Ala Ala Ala Gln Ala Tyr
            35                  40                  45
Ala Lys Glu Lys Gly Leu Thr Pro Pro Ser Thr Asp His Gln Asp Ser

50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp Ser
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Gly Pro Gln Ile Gly Gln Pro Thr Leu Pro Asn Asn Ser Leu Ala Thr
        275                 280                 285
Pro Ser Pro Ser Leu Pro Ile Asn Pro Gly Thr Ser His Glu Lys His
    290                 295                 300
Glu Glu Asp Gly Tyr Gly Phe Asp Ala Asn Arg Ile Ile Ala Glu Asp
305                 310                 315                 320
Glu Ser Gly Phe Val Met Ser Tyr Phe Phe Lys Lys Asp Leu Thr Glu
                325                 330                 335
Glu Gln Ile Lys Ala Ala Gln Lys His Leu Glu Glu Val Lys Thr Ser
            340                 345                 350
His Asn Gly Leu Asp Ser Leu Ser Ser His Glu Gln Asp Tyr Pro Gly
        355                 360                 365
Asn Ala Lys Glu Met Lys Asp Leu Asp Lys Lys Ile Glu Glu Lys Ile
    370                 375                 380
Ala Gly Ile Met Lys Gln Tyr Gly Val Lys Arg Glu Ser Ile Val Val
385                 390                 395                 400
Asn Lys Glu Lys Asn Ala Ile Ile Tyr Pro His Gly Asp His His His
                405                 410                 415
Ala Asp Pro Ile Asp Glu His Lys Pro Val Gly Ile Gly His Ser His
            420                 425                 430
Ser Asn Tyr Glu Leu Phe Lys Pro Glu Glu Gly Val Ala Lys Lys Glu
        435                 440                 445
Gly Asn Lys Val Tyr Thr Gly Glu Glu Leu Thr Asn Val Val Asn Leu
    450                 455                 460
Leu Lys Asn Ser Thr Phe Asn Asn Gln Asn Phe Thr Leu Ala Asn Gly
465                 470                 475                 480
Gln Lys Arg Val Ser Phe Ser Phe Pro Pro Glu Leu Glu Lys Lys Leu
                485                 490                 495
Gly Ile Asn Met Leu Val Lys Leu Ile Thr Pro Asp Gly Lys Val Leu
            500                 505                 510
Glu Lys Val Ser Gly Lys Val Phe Gly Glu Gly Val Gly Asn Ile Ala
        515                 520                 525
Asn Phe Glu Leu Asp Gln Pro Tyr Leu Pro Gly Gln Thr Phe Lys Tyr
    530                 535                 540
Thr Ile Ala Ser Lys Asp Tyr Pro Glu Val Ser Tyr Asp Gly Thr Phe
545                 550                 555                 560
Thr Val Pro Thr Ser Leu Ala Tyr Lys Met Ala Ser Gln Thr Ile Phe
                565                 570                 575
Tyr Pro Phe His Ala Gly Asp Thr Tyr Leu Arg Val Asn Pro Gln Phe
            580                 585                 590
Ala Val Pro Lys Gly Thr Asp Ala Leu Val Arg Val Phe Asp Glu Phe

595                 600                 605
His Gly Asn Ala Tyr Leu Glu Asn Asn Tyr Lys Val Gly Glu Ile Lys
    610                 615                 620
Leu Pro Ile Pro Lys Leu Asn Gln Gly Thr Thr Arg Thr Ala Gly Asn
625                 630                 635                 640
Lys Ile Pro Val Thr Phe Met Ala Asn Ala Tyr Leu Asp Asn Gln Ser
                645                 650                 655
Thr Tyr Ile Val Glu Val Pro Ile Leu Glu Lys Glu Asn Gln Thr Asp
            660                 665                 670
Lys Pro Ser Ile Leu Pro Gln Phe Lys Arg Asn Lys Ala Gln Glu Asn
        675                 680                 685
Ser Lys Leu Asp Glu Lys Val Glu Glu Pro Lys Thr Ser Glu Lys Val
    690                 695                 700
Glu Lys Glu Lys Leu Ser Glu Thr Gly Asn Ser Thr Ser Asn Ser Thr
705                 710                 715                 720
Leu Glu Glu Val Pro Thr Val Asp Pro Val Gln Glu Lys Val Ala Lys
                725                 730                 735
Phe Ala Glu Ser Tyr Gly Met Lys Leu Glu Asn Val Leu Phe Asn Met
            740                 745                 750
Asp Gly Thr Ile Glu Leu Tyr Leu Pro Ser Gly Glu Val Ile Lys Lys
        755                 760                 765
Asn Met Ala Asp Phe Thr Gly Glu Ala Pro Gln Gly Asn Gly Glu Asn
    770                 775                 780
Lys Pro Ser Glu Asn Gly Lys Val Ser Thr Gly Thr Val Glu Asn Gln
785                 790                 795                 800
Pro Thr Glu Asn Lys Pro Ala Asp Ser Leu Pro Glu Ala Pro Asn Glu
                805                 810                 815
Lys Pro Val Lys Pro Glu Asn Ser Thr Asp Asn Gly Met Leu Asn Pro
            820                 825                 830
Glu Gly Asn Val Gly Ser Asp Pro Met Leu Asp Pro Ala Leu Glu Glu
        835                 840                 845
Ala Pro Ala Val Asp Pro Val Gln Glu Lys Leu Glu Lys Phe Thr Ala
    850                 855                 860
Ser Tyr Gly Leu Gly Leu Asp Ser Val Ile Phe Asn Met Asp Gly Thr
865                 870                 875                 880
Ile Glu Leu Arg Leu Pro Ser Gly Glu Val Ile Lys Lys Asn Leu Ser
                885                 890                 895
Asp Phe Ile Ala
            900

<210> 340

<211> 894
<212> PRT
<213> Unknown

<220>
<223> VP 115

<400> 340
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp Ser
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140

Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Gly Pro Gln Ile Gly Gln Pro Thr Leu Pro Asn Asn Ser Leu Ala Thr
        275                 280                 285
Pro Ser Pro Ser Leu Pro Ile Asn Pro Gly Thr Ser His Glu Lys His
    290                 295                 300
Glu Glu Asp Gly Tyr Gly Phe Asp Ala Asn Arg Ile Ile Ala Glu Asp
305                 310                 315                 320
Glu Ser Gly Phe Val Met Ser Tyr Phe Phe Lys Lys Asp Leu Thr Glu
                325                 330                 335
Glu Gln Ile Lys Ala Ala Gln Lys His Leu Glu Glu Val Lys Thr Ser
            340                 345                 350
His Asn Gly Leu Asp Ser Leu Ser Ser His Glu Gln Asp Tyr Pro Gly
        355                 360                 365
Asn Ala Lys Glu Met Lys Asp Leu Asp Lys Lys Ile Glu Glu Lys Ile
    370                 375                 380
Ala Gly Ile Met Lys Gln Tyr Gly Val Lys Arg Glu Ser Ile Val Val
385                 390                 395                 400
Asn Lys Glu Lys Asn Ala Ile Ile Tyr Pro Ala Asp Pro Ile Asp Glu
                405                 410                 415
His Lys Pro Val Gly Ile Gly His Ser His Ser Asn Tyr Glu Leu Phe
            420                 425                 430
Lys Pro Glu Glu Gly Val Ala Lys Lys Glu Gly Asn Lys Val Tyr Thr
        435                 440                 445
Gly Glu Glu Leu Thr Asn Val Val Asn Leu Leu Lys Asn Ser Thr Phe
    450                 455                 460
Asn Asn Gln Asn Phe Thr Leu Ala Asn Gly Gln Lys Arg Val Ser Phe
465                 470                 475                 480
Ser Phe Pro Pro Glu Leu Glu Lys Lys Leu Gly Ile Asn Met Leu Val
                485                 490                 495
Lys Leu Ile Thr Pro Asp Gly Lys Val Leu Glu Lys Val Ser Gly Lys
            500                 505                 510
Val Phe Gly Glu Gly Val Gly Asn Ile Ala Asn Phe Glu Leu Asp Gln
        515                 520                 525
Pro Tyr Leu Pro Gly Gln Thr Phe Lys Tyr Thr Ile Ala Ser Lys Asp
    530                 535                 540
Tyr Pro Glu Val Ser Tyr Asp Gly Thr Phe Thr Val Pro Thr Ser Leu
545                 550                 555                 560
Ala Tyr Lys Met Ala Ser Gln Thr Ile Phe Tyr Pro Phe His Ala Gly
                565                 570                 575
Asp Thr Tyr Leu Arg Val Asn Pro Gln Phe Ala Val Pro Lys Gly Thr
            580                 585                 590
Asp Ala Leu Val Arg Val Phe Asp Glu Phe His Gly Asn Ala Tyr Leu
        595                 600                 605
Glu Asn Asn Tyr Lys Val Gly Glu Ile Lys Leu Pro Ile Pro Lys Leu
    610                 615                 620
Asn Gln Gly Thr Thr Arg Thr Ala Gly Asn Lys Ile Pro Val Thr Phe
625                 630                 635                 640
Met Ala Asn Ala Tyr Leu Asp Asn Gln Ser Thr Tyr Ile Val Glu Val
                645                 650                 655
Pro Ile Leu Glu Lys Glu Asn Gln Thr Asp Lys Pro Ser Ile Leu Pro
            660                 665                 670
Gln Phe Lys Arg Asn Lys Ala Gln Glu Asn Ser Lys Leu Asp Glu Lys
        675                 680                 685

Val Glu Glu Pro Lys Thr Ser Glu Lys Val Glu Lys Glu Lys Leu Ser
    690                 695                 700
Glu Thr Gly Asn Ser Thr Ser Asn Ser Thr Leu Glu Glu Val Pro Thr
705                 710                 715                 720
Val Asp Pro Val Gln Glu Lys Val Ala Lys Phe Ala Glu Ser Tyr Gly
                725                 730                 735
Met Lys Leu Glu Asn Val Leu Phe Asn Met Asp Gly Thr Ile Glu Leu
            740                 745                 750
Tyr Leu Pro Ser Gly Glu Val Ile Lys Lys Asn Met Ala Asp Phe Thr
        755                 760                 765
Gly Glu Ala Pro Gln Gly Asn Gly Glu Asn Lys Pro Ser Glu Asn Gly
    770                 775                 780
Lys Val Ser Thr Gly Thr Val Glu Asn Gln Pro Thr Glu Asn Lys Pro
785                 790                 795                 800
Ala Asp Ser Leu Pro Glu Ala Pro Asn Glu Lys Pro Val Lys Pro Glu
                805                 810                 815
Asn Ser Thr Asp Asn Gly Met Leu Asn Pro Glu Gly Asn Val Gly Ser
            820                 825                 830
Asp Pro Met Leu Asp Pro Ala Leu Glu Glu Ala Pro Ala Val Asp Pro
        835                 840                 845
Val Gln Glu Lys Leu Glu Lys Phe Thr Ala Ser Tyr Gly Leu Gly Leu
    850                 855                 860
Asp Ser Val Ile Phe Asn Met Asp Gly Thr Ile Glu Leu Arg Leu Pro
865                 870                 875                 880
Ser Gly Glu Val Ile Lys Lys Asn Leu Ser Asp Phe Ile Ala
                885                 890

<210> 341

<211> 900
<212> PRT
<213> Unknown

<220>
<223> VP 116

<400> 341
Met Gln Ile Thr Tyr Thr Asp Asp Glu Ile Gln Val Ala Lys Leu Ala
 1               5                  10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp His
            100                 105                 110
Tyr Ser Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val

                245                 250                 255
Asp Ser Leu Leu Ala Leu Leu Lys Glu Ser Gln Pro Ala Pro Ile Gln
            260                 265                 270
Gly Pro Gln Ile Gly Gln Pro Thr Leu Pro Asn Asn Ser Leu Ala Thr
        275                 280                 285
Pro Ser Pro Ser Leu Pro Ile Asn Pro Gly Thr Ser His Glu Lys His
    290                 295                 300
Glu Glu Asp Gly Tyr Gly Phe Asp Ala Asn Arg Ile Ile Ala Glu Asp
305                 310                 315                 320
Glu Ser Gly Phe Val Met Ser Tyr Phe Phe Lys Lys Asp Leu Thr Glu
                325                 330                 335
Glu Gln Ile Lys Ala Ala Gln Lys His Leu Glu Glu Val Lys Thr Ser
            340                 345                 350
His Asn Gly Leu Asp Ser Leu Ser Ser His Glu Gln Asp Tyr Pro Gly
        355                 360                 365
Asn Ala Lys Glu Met Lys Asp Leu Asp Lys Lys Ile Glu Glu Lys Ile
    370                 375                 380
Ala Gly Ile Met Lys Gln Tyr Gly Val Lys Arg Glu Ser Ile Val Val
385                 390                 395                 400
Asn Lys Glu Lys Asn Ala Ile Ile Tyr Pro His Gly Asp His His His
                405                 410                 415
Ala Asp Pro Ile Asp Glu His Lys Pro Val Gly Ile Gly His Ser His
            420                 425                 430
Ser Asn Tyr Glu Leu Phe Lys Pro Glu Glu Gly Val Ala Lys Lys Glu
        435                 440                 445
Gly Asn Lys Val Tyr Thr Gly Glu Glu Leu Thr Asn Val Val Asn Leu
    450                 455                 460
Leu Lys Asn Ser Thr Phe Asn Asn Gln Asn Phe Thr Leu Ala Asn Gly
465                 470                 475                 480
Gln Lys Arg Val Ser Phe Ser Phe Pro Pro Glu Leu Glu Lys Lys Leu
                485                 490                 495
Gly Ile Asn Met Leu Val Lys Leu Ile Thr Pro Asp Gly Lys Val Leu
            500                 505                 510
Glu Lys Val Ser Gly Lys Val Phe Gly Glu Gly Val Gly Asn Ile Ala
        515                 520                 525
Asn Phe Glu Leu Asp Gln Pro Tyr Leu Pro Gly Gln Thr Phe Lys Tyr
    530                 535                 540
Thr Ile Ala Ser Lys Asp Tyr Pro Glu Val Ser Tyr Asp Gly Thr Phe
545                 550                 555                 560
Thr Val Pro Thr Ser Leu Ala Tyr Lys Met Ala Ser Gln Thr Ile Phe
                565                 570                 575
Tyr Pro Phe His Ala Gly Asp Thr Tyr Leu Arg Val Asn Pro Gln Phe
            580                 585                 590
Ala Val Pro Lys Gly Thr Asp Ala Leu Val Arg Val Phe Asp Glu Phe
        595                 600                 605
His Gly Asn Ala Tyr Leu Glu Asn Asn Tyr Lys Val Gly Glu Ile Lys
    610                 615                 620
Leu Pro Ile Pro Lys Leu Asn Gln Gly Thr Thr Arg Thr Ala Gly Asn
625                 630                 635                 640
Lys Ile Pro Val Thr Phe Met Ala Asn Ala Tyr Leu Asp Asn Gln Ser
                645                 650                 655
Thr Tyr Ile Val Glu Val Pro Ile Leu Glu Lys Glu Asn Gln Thr Asp
            660                 665                 670
Lys Pro Ser Ile Leu Pro Gln Phe Lys Arg Asn Lys Ala Gln Glu Asn
        675                 680                 685
Ser Lys Leu Asp Glu Lys Val Glu Glu Pro Lys Thr Ser Glu Lys Val
    690                 695                 700
Glu Lys Glu Lys Leu Ser Glu Thr Gly Asn Ser Thr Ser Asn Ser Thr
705                 710                 715                 720
Leu Glu Glu Val Pro Thr Val Asp Pro Val Gln Glu Lys Val Ala Lys
                725                 730                 735
Phe Ala Glu Ser Tyr Gly Met Lys Leu Glu Asn Val Leu Phe Asn Met
            740                 745                 750
Asp Gly Thr Ile Glu Leu Tyr Leu Pro Ser Gly Glu Val Ile Lys Lys
        755                 760                 765
Asn Met Ala Asp Phe Thr Gly Glu Ala Pro Gln Gly Asn Gly Glu Asn
    770                 775                 780
Lys Pro Ser Glu Asn Gly Lys Val Ser Thr Gly Thr Val Glu Asn Gln

785                 790                 795                 800
Pro Thr Glu Asn Lys Pro Ala Asp Ser Leu Pro Glu Ala Pro Asn Glu
                805                 810                 815
Lys Pro Val Lys Pro Glu Asn Ser Thr Asp Asn Gly Met Leu Asn Pro
            820                 825                 830
Glu Gly Asn Val Gly Ser Asp Pro Met Leu Asp Pro Ala Leu Glu Glu
        835                 840                 845
Ala Pro Ala Val Asp Pro Val Gln Glu Lys Leu Glu Lys Phe Thr Ala
    850                 855                 860
Ser Tyr Gly Leu Gly Leu Asp Ser Val Ile Phe Asn Met Asp Gly Thr
865                 870                 875                 880
Ile Glu Leu Arg Leu Pro Ser Gly Glu Val Ile Lys Lys Asn Leu Ser
                885                 890                 895
Asp Phe Ile Ala
            900

<210> 342

<211> 894
<212> PRT
<213> Unknown

<220>
<223> VP 117

<400> 342
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp His
            100                 105                 110
Tyr Ser Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Gly Pro Gln Ile Gly Gln Pro Thr Leu Pro Asn Asn Ser Leu Ala Thr
        275                 280                 285
Pro Ser Pro Ser Leu Pro Ile Asn Pro Gly Thr Ser His Glu Lys His
    290                 295                 300
Glu Glu Asp Gly Tyr Gly Phe Asp Ala Asn Arg Ile Ile Ala Glu Asp
305                 310                 315                 320

Glu Ser Gly Phe Val Met Ser Tyr Phe Phe Lys Lys Asp Leu Thr Glu
                325                 330                 335
Glu Gln Ile Lys Ala Ala Gln Lys His Leu Glu Glu Val Lys Thr Ser
            340                 345                 350
His Asn Gly Leu Asp Ser Leu Ser Ser His Glu Gln Asp Tyr Pro Gly
        355                 360                 365
Asn Ala Lys Glu Met Lys Asp Leu Asp Lys Lys Ile Glu Glu Lys Ile
    370                 375                 380
Ala Gly Ile Met Lys Gln Tyr Gly Val Lys Arg Glu Ser Ile Val Val
385                 390                 395                 400
Asn Lys Glu Lys Asn Ala Ile Ile Tyr Pro Ala Asp Pro Ile Asp Glu
                405                 410                 415
His Lys Pro Val Gly Ile Gly His Ser His Ser Asn Tyr Glu Leu Phe
            420                 425                 430
Lys Pro Glu Glu Gly Val Ala Lys Lys Glu Gly Asn Lys Val Tyr Thr
        435                 440                 445
Gly Glu Glu Leu Thr Asn Val Val Asn Leu Leu Lys Asn Ser Thr Phe
    450                 455                 460
Asn Asn Gln Asn Phe Thr Leu Ala Asn Gly Gln Lys Arg Val Ser Phe
465                 470                 475                 480
Ser Phe Pro Pro Glu Leu Glu Lys Lys Leu Gly Ile Asn Met Leu Val
                485                 490                 495
Lys Leu Ile Thr Pro Asp Gly Lys Val Leu Glu Lys Val Ser Gly Lys
            500                 505                 510
Val Phe Gly Glu Gly Val Gly Asn Ile Ala Asn Phe Glu Leu Asp Gln
        515                 520                 525
Pro Tyr Leu Pro Gly Gln Thr Phe Lys Tyr Thr Ile Ala Ser Lys Asp
    530                 535                 540
Tyr Pro Glu Val Ser Tyr Asp Gly Thr Phe Thr Val Pro Thr Ser Leu
545                 550                 555                 560
Ala Tyr Lys Met Ala Ser Gln Thr Ile Phe Tyr Pro Phe His Ala Gly
                565                 570                 575
Asp Thr Tyr Leu Arg Val Asn Pro Gln Phe Ala Val Pro Lys Gly Thr
            580                 585                 590
Asp Ala Leu Val Arg Val Phe Asp Glu Phe His Gly Asn Ala Tyr Leu
        595                 600                 605
Glu Asn Asn Tyr Lys Val Gly Glu Ile Lys Leu Pro Ile Pro Lys Leu
    610                 615                 620
Asn Gln Gly Thr Thr Arg Thr Ala Gly Asn Lys Ile Pro Val Thr Phe
625                 630                 635                 640
Met Ala Asn Ala Tyr Leu Asp Asn Gln Ser Thr Tyr Ile Val Glu Val
                645                 650                 655
Pro Ile Leu Glu Lys Glu Asn Gln Thr Asp Lys Pro Ser Ile Leu Pro
            660                 665                 670
Gln Phe Lys Arg Asn Lys Ala Gln Glu Asn Ser Lys Leu Asp Glu Lys
        675                 680                 685
Val Glu Glu Pro Lys Thr Ser Glu Lys Val Glu Lys Glu Lys Leu Ser
    690                 695                 700
Glu Thr Gly Asn Ser Thr Ser Asn Ser Thr Leu Glu Glu Val Pro Thr
705                 710                 715                 720
Val Asp Pro Val Gln Glu Lys Val Ala Lys Phe Ala Glu Ser Tyr Gly
                725                 730                 735
Met Lys Leu Glu Asn Val Leu Phe Asn Met Asp Gly Thr Ile Glu Leu
            740                 745                 750
Tyr Leu Pro Ser Gly Glu Val Ile Lys Lys Asn Met Ala Asp Phe Thr
        755                 760                 765
Gly Glu Ala Pro Gln Gly Asn Gly Glu Asn Lys Pro Ser Glu Asn Gly
    770                 775                 780
Lys Val Ser Thr Gly Thr Val Glu Asn Gln Pro Thr Glu Asn Lys Pro
785                 790                 795                 800
Ala Asp Ser Leu Pro Glu Ala Pro Asn Glu Lys Pro Val Lys Pro Glu
                805                 810                 815
Asn Ser Thr Asp Asn Gly Met Leu Asn Pro Glu Gly Asn Val Gly Ser
            820                 825                 830
Asp Pro Met Leu Asp Pro Ala Leu Glu Glu Ala Pro Ala Val Asp Pro
        835                 840                 845
Val Gln Glu Lys Leu Glu Lys Phe Thr Ala Ser Tyr Gly Leu Gly Leu
    850                 855                 860

Asp Ser Val Ile Phe Asn Met Asp Gly Thr Ile Glu Leu Arg Leu Pro
865                 870                 875                 880
Ser Gly Glu Val Ile Lys Lys Asn Leu Ser Asp Phe Ile Ala
                885                 890

<210> 343

<211> 901
<212> PRT
<213> Unknown

<220>
<223> VP 119

<400> 343

Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro His Tyr Asp His
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Ser Gly Pro Gln Ile Gly Gln Pro Thr Leu Pro Asn Asn Ser Leu Ala
        275                 280                 285
Thr Pro Ser Pro Ser Leu Pro Ile Asn Pro Gly Thr Ser His Glu Lys
    290                 295                 300
His Glu Glu Asp Gly Tyr Gly Phe Asp Ala Asn Arg Ile Ile Ala Glu
305                 310                 315                 320
Asp Glu Ser Gly Phe Val Met Ser Tyr Phe Phe Lys Lys Asp Leu Thr
                325                 330                 335
Glu Glu Gln Ile Lys Ala Ala Gln Lys His Leu Glu Glu Val Lys Thr
            340                 345                 350
Ser His Asn Gly Leu Asp Ser Leu Ser Ser His Glu Gln Asp Tyr Pro
        355                 360                 365
Gly Asn Ala Lys Glu Met Lys Asp Leu Asp Lys Lys Ile Glu Glu Lys
    370                 375                 380
Ile Ala Gly Ile Met Lys Gln Tyr Gly Val Lys Arg Glu Ser Ile Val
385                 390                 395                 400
Val Asn Lys Glu Lys Asn Ala Ile Ile Tyr Pro His Gly Asp His His
                405                 410                 415

His Ala Asp Pro Ile Asp Glu His Lys Pro Val Gly Ile Gly His Ser
            420                 425                 430
His Ser Asn Tyr Glu Leu Phe Lys Pro Glu Glu Gly Val Ala Lys Lys
        435                 440                 445
Glu Gly Asn Lys Val Tyr Thr Gly Glu Glu Leu Thr Asn Val Val Asn
    450                 455                 460
Leu Leu Lys Asn Ser Thr Phe Asn Asn Gln Asn Phe Thr Leu Ala Asn
465                 470                 475                 480
Gly Gln Lys Arg Val Ser Phe Ser Phe Pro Pro Glu Leu Glu Lys Lys
                485                 490                 495
Leu Gly Ile Asn Met Leu Val Lys Leu Ile Thr Pro Asp Gly Lys Val
            500                 505                 510
Leu Glu Lys Val Ser Gly Lys Val Phe Gly Glu Gly Val Gly Asn Ile
        515                 520                 525
Ala Asn Phe Glu Leu Asp Gln Pro Tyr Leu Pro Gly Gln Thr Phe Lys
    530                 535                 540
Tyr Thr Ile Ala Ser Lys Asp Tyr Pro Glu Val Ser Tyr Asp Gly Thr
545                 550                 555                 560
Phe Thr Val Pro Thr Ser Leu Ala Tyr Lys Met Ala Ser Gln Thr Ile
                565                 570                 575
Phe Tyr Pro Phe His Ala Gly Asp Thr Tyr Leu Arg Val Asn Pro Gln
            580                 585                 590
Phe Ala Val Pro Lys Gly Thr Asp Ala Leu Val Arg Val Phe Asp Glu
        595                 600                 605
Phe His Gly Asn Ala Tyr Leu Glu Asn Asn Tyr Lys Val Gly Glu Ile
    610                 615                 620
Lys Leu Pro Ile Pro Lys Leu Asn Gln Gly Thr Thr Arg Thr Ala Gly
625                 630                 635                 640
Asn Lys Ile Pro Val Thr Phe Met Ala Asn Ala Tyr Leu Asp Asn Gln
                645                 650                 655
Ser Thr Tyr Ile Val Glu Val Pro Ile Leu Glu Lys Glu Asn Gln Thr
            660                 665                 670
Asp Lys Pro Ser Ile Leu Pro Gln Phe Lys Arg Asn Lys Ala Gln Glu
        675                 680                 685
Asn Ser Lys Leu Asp Glu Lys Val Glu Glu Pro Lys Thr Ser Glu Lys
    690                 695                 700
Val Glu Lys Glu Lys Leu Ser Glu Thr Gly Asn Ser Thr Ser Asn Ser
705                 710                 715                 720
Thr Leu Glu Glu Val Pro Thr Val Asp Pro Val Gln Glu Lys Val Ala
                725                 730                 735
Lys Phe Ala Glu Ser Tyr Gly Met Lys Leu Glu Asn Val Leu Phe Asn
            740                 745                 750
Met Asp Gly Thr Ile Glu Leu Tyr Leu Pro Ser Gly Glu Val Ile Lys
        755                 760                 765
Lys Asn Met Ala Asp Phe Thr Gly Glu Ala Pro Gln Gly Asn Gly Glu
    770                 775                 780
Asn Lys Pro Ser Glu Asn Gly Lys Val Ser Thr Gly Thr Val Glu Asn
785                 790                 795                 800
Gln Pro Thr Glu Asn Lys Pro Ala Asp Ser Leu Pro Glu Ala Pro Asn
                805                 810                 815
Glu Lys Pro Val Lys Pro Glu Asn Ser Thr Asp Asn Gly Met Leu Asn
            820                 825                 830
Pro Glu Gly Asn Val Gly Ser Asp Pro Met Leu Asp Pro Ala Leu Glu
        835                 840                 845
Glu Ala Pro Ala Val Asp Pro Val Gln Glu Lys Leu Glu Lys Phe Thr
    850                 855                 860
Ala Ser Tyr Gly Leu Gly Leu Asp Ser Val Ile Phe Asn Met Asp Gly
865                 870                 875                 880
Thr Ile Glu Leu Arg Leu Pro Ser Gly Glu Val Ile Lys Lys Asn Leu
                885                 890                 895
Ser Asp Phe Ile Ala
            900

<210> 344

<211> 895
<212> PRT
<213> Unknown

<220>
<223> VP 120

<400> 344
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro His Tyr Asp His
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Ser Gly Pro Gln Ile Gly Gln Pro Thr Leu Pro Asn Asn Ser Leu Ala
        275                 280                 285
Thr Pro Ser Pro Ser Leu Pro Ile Asn Pro Gly Thr Ser His Glu Lys
    290                 295                 300
His Glu Glu Asp Gly Tyr Gly Phe Asp Ala Asn Arg Ile Ile Ala Glu
305                 310                 315                 320
Asp Glu Ser Gly Phe Val Met Ser Tyr Phe Phe Lys Lys Asp Leu Thr
                325                 330                 335
Glu Glu Gln Ile Lys Ala Ala Gln Lys His Leu Glu Glu Val Lys Thr
            340                 345                 350
Ser His Asn Gly Leu Asp Ser Leu Ser Ser His Glu Gln Asp Tyr Pro
        355                 360                 365
Gly Asn Ala Lys Glu Met Lys Asp Leu Asp Lys Lys Ile Glu Glu Lys
    370                 375                 380
Ile Ala Gly Ile Met Lys Gln Tyr Gly Val Lys Arg Glu Ser Ile Val
385                 390                 395                 400
Val Asn Lys Glu Lys Asn Ala Ile Ile Tyr Pro Ala Asp Pro Ile Asp
                405                 410                 415
Glu His Lys Pro Val Gly Ile Gly His Ser His Ser Asn Tyr Glu Leu
            420                 425                 430
Phe Lys Pro Glu Glu Gly Val Ala Lys Lys Glu Gly Asn Lys Val Tyr
        435                 440                 445
Thr Gly Glu Glu Leu Thr Asn Val Val Asn Leu Leu Lys Asn Ser Thr
    450                 455                 460
Phe Asn Asn Gln Asn Phe Thr Leu Ala Asn Gly Gln Lys Arg Val Ser
465                 470                 475                 480
Phe Ser Phe Pro Pro Glu Leu Glu Lys Lys Leu Gly Ile Asn Met Leu
                485                 490                 495
Val Lys Leu Ile Thr Pro Asp Gly Lys Val Leu Glu Lys Val Ser Gly

                500                 505                 510
Lys Val Phe Gly Glu Gly Val Gly Asn Ile Ala Asn Phe Glu Leu Asp
            515                 520                 525
Gln Pro Tyr Leu Pro Gly Gln Thr Phe Lys Tyr Thr Ile Ala Ser Lys
        530                 535                 540
Asp Tyr Pro Glu Val Ser Tyr Asp Gly Thr Phe Thr Val Pro Thr Ser
545                 550                 555                 560
Leu Ala Tyr Lys Met Ala Ser Gln Thr Ile Phe Tyr Pro Phe His Ala
                565                 570                 575
Gly Asp Thr Tyr Leu Arg Val Asn Pro Gln Phe Ala Val Pro Lys Gly
            580                 585                 590
Thr Asp Ala Leu Val Arg Val Phe Asp Glu Phe His Gly Asn Ala Tyr
        595                 600                 605
Leu Glu Asn Asn Tyr Lys Val Gly Glu Ile Lys Leu Pro Ile Pro Lys
    610                 615                 620
Leu Asn Gln Gly Thr Thr Arg Thr Ala Gly Asn Lys Ile Pro Val Thr
625                 630                 635                 640
Phe Met Ala Asn Ala Tyr Leu Asp Asn Gln Ser Thr Tyr Ile Val Glu
                645                 650                 655
Val Pro Ile Leu Glu Lys Glu Asn Gln Thr Asp Lys Pro Ser Ile Leu
            660                 665                 670
Pro Gln Phe Lys Arg Asn Lys Ala Gln Glu Asn Ser Lys Leu Asp Glu
        675                 680                 685
Lys Val Glu Glu Pro Lys Thr Ser Glu Lys Val Glu Lys Glu Lys Leu
    690                 695                 700
Ser Glu Thr Gly Asn Ser Thr Ser Asn Ser Thr Leu Glu Glu Val Pro
705                 710                 715                 720
Thr Val Asp Pro Val Gln Glu Lys Val Ala Lys Phe Ala Glu Ser Tyr
                725                 730                 735
Gly Met Lys Leu Glu Asn Val Leu Phe Asn Met Asp Gly Thr Ile Glu
            740                 745                 750
Leu Tyr Leu Pro Ser Gly Glu Val Ile Lys Lys Asn Met Ala Asp Phe
        755                 760                 765
Thr Gly Glu Ala Pro Gln Gly Asn Gly Glu Asn Lys Pro Ser Glu Asn
    770                 775                 780
Gly Lys Val Ser Thr Gly Thr Val Glu Asn Gln Pro Thr Glu Asn Lys
785                 790                 795                 800
Pro Ala Asp Ser Leu Pro Glu Ala Pro Asn Glu Lys Pro Val Lys Pro
                805                 810                 815
Glu Asn Ser Thr Asp Asn Gly Met Leu Asn Pro Glu Gly Asn Val Gly
            820                 825                 830
Ser Asp Pro Met Leu Asp Pro Ala Leu Glu Glu Ala Pro Ala Val Asp
        835                 840                 845
Pro Val Gln Glu Lys Leu Glu Lys Phe Thr Ala Ser Tyr Gly Leu Gly
    850                 855                 860
Leu Asp Ser Val Ile Phe Asn Met Asp Gly Thr Ile Glu Leu Arg Leu
865                 870                 875                 880
Pro Ser Gly Glu Val Ile Lys Lys Asn Leu Ser Asp Phe Ile Ala
                885                 890                 895

<210> 345

<211> 901
<212> PRT
<213> Unknown

<220>
<223> VP 121

<400> 345
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60

Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp Ser
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Ser Gly Pro Gln Ile Gly Gln Pro Thr Leu Pro Asn Asn Ser Leu Ala
        275                 280                 285
Thr Pro Ser Pro Ser Leu Pro Ile Asn Pro Gly Thr Ser His Glu Lys
    290                 295                 300
His Glu Glu Asp Gly Tyr Gly Phe Asp Ala Asn Arg Ile Ile Ala Glu
305                 310                 315                 320
Asp Glu Ser Gly Phe Val Met Ser Tyr Phe Phe Lys Lys Asp Leu Thr
                325                 330                 335
Glu Glu Gln Ile Lys Ala Ala Gln Lys His Leu Glu Glu Val Lys Thr
            340                 345                 350
Ser His Asn Gly Leu Asp Ser Leu Ser Ser His Glu Gln Asp Tyr Pro
        355                 360                 365
Gly Asn Ala Lys Glu Met Lys Asp Leu Asp Lys Lys Ile Glu Glu Lys
    370                 375                 380
Ile Ala Gly Ile Met Lys Gln Tyr Gly Val Lys Arg Glu Ser Ile Val
385                 390                 395                 400
Val Asn Lys Glu Lys Asn Ala Ile Ile Tyr Pro His Gly Asp His His
                405                 410                 415
His Ala Asp Pro Ile Asp Glu His Lys Pro Val Gly Ile Gly His Ser
            420                 425                 430
His Ser Asn Tyr Glu Leu Phe Lys Pro Glu Glu Gly Val Ala Lys Lys
        435                 440                 445
Glu Gly Asn Lys Val Tyr Thr Gly Glu Glu Leu Thr Asn Val Val Asn
    450                 455                 460
Leu Leu Lys Asn Ser Thr Phe Asn Asn Gln Asn Phe Thr Leu Ala Asn
465                 470                 475                 480
Gly Gln Lys Arg Val Ser Phe Ser Phe Pro Pro Glu Leu Glu Lys Lys
                485                 490                 495
Leu Gly Ile Asn Met Leu Val Lys Leu Ile Thr Pro Asp Gly Lys Val
            500                 505                 510
Leu Glu Lys Val Ser Gly Lys Val Phe Gly Glu Gly Val Gly Asn Ile
        515                 520                 525
Ala Asn Phe Glu Leu Asp Gln Pro Tyr Leu Pro Gly Gln Thr Phe Lys
    530                 535                 540
Tyr Thr Ile Ala Ser Lys Asp Tyr Pro Glu Val Ser Tyr Asp Gly Thr
545                 550                 555                 560
Phe Thr Val Pro Thr Ser Leu Ala Tyr Lys Met Ala Ser Gln Thr Ile
                565                 570                 575
Phe Tyr Pro Phe His Ala Gly Asp Thr Tyr Leu Arg Val Asn Pro Gln
            580                 585                 590
Phe Ala Val Pro Lys Gly Thr Asp Ala Leu Val Arg Val Phe Asp Glu
        595                 600                 605

Phe His Gly Asn Ala Tyr Leu Glu Asn Asn Tyr Lys Val Gly Glu Ile
    610                 615                 620
Lys Leu Pro Ile Pro Lys Leu Asn Gln Gly Thr Thr Arg Thr Ala Gly
625                 630                 635                 640
Asn Lys Ile Pro Val Thr Phe Met Ala Asn Ala Tyr Leu Asp Asn Gln
                645                 650                 655
Ser Thr Tyr Ile Val Glu Val Pro Ile Leu Glu Lys Glu Asn Gln Thr
            660                 665                 670
Asp Lys Pro Ser Ile Leu Pro Gln Phe Lys Arg Asn Lys Ala Gln Glu
        675                 680                 685
Asn Ser Lys Leu Asp Glu Lys Val Glu Glu Pro Lys Thr Ser Glu Lys
    690                 695                 700
Val Glu Lys Glu Lys Leu Ser Glu Thr Gly Asn Ser Thr Ser Asn Ser
705                 710                 715                 720
Thr Leu Glu Glu Val Pro Thr Val Asp Pro Val Gln Glu Lys Val Ala
                725                 730                 735
Lys Phe Ala Glu Ser Tyr Gly Met Lys Leu Glu Asn Val Leu Phe Asn
            740                 745                 750
Met Asp Gly Thr Ile Glu Leu Tyr Leu Pro Ser Gly Glu Val Ile Lys
        755                 760                 765
Lys Asn Met Ala Asp Phe Thr Gly Glu Ala Pro Gln Gly Asn Gly Glu
    770                 775                 780
Asn Lys Pro Ser Glu Asn Gly Lys Val Ser Thr Gly Thr Val Glu Asn
785                 790                 795                 800
Gln Pro Thr Glu Asn Lys Pro Ala Asp Ser Leu Pro Glu Ala Pro Asn
                805                 810                 815
Glu Lys Pro Val Lys Pro Glu Asn Ser Thr Asp Asn Gly Met Leu Asn
            820                 825                 830
Pro Glu Gly Asn Val Gly Ser Asp Pro Met Leu Asp Pro Ala Leu Glu
        835                 840                 845
Glu Ala Pro Ala Val Asp Pro Val Gln Glu Lys Leu Glu Lys Phe Thr
    850                 855                 860
Ala Ser Tyr Gly Leu Gly Leu Asp Ser Val Ile Phe Asn Met Asp Gly
865                 870                 875                 880
Thr Ile Glu Leu Arg Leu Pro Ser Gly Glu Val Ile Lys Lys Asn Leu
                885                 890                 895
Ser Asp Phe Ile Ala
            900

<210> 346

<211> 895
<212> PRT
<213> Unknown

<220>
<223> VP 122

<400> 346
Met Gln Ile Thr Tyr Thr Asp Asp Glu Ile Gln Val Ala Lys Leu Ala
 1               5                  10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Ser Tyr Asp Ser
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp

145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Ser Gly Pro Gln Ile Gly Gln Pro Thr Leu Pro Asn Asn Ser Leu Ala
        275                 280                 285
Thr Pro Ser Pro Ser Leu Pro Ile Asn Pro Gly Thr Ser His Glu Lys
    290                 295                 300
His Glu Glu Asp Gly Tyr Gly Phe Asp Ala Asn Arg Ile Ile Ala Glu
305                 310                 315                 320
Asp Glu Ser Gly Phe Val Met Ser Tyr Phe Phe Lys Lys Asp Leu Thr
                325                 330                 335
Glu Glu Gln Ile Lys Ala Ala Gln Lys His Leu Glu Glu Val Lys Thr
            340                 345                 350
Ser His Asn Gly Leu Asp Ser Leu Ser Ser His Glu Gln Asp Tyr Pro
        355                 360                 365
Gly Asn Ala Lys Glu Met Lys Asp Leu Asp Lys Lys Ile Glu Glu Lys
    370                 375                 380
Ile Ala Gly Ile Met Lys Gln Tyr Gly Val Lys Arg Glu Ser Ile Val
385                 390                 395                 400
Val Asn Lys Glu Lys Asn Ala Ile Ile Tyr Pro Ala Asp Pro Ile Asp
                405                 410                 415
Glu His Lys Pro Val Gly Ile Gly His Ser His Ser Asn Tyr Glu Leu
            420                 425                 430
Phe Lys Pro Glu Glu Gly Val Ala Lys Lys Glu Gly Asn Lys Val Tyr
        435                 440                 445
Thr Gly Glu Glu Leu Thr Asn Val Val Asn Leu Leu Lys Asn Ser Thr
    450                 455                 460
Phe Asn Asn Gln Asn Phe Thr Leu Ala Asn Gly Gln Lys Arg Val Ser
465                 470                 475                 480
Phe Ser Phe Pro Pro Glu Leu Glu Lys Lys Leu Gly Ile Asn Met Leu
                485                 490                 495
Val Lys Leu Ile Thr Pro Asp Gly Lys Val Leu Glu Lys Val Ser Gly
            500                 505                 510
Lys Val Phe Gly Glu Gly Val Gly Asn Ile Ala Asn Phe Glu Leu Asp
        515                 520                 525
Gln Pro Tyr Leu Pro Gly Gln Thr Phe Lys Tyr Thr Ile Ala Ser Lys
    530                 535                 540
Asp Tyr Pro Glu Val Ser Tyr Asp Gly Thr Phe Thr Val Pro Thr Ser
545                 550                 555                 560
Leu Ala Tyr Lys Met Ala Ser Gln Thr Ile Phe Tyr Pro Phe His Ala
                565                 570                 575
Gly Asp Thr Tyr Leu Arg Val Asn Pro Gln Phe Ala Val Pro Lys Gly
            580                 585                 590
Thr Asp Ala Leu Val Arg Val Phe Asp Glu Phe His Gly Asn Ala Tyr
        595                 600                 605
Leu Glu Asn Asn Tyr Lys Val Gly Glu Ile Lys Leu Pro Ile Pro Lys
    610                 615                 620
Leu Asn Gln Gly Thr Thr Arg Thr Ala Gly Asn Lys Ile Pro Val Thr
625                 630                 635                 640
Phe Met Ala Asn Ala Tyr Leu Asp Asn Gln Ser Thr Tyr Ile Val Glu
                645                 650                 655
Val Pro Ile Leu Glu Lys Glu Asn Gln Thr Asp Lys Pro Ser Ile Leu
            660                 665                 670
Pro Gln Phe Lys Arg Asn Lys Ala Gln Glu Asn Ser Lys Leu Asp Glu
        675                 680                 685
Lys Val Glu Glu Pro Lys Thr Ser Glu Lys Val Glu Lys Glu Lys Leu

690                 695                 700
Ser Glu Thr Gly Asn Ser Thr Ser Asn Ser Thr Leu Glu Glu Val Pro
705                 710                 715                 720
Thr Val Asp Pro Val Gln Glu Lys Val Ala Lys Phe Ala Glu Ser Tyr
                725                 730                 735
Gly Met Lys Leu Glu Asn Val Leu Phe Asn Met Asp Gly Thr Ile Glu
            740                 745                 750
Leu Tyr Leu Pro Ser Gly Glu Val Ile Lys Lys Asn Met Ala Asp Phe
        755                 760                 765
Thr Gly Glu Ala Pro Gln Gly Asn Gly Glu Asn Lys Pro Ser Glu Asn
    770                 775                 780
Gly Lys Val Ser Thr Gly Thr Val Glu Asn Gln Pro Thr Glu Asn Lys
785                 790                 795                 800
Pro Ala Asp Ser Leu Pro Glu Ala Pro Asn Glu Lys Pro Val Lys Pro
                805                 810                 815
Glu Asn Ser Thr Asp Asn Gly Met Leu Asn Pro Glu Gly Asn Val Gly
            820                 825                 830
Ser Asp Pro Met Leu Asp Pro Ala Leu Glu Glu Ala Pro Ala Val Asp
        835                 840                 845
Pro Val Gln Glu Lys Leu Glu Lys Phe Thr Ala Ser Tyr Gly Leu Gly
    850                 855                 860
Leu Asp Ser Val Ile Phe Asn Met Asp Gly Thr Ile Glu Leu Arg Leu
865                 870                 875                 880
Pro Ser Gly Glu Val Ile Lys Lys Asn Leu Ser Asp Phe Ile Ala
                885                 890                 895

<210> 347

<211> 895
<212> PRT
<213> Unknown

<220>
<223> VP 123

<400> 347
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Asn Ile Lys Phe
            100                 105                 110
Glu Trp Phe Asp Glu Gly Leu Tyr Glu Ala Pro Lys Gly Tyr Ser Leu
        115                 120                 125
Glu Asp Leu Leu Ala Thr Val Lys Tyr Tyr Val Glu Pro Arg Asn Ala
    130                 135                 140
Ser Asp His Val Arg Lys Asn Lys Ala Asp Gln Asp Ser Lys Pro Asp
145                 150                 155                 160
Glu Asp Lys Glu His Asp Glu Val Ser Glu Pro Thr His Pro Glu Ser
                165                 170                 175
Asp Glu Lys Glu Asn His Ala Gly Leu Asn Pro Ser Ala Asp Asn Leu
            180                 185                 190
Tyr Lys Pro Ser Thr Asp Thr Glu Glu Thr Glu Glu Glu Ala Glu Asp
        195                 200                 205
Thr Thr Asp Glu Ala Glu Ile Pro Gly Thr Pro Ser Ile Arg Gln Asn
    210                 215                 220
Ala Met Glu Thr Leu Thr Gly Leu Lys Ser Ser Leu Leu Leu Gly Thr
225                 230                 235                 240
Lys Asp Asn Asn Thr Ile Ser Ala Glu Val Asp Ser Leu Leu Ala Leu
                245                 250                 255

Leu Lys Glu Ser Gln Pro Ala Pro Ile Gln Ser Gly Pro Gln Ile Gly
            260                 265                 270
Gln Pro Thr Leu Pro Asn Asn Ser Leu Ala Thr Pro Ser Pro Ser Leu
        275                 280                 285
Pro Ile Asn Pro Gly Thr Ser His Glu Lys His Glu Glu Asp Gly Tyr
    290                 295                 300
Gly Phe Asp Ala Asn Arg Ile Ile Ala Glu Asp Glu Ser Gly Phe Val
305                 310                 315                 320
Met Ser Tyr Phe Phe Lys Lys Asp Leu Thr Glu Glu Gln Ile Lys Ala
                325                 330                 335
Ala Gln Lys His Leu Glu Glu Val Lys Thr Ser His Asn Gly Leu Asp
            340                 345                 350
Ser Leu Ser Ser His Glu Gln Asp Tyr Pro Gly Asn Ala Lys Glu Met
        355                 360                 365
Lys Asp Leu Asp Lys Lys Ile Glu Glu Lys Ile Ala Gly Ile Met Lys
    370                 375                 380
Gln Tyr Gly Val Lys Arg Glu Ser Ile Val Val Asn Lys Glu Lys Asn
385                 390                 395                 400
Ala Ile Ile Tyr Pro His Gly Asp His His His Ala Asp Pro Ile Asp
                405                 410                 415
Glu His Lys Pro Val Gly Ile Gly His Ser His Ser Asn Tyr Glu Leu
            420                 425                 430
Phe Lys Pro Glu Glu Gly Val Ala Lys Lys Glu Gly Asn Lys Val Tyr
        435                 440                 445
Thr Gly Glu Glu Leu Thr Asn Val Val Asn Leu Leu Lys Asn Ser Thr
    450                 455                 460
Phe Asn Asn Gln Asn Phe Thr Leu Ala Asn Gly Gln Lys Arg Val Ser
465                 470                 475                 480
Phe Ser Phe Pro Pro Glu Leu Glu Lys Lys Leu Gly Ile Asn Met Leu
                485                 490                 495
Val Lys Leu Ile Thr Pro Asp Gly Lys Val Leu Glu Lys Val Ser Gly
            500                 505                 510
Lys Val Phe Gly Glu Gly Val Gly Asn Ile Ala Asn Phe Glu Leu Asp
        515                 520                 525
Gln Pro Tyr Leu Pro Gly Gln Thr Phe Lys Tyr Thr Ile Ala Ser Lys
    530                 535                 540
Asp Tyr Pro Glu Val Ser Tyr Asp Gly Thr Phe Thr Val Pro Thr Ser
545                 550                 555                 560
Leu Ala Tyr Lys Met Ala Ser Gln Thr Ile Phe Tyr Pro Phe His Ala
                565                 570                 575
Gly Asp Thr Tyr Leu Arg Val Asn Pro Gln Phe Ala Val Pro Lys Gly
            580                 585                 590
Thr Asp Ala Leu Val Arg Val Phe Asp Glu Phe His Gly Asn Ala Tyr
        595                 600                 605
Leu Glu Asn Asn Tyr Lys Val Gly Glu Ile Lys Leu Pro Ile Pro Lys
    610                 615                 620
Leu Asn Gln Gly Thr Thr Arg Thr Ala Gly Asn Lys Ile Pro Val Thr
625                 630                 635                 640
Phe Met Ala Asn Ala Tyr Leu Asp Asn Gln Ser Thr Tyr Ile Val Glu
                645                 650                 655
Val Pro Ile Leu Glu Lys Glu Asn Gln Thr Asp Lys Pro Ser Ile Leu
            660                 665                 670
Pro Gln Phe Lys Arg Asn Lys Ala Gln Glu Asn Ser Lys Leu Asp Glu
        675                 680                 685
Lys Val Glu Glu Pro Lys Thr Ser Glu Lys Val Glu Lys Glu Lys Leu
    690                 695                 700
Ser Glu Thr Gly Asn Ser Thr Ser Asn Ser Thr Leu Glu Glu Val Pro
705                 710                 715                 720
Thr Val Asp Pro Val Gln Glu Lys Val Ala Lys Phe Ala Glu Ser Tyr
                725                 730                 735
Gly Met Lys Leu Glu Asn Val Leu Phe Asn Met Asp Gly Thr Ile Glu
            740                 745                 750
Leu Tyr Leu Pro Ser Gly Glu Val Ile Lys Lys Asn Met Ala Asp Phe
        755                 760                 765
Thr Gly Glu Ala Pro Gln Gly Asn Gly Glu Asn Lys Pro Ser Glu Asn
    770                 775                 780
Gly Lys Val Ser Thr Gly Thr Val Glu Asn Gln Pro Thr Glu Asn Lys
785                 790                 795                 800

Pro Ala Asp Ser Leu Pro Glu Ala Pro Asn Glu Lys Pro Val Lys Pro
                805                 810                 815
Glu Asn Ser Thr Asp Asn Gly Met Leu Asn Pro Glu Gly Asn Val Gly
            820                 825                 830
Ser Asp Pro Met Leu Asp Pro Ala Leu Glu Glu Ala Pro Ala Val Asp
        835                 840                 845
Pro Val Gln Glu Lys Leu Glu Lys Phe Thr Ala Ser Tyr Gly Leu Gly
    850                 855                 860
Leu Asp Ser Val Ile Phe Asn Met Asp Gly Thr Ile Glu Leu Arg Leu
865                 870                 875                 880
Pro Ser Gly Glu Val Ile Lys Lys Asn Leu Ser Asp Phe Ile Ala
                885                 890                 895

<210> 348

<211> 889
<212> PRT
<213> Unknown

<220>
<223> VP 124

<400> 348
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Asn Ile Lys Phe
            100                 105                 110
Glu Trp Phe Asp Glu Gly Leu Tyr Glu Ala Pro Lys Gly Tyr Ser Leu
        115                 120                 125
Glu Asp Leu Leu Ala Thr Val Lys Tyr Tyr Val Glu Pro Arg Asn Ala
    130                 135                 140
Ser Asp His Val Arg Lys Asn Lys Ala Asp Gln Asp Ser Lys Pro Asp
145                 150                 155                 160
Glu Asp Lys Glu His Asp Glu Val Ser Glu Pro Thr His Pro Glu Ser
                165                 170                 175
Asp Glu Lys Glu Asn His Ala Gly Leu Asn Pro Ser Ala Asp Asn Leu
            180                 185                 190
Tyr Lys Pro Ser Thr Asp Thr Glu Glu Thr Glu Glu Glu Ala Glu Asp
        195                 200                 205
Thr Thr Asp Glu Ala Glu Ile Pro Gly Thr Pro Ser Ile Arg Gln Asn
    210                 215                 220
Ala Met Glu Thr Leu Thr Gly Leu Lys Ser Ser Leu Leu Leu Gly Thr
225                 230                 235                 240
Lys Asp Asn Asn Thr Ile Ser Ala Glu Val Asp Ser Leu Leu Ala Leu
                245                 250                 255
Leu Lys Glu Ser Gln Pro Ala Pro Ile Gln Ser Gly Pro Gln Ile Gly
            260                 265                 270
Gln Pro Thr Leu Pro Asn Asn Ser Leu Ala Thr Pro Ser Pro Ser Leu
        275                 280                 285
Pro Ile Asn Pro Gly Thr Ser His Glu Lys His Glu Glu Asp Gly Tyr
    290                 295                 300
Gly Phe Asp Ala Asn Arg Ile Ile Ala Glu Asp Glu Ser Gly Phe Val
305                 310                 315                 320
Met Ser Tyr Phe Phe Lys Lys Asp Leu Thr Glu Glu Gln Ile Lys Ala
                325                 330                 335
Ala Gln Lys His Leu Glu Glu Val Lys Thr Ser His Asn Gly Leu Asp
            340                 345                 350
Ser Leu Ser Ser His Glu Gln Asp Tyr Pro Gly Asn Ala Lys Glu Met

        355                 360                 365
Lys Asp Leu Asp Lys Lys Ile Glu Glu Lys Ile Ala Gly Ile Met Lys
    370                 375                 380
Gln Tyr Gly Val Lys Arg Glu Ser Ile Val Val Asn Lys Glu Lys Asn
385                 390                 395                 400
Ala Ile Ile Tyr Pro Ala Asp Pro Ile Asp Glu His Lys Pro Val Gly
                405                 410                 415
Ile Gly His Ser His Ser Asn Tyr Glu Leu Phe Lys Pro Glu Glu Gly
            420                 425                 430
Val Ala Lys Lys Glu Gly Asn Lys Val Tyr Thr Gly Glu Glu Leu Thr
        435                 440                 445
Asn Val Val Asn Leu Leu Lys Asn Ser Thr Phe Asn Asn Gln Asn Phe
    450                 455                 460
Thr Leu Ala Asn Gly Gln Lys Arg Val Ser Phe Ser Phe Pro Pro Glu
465                 470                 475                 480
Leu Glu Lys Lys Leu Gly Ile Asn Met Leu Val Lys Leu Ile Thr Pro
                485                 490                 495
Asp Gly Lys Val Leu Glu Lys Val Ser Gly Lys Val Phe Gly Glu Gly
            500                 505                 510
Val Gly Asn Ile Ala Asn Phe Glu Leu Asp Gln Pro Tyr Leu Pro Gly
        515                 520                 525
Gln Thr Phe Lys Tyr Thr Ile Ala Ser Lys Asp Tyr Pro Glu Val Ser
    530                 535                 540
Tyr Asp Gly Thr Phe Thr Val Pro Thr Ser Leu Ala Tyr Lys Met Ala
545                 550                 555                 560
Ser Gln Thr Ile Phe Tyr Pro Phe His Ala Gly Asp Thr Tyr Leu Arg
                565                 570                 575
Val Asn Pro Gln Phe Ala Val Pro Lys Gly Thr Asp Ala Leu Val Arg
            580                 585                 590
Val Phe Asp Glu Phe His Gly Asn Ala Tyr Leu Glu Asn Asn Tyr Lys
        595                 600                 605
Val Gly Glu Ile Lys Leu Pro Ile Pro Lys Leu Asn Gln Gly Thr Thr
    610                 615                 620
Arg Thr Ala Gly Asn Lys Ile Pro Val Thr Phe Met Ala Asn Ala Tyr
625                 630                 635                 640
Leu Asp Asn Gln Ser Thr Tyr Ile Val Glu Val Pro Ile Leu Glu Lys
                645                 650                 655
Glu Asn Gln Thr Asp Lys Pro Ser Ile Leu Pro Gln Phe Lys Arg Asn
            660                 665                 670
Lys Ala Gln Glu Asn Ser Lys Leu Asp Glu Lys Val Glu Glu Pro Lys
        675                 680                 685
Thr Ser Glu Lys Val Glu Lys Glu Lys Leu Ser Glu Thr Gly Asn Ser
    690                 695                 700
Thr Ser Asn Ser Thr Leu Glu Glu Val Pro Thr Val Asp Pro Val Gln
705                 710                 715                 720
Glu Lys Val Ala Lys Phe Ala Glu Ser Tyr Gly Met Lys Leu Glu Asn
                725                 730                 735
Val Leu Phe Asn Met Asp Gly Thr Ile Glu Leu Tyr Leu Pro Ser Gly
            740                 745                 750
Glu Val Ile Lys Lys Asn Met Ala Asp Phe Thr Gly Glu Ala Pro Gln
        755                 760                 765
Gly Asn Gly Glu Asn Lys Pro Ser Glu Asn Gly Lys Val Ser Thr Gly
    770                 775                 780
Thr Val Glu Asn Gln Pro Thr Glu Asn Lys Pro Ala Asp Ser Leu Pro
785                 790                 795                 800
Glu Ala Pro Asn Glu Lys Pro Val Lys Pro Glu Asn Ser Thr Asp Asn
                805                 810                 815
Gly Met Leu Asn Pro Glu Gly Asn Val Gly Ser Asp Pro Met Leu Asp
            820                 825                 830
Pro Ala Leu Glu Glu Ala Pro Ala Val Asp Pro Val Gln Glu Lys Leu
        835                 840                 845
Glu Lys Phe Thr Ala Ser Tyr Gly Leu Gly Leu Asp Ser Val Ile Phe
    850                 855                 860
Asn Met Asp Gly Thr Ile Glu Leu Arg Leu Pro Ser Gly Glu Val Ile
865                 870                 875                 880
Lys Lys Asn Leu Ser Asp Phe Ile Ala
                885

<210> 349

<211> 633
<212> PRT
<213> Unknown

<220>
<223> NEW 42

<400> 349
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser Ser Gly Asp Ser Asn Ser Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser
    130                 135                 140
Gly Asp Gly Thr Ser Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460

Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 350

<211> 633
<212> PRT
<213> Unknown

<220>
<223> NEW 49

<400> 350
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser Ser Gly Asp His Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro His
    130                 135                 140
Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser

        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 351

<211> 633
<212> PRT
<213> Unknown

<220>
<223> NEW 50

<400> 351
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser Ser Gly Asp Ser Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95

Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro His
    130                 135                 140
Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 352

<211> 633
<212> PRT
<213> Unknown

<220>
<223> NEW 51

<400> 352
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser Ser Gly Asp His Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser
    130                 135                 140
Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln

450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 353

<211> 633
<212> PRT
<213> Unknown

<220>
<223> NEW 52

<400> 353
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser Ser Gly Asp Ser Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser
    130                 135                 140
Gly Asp Gly His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270

Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 354

<211> 633
<212> PRT
<213> Unknown

<220>
<223> NEW 53

<400> 354
Met Gln Ile Gly Gln Pro Thr Leu Pro Asn Asn Ser Leu Ala Thr Pro
 1               5                  10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp His Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu

85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser
    130                 135                 140
Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala

625                 630

<210> 355

<211> 633
<212> PRT
<213> Unknown

<220>
<223> NEW 54

<400> 355
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp His Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser
    130                 135                 140
Gly Asp Gly His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445

Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 356

<211> 569
<212> PRT
<213> Unknown

<220>
<223> NEW 55

<400> 356
Met Asp Leu Thr Glu Glu Gln Ile Lys Ala Ala Gln Lys His Leu Glu
 1               5                  10                  15
Glu Val Lys Thr Ser His Asn Gly Leu Asp Ser Leu Ser Ser His Glu
            20                  25                  30
Gln Asp Tyr Pro Gly Asn Ala Lys Glu Met Lys Asp Leu Asp Lys Lys
        35                  40                  45
Ile Glu Glu Lys Ile Ala Gly Ile Met Lys Gln Tyr Gly Val Lys Arg
    50                  55                  60
Glu Ser Ile Val Val Asn Lys Glu Lys Asn Ala Ile Ile Tyr Pro His
65                  70                  75                  80
Gly Asp Gly His His Ala Asp Pro Ile Asp Glu His Lys Pro Val Gly
                85                  90                  95
Ile Gly His Ser His Ser Asn Tyr Glu Leu Phe Lys Pro Glu Glu Gly
            100                 105                 110
Val Ala Lys Lys Glu Gly Asn Lys Val Tyr Thr Gly Glu Glu Leu Thr
        115                 120                 125
Asn Val Val Asn Leu Leu Lys Asn Ser Thr Phe Asn Asn Gln Asn Phe
    130                 135                 140
Thr Leu Ala Asn Gly Gln Lys Arg Val Ser Phe Ser Phe Pro Pro Glu
145                 150                 155                 160
Leu Glu Lys Lys Leu Gly Ile Asn Met Leu Val Lys Leu Ile Thr Pro
                165                 170                 175
Asp Gly Lys Val Leu Glu Lys Val Ser Gly Lys Val Phe Gly Glu Gly
            180                 185                 190
Val Gly Asn Ile Ala Asn Phe Glu Leu Asp Gln Pro Tyr Leu Pro Gly
        195                 200                 205
Gln Thr Phe Lys Tyr Thr Ile Ala Ser Lys Asp Tyr Pro Glu Val Ser
    210                 215                 220
Tyr Asp Gly Thr Phe Thr Val Pro Thr Ser Leu Ala Tyr Lys Met Ala
225                 230                 235                 240
Ser Gln Thr Ile Phe Tyr Pro Phe His Ala Gly Asp Thr Tyr Leu Arg
                245                 250                 255
Val Asn Pro Gln Phe Ala Val Pro Lys Gly Thr Asp Ala Leu Val Arg

260                 265                 270
Val Phe Asp Glu Phe His Gly Asn Ala Tyr Leu Glu Asn Asn Tyr Lys
        275                 280                 285
Val Gly Glu Ile Lys Leu Pro Ile Pro Lys Leu Asn Gln Gly Thr Thr
    290                 295                 300
Arg Thr Ala Gly Asn Lys Ile Pro Val Thr Phe Met Ala Asn Ala Tyr
305                 310                 315                 320
Leu Asp Asn Gln Ser Thr Tyr Ile Val Glu Val Pro Ile Leu Glu Lys
                325                 330                 335
Glu Asn Gln Thr Asp Lys Pro Ser Ile Leu Pro Gln Phe Lys Arg Asn
            340                 345                 350
Lys Ala Gln Glu Asn Ser Lys Leu Asp Glu Lys Val Glu Glu Pro Lys
        355                 360                 365
Thr Ser Glu Lys Val Glu Lys Glu Lys Leu Ser Glu Thr Gly Asn Ser
    370                 375                 380
Thr Ser Asn Ser Thr Leu Glu Glu Val Pro Thr Val Asp Pro Val Gln
385                 390                 395                 400
Glu Lys Val Ala Lys Phe Ala Glu Ser Tyr Gly Met Lys Leu Glu Asn
                405                 410                 415
Val Leu Phe Asn Met Asp Gly Thr Ile Glu Leu Tyr Leu Pro Ser Gly
            420                 425                 430
Glu Val Ile Lys Lys Asn Met Ala Asp Phe Thr Gly Glu Ala Pro Gln
        435                 440                 445
Gly Asn Gly Glu Asn Lys Pro Ser Glu Asn Gly Lys Val Ser Thr Gly
    450                 455                 460
Thr Val Glu Asn Gln Pro Thr Glu Asn Lys Pro Ala Asp Ser Leu Pro
465                 470                 475                 480
Glu Ala Pro Asn Glu Lys Pro Val Lys Pro Glu Asn Ser Thr Asp Asn
                485                 490                 495
Gly Met Leu Asn Pro Glu Gly Asn Val Gly Ser Asp Pro Met Leu Asp
            500                 505                 510
Pro Ala Leu Glu Glu Ala Pro Ala Val Asp Pro Val Gln Glu Lys Leu
        515                 520                 525
Glu Lys Phe Thr Ala Ser Tyr Gly Leu Gly Leu Asp Ser Val Ile Phe
    530                 535                 540
Asn Met Asp Gly Thr Ile Glu Leu Arg Leu Pro Ser Gly Glu Val Ile
545                 550                 555                 560
Lys Lys Asn Leu Ser Asp Phe Ile Ala
                565

<210> 357

<211> 633
<212> PRT
<213> Unknown

<220>
<223> NEW 56

<400> 357
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp Ser Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser
    130                 135                 140

Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 358

<211> 633
<212> PRT
<213> Unknown

<220>
<223> NEW 56

<400> 358
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp Ser Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser
    130                 135                 140
Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln

                500                 505                 510
Gly Asn Gly Glu Asn Lys Pro Ser Glu Asn Gly Lys Val Ser Thr Gly
            515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
            595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 359

<211> 633
<212> PRT
<213> Unknown

<220>
<223> NEW 56

<400> 359
Met Gln Ile Gly Gln Pro Thr Leu Pro Asn Asn Ser Leu Ala Thr Pro
 1               5                  10                  15
Ser Pro Ser Leu Pro Ile Asn Pro Gly Thr Ser His Glu Lys His Glu
                20                  25                  30
Glu Asp Gly Tyr Gly Phe Asp Ala Asn Arg Ile Ile Ala Glu Asp Glu
            35                  40                  45
Ser Gly Phe Val Met Ser His Gly Asp Ser Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
            115                 120                 125
Glu Ser Ile Val Val Asn Lys Glu Lys Asn Ala Ile Ile Tyr Pro Ser
    130                 135                 140
Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
            195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
            275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320

Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 360

<211> 633
<212> PRT
<213> Unknown

<220>
<223> NEW 57

<400> 360
Met Gln Ile Gly Gln Pro Thr Leu Pro Asn Asn Ser Leu Ala Thr Pro
 1               5                  10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp His Asn Ser Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser

130                 135                 140
Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 361

<211> 633
<212> PRT

<213> Unknown

<220>
<223> NEW 63

<400> 361
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp Ser Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro His
    130                 135                 140
Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495

Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 362

<211> 633
<212> PRT
<213> Unknown

<220>
<223> NEW 64

<400> 362
Met Gln Ile Gly Gln Pro Thr Leu Pro Asn Asn Ser Leu Ala Thr Pro
 1               5                  10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp His Asn Ser Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro His
    130                 135                 140
Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg

305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 363

<211> 633
<212> PRT
<213> Unknown

<220>
<223> NEW 65

<400> 363
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp Ser Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125

Glu Ser Ile Val Val Asn Lys Glu Lys Asn Ala Ile Ile Tyr Pro His
        130                 135                 140
Gly Asp Gly His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 364

<211> 633

<212> PRT
<213> Unknown

<220>
<223> NEW 66

<400> 364
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp His Asn Ser Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro His
    130                 135                 140
Gly Asp Gly His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly

485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 365

<211> 633
<212> PRT
<213> Unknown

<220>
<223> NEW 76

<400> 365
Met Gln Ile Gly Gln Pro Thr Leu Pro Asn Asn Ser Leu Ala Thr Pro
 1               5                  10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp His Asn Ser Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser
    130                 135                 140
Gly Asp Gly His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300

Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala
625                 630

<210> 366

<211> 627
<212> PRT
<213> Unknown

<220>
<223> NEW 105

<400> 366
Met Gln Ile Gly Gln Pro Thr Leu Pro Asn Asn Ser Leu Ala Thr Pro
 1               5                  10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser Tyr Phe Phe Lys Lys Asp Leu Thr Glu Glu
    50                  55                  60
Gln Ile Lys Ala Ala Gln Lys His Leu Glu Glu Val Lys Thr Ser His
65                  70                  75                  80
Asn Gly Leu Asp Ser Leu Ser Ser His Glu Gln Asp Tyr Pro Gly Asn
                85                  90                  95
Ala Lys Glu Met Lys Asp Leu Asp Lys Lys Ile Glu Glu Lys Ile Ala
            100                 105                 110
Gly Ile Met Lys Gln Tyr Gly Val Lys Arg Glu Ser Ile Val Val Asn

        115                 120                 125
Lys Glu Lys Asn Ala Ile Ile Tyr Pro His Gly Asp His His His Ala
    130                 135                 140
Asp Pro Ile Asp Glu His Lys Pro Val Gly Ile Gly His Ser His Ser
145                 150                 155                 160
Asn Tyr Glu Leu Phe Lys Pro Glu Glu Gly Val Ala Lys Lys Glu Gly
                165                 170                 175
Asn Lys Val Tyr Thr Gly Glu Glu Leu Thr Asn Val Val Asn Leu Leu
            180                 185                 190
Lys Asn Ser Thr Phe Asn Asn Gln Asn Phe Thr Leu Ala Asn Gly Gln
        195                 200                 205
Lys Arg Val Ser Phe Ser Phe Pro Pro Glu Leu Glu Lys Lys Leu Gly
    210                 215                 220
Ile Asn Met Leu Val Lys Leu Ile Thr Pro Asp Gly Lys Val Leu Glu
225                 230                 235                 240
Lys Val Ser Gly Lys Val Phe Gly Glu Gly Val Gly Asn Ile Ala Asn
                245                 250                 255
Phe Glu Leu Asp Gln Pro Tyr Leu Pro Gly Gln Thr Phe Lys Tyr Thr
            260                 265                 270
Ile Ala Ser Lys Asp Tyr Pro Glu Val Ser Tyr Asp Gly Thr Phe Thr
        275                 280                 285
Val Pro Thr Ser Leu Ala Tyr Lys Met Ala Ser Gln Thr Ile Phe Tyr
    290                 295                 300
Pro Phe His Ala Gly Asp Thr Tyr Leu Arg Val Asn Pro Gln Phe Ala
305                 310                 315                 320
Val Pro Lys Gly Thr Asp Ala Leu Val Arg Val Phe Asp Glu Phe His
                325                 330                 335
Gly Asn Ala Tyr Leu Glu Asn Asn Tyr Lys Val Gly Glu Ile Lys Leu
            340                 345                 350
Pro Ile Pro Lys Leu Asn Gln Gly Thr Thr Arg Thr Ala Gly Asn Lys
        355                 360                 365
Ile Pro Val Thr Phe Met Ala Asn Ala Tyr Leu Asp Asn Gln Ser Thr
    370                 375                 380
Tyr Ile Val Glu Val Pro Ile Leu Glu Lys Glu Asn Gln Thr Asp Lys
385                 390                 395                 400
Pro Ser Ile Leu Pro Gln Phe Lys Arg Asn Lys Ala Gln Glu Asn Ser
                405                 410                 415
Lys Leu Asp Glu Lys Val Glu Glu Pro Lys Thr Ser Glu Lys Val Glu
            420                 425                 430
Lys Glu Lys Leu Ser Glu Thr Gly Asn Ser Thr Ser Asn Ser Thr Leu
        435                 440                 445
Glu Glu Val Pro Thr Val Asp Pro Val Gln Glu Lys Val Ala Lys Phe
    450                 455                 460
Ala Glu Ser Tyr Gly Met Lys Leu Glu Asn Val Leu Phe Asn Met Asp
465                 470                 475                 480
Gly Thr Ile Glu Leu Tyr Leu Pro Ser Gly Glu Val Ile Lys Lys Asn
                485                 490                 495
Met Ala Asp Phe Thr Gly Glu Ala Pro Gln Gly Asn Gly Glu Asn Lys
            500                 505                 510
Pro Ser Glu Asn Gly Lys Val Ser Thr Gly Thr Val Glu Asn Gln Pro
        515                 520                 525
Thr Glu Asn Lys Pro Ala Asp Ser Leu Pro Glu Ala Pro Asn Glu Lys
    530                 535                 540
Pro Val Lys Pro Glu Asn Ser Thr Asp Asn Gly Met Leu Asn Pro Glu
545                 550                 555                 560
Gly Asn Val Gly Ser Asp Pro Met Leu Asp Pro Ala Leu Glu Glu Ala
                565                 570                 575
Pro Ala Val Asp Pro Val Gln Glu Lys Leu Glu Lys Phe Thr Ala Ser
            580                 585                 590
Tyr Gly Leu Gly Leu Asp Ser Val Ile Phe Asn Met Asp Gly Thr Ile
        595                 600                 605
Glu Leu Arg Leu Pro Ser Gly Glu Val Ile Lys Lys Asn Leu Ser Asp
    610                 615                 620
Phe Ile Ala
625

<210> 367

<211> 627
<212> PRT
<213> Unknown

<220>
<223> NEW 106

<400> 367
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp His Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ala
    130                 135                 140
Asp Pro Ile Asp Glu His Lys Pro Val Gly Ile Gly His Ser His Ser
145                 150                 155                 160
Asn Tyr Glu Leu Phe Lys Pro Glu Glu Gly Val Ala Lys Lys Glu Gly
                165                 170                 175
Asn Lys Val Tyr Thr Gly Glu Glu Leu Thr Asn Val Val Asn Leu Leu
            180                 185                 190
Lys Asn Ser Thr Phe Asn Asn Gln Asn Phe Thr Leu Ala Asn Gly Gln
        195                 200                 205
Lys Arg Val Ser Phe Ser Phe Pro Pro Glu Leu Glu Lys Lys Leu Gly
    210                 215                 220
Ile Asn Met Leu Val Lys Leu Ile Thr Pro Asp Gly Lys Val Leu Glu
225                 230                 235                 240
Lys Val Ser Gly Lys Val Phe Gly Glu Gly Val Gly Asn Ile Ala Asn
                245                 250                 255
Phe Glu Leu Asp Gln Pro Tyr Leu Pro Gly Gln Thr Phe Lys Tyr Thr
            260                 265                 270
Ile Ala Ser Lys Asp Tyr Pro Glu Val Ser Tyr Asp Gly Thr Phe Thr
        275                 280                 285
Val Pro Thr Ser Leu Ala Tyr Lys Met Ala Ser Gln Thr Ile Phe Tyr
    290                 295                 300
Pro Phe His Ala Gly Asp Thr Tyr Leu Arg Val Asn Pro Gln Phe Ala
305                 310                 315                 320
Val Pro Lys Gly Thr Asp Ala Leu Val Arg Val Phe Asp Glu Phe His
                325                 330                 335
Gly Asn Ala Tyr Leu Glu Asn Asn Tyr Lys Val Gly Glu Ile Lys Leu
            340                 345                 350
Pro Ile Pro Lys Leu Asn Gln Gly Thr Thr Arg Thr Ala Gly Asn Lys
        355                 360                 365
Ile Pro Val Thr Phe Met Ala Asn Ala Tyr Leu Asp Asn Gln Ser Thr
    370                 375                 380
Tyr Ile Val Glu Val Pro Ile Leu Glu Lys Glu Asn Gln Thr Asp Lys
385                 390                 395                 400
Pro Ser Ile Leu Pro Gln Phe Lys Arg Asn Lys Ala Gln Glu Asn Ser
                405                 410                 415
Lys Leu Asp Glu Lys Val Glu Glu Pro Lys Thr Ser Glu Lys Val Glu
            420                 425                 430
Lys Glu Lys Leu Ser Glu Thr Gly Asn Ser Thr Ser Asn Ser Thr Leu
        435                 440                 445
Glu Glu Val Pro Thr Val Asp Pro Val Gln Glu Lys Val Ala Lys Phe
    450                 455                 460
Ala Glu Ser Tyr Gly Met Lys Leu Glu Asn Val Leu Phe Asn Met Asp
465                 470                 475                 480

Gly Thr Ile Glu Leu Tyr Leu Pro Ser Gly Glu Val Ile Lys Lys Asn
                485                 490                 495
Met Ala Asp Phe Thr Gly Glu Ala Pro Gln Gly Asn Gly Glu Asn Lys
            500                 505                 510
Pro Ser Glu Asn Gly Lys Val Ser Thr Gly Thr Val Glu Asn Gln Pro
        515                 520                 525
Thr Glu Asn Lys Pro Ala Asp Ser Leu Pro Glu Ala Pro Asn Glu Lys
    530                 535                 540
Pro Val Lys Pro Glu Asn Ser Thr Asp Asn Gly Met Leu Asn Pro Glu
545                 550                 555                 560
Gly Asn Val Gly Ser Asp Pro Met Leu Asp Pro Ala Leu Glu Glu Ala
                565                 570                 575
Pro Ala Val Asp Pro Val Gln Glu Lys Leu Glu Lys Phe Thr Ala Ser
            580                 585                 590
Tyr Gly Leu Gly Leu Asp Ser Val Ile Phe Asn Met Asp Gly Thr Ile
        595                 600                 605
Glu Leu Arg Leu Pro Ser Gly Glu Val Ile Lys Lys Asn Leu Ser Asp
    610                 615                 620
Phe Ile Ala
625

<210> 368


<211> 621
<212> PRT
<213> Unknown

<220>
<223> NEW 107

<400> 368
Met Gln Ile Gly Gln Pro Thr Leu Pro Asn Asn Ser Leu Ala Thr Pro
 1               5                  10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser Tyr Phe Phe Lys Lys Asp Leu Thr Glu Glu
    50                  55                  60
Gln Ile Lys Ala Ala Gln Lys His Leu Glu Glu Val Lys Thr Ser His
65                  70                  75                  80
Asn Gly Leu Asp Ser Leu Ser Ser His Glu Gln Asp Tyr Pro Gly Asn
                85                  90                  95
Ala Lys Glu Met Lys Asp Leu Asp Lys Lys Ile Glu Glu Lys Ile Ala
            100                 105                 110
Gly Ile Met Lys Gln Tyr Gly Val Lys Arg Glu Ser Ile Val Val Asn
        115                 120                 125
Lys Glu Lys Asn Ala Ile Ile Tyr Pro Ala Asp Pro Ile Asp Glu His
    130                 135                 140
Lys Pro Val Gly Ile Gly His Ser His Ser Asn Tyr Glu Leu Phe Lys
145                 150                 155                 160
Pro Glu Glu Gly Val Ala Lys Lys Glu Gly Asn Lys Val Tyr Thr Gly
                165                 170                 175
Glu Glu Leu Thr Asn Val Val Asn Leu Leu Lys Asn Ser Thr Phe Asn
            180                 185                 190
Asn Gln Asn Phe Thr Leu Ala Asn Gly Gln Lys Arg Val Ser Phe Ser
        195                 200                 205
Phe Pro Pro Glu Leu Glu Lys Lys Leu Gly Ile Asn Met Leu Val Lys
    210                 215                 220
Leu Ile Thr Pro Asp Gly Lys Val Leu Glu Lys Val Ser Gly Lys Val
225                 230                 235                 240
Phe Gly Glu Gly Val Gly Asn Ile Ala Asn Phe Glu Leu Asp Gln Pro
                245                 250                 255
Tyr Leu Pro Gly Gln Thr Phe Lys Tyr Thr Ile Ala Ser Lys Asp Tyr
            260                 265                 270
Pro Glu Val Ser Tyr Asp Gly Thr Phe Thr Val Pro Thr Ser Leu Ala
        275                 280                 285

Tyr Lys Met Ala Ser Gln Thr Ile Phe Tyr Pro Phe His Ala Gly Asp
    290                 295                 300
Thr Tyr Leu Arg Val Asn Pro Gln Phe Ala Val Pro Lys Gly Thr Asp
305                 310                 315                 320
Ala Leu Val Arg Val Phe Asp Glu Phe His Gly Asn Ala Tyr Leu Glu
                325                 330                 335
Asn Asn Tyr Lys Val Gly Glu Ile Lys Leu Pro Ile Pro Lys Leu Asn
            340                 345                 350
Gln Gly Thr Thr Arg Thr Ala Gly Asn Lys Ile Pro Val Thr Phe Met
        355                 360                 365
Ala Asn Ala Tyr Leu Asp Asn Gln Ser Thr Tyr Ile Val Glu Val Pro
    370                 375                 380
Ile Leu Glu Lys Glu Asn Gln Thr Asp Lys Pro Ser Ile Leu Pro Gln
385                 390                 395                 400
Phe Lys Arg Asn Lys Ala Gln Glu Asn Ser Lys Leu Asp Glu Lys Val
                405                 410                 415
Glu Glu Pro Lys Thr Ser Glu Lys Val Glu Lys Glu Lys Leu Ser Glu
            420                 425                 430
Thr Gly Asn Ser Thr Ser Asn Ser Thr Leu Glu Glu Val Pro Thr Val
        435                 440                 445
Asp Pro Val Gln Glu Lys Val Ala Lys Phe Ala Glu Ser Tyr Gly Met
    450                 455                 460
Lys Leu Glu Asn Val Leu Phe Asn Met Asp Gly Thr Ile Glu Leu Tyr
465                 470                 475                 480
Leu Pro Ser Gly Glu Val Ile Lys Lys Asn Met Ala Asp Phe Thr Gly
                485                 490                 495
Glu Ala Pro Gln Gly Asn Gly Glu Asn Lys Pro Ser Glu Asn Gly Lys
            500                 505                 510
Val Ser Thr Gly Thr Val Glu Asn Gln Pro Thr Glu Asn Lys Pro Ala
        515                 520                 525
Asp Ser Leu Pro Glu Ala Pro Asn Glu Lys Pro Val Lys Pro Glu Asn
    530                 535                 540
Ser Thr Asp Asn Gly Met Leu Asn Pro Glu Gly Asn Val Gly Ser Asp
545                 550                 555                 560
Pro Met Leu Asp Pro Ala Leu Glu Glu Ala Pro Ala Val Asp Pro Val
                565                 570                 575
Gln Glu Lys Leu Glu Lys Phe Thr Ala Ser Tyr Gly Leu Gly Leu Asp
            580                 585                 590
Ser Val Ile Phe Asn Met Asp Gly Thr Ile Glu Leu Arg Leu Pro Ser
        595                 600                 605
Gly Glu Val Ile Lys Lys Asn Leu Ser Asp Phe Ile Ala
    610                 615                 620

<210> 369

<211> 906
<212> PRT
<213> Unknown

<220>
<223> VP 89

<400> 369
   Met Gln Ile Gly Gln Pro Thr Leu Pro Asn Asn Ser Leu Ala Thr Pro
   1               5                   10                  15
   Ser Pro Ser Leu Pro Ile Asn Pro Gly Thr Ser His Glu Lys His Glu
               20                  25                  30
   Glu Asp Gly Tyr Gly Phe Asp Ala Asn Arg Ile Ile Ala Glu Asp Glu
           35                  40                  45
   Ser Gly Phe Val Met Ser His Gly Asp Ser Asn His Tyr Phe Phe Lys
       50                  55                  60
   Lys Asp Leu Thr Glu Glu Gln Ile Lys Ala Ala Gln Lys His Leu Glu
   65                  70                  75                  80
   Glu Val Lys Thr Ser His Asn Gly Leu Asp Ser Leu Ser Ser His Glu
                   85                  90                  95
   Gln Asp Tyr Pro Gly Asn Ala Lys Glu Met Lys Asp Leu Asp Lys Lys
               100                 105                 110
   Ile Glu Glu Lys Ile Ala Gly Ile Met Lys Gln Tyr Gly Val Lys Arg
   115 120 125
   Glu Ser Ile Val Val Asn Lys Glu Lys Asn Ala Ile Ile Tyr Pro Ser
   130 135 140
   Gly Asp His His His Ala Asp Pro Ile Asp Glu His Lys Pro Val Gly
   145 150 155 160
   Ile Gly His Ser His Ser Asn Tyr Glu Leu Phe Lys Pro Glu Glu Gly
   165 170 175
   Val Ala Lys Lys Glu Gly Asn Lys Val Tyr Thr Gly Glu Glu Leu Thr
   180 185 190
   Asn Val Val Asn Leu Leu Lys Asn Ser Thr Phe Asn Asn Gln Asn Phe
   195 200 205
   Thr Leu Ala Asn Gly Gln Lys Arg Val Ser Phe Ser Phe Pro Pro Glu
   210 215 220
   Leu Glu Lys Lys Leu Gly Ile Asn Met Leu Val Lys Leu Ile Thr Pro
   225 230 235 240
   Asp Gly Lys Val Leu Glu Lys Val Ser Gly Lys Val Phe Gly Glu Gly
   245 250 255
   Val Gly Asn Ile Ala Asn Phe Glu Leu Asp Gln Pro Tyr Leu Pro Gly
   260 265 270
   Gln Thr Phe Lys Tyr Thr Ile Ala Ser Lys Asp Tyr Pro Glu Val Ser
   275 280 285
   Tyr Asp Gly Thr Phe Thr Val Pro Thr Ser Leu Ala Tyr Lys Met Ala
   290 295 300
   Ser Gln Thr Ile Phe Tyr Pro Phe His Ala Gly Asp Thr Tyr Leu Arg
   305 310 315 320
   Val Asn Pro Gln Phe Ala Val Pro Lys Gly Thr Asp Ala Leu Val Arg
   325 330 335
   Val Phe Asp Glu Phe His Gly Asn Ala Tyr Leu Glu Asn Asn Tyr Lys
   340 345 350
   Val Gly Glu Ile Lys Leu Pro Ile Pro Lys Leu Asn Gln Gly Thr Thr
   355 360 365
   Arg Thr Ala Gly Asn Lys Ile Pro Val Thr Phe Met Ala Asn Ala Tyr
   370 375 380
   Leu Asp Asn Gln Ser Thr Tyr Ile Val Glu Val Pro Ile Leu Glu Lys
   385 390 395 400
   Glu Asn Gln Thr Asp Lys Pro Ser Ile Leu Pro Gln Phe Lys Arg Asn
   405 410 415
   Lys Ala Gln Glu Asn Ser Lys Leu Asp Glu Lys Val Glu Glu Pro Lys
   420 425 430
   Thr Ser Glu Lys Val Glu Lys Glu Lys Leu Ser Glu Thr Gly Asn Ser
   435 440 445
   Thr Ser Asn Ser Thr Leu Glu Glu Val Pro Thr Val Asp Pro Val Gln
   450 455 460
   Glu Lys Val Ala Lys Phe Ala Glu Ser Tyr Gly Met Lys Leu Glu Asn
   465 470 475 480
   Val Leu Phe Asn Met Asp Gly Thr Ile Glu Leu Tyr Leu Pro Ser Gly
   485 490 495
   Glu Val Ile Lys Lys Asn Met Ala Asp Phe Thr Gly Glu Ala Pro Gln
   500 505 510
   Gly Asn Gly Glu Asn Lys Pro Ser Glu Asn Gly Lys Val Ser Thr Gly
   515 520 525
   Thr Val Glu Asn Gln Pro Thr Glu Asn Lys Pro Ala Asp Ser Leu Pro
   530 535 540
   Glu Ala Pro Asn Glu Lys Pro Val Lys Pro Glu Asn Ser Thr Asp Asn
   545 550 555 560
   Gly Met Leu Asn Pro Glu Gly Asn Val Gly Ser Asp Pro Met Leu Asp
   565 570 575
   Pro Ala Leu Glu Glu Ala Pro Ala Val Asp Pro Val Gln Glu Lys Leu
   580 585 590
   Glu Lys Phe Thr Ala Ser Tyr Gly Leu Gly Leu Asp Ser Val Ile Phe
   595 600 605
   Asn Met Asp Gly Thr Ile Glu Leu Arg Leu Pro Ser Gly Glu Val Ile
   610 615 620
   Lys Lys Asn Leu Ser Asp Phe Ile Ala Gly Pro Gln Ile Thr Tyr Thr
   625 630 635 640
   Asp Asp Glu Ile Gln Val Ala Lys Leu Ala Gly Lys Tyr Thr Thr Glu
   645 650 655
   Asp Gly Tyr Ile Phe Asp Thr Ser Trp Ile Lys Lys Asp Ser Leu Ser
   660 665 670
   Glu Ala Glu Arg Ala Ala Ala Gln Ala Tyr Ala Lys Glu Lys Gly Leu
   675 680 685
   Thr Pro Pro Ser Thr Asp His Gln Asp Ser Gly Asn Thr Glu Ala Lys
   690 695 700
   Gly Ala Glu Ala Ile Tyr Asn Arg Val Lys Ala Ala Lys Lys Val Pro
   705 710 715 720
   Leu Asp Arg Met Pro Tyr Asn Leu Gln Tyr Thr Val Glu Val Lys Asn
   725 730 735
   Gly Ser Leu Ile Ile Pro His Tyr Asp His Tyr His Asn Ile Lys Phe
   740 745 750
   Glu Trp Phe Asp Glu Gly Leu Tyr Glu Ala Pro Lys Gly Tyr Ser Leu
   755 760 765
   Glu Asp Leu Leu Ala Thr Val Lys Tyr Tyr Val Glu Pro Arg Asn Ala
   770 775 780
   Ser Asp His Val Arg Lys Asn Lys Ala Asp Gln Asp Ser Lys Pro Asp
   785 790 795 800
   Glu Asp Lys Glu His Asp Glu Val Ser Glu Pro Thr His Pro Glu Ser
   805 810 815
   Asp Glu Lys Glu Asn His Ala Gly Leu Asn Pro Ser Ala Asp Asn Leu
   820 825 830
   Tyr Lys Pro Ser Thr Asp Thr Glu Glu Thr Glu Glu Glu Ala Glu Asp
   835 840 845
   Thr Thr Asp Glu Ala Glu Ile Pro Gly Thr Pro Ser Ile Arg Gln Asn
   850 855 860
   Ala Met Glu Thr Leu Thr Gly Leu Lys Ser Ser Leu Leu Leu Gly Thr
   865 870 875 880
   Lys Asp Asn Asn Thr Ile Ser Ala Glu Val Asp Ser Leu Leu Ala Leu
   885 890 895
   Leu Lys Glu Ser Gln Pro Ala Pro Ile Gln
   900 905

<210> 370

<211> 906
<212> PRT
<213> Unknown

<220>
<223> VP 90

<400> 370
Met Gln Ile Thr Tyr Thr Asp Asp Glu Ile Gln Val Ala Lys Leu Ala
 1               5                  10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro His Tyr Asp His
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205

Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Gly Pro Gln Ile Gly Gln Pro Thr Leu Pro Asn Asn Ser Leu Ala Thr
        275                 280                 285
Pro Ser Pro Ser Leu Pro Ile Asn Pro Gly Thr Ser His Glu Lys His
    290                 295                 300
Glu Glu Asp Gly Tyr Gly Phe Asp Ala Asn Arg Ile Ile Ala Glu Asp
305                 310                 315                 320
Glu Ser Gly Phe Val Met Ser His Gly Asp Ser Asn His Tyr Phe Phe
                325                 330                 335
Lys Lys Asp Leu Thr Glu Glu Gln Ile Lys Ala Ala Gln Lys His Leu
            340                 345                 350
Glu Glu Val Lys Thr Ser His Asn Gly Leu Asp Ser Leu Ser Ser His
        355                 360                 365
Glu Gln Asp Tyr Pro Gly Asn Ala Lys Glu Met Lys Asp Leu Asp Lys
    370                 375                 380
Lys Ile Glu Glu Lys Ile Ala Gly Ile Met Lys Gln Tyr Gly Val Lys
385                 390                 395                 400
Arg Glu Ser Ile Val Val Asn Lys Glu Lys Asn Ala Ile Ile Tyr Pro
                405                 410                 415
Ser Gly Asp His His His Ala Asp Pro Ile Asp Glu His Lys Pro Val
            420                 425                 430
Gly Ile Gly His Ser His Ser Asn Tyr Glu Leu Phe Lys Pro Glu Glu
        435                 440                 445
Gly Val Ala Lys Lys Glu Gly Asn Lys Val Tyr Thr Gly Glu Glu Leu
    450                 455                 460
Thr Asn Val Val Asn Leu Leu Lys Asn Ser Thr Phe Asn Asn Gln Asn
465                 470                 475                 480
Phe Thr Leu Ala Asn Gly Gln Lys Arg Val Ser Phe Ser Phe Pro Pro
                485                 490                 495
Glu Leu Glu Lys Lys Leu Gly Ile Asn Met Leu Val Lys Leu Ile Thr
            500                 505                 510
Pro Asp Gly Lys Val Leu Glu Lys Val Ser Gly Lys Val Phe Gly Glu
        515                 520                 525
Gly Val Gly Asn Ile Ala Asn Phe Glu Leu Asp Gln Pro Tyr Leu Pro
    530                 535                 540
Gly Gln Thr Phe Lys Tyr Thr Ile Ala Ser Lys Asp Tyr Pro Glu Val
545                 550                 555                 560
Ser Tyr Asp Gly Thr Phe Thr Val Pro Thr Ser Leu Ala Tyr Lys Met
                565                 570                 575
Ala Ser Gln Thr Ile Phe Tyr Pro Phe His Ala Gly Asp Thr Tyr Leu
            580                 585                 590
Arg Val Asn Pro Gln Phe Ala Val Pro Lys Gly Thr Asp Ala Leu Val
        595                 600                 605
Arg Val Phe Asp Glu Phe His Gly Asn Ala Tyr Leu Glu Asn Asn Tyr
    610                 615                 620
Lys Val Gly Glu Ile Lys Leu Pro Ile Pro Lys Leu Asn Gln Gly Thr
625                 630                 635                 640
Thr Arg Thr Ala Gly Asn Lys Ile Pro Val Thr Phe Met Ala Asn Ala
                645                 650                 655
Tyr Leu Asp Asn Gln Ser Thr Tyr Ile Val Glu Val Pro Ile Leu Glu
            660                 665                 670
Lys Glu Asn Gln Thr Asp Lys Pro Ser Ile Leu Pro Gln Phe Lys Arg
        675                 680                 685
Asn Lys Ala Gln Glu Asn Ser Lys Leu Asp Glu Lys Val Glu Glu Pro
    690                 695                 700
Lys Thr Ser Glu Lys Val Glu Lys Glu Lys Leu Ser Glu Thr Gly Asn
705                 710                 715                 720
Ser Thr Ser Asn Ser Thr Leu Glu Glu Val Pro Thr Val Asp Pro Val
                725                 730                 735
Gln Glu Lys Val Ala Lys Phe Ala Glu Ser Tyr Gly Met Lys Leu Glu
            740                 745                 750

Asn Val Leu Phe Asn Met Asp Gly Thr Ile Glu Leu Tyr Leu Pro Ser
        755                 760                 765
Gly Glu Val Ile Lys Lys Asn Met Ala Asp Phe Thr Gly Glu Ala Pro
    770                 775                 780
Gln Gly Asn Gly Glu Asn Lys Pro Ser Glu Asn Gly Lys Val Ser Thr
785                 790                 795                 800
Gly Thr Val Glu Asn Gln Pro Thr Glu Asn Lys Pro Ala Asp Ser Leu
                805                 810                 815
Pro Glu Ala Pro Asn Glu Lys Pro Val Lys Pro Glu Asn Ser Thr Asp
            820                 825                 830
Asn Gly Met Leu Asn Pro Glu Gly Asn Val Gly Ser Asp Pro Met Leu
        835                 840                 845
Asp Pro Ala Leu Glu Glu Ala Pro Ala Val Asp Pro Val Gln Glu Lys
    850                 855                 860
Leu Glu Lys Phe Thr Ala Ser Tyr Gly Leu Gly Leu Asp Ser Val Ile
865                 870                 875                 880
Phe Asn Met Asp Gly Thr Ile Glu Leu Arg Leu Pro Ser Gly Glu Val
                885                 890                 895
Ile Lys Lys Asn Leu Ser Asp Phe Ile Ala
            900                 905

<210> 371

<211> 906
<212> PRT
<213> Unknown

<220>
<223> VP 91

<400> 371
Met Gln Ile Gly Gln Pro Thr Leu Pro Asn Asn Ser Leu Ala Thr Pro
 1               5                  10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser Ser Gly Asp Ser Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser
    130                 135                 140
Gly Asp Gly His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220

Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285

Tyr Asp Gly Thr Phe Thr Val Pro Thr Ser Leu Ala Tyr Lys Met Ala
        290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln
    450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala Gly Pro Gln Ile Thr Tyr Thr
625                 630                 635                 640
Asp Asp Glu Ile Gln Val Ala Lys Leu Ala Gly Lys Tyr Thr Thr Glu
                645                 650                 655
Asp Gly Tyr Ile Phe Asp Thr Ser Trp Ile Lys Lys Asp Ser Leu Ser
            660                 665                 670
Glu Ala Glu Arg Ala Ala Ala Gln Ala Tyr Ala Lys Glu Lys Gly Leu
        675                 680                 685
Thr Pro Pro Ser Thr Asp His Gln Asp Ser Gly Asn Thr Glu Ala Lys
    690                 695                 700
Gly Ala Glu Ala Ile Tyr Asn Arg Val Lys Ala Ala Lys Lys Val Pro
705                 710                 715                 720
Leu Asp Arg Met Pro Tyr Asn Leu Gln Tyr Thr Val Glu Val Lys Asn
                725                 730                 735
Gly Ser Leu Ile Ile Pro His Tyr Asp His Tyr His Asn Ile Lys Phe
            740                 745                 750
Glu Trp Phe Asp Glu Gly Leu Tyr Glu Ala Pro Lys Gly Tyr Ser Leu
        755                 760                 765
Glu Asp Leu Leu Ala Thr Val Lys Tyr Tyr Val Glu Pro Arg Asn Ala
    770                 775                 780
Ser Asp His Val Arg Lys Asn Lys Ala Asp Gln Asp Ser Lys Pro Asp
785                 790                 795                 800
Glu Asp Lys Glu His Asp Glu Val Ser Glu Pro Thr His Pro Glu Ser
                805                 810                 815
Asp Glu Lys Glu Asn His Ala Gly Leu Asn Pro Ser Ala Asp Asn Leu
            820                 825                 830

Tyr Lys Pro Ser Thr Asp Thr Glu Glu Thr Glu Glu Glu Ala Glu Asp
        835                 840                 845
Thr Thr Asp Glu Ala Glu Ile Pro Gly Thr Pro Ser Ile Arg Gln Asn
    850                 855                 860
Ala Met Glu Thr Leu Thr Gly Leu Lys Ser Ser Leu Leu Leu Gly Thr
865                 870                 875                 880

Lys Asp Asn Asn Thr Ile Ser Ala Glu Val Asp Ser Leu Leu Ala Leu
                885                 890                 895
Leu Lys Glu Ser Gln Pro Ala Pro Ile Gln
            900                 905

<210> 372

<211> 906
<212> PRT
<213> Unknown

<220>
<223> VP 92

<400> 372
Met Gln Ile Thr Tyr Thr Asp Asp Glu Ile Gln Val Ala Lys Leu Ala
 1               5                  10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro His Tyr Asp His
            100                 105                 110
Tyr His Asn Ile Lys Phe Glu Trp Phe Asp Glu Gly Leu Tyr Glu Ala
        115                 120                 125
Pro Lys Gly Tyr Ser Leu Glu Asp Leu Leu Ala Thr Val Lys Tyr Tyr
    130                 135                 140
Val Glu Pro Arg Asn Ala Ser Asp His Val Arg Lys Asn Lys Ala Asp
145                 150                 155                 160
Gln Asp Ser Lys Pro Asp Glu Asp Lys Glu His Asp Glu Val Ser Glu
                165                 170                 175
Pro Thr His Pro Glu Ser Asp Glu Lys Glu Asn His Ala Gly Leu Asn
            180                 185                 190
Pro Ser Ala Asp Asn Leu Tyr Lys Pro Ser Thr Asp Thr Glu Glu Thr
        195                 200                 205
Glu Glu Glu Ala Glu Asp Thr Thr Asp Glu Ala Glu Ile Pro Gly Thr
    210                 215                 220
Pro Ser Ile Arg Gln Asn Ala Met Glu Thr Leu Thr Gly Leu Lys Ser
225                 230                 235                 240
Ser Leu Leu Leu Gly Thr Lys Asp Asn Asn Thr Ile Ser Ala Glu Val
                245                 250                 255
Asp Ser Leu Leu Ala Leu Leu Lys Glu Ser Gln Pro Ala Pro Ile Gln
            260                 265                 270
Gly Pro Gln Ile Gly Gln Pro Thr Leu Pro Asn Asn Ser Leu Ala Thr
        275                 280                 285
Pro Ser Pro Ser Leu Pro Ile Asn Pro Gly Thr Ser His Glu Lys His
    290                 295                 300
Glu Glu Asp Gly Tyr Gly Phe Asp Ala Asn Arg Ile Ile Ala Glu Asp
305                 310                 315                 320
Glu Ser Gly Phe Val Met Ser Ser Gly Asp Ser Asn His Tyr Phe Phe
                325                 330                 335
Lys Lys Asp Leu Thr Glu Glu Gln Ile Lys Ala Ala Gln Lys His Leu
            340                 345                 350
Glu Glu Val Lys Thr Ser His Asn Gly Leu Asp Ser Leu Ser Ser His
        355                 360                 365

Glu Gln Asp Tyr Pro Gly Asn Ala Lys Glu Met Lys Asp Leu Asp Lys
    370                 375                 380
Lys Ile Glu Glu Lys Ile Ala Gly Ile Met Lys Gln Tyr Gly Val Lys
385                 390                 395                 400
Arg Glu Ser Ile Val Val Asn Lys Glu Lys Asn Ala Ile Ile Tyr Pro
                405                 410                 415
Ser Gly Asp Gly His His Ala Asp Pro Ile Asp Glu His Lys Pro Val
            420                 425                 430
Gly Ile Gly His Ser His Ser Asn Tyr Glu Leu Phe Lys Pro Glu Glu
        435                 440                 445
Gly Val Ala Lys Lys Glu Gly Asn Lys Val Tyr Thr Gly Glu Glu Leu
    450                 455                 460
Thr Asn Val Val Asn Leu Leu Lys Asn Ser Thr Phe Asn Asn Gln Asn
465                 470                 475                 480
Phe Thr Leu Ala Asn Gly Gln Lys Arg Val Ser Phe Ser Phe Pro Pro
                485                 490                 495
Glu Leu Glu Lys Lys Leu Gly Ile Asn Met Leu Val Lys Leu Ile Thr
            500                 505                 510
Pro Asp Gly Lys Val Leu Glu Lys Val Ser Gly Lys Val Phe Gly Glu
        515                 520                 525
Gly Val Gly Asn Ile Ala Asn Phe Glu Leu Asp Gln Pro Tyr Leu Pro
    530                 535                 540
Gly Gln Thr Phe Lys Tyr Thr Ile Ala Ser Lys Asp Tyr Pro Glu Val
545                 550                 555                 560
Ser Tyr Asp Gly Thr Phe Thr Val Pro Thr Ser Leu Ala Tyr Lys Met
                565                 570                 575
Ala Ser Gln Thr Ile Phe Tyr Pro Phe His Ala Gly Asp Thr Tyr Leu
            580                 585                 590
Arg Val Asn Pro Gln Phe Ala Val Pro Lys Gly Thr Asp Ala Leu Val
        595                 600                 605
Arg Val Phe Asp Glu Phe His Gly Asn Ala Tyr Leu Glu Asn Asn Tyr
    610                 615                 620
Lys Val Gly Glu Ile Lys Leu Pro Ile Pro Lys Leu Asn Gln Gly Thr
625                 630                 635                 640
Thr Arg Thr Ala Gly Asn Lys Ile Pro Val Thr Phe Met Ala Asn Ala
                645                 650                 655
Tyr Leu Asp Asn Gln Ser Thr Tyr Ile Val Glu Val Pro Ile Leu Glu
            660                 665                 670
Lys Glu Asn Gln Thr Asp Lys Pro Ser Ile Leu Pro Gln Phe Lys Arg
        675                 680                 685
Asn Lys Ala Gln Glu Asn Ser Lys Leu Asp Glu Lys Val Glu Glu Pro
    690                 695                 700
Lys Thr Ser Glu Lys Val Glu Lys Glu Lys Leu Ser Glu Thr Gly Asn
705                 710                 715                 720
Ser Thr Ser Asn Ser Thr Leu Glu Glu Val Pro Thr Val Asp Pro Val
                725                 730                 735
Gln Glu Lys Val Ala Lys Phe Ala Glu Ser Tyr Gly Met Lys Leu Glu
            740                 745                 750
Asn Val Leu Phe Asn Met Asp Gly Thr Ile Glu Leu Tyr Leu Pro Ser
        755                 760                 765
Gly Glu Val Ile Lys Lys Asn Met Ala Asp Phe Thr Gly Glu Ala Pro
    770                 775                 780
Gln Gly Asn Gly Glu Asn Lys Pro Ser Glu Asn Gly Lys Val Ser Thr
785                 790                 795                 800
Gly Thr Val Glu Asn Gln Pro Thr Glu Asn Lys Pro Ala Asp Ser Leu
                805                 810                 815
Pro Glu Ala Pro Asn Glu Lys Pro Val Lys Pro Glu Asn Ser Thr Asp
            820                 825                 830
Asn Gly Met Leu Asn Pro Glu Gly Asn Val Gly Ser Asp Pro Met Leu
        835                 840                 845
Asp Pro Ala Leu Glu Glu Ala Pro Ala Val Asp Pro Val Gln Glu Lys
    850                 855                 860
Leu Glu Lys Phe Thr Ala Ser Tyr Gly Leu Gly Leu Asp Ser Val Ile
865                 870                 875                 880
Phe Asn Met Asp Gly Thr Ile Glu Leu Arg Leu Pro Ser Gly Glu Val
                885                 890                 895
Ile Lys Lys Asn Leu Ser Asp Phe Ile Ala
            900                 905

<210> 373

<211> 906
<212> PRT
<213> Unknown

<220>
<223> VP 93

<400> 373
Met Gln Ile Gly Gln Pro Thr Leu Pro Asn Asn Ser Leu Ala Thr Pro
1               5                   10                  15
Ser Pro Ser Leu Pro Ile Asn Pro Gly Thr Ser His Glu Lys His Glu
            20                  25                  30
Glu Asp Gly Tyr Gly Phe Asp Ala Asn Arg Ile Ile Ala Glu Asp Glu
        35                  40                  45
Ser Gly Phe Val Met Ser His Gly Asp Ser Asn His Tyr Phe Phe Lys
    50                  55                  60
Lys Asp Leu Thr Glu Glu Gln Ile Lys Ala Ala Gln Lys His Leu Glu
65                  70                  75                  80
Glu Val Lys Thr Ser His Asn Gly Leu Asp Ser Leu Ser Ser His Glu
                85                  90                  95
Gln Asp Tyr Pro Gly Asn Ala Lys Glu Met Lys Asp Leu Asp Lys Lys
            100                 105                 110
Ile Glu Glu Lys Ile Ala Gly Ile Met Lys Gln Tyr Gly Val Lys Arg
        115                 120                 125
Glu Ser Ile Val Val Asn Lys Glu Lys Asn Ala Ile Ile Tyr Pro Ser
    130                 135                 140
Gly Asp His His His Ala Asp Pro Ile Asp Glu His Lys Pro Val Gly
145                 150                 155                 160
Ile Gly His Ser His Ser Asn Tyr Glu Leu Phe Lys Pro Glu Glu Gly
                165                 170                 175
Val Ala Lys Lys Glu Gly Asn Lys Val Tyr Thr Gly Glu Glu Leu Thr
            180                 185                 190
Asn Val Val Asn Leu Leu Lys Asn Ser Thr Phe Asn Asn Gln Asn Phe
        195                 200                 205
Thr Leu Ala Asn Gly Gln Lys Arg Val Ser Phe Ser Phe Pro Pro Glu
    210                 215                 220
Leu Glu Lys Lys Leu Gly Ile Asn Met Leu Val Lys Leu Ile Thr Pro
225                 230                 235                 240
Asp Gly Lys Val Leu Glu Lys Val Ser Gly Lys Val Phe Gly Glu Gly
                245                 250                 255
Val Gly Asn Ile Ala Asn Phe Glu Leu Asp Gln Pro Tyr Leu Pro Gly
            260                 265                 270
Gln Thr Phe Lys Tyr Thr Ile Ala Ser Lys Asp Tyr Pro Glu Val Ser
        275                 280                 285
Tyr Asp Gly Thr Phe Thr Val Pro Thr Ser Leu Ala Tyr Lys Met Ala
    290                 295                 300
Ser Gln Thr Ile Phe Tyr Pro Phe His Ala Gly Asp Thr Tyr Leu Arg
305                 310                 315                 320
Val Asn Pro Gln Phe Ala Val Pro Lys Gly Thr Asp Ala Leu Val Arg
                325                 330                 335
Val Phe Asp Glu Phe His Gly Asn Ala Tyr Leu Glu Asn Asn Tyr Lys
            340                 345                 350
Val Gly Glu Ile Lys Leu Pro Ile Pro Lys Leu Asn Gln Gly Thr Thr
        355                 360                 365
Arg Thr Ala Gly Asn Lys Ile Pro Val Thr Phe Met Ala Asn Ala Tyr
    370                 375                 380
Leu Asp Asn Gln Ser Thr Tyr Ile Val Glu Val Pro Ile Leu Glu Lys
385                 390                 395                 400
Glu Asn Gln Thr Asp Lys Pro Ser Ile Leu Pro Gln Phe Lys Arg Asn
                405                 410                 415
Lys Ala Gln Glu Asn Ser Lys Leu Asp Glu Lys Val Glu Glu Pro Lys
            420                 425                 430
Thr Ser Glu Lys Val Glu Lys Glu Lys Leu Ser Glu Thr Gly Asn Ser
        435                 440                 445
Thr Ser Asn Ser Thr Leu Glu Glu Val Pro Thr Val Asp Pro Val Gln

450                 455                 460
Glu Lys Val Ala Lys Phe Ala Glu Ser Tyr Gly Met Lys Leu Glu Asn
465                 470                 475                 480
Val Leu Phe Asn Met Asp Gly Thr Ile Glu Leu Tyr Leu Pro Ser Gly
                485                 490                 495
Glu Val Ile Lys Lys Asn Met Ala Asp Phe Thr Gly Glu Ala Pro Gln
            500                 505                 510
Gly Asn Gly Glu Asn Lys Pro Ser Glu Asn Gly Lys Val Ser Thr Gly
        515                 520                 525
Thr Val Glu Asn Gln Pro Thr Glu Asn Lys Pro Ala Asp Ser Leu Pro
    530                 535                 540
Glu Ala Pro Asn Glu Lys Pro Val Lys Pro Glu Asn Ser Thr Asp Asn
545                 550                 555                 560
Gly Met Leu Asn Pro Glu Gly Asn Val Gly Ser Asp Pro Met Leu Asp
                565                 570                 575
Pro Ala Leu Glu Glu Ala Pro Ala Val Asp Pro Val Gln Glu Lys Leu
            580                 585                 590
Glu Lys Phe Thr Ala Ser Tyr Gly Leu Gly Leu Asp Ser Val Ile Phe
        595                 600                 605
Asn Met Asp Gly Thr Ile Glu Leu Arg Leu Pro Ser Gly Glu Val Ile
    610                 615                 620
Lys Lys Asn Leu Ser Asp Phe Ile Ala Gly Pro Gln Ile Thr Tyr Thr
625                 630                 635                 640
Asp Asp Glu Ile Gln Val Ala Lys Leu Ala Gly Lys Tyr Thr Thr Glu
                645                 650                 655
Asp Gly Tyr Ile Phe Asp Thr Ser Trp Ile Lys Lys Asp Ser Leu Ser
            660                 665                 670
Glu Ala Glu Arg Ala Ala Ala Gln Ala Tyr Ala Lys Glu Lys Gly Leu
        675                 680                 685
Thr Pro Pro Ser Thr Asp His Gln Asp Ser Gly Asn Thr Glu Ala Lys
    690                 695                 700
Gly Ala Glu Ala Ile Tyr Asn Arg Val Lys Ala Ala Lys Lys Val Pro
705                 710                 715                 720
Leu Asp Arg Met Pro Tyr Asn Leu Gln Tyr Thr Val Glu Val Lys Asn
                725                 730                 735
Gly Ser Leu Ile Ile Pro Ser Tyr Asp His Tyr His Asn Ile Lys Phe
            740                 745                 750
Glu Trp Phe Asp Glu Gly Leu Tyr Glu Ala Pro Lys Gly Tyr Ser Leu
        755                 760                 765
Glu Asp Leu Leu Ala Thr Val Lys Tyr Tyr Val Glu Pro Arg Asn Ala
    770                 775                 780
Ser Asp His Val Arg Lys Asn Lys Ala Asp Gln Asp Ser Lys Pro Asp
785                 790                 795                 800
Glu Asp Lys Glu His Asp Glu Val Ser Glu Pro Thr His Pro Glu Ser
                805                 810                 815
Asp Glu Lys Glu Asn His Ala Gly Leu Asn Pro Ser Ala Asp Asn Leu
            820                 825                 830
Tyr Lys Pro Ser Thr Asp Thr Glu Glu Thr Glu Glu Glu Ala Glu Asp
        835                 840                 845
Thr Thr Asp Glu Ala Glu Ile Pro Gly Thr Pro Ser Ile Arg Gln Asn
    850                 855                 860
Ala Met Glu Thr Leu Thr Gly Leu Lys Ser Ser Leu Leu Leu Gly Thr
865                 870                 875                 880
Lys Asp Asn Asn Thr Ile Ser Ala Glu Val Asp Ser Leu Leu Ala Leu
                885                 890                 895
Leu Lys Glu Ser Gln Pro Ala Pro Ile Gln
            900                 905

<210> 374

<211> 259
<212> PRT
<213> Unknown

<220>
<223> VP43S

<400> 374
Met Leu Ala Gly Lys Tyr Thr Thr Glu Asp Gly Tyr Ile Phe Asp Thr
1               5                   10                  15
Ser Trp Ile Lys Lys Asp Ser Leu Ser Glu Ala Glu Arg Ala Ala Ala
            20                  25                  30
Gln Ala Tyr Ala Lys Glu Lys Gly Leu Thr Pro Pro Ser Thr Asp His
        35                  40                  45
Gln Asp Ser Gly Asn Thr Glu Ala Lys Gly Ala Glu Ala Ile Tyr Asn
    50                  55                  60
Arg Val Lys Ala Ala Lys Lys Val Pro Leu Asp Arg Met Pro Tyr Asn
65                  70                  75                  80
Leu Gln Tyr Thr Val Glu Val Lys Asn Gly Ser Leu Ile Ile Pro His
                85                  90                  95
Tyr Asp His Tyr His Asn Ile Lys Phe Glu Trp Phe Asp Glu Gly Leu
            100                 105                 110
Tyr Glu Ala Pro Lys Gly Tyr Ser Leu Glu Asp Leu Leu Ala Thr Val
        115                 120                 125
Lys Tyr Tyr Val Glu Pro Arg Asn Ala Ser Asp His Val Arg Lys Asn
    130                 135                 140
Lys Ala Asp Gln Asp Ser Lys Pro Asp Glu Asp Lys Glu His Asp Glu
145                 150                 155                 160
Val Ser Glu Pro Thr His Pro Glu Ser Asp Glu Lys Glu Asn His Ala
                165                 170                 175
Gly Leu Asn Pro Ser Ala Asp Asn Leu Tyr Lys Pro Ser Thr Asp Thr
            180                 185                 190
Glu Glu Thr Glu Glu Glu Ala Glu Asp Thr Thr Asp Glu Ala Glu Ile
        195                 200                 205
Pro Gly Thr Pro Ser Ile Arg Gln Asn Ala Met Glu Thr Leu Thr Gly
    210                 215                 220
Leu Lys Ser Ser Leu Leu Leu Gly Thr Lys Asp Asn Asn Thr Ile Ser
225                 230                 235                 240
Ala Glu Val Asp Ser Leu Leu Ala Leu Leu Lys Glu Ser Gln Pro Ala
                245                 250                 255
Pro Ile Gln

<210> 375

<211> 268
<212> PRT
<213> Unknown

<220>
<223> NEW 86

<400> 375
Met Gln Ile Thr Tyr Thr Asp Asp Glu Ile Gln Val Ala Lys Leu Ala
1               5                   10                  15
Gly Lys Tyr Thr Thr Glu Asp Gly Tyr Ile Phe Asp Thr Ser Trp Ile
            20                  25                  30
Lys Lys Asp Ser Leu Ser Glu Ala Glu Arg Ala Ala Ala Gln Ala Tyr
        35                  40                  45
Ala Lys Glu Lys Gly Leu Thr Pro Pro Ser Thr Asp His Gln Asp Ser
    50                  55                  60
Gly Asn Thr Glu Ala Lys Gly Ala Glu Ala Ile Tyr Asn Arg Val Lys
65                  70                  75                  80
Ala Ala Lys Lys Val Pro Leu Asp Arg Met Pro Tyr Asn Leu Gln Tyr
                85                  90                  95
Thr Val Glu Val Lys Asn Gly Ser Leu Ile Ile Pro Pro Gly Asn Ile
            100                 105                 110
Lys Phe Glu Trp Phe Asp Glu Gly Leu Tyr Glu Ala Pro Lys Gly Tyr
        115                 120                 125
Ser Leu Glu Asp Leu Leu Ala Thr Val Lys Tyr Tyr Val Glu Pro Arg
    130                 135                 140
Asn Ala Ser Asp His Val Arg Lys Asn Lys Ala Asp Gln Asp Ser Lys
145                 150                 155                 160
Pro Asp Glu Asp Lys Glu His Asp Glu Val Ser Glu Pro Thr His Pro

                    165                 170                 175
Glu Ser Asp Glu Lys Glu Asn His Ala Gly Leu Asn Pro Ser Ala Asp
            180                 185                 190
Asn Leu Tyr Lys Pro Ser Thr Asp Thr Glu Glu Thr Glu Glu Glu Ala
        195                 200                 205
Glu Asp Thr Thr Asp Glu Ala Glu Ile Pro Gly Thr Pro Ser Ile Arg
    210                 215                 220
Gln Asn Ala Met Glu Thr Leu Thr Gly Leu Lys Ser Ser Leu Leu Leu
225                 230                 235                 240
Gly Thr Lys Asp Asn Asn Thr Ile Ser Ala Glu Val Asp Ser Leu Leu
                245                 250                 255
Ala Leu Leu Lys Glu Ser Gln Pro Ala Pro Ile Gln
            260                 265

<210> 376

<211> 999
<212> PRT
<213> Unknown

<220>
<223> NEW 17

<221> VARIANT
<222> (1)...(1)
<223> Xaa = Methionine or nothing

<221> VARIANT
<222> (430)...(430)
<223> Xaa = Glycine or nothing

<221> VARIANT
<222> (431)...(431)
<223> Xaa = Proline or nothing

<400> 376
Xaa Asp Ile Asp Ser Leu Leu Lys Gln Leu Tyr Lys Leu Pro Leu Ser
 1               5                  10                  15
Gln Arg His Val Glu Ser Asp Gly Leu Ile Phe Asp Pro Ala Gln Ile
            20                  25                  30
Thr Ser Arg Thr Ala Arg Gly Val Ala Val Pro His Gly Asn His Tyr
        35                  40                  45
His Phe Ile Pro Tyr Glu Gln Met Ser Glu Leu Glu Lys Arg Ile Ala
    50                  55                  60
Arg Ile Ile Pro Leu Arg Tyr Arg Ser Asn His Trp Val Pro Asp Ser
65                  70                  75                  80
Arg Pro Glu Glu Pro Ser Pro Gln Pro Thr Pro Glu Pro Ser Pro Ser
                85                  90                  95
Pro Gln Pro Ala Pro Asn Pro Gln Pro Ala Pro Ser Asn Pro Ile Asp
            100                 105                 110
Glu Lys Leu Val Lys Glu Ala Val Arg Lys Val Gly Asp Gly Tyr Val
        115                 120                 125
Phe Glu Glu Asn Gly Val Ser Arg Tyr Ile Pro Ala Lys Asn Leu Ser
    130                 135                 140
Ala Glu Thr Ala Ala Gly Ile Asp Ser Lys Leu Ala Lys Gln Glu Ser
145                 150                 155                 160
Leu Ser His Lys Leu Gly Ala Lys Lys Thr Asp Leu Pro Ser Ser Asp
                165                 170                 175
Arg Glu Phe Tyr Asn Lys Ala Tyr Asp Leu Leu Ala Arg Ile His Gln
            180                 185                 190
Asp Leu Leu Asp Asn Lys Gly Arg Gln Val Asp Phe Glu Ala Leu Asp
        195                 200                 205
Asn Leu Leu Glu Arg Leu Lys Asp Val Ser Ser Asp Lys Val Lys Leu
    210                 215                 220
Val Asp Asp Ile Leu Ala Phe Leu Ala Pro Ile Arg His Pro Glu Arg
225                 230                 235                 240
Leu Gly Lys Pro Asn Ala Gln Ile Thr Tyr Thr Asp Asp Glu Ile Gln

                245                 250                 255
Val Ala Lys Leu Ala Gly Lys Tyr Thr Thr Glu Asp Gly Tyr Ile Phe
            260                 265                 270
Asp Pro Arg Asp Ile Thr Ser Asp Glu Gly Asp Ala Tyr Val Thr Pro
        275                 280                 285
His Met Thr His Ser His Trp Ile Lys Lys Asp Ser Leu Ser Glu Ala
    290                 295                 300
Glu Arg Ala Ala Ala Gln Ala Tyr Ala Lys Glu Lys Gly Leu Thr Pro
305                 310                 315                 320
Pro Ser Thr Asp His Gln Asp Ser Gly Asn Thr Glu Ala Lys Gly Ala
                325                 330                 335
Glu Ala Ile Tyr Asn Arg Val Lys Ala Ala Lys Lys Val Pro Leu Asp
            340                 345                 350
Arg Met Pro Tyr Asn Leu Gln Tyr Thr Val Glu Val Lys Asn Gly Ser
        355                 360                 365
Leu Ile Ile Pro His Tyr Asp His Tyr His Asn Ile Lys Phe Glu Trp
    370                 375                 380
Phe Asp Glu Gly Leu Tyr Glu Ala Pro Lys Gly Tyr Thr Leu Glu Asp
385                 390                 395                 400
Leu Leu Ala Thr Val Lys Tyr Tyr Val Glu His Pro Asn Glu Arg Pro
                405                 410                 415
His Ser Asp Asn Gly Phe Gly Asn Ala Ser Asp His Val Xaa Xaa Asp
            420                 425                 430
Leu Thr Glu Glu Gln Ile Lys Ala Ala Gln Lys His Leu Glu Glu Val
        435                 440                 445
Lys Thr Ser His Asn Gly Leu Asp Ser Leu Ser Ser His Glu Gln Asp
    450                 455                 460
Tyr Pro Gly Asn Ala Lys Glu Met Lys Asp Leu Asp Lys Lys Ile Glu
465                 470                 475                 480
Glu Lys Ile Ala Gly Ile Met Lys Gln Tyr Gly Val Lys Arg Glu Ser
                485                 490                 495
Ile Val Val Asn Lys Glu Lys Asn Ala Ile Ile Tyr Pro His Gly Asp
            500                 505                 510
His His His Ala Asp Pro Ile Asp Glu His Lys Pro Val Gly Ile Gly
        515                 520                 525
His Ser His Ser Asn Tyr Glu Leu Phe Lys Pro Glu Glu Gly Val Ala
    530                 535                 540
Lys Lys Glu Gly Asn Lys Val Tyr Thr Gly Glu Glu Leu Thr Asn Val
545                 550                 555                 560
Val Asn Leu Leu Lys Asn Ser Thr Phe Asn Asn Gln Asn Phe Thr Leu
                565                 570                 575
Ala Asn Gly Gln Lys Arg Val Ser Phe Ser Phe Pro Pro Glu Leu Glu
            580                 585                 590
Lys Lys Leu Gly Ile Asn Met Leu Val Lys Leu Ile Thr Pro Asp Gly
        595                 600                 605
Lys Val Leu Glu Lys Val Ser Gly Lys Val Phe Gly Glu Gly Val Gly
    610                 615                 620
Asn Ile Ala Asn Phe Glu Leu Asp Gln Pro Tyr Leu Pro Gly Gln Thr
625                 630                 635                 640
Phe Lys Tyr Thr Ile Ala Ser Lys Asp Tyr Pro Glu Val Ser Tyr Asp
                645                 650                 655
Gly Thr Phe Thr Val Pro Thr Ser Leu Ala Tyr Lys Met Ala Ser Gln
            660                 665                 670
Thr Ile Phe Tyr Pro Phe His Ala Gly Asp Thr Tyr Leu Arg Val Asn
        675                 680                 685
Pro Gln Phe Ala Val Pro Lys Gly Thr Asp Ala Leu Val Arg Val Phe
    690                 695                 700
Asp Glu Phe His Gly Asn Ala Tyr Leu Glu Asn Asn Tyr Lys Val Gly
705                 710                 715                 720
Glu Ile Lys Leu Pro Ile Pro Lys Leu Asn Gln Gly Thr Thr Arg Thr
                725                 730                 735
Ala Gly Asn Lys Ile Pro Val Thr Phe Met Ala Asn Ala Tyr Leu Asp
            740                 745                 750
Asn Gln Ser Thr Tyr Ile Val Glu Val Pro Ile Leu Glu Lys Glu Asn
        755                 760                 765
Gln Thr Asp Lys Pro Ser Ile Leu Pro Gln Phe Lys Arg Asn Lys Ala
    770                 775                 780
Gln Glu Asn Ser Lys Leu Asp Glu Lys Val Glu Glu Pro Lys Thr Ser

785                 790                 795                 800
Glu Lys Val Glu Lys Glu Lys Leu Ser Glu Thr Gly Asn Ser Thr Ser
                805                 810                 815
Asn Ser Thr Leu Glu Glu Val Pro Thr Val Asp Pro Val Gln Glu Lys
            820                 825                 830
Val Ala Lys Phe Ala Glu Ser Tyr Gly Met Lys Leu Glu Asn Val Leu
        835                 840                 845
Phe Asn Met Asp Gly Thr Ile Glu Leu Tyr Leu Pro Ser Gly Glu Val
    850                 855                 860
Ile Lys Lys Asn Met Ala Asp Phe Thr Gly Glu Ala Pro Gln Gly Asn
865                 870                 875                 880
Gly Glu Asn Lys Pro Ser Glu Asn Gly Lys Val Ser Thr Gly Thr Val
                885                 890                 895
Glu Asn Gln Pro Thr Glu Asn Lys Pro Ala Asp Ser Leu Pro Glu Ala
            900                 905                 910
Pro Asn Glu Lys Pro Val Lys Pro Glu Asn Ser Thr Asp Asn Gly Met
        915                 920                 925
Leu Asn Pro Glu Gly Asn Val Gly Ser Asp Pro Met Leu Asp Pro Ala
    930                 935                 940
Leu Glu Glu Ala Pro Ala Val Asp Pro Val Gln Glu Lys Leu Glu Lys
945                 950                 955                 960
Phe Thr Ala Ser Tyr Gly Leu Gly Leu Asp Ser Val Ile Phe Asn Met
                965                 970                 975
Asp Gly Thr Ile Glu Leu Arg Leu Pro Ser Gly Glu Val Ile Lys Lys
            980                 985                 990
Asn Leu Ser Asp Phe Ile Ala
        995

<210> 377

<211> 999
<212> PRT
<213> Unknown

<220>
<223> NEW 20

<221> VARIANT
<222> (1)...(1)
<223> Xaa = Methionine or nothing

<221> VARIANT
<222> (570)...(570)
<223> Xaa = Glycine or nothing

<221> VARIANT
<222> (571)...(571)
<223> Xaa = Proline or nothing

<400> 377
Xaa Asp Leu Thr Glu Glu Gln Ile Lys Ala Ala Gln Lys His Leu Glu
 1               5                  10                  15
Glu Val Lys Thr Ser His Asn Gly Leu Asp Ser Leu Ser Ser His Glu
            20                  25                  30
Gln Asp Tyr Pro Gly Asn Ala Lys Glu Met Lys Asp Leu Asp Lys Lys
        35                  40                  45
Ile Glu Glu Lys Ile Ala Gly Ile Met Lys Gln Tyr Gly Val Lys Arg
    50                  55                  60
Glu Ser Ile Val Val Asn Lys Glu Lys Asn Ala Ile Ile Tyr Pro His
65                  70                  75                  80
Gly Asp His His His Ala Asp Pro Ile Asp Glu His Lys Pro Val Gly
                85                  90                  95
Ile Gly His Ser His Ser Asn Tyr Glu Leu Phe Lys Pro Glu Glu Gly
            100                 105                 110
Val Ala Lys Lys Glu Gly Asn Lys Val Tyr Thr Gly Glu Glu Leu Thr
        115                 120                 125
Asn Val Val Asn Leu Leu Lys Asn Ser Thr Phe Asn Asn Gln Asn Phe

130                 135                 [?]                 140
Thr Leu Ala Asn Gly Gln Lys Arg Val Ser Phe Ser Phe Pro Pro Glu
145                 150                 155                 160
Leu Glu Lys Lys Leu Gly Ile Asn Met Leu Val Lys Leu Ile Thr Pro
                165                 170                 175
Asp Gly Lys Val Leu Glu Lys Val Ser Gly Lys Val Phe Gly Glu Gly
            180                 185                 190
Val Gly Asn Ile Ala Asn Phe Glu Leu Asp Gln Pro Tyr Leu Pro Gly
        195                 200                 205
Gln Thr Phe Lys Tyr Thr Ile Ala Ser Lys Asp Tyr Pro Glu Val Ser
    210                 215                 220
Tyr Asp Gly Thr Phe Thr Val Pro Thr Ser Leu Ala Tyr Lys Met Ala
225                 230                 235                 240
Ser Gln Thr Ile Phe Tyr Pro Phe His Ala Gly Asp Thr Tyr Leu Arg
                245                 250                 255
Val Asn Pro Gln Phe Ala Val Pro Lys Gly Thr Asp Ala Leu Val Arg
            260                 265                 270
Val Phe Asp Glu Phe His Gly Asn Ala Tyr Leu Glu Asn Asn Tyr Lys
        275                 280                 285
Val Gly Glu Ile Lys Leu Pro Ile Pro Lys Leu Asn Gln Gly Thr Thr
    290                 295                 300
Arg Thr Ala Gly Asn Lys Ile Pro Val Thr Phe Met Ala Asn Ala Tyr
305                 310                 315                 320
Leu Asp Asn Gln Ser Thr Tyr Ile Val Glu Val Pro Ile Leu Glu Lys
                325                 330                 335
Glu Asn Gln Thr Asp Lys Pro Ser Ile Leu Pro Gln Phe Lys Arg Asn
            340                 345                 350
Lys Ala Gln Glu Asn Ser Lys Leu Asp Glu Lys Val Glu Glu Pro Lys
        355                 360                 365
Thr Ser Glu Lys Val Glu Lys Glu Lys Leu Ser Glu Thr Gly Asn Ser
    370                 375                 380
Thr Ser Asn Ser Thr Leu Glu Glu Val Pro Thr Val Asp Pro Val Gln
385                 390                 395                 400
Glu Lys Val Ala Lys Phe Ala Glu Ser Tyr Gly Met Lys Leu Glu Asn
                405                 410                 415
Val Leu Phe Asn Met Asp Gly Thr Ile Glu Leu Tyr Leu Pro Ser Gly
            420                 425                 430
Glu Val Ile Lys Lys Asn Met Ala Asp Phe Thr Gly Glu Ala Pro Gln
        435                 440                 445
Gly Asn Gly Glu Asn Lys Pro Ser Glu Asn Gly Lys Val Ser Thr Gly
    450                 455                 460
Thr Val Glu Asn Gln Pro Thr Glu Asn Lys Pro Ala Asp Ser Leu Pro
465                 470                 475                 480
Glu Ala Pro Asn Glu Lys Pro Val Lys Pro Glu Asn Ser Thr Asp Asn
                485                 490                 495
Gly Met Leu Asn Pro Glu Gly Asn Val Gly Ser Asp Pro Met Leu Asp
            500                 505                 510
Pro Ala Leu Glu Glu Ala Pro Ala Val Asp Pro Val Gln Glu Lys Leu
        515                 520                 525
Glu Lys Phe Thr Ala Ser Tyr Gly Leu Gly Leu Asp Ser Val Ile Phe
    530                 535                 540
Asn Met Asp Gly Thr Ile Glu Leu Arg Leu Pro Ser Gly Glu Val Ile
545                 550                 555                 560
Lys Lys Asn Leu Ser Asp Phe Ile Ala Xaa Xaa Asp Ile Asp Ser Leu
                565                 570                 575
Leu Lys Gln Leu Tyr Lys Leu Pro Leu Ser Gln Arg His Val Glu Ser
            580                 585                 590
Asp Gly Leu Ile Phe Asp Pro Ala Gln Ile Thr Ser Arg Thr Ala Arg
        595                 600                 605
Gly Val Ala Val Pro His Gly Asn His Tyr His Phe Ile Pro Tyr Glu
    610                 615                 620
Gln Met Ser Glu Leu Glu Lys Arg Ile Ala Arg Ile Ile Pro Leu Arg
625                 630                 635                 640
Tyr Arg Ser Asn His Trp Val Pro Asp Ser Arg Pro Glu Glu Pro Ser
                645                 650                 655
Pro Gln Pro Thr Pro Glu Pro Ser Pro Ser Pro Gln Pro Ala Pro Asn
            660                 665                 670
Pro Gln Pro Ala Pro Ser Asn Pro Ile Asp Glu Lys Leu Val Lys Glu

675                 680                 685
Ala Val Arg Lys Val Gly Asp Gly Tyr Val Phe Glu Glu Asn Gly Val
    690                 695                 700
Ser Arg Tyr Ile Pro Ala Lys Asn Leu Ser Ala Glu Thr Ala Ala Gly
705                 710                 715                 720
Ile Asp Ser Lys Leu Ala Lys Gln Glu Ser Leu Ser His Lys Leu Gly
                725                 730                 735
Ala Lys Lys Thr Asp Leu Pro Ser Ser Asp Arg Glu Phe Tyr Asn Lys
            740                 745                 750
Ala Tyr Asp Leu Leu Ala Arg Ile His Gln Asp Leu Leu Asp Asn Lys
        755                 760                 765
Gly Arg Gln Val Asp Phe Glu Ala Leu Asp Asn Leu Leu Glu Arg Leu
    770                 775                 780
Lys Asp Val Ser Ser Asp Lys Val Lys Leu Val Asp Asp Ile Leu Ala
785                 790                 795                 800
Phe Leu Ala Pro Ile Arg His Pro Glu Arg Leu Gly Lys Pro Asn Ala
                805                 810                 815
Gln Ile Thr Tyr Thr Asp Asp Glu Ile Gln Val Ala Lys Leu Ala Gly
            820                 825                 830
Lys Tyr Thr Thr Glu Asp Gly Tyr Ile Phe Asp Pro Arg Asp Ile Thr
        835                 840                 845
Ser Asp Glu Gly Asp Ala Tyr Val Thr Pro His Met Thr His Ser His
    850                 855                 860
Trp Ile Lys Lys Asp Ser Leu Ser Glu Ala Glu Arg Ala Ala Ala Gln
865                 870                 875                 880
Ala Tyr Ala Lys Glu Lys Gly Leu Thr Pro Pro Ser Thr Asp His Gln
                885                 890                 895
Asp Ser Gly Asn Thr Glu Ala Lys Gly Ala Glu Ala Ile Tyr Asn Arg
            900                 905                 910
Val Lys Ala Ala Lys Lys Val Pro Leu Asp Arg Met Pro Tyr Asn Leu
        915                 920                 925
Gln Tyr Thr Val Glu Val Lys Asn Gly Ser Leu Ile Ile Pro His Tyr
    930                 935                 940
Asp His Tyr His Asn Ile Lys Phe Glu Trp Phe Asp Glu Gly Leu Tyr
945                 950                 955                 960
Glu Ala Pro Lys Gly Tyr Thr Leu Glu Asp Leu Leu Ala Thr Val Lys
                965                 970                 975
Tyr Tyr Val Glu His Pro Asn Glu Arg Pro His Ser Asp Asn Gly Phe
            980                 985                 990
Gly Asn Ala Ser Asp His Val
        995

<210> 378

<211> 1378
<212> PRT
<213> Unknown

<220>
<223> NEW 26

<221> VARIANT
<222> (1)...(1)
<223> Xaa = Methionine or nothing

<221> VARIANT
<222> (570)...(570)
<223> Xaa = Glycine or nothing

<221> VARIANT
<222> (571)...(571)
<223> Xaa = Proline or nothing

<400> 378
Xaa Glu Asn Ile Ser Ser Leu Leu Arg Glu Leu Tyr Ala Lys Pro Leu
 1               5                  10                  15
Ser Glu Arg His Val Glu Ser Asp Gly Leu Ile Phe Asp Pro Ala Gln

            20                  25                  30
Ile Thr Ser Arg Thr Ala Arg Gly Val Ala Val Pro His Gly Asn His
        35                  40                  45
Tyr His Phe Ile Pro Tyr Glu Gln Met Ser Glu Leu Glu Lys Arg Ile
    50                  55                  60
Ala Arg Ile Ile Pro Leu Arg Tyr Arg Ser Asn His Trp Val Pro Asp
65                  70                  75                  80
Ser Arg Pro Glu Gln Pro Ser Pro Gln Ser Thr Pro Glu Pro Ser Pro
                85                  90                  95
Ser Leu Gln Pro Ala Pro Asn Pro Gln Pro Ala Pro Ser Asn Pro Ile
            100                 105                 110
Asp Glu Lys Leu Val Lys Glu Ala Val Arg Lys Val Gly Asp Gly Tyr
        115                 120                 125
Val Phe Glu Glu Asn Gly Val Ser Arg Tyr Ile Pro Ala Lys Asp Leu
    130                 135                 140
Ser Ala Glu Thr Ala Ala Gly Ile Asp Ser Lys Leu Ala Lys Gln Glu
145                 150                 155                 160
Ser Leu Ser His Lys Leu Gly Ala Lys Lys Thr Asp Leu Pro Ser Ser
                165                 170                 175
Asp Arg Glu Phe Tyr Asn Lys Ala Tyr Asp Leu Leu Ala Arg Ile His
            180                 185                 190
Gln Asp Leu Leu Asp Asn Lys Gly Arg Gln Val Asp Phe Glu Val Leu
        195                 200                 205
Asp Asn Leu Leu Glu Arg Leu Lys Asp Val Ser Ser Asp Lys Val Lys
    210                 215                 220
Leu Val Asp Asp Ile Leu Ala Phe Leu Ala Pro Ile Arg His Pro Glu
225                 230                 235                 240
Arg Leu Gly Lys Pro Asn Ala Gln Ile Thr Tyr Thr Asp Asp Glu Ile
                245                 250                 255
Gln Val Ala Lys Leu Ala Gly Lys Tyr Thr Thr Glu Asp Gly Tyr Ile
            260                 265                 270
Phe Asp Pro Arg Asp Ile Thr Ser Asp Glu Gly Asp Ala Tyr Val Thr
        275                 280                 285
Pro His Met Thr His Ser His Trp Ile Lys Lys Asp Ser Leu Ser Glu
    290                 295                 300
Ala Glu Arg Ala Ala Ala Gln Ala Tyr Ala Lys Glu Lys Gly Leu Thr
305                 310                 315                 320
Pro Pro Ser Thr Asp His Gln Asp Ser Gly Asn Thr Glu Ala Lys Gly
                325                 330                 335
Ala Glu Ala Ile Tyr Asn Arg Val Lys Ala Ala Lys Lys Val Pro Leu
            340                 345                 350
Asp Arg Met Pro Tyr Asn Leu Gln Tyr Thr Val Glu Val Lys Asn Gly
        355                 360                 365
Ser Leu Ile Ile Pro His Tyr Asp His Tyr His Asn Ile Lys Phe Glu
    370                 375                 380
Trp Phe Asp Glu Gly Leu Tyr Glu Ala Pro Lys Gly Tyr Ser Leu Glu
385                 390                 395                 400
Asp Leu Leu Ala Thr Val Lys Tyr Tyr Val Glu His Pro Asn Glu Arg
                405                 410                 415
Pro His Ser Asp Asn Gly Phe Gly Asn Ala Ser Asp His Val Arg Lys
            420                 425                 430
Asn Lys Ala Asp Gln Asp Ser Lys Pro Asp Glu Asp Lys Glu His Asp
        435                 440                 445
Glu Val Ser Glu Pro Thr His Pro Glu Ser Asp Glu Lys Glu Asn His
    450                 455                 460
Ala Gly Leu Asn Pro Ser Ala Asp Asn Leu Tyr Lys Pro Ser Thr Asp
465                 470                 475                 480
Thr Glu Glu Thr Glu Glu Glu Ala Glu Asp Thr Thr Asp Glu Ala Glu
                485                 490                 495
Ile Pro Gln Val Glu Asn Ser Val Ile Asn Ala Lys Ile Ala Asp Ala
            500                 505                 510
Glu Ala Leu Leu Glu Lys Val Thr Asp Pro Ser Ile Arg Gln Asn Ala
        515                 520                 525
Met Glu Thr Leu Thr Gly Leu Lys Ser Ser Leu Leu Leu Gly Thr Lys
    530                 535                 540
Asp Asn Asn Thr Ile Ser Ala Glu Val Asp Ser Leu Leu Ala Leu Leu
545                 550                 555                 560
Lys Glu Ser Gln Pro Ala Pro Ile Gln Xaa Xaa Asn Met Gln Pro Ser

                565                 570                 575
Gln Leu Ser Tyr Ser Ser Thr Ala Ser Asp Asn Asn Thr Gln Ser Val
            580                 585                 590
Ala Lys Gly Ser Thr Ser Lys Pro Ala Asn Lys Ser Glu Asn Leu Gln
        595                 600                 605
Ser Leu Leu Lys Glu Leu Tyr Asp Ser Pro Ser Ala Gln Arg Tyr Ser
    610                 615                 620
Glu Ser Asp Gly Leu Val Phe Asp Pro Ala Lys Ile Ile Ser Arg Thr
625                 630                 635                 640
Pro Asn Gly Val Ala Ile Pro His Gly Asp His Tyr His Phe Ile Pro
                645                 650                 655
Tyr Ser Lys Leu Ser Ala Leu Glu Glu Lys Ile Ala Arg Met Val Pro
            660                 665                 670
Ile Ser Gly Thr Gly Ser Thr Val Ser Thr Asn Ala Lys Pro Asn Glu
        675                 680                 685
Val Val Ser Ser Leu Gly Ser Leu Ser Ser Asn Pro Ser Ser Leu Thr
    690                 695                 700
Thr Ser Lys Glu Leu Ser Ser Ala Ser Asp Gly Tyr Ile Phe Asn Pro
705                 710                 715                 720
Lys Asp Ile Val Glu Glu Thr Ala Thr Ala Tyr Ile Val Arg His Gly
                725                 730                 735
Asp His Phe His Tyr Ile Pro Lys Ser Asn Gln Ile Gly Gln Pro Thr
            740                 745                 750
Leu Pro Asn Asn Ser Leu Ala Thr Pro Ser Pro Ser Leu Pro Ile Asn
        755                 760                 765
Pro Gly Thr Ser His Glu Lys His Glu Glu Asp Gly Tyr Gly Phe Asp
    770                 775                 780
Ala Asn Arg Ile Ile Ala Glu Asp Glu Ser Gly Phe Val Met Ser His
785                 790                 795                 800
Gly Asp His Asn His Tyr Phe Phe Lys Lys Asp Leu Thr Glu Glu Gln
                805                 810                 815
Ile Lys Ala Ala Gln Lys His Leu Glu Glu Val Lys Thr Ser His Asn
            820                 825                 830
Gly Leu Asp Ser Leu Ser Ser His Glu Gln Asp Tyr Pro Gly Asn Ala
        835                 840                 845
Lys Glu Met Lys Asp Leu Asp Lys Lys Ile Glu Glu Lys Ile Ala Gly
    850                 855                 860
Ile Met Lys Gln Tyr Gly Val Lys Arg Glu Ser Ile Val Val Asn Lys
865                 870                 875                 880
Glu Lys Asn Ala Ile Ile Tyr Pro His Gly Asp His His His Ala Asp
                885                 890                 895
Pro Ile Asp Glu His Lys Pro Val Gly Ile Gly His Ser His Ser Asn
            900                 905                 910
Tyr Glu Leu Phe Lys Pro Glu Glu Gly Val Ala Lys Lys Glu Gly Asn
        915                 920                 925
Lys Val Tyr Thr Gly Glu Glu Leu Thr Asn Val Val Asn Leu Leu Lys
    930                 935                 940
Asn Ser Thr Phe Asn Asn Gln Asn Phe Thr Leu Ala Asn Gly Gln Lys
945                 950                 955                 960
Arg Val Ser Phe Ser Phe Pro Pro Glu Leu Glu Lys Lys Leu Gly Ile
                965                 970                 975

Asn Met Leu Val Lys Leu Ile Thr Pro Asp Gly Lys Val Leu Glu Lys
            980                 985                 990
Val Ser Gly Lys Val Phe Gly Glu Gly Val Gly Asn Ile Ala Asn Phe
        995                 1000                1005
Glu Leu Asp Gln Pro Tyr Leu Pro Gly Gln Thr Phe Lys Tyr Thr Ile
    1010                1015                1020
Ala Ser Lys Asp Tyr Pro Glu Val Ser Tyr Asp Gly Thr Phe Thr Val
1025                1030                1035                1040
Pro Thr Ser Leu Ala Tyr Lys Met Ala Ser Gln Thr Ile Phe Tyr Pro
                1045                1050                1055
Phe His Ala Gly Asp Thr Tyr Leu Arg Val Asn Pro Gln Phe Ala Val
            1060                1065                1070
Pro Lys Gly Thr Asp Ala Leu Val Arg Val Phe Asp Glu Phe His Gly
        1075                1080                1085
Asn Ala Tyr Leu Glu Asn Asn Tyr Lys Val Gly Glu Ile Lys Leu Pro
    1090                1095                1100

Ile Pro Lys Leu Asn Gln Gly Thr Thr Arg Thr Ala Gly Asn Lys Ile
1105                1110                1115                1120
Pro Val Thr Phe Met Ala Asn Ala Tyr Leu Asp Asn Gln Ser Thr Tyr
                1125                1130                1135
Ile Val Glu Val Pro Ile Leu Glu Lys Glu Asn Gln Thr Asp Lys Pro
            1140                1145                1150
Ser Ile Leu Pro Gln Phe Lys Arg Asn Lys Ala Gln Glu Asn Ser Lys
        1155                1160                1165
Leu Asp Glu Lys Val Glu Glu Pro Lys Thr Ser Glu Lys Val Glu Lys
    1170                1175                1180
Glu Lys Leu Ser Glu Thr Gly Asn Ser Thr Ser Asn Ser Thr Leu Glu
1185                1190                1195                1200
Glu Val Pro Thr Val Asp Pro Val Gln Glu Lys Val Ala Lys Phe Ala
                1205                1210                1215
Glu Ser Tyr Gly Met Lys Leu Glu Asn Val Leu Phe Asn Met Asp Gly
            1220                1225                1230
Thr Ile Glu Leu Tyr Leu Pro Ser Gly Glu Val Ile Lys Lys Asn Met
        1235                1240                1245
Ala Asp Phe Thr Gly Glu Ala Pro Gln Gly Asn Gly Glu Asn Lys Pro
    1250                1255                1260
Ser Glu Asn Gly Lys Val Ser Thr Gly Thr Val Glu Asn Gln Pro Thr
1265                1270                1275                1280
Glu Asn Lys Pro Ala Asp Ser Leu Pro Glu Ala Pro Asn Glu Lys Pro
                1285                1290                1295
Val Lys Pro Glu Asn Ser Thr Asp Asn Gly Met Leu Asn Pro Glu Gly
            1300                1305                1310
Asn Val Gly Ser Asp Pro Met Leu Asp Pro Ala Leu Glu Glu Ala Pro
        1315                1320                1325
Ala Val Asp Pro Val Gln Glu Lys Leu Glu Lys Phe Thr Ala Ser Tyr
    1330                1335                1340
Gly Leu Gly Leu Asp Ser Val Ile Phe Asn Met Asp Gly Thr Ile Glu
1345                1350                1355                1360
Leu Arg Leu Pro Ser Gly Glu Val Ile Lys Lys Asn Leu Ser Asp Phe
                1365                1370                1375
Ile Ala

<210> 379

<211> 1152
<212> PRT
<213> Unknown

<220>
<223> NEW 27

<221> VARIANT
<222> (1)...(1)
<223> Xaa = Methionine or nothing

<221> VARIANT
<222> (344)...(344)
<223> Xaa = Glycine or nothing

<221> VARIANT
<222> (345)...(345)
<223> Xaa = Proline or nothing

<400> 379
Xaa Asp Ile Leu Ala Phe Leu Ala Pro Ile Arg His Pro Glu Arg Leu
 1               5                  10                  15
Gly Lys Pro Asn Ala Gln Ile Thr Tyr Thr Asp Asp Glu Ile Gln Val
            20                  25                  30
Ala Lys Leu Ala Gly Lys Tyr Thr Thr Glu Asp Gly Tyr Ile Phe Asp
        35                  40                  45
Pro Arg Asp Ile Thr Ser Asp Glu Gly Asp Ala Tyr Val Thr Pro His
    50                  55                  60

Met Thr His Ser His Trp Ile Lys Lys Asp Ser Leu Ser Glu Ala Glu
65                  70                  75                  80
Arg Ala Ala Ala Gln Ala Tyr Ala Lys Glu Lys Gly Leu Thr Pro Pro
                85                  90                  95
Ser Thr Asp His Gln Asp Ser Gly Asn Thr Glu Ala Lys Gly Ala Glu
            100                 105                 110
Ala Ile Tyr Asn Arg Val Lys Ala Ala Lys Lys Val Pro Leu Asp Arg
        115                 120                 125
Met Pro Tyr Asn Leu Gln Tyr Thr Val Glu Val Lys Asn Gly Ser Leu
    130                 135                 140
Ile Ile Pro His Tyr Asp His Tyr His Asn Ile Lys Phe Glu Trp Phe
145                 150                 155                 160
Asp Glu Gly Leu Tyr Glu Ala Pro Lys Gly Tyr Ser Leu Glu Asp Leu
                165                 170                 175
Leu Ala Thr Val Lys Tyr Tyr Val Glu His Pro Asn Glu Arg Pro His
            180                 185                 190
Ser Asp Asn Gly Phe Gly Asn Ala Ser Asp His Val Arg Lys Asn Lys
        195                 200                 205
Ala Asp Gln Asp Ser Lys Pro Asp Glu Asp Lys Glu His Asp Glu Val
    210                 215                 220
Ser Glu Pro Thr His Pro Glu Ser Asp Glu Lys Glu Asn His Ala Gly
225                 230                 235                 240
Leu Asn Pro Ser Ala Asp Asn Leu Tyr Lys Pro Ser Thr Asp Thr Glu
                245                 250                 255
Glu Thr Glu Glu Glu Ala Glu Asp Thr Thr Asp Glu Ala Glu Ile Pro
            260                 265                 270
Gln Val Glu Asn Ser Val Ile Asn Ala Lys Ile Ala Asp Ala Glu Ala
        275                 280                 285
Leu Leu Glu Lys Val Thr Asp Pro Ser Ile Arg Gln Asn Ala Met Glu
    290                 295                 300
Thr Leu Thr Gly Leu Lys Ser Ser Leu Leu Leu Gly Thr Lys Asp Asn
305                 310                 315                 320
Asn Thr Ile Ser Ala Glu Val Asp Ser Leu Leu Ala Leu Leu Lys Glu
                325                 330                 335
Ser Gln Pro Ala Pro Ile Gln Xaa Xaa Asn Met Gln Pro Ser Gln Leu
            340                 345                 350
Ser Tyr Ser Ser Thr Ala Ser Asp Asn Asn Thr Gln Ser Val Ala Lys
        355                 360                 365
Gly Ser Thr Ser Lys Pro Ala Asn Lys Ser Glu Asn Leu Gln Ser Leu
    370                 375                 380
Leu Lys Glu Leu Tyr Asp Ser Pro Ser Ala Gln Arg Tyr Ser Glu Ser
385                 390                 395                 400
Asp Gly Leu Val Phe Asp Pro Ala Lys Ile Ile Ser Arg Thr Pro Asn
                405                 410                 415
Gly Val Ala Ile Pro His Gly Asp His Tyr His Phe Ile Pro Tyr Ser
            420                 425                 430
Lys Leu Ser Ala Leu Glu Glu Lys Ile Ala Arg Met Val Pro Ile Ser
        435                 440                 445
Gly Thr Gly Ser Thr Val Ser Thr Asn Ala Lys Pro Asn Glu Val Val
    450                 455                 460
Ser Ser Leu Gly Ser Leu Ser Ser Asn Pro Ser Ser Leu Thr Thr Ser
465                 470                 475                 480
Lys Glu Leu Ser Ser Ala Ser Asp Gly Tyr Ile Phe Asn Pro Lys Asp
                485                 490                 495
Ile Val Glu Glu Thr Ala Thr Ala Tyr Ile Val Arg His Gly Asp His
            500                 505                 510
Phe His Tyr Ile Pro Lys Ser Asn Gln Ile Gly Gln Pro Thr Leu Pro
        515                 520                 525
Asn Asn Ser Leu Ala Thr Pro Ser Pro Ser Leu Pro Ile Asn Pro Gly
    530                 535                 540
Thr Ser His Glu Lys His Glu Glu Asp Gly Tyr Gly Phe Asp Ala Asn
545                 550                 555                 560
Arg Ile Ile Ala Glu Asp Glu Ser Gly Phe Val Met Ser His Gly Asp
                565                 570                 575
His Asn His Tyr Phe Phe Lys Lys Asp Leu Thr Glu Glu Gln Ile Lys
            580                 585                 590
Ala Ala Gln Lys His Leu Glu Glu Val Lys Thr Ser His Asn Gly Leu
        595                 600                 605

Asp Ser Leu Ser Ser His Glu Gln Asp Tyr Pro Gly Asn Ala Lys Glu
    610                 615                 620
Met Lys Asp Leu Asp Lys Lys Ile Glu Glu Lys Ile Ala Gly Ile Met
625                 630                 635                 640
Lys Gln Tyr Gly Val Lys Arg Glu Ser Ile Val Val Asn Lys Glu Lys
                645                 650                 655
Asn Ala Ile Ile Tyr Pro His Gly Asp His His His Ala Asp Pro Ile
            660                 665                 670
Asp Glu His Lys Pro Val Gly Ile Gly His Ser His Ser Asn Tyr Glu
        675                 680                 685
Leu Phe Lys Pro Glu Glu Gly Val Ala Lys Lys Glu Gly Asn Lys Val
    690                 695                 700
Tyr Thr Gly Glu Glu Leu Thr Asn Val Val Asn Leu Leu Lys Asn Ser
705                 710                 715                 720
Thr Phe Asn Asn Gln Asn Phe Thr Leu Ala Asn Gly Gln Lys Arg Val
                725                 730                 735
Ser Phe Ser Phe Pro Pro Glu Leu Glu Lys Lys Leu Gly Ile Asn Met
            740                 745                 750
Leu Val Lys Leu Ile Thr Pro Asp Gly Lys Val Leu Glu Lys Val Ser
        755                 760                 765
Gly Lys Val Phe Gly Glu Gly Val Gly Asn Ile Ala Asn Phe Glu Leu
    770                 775                 780
Asp Gln Pro Tyr Leu Pro Gly Gln Thr Phe Lys Tyr Thr Ile Ala Ser
785                 790                 795                 800
Lys Asp Tyr Pro Glu Val Ser Tyr Asp Gly Thr Phe Thr Val Pro Thr
                805                 810                 815
Ser Leu Ala Tyr Lys Met Ala Ser Gln Thr Ile Phe Tyr Pro Phe His
            820                 825                 830
Ala Gly Asp Thr Tyr Leu Arg Val Asn Pro Gln Phe Ala Val Pro Lys
        835                 840                 845
Gly Thr Asp Ala Leu Val Arg Val Phe Asp Glu Phe His Gly Asn Ala
    850                 855                 860
Tyr Leu Glu Asn Asn Tyr Lys Val Gly Glu Ile Lys Leu Pro Ile Pro
865                 870                 875                 880
Lys Leu Asn Gln Gly Thr Thr Arg Thr Ala Gly Asn Lys Ile Pro Val
                885                 890                 895
Thr Phe Met Ala Asn Ala Tyr Leu Asp Asn Gln Ser Thr Tyr Ile Val
            900                 905                 910
Glu Val Pro Ile Leu Glu Lys Glu Asn Gln Thr Asp Lys Pro Ser Ile
        915                 920                 925
Leu Pro Gln Phe Lys Arg Asn Lys Ala Gln Glu Asn Ser Lys Leu Asp
    930                 935                 940
Glu Lys Val Glu Glu Pro Lys Thr Ser Glu Lys Val Glu Lys Glu Lys
945                 950                 955                 960
Leu Ser Glu Thr Gly Asn Ser Thr Ser Asn Ser Thr Leu Glu Glu Val
                965                 970                 975
Pro Thr Val Asp Pro Val Gln Glu Lys Val Ala Lys Phe Ala Glu Ser
            980                 985                 990
Tyr Gly Met Lys Leu Glu Asn Val Leu Phe Asn Met Asp Gly Thr Ile
        995                 1000                1005
Glu Leu Tyr Leu Pro Ser Gly Glu Val Ile Lys Lys Asn Met Ala Asp
    1010                1015                1020
Phe Thr Gly Glu Ala Pro Gln Gly Asn Gly Glu Asn Lys Pro Ser Glu
1025                1030                1035                1040
Asn Gly Lys Val Ser Thr Gly Thr Val Glu Asn Gln Pro Thr Glu Asn
                1045                1050                1055
Lys Pro Ala Asp Ser Leu Pro Glu Ala Pro Asn Glu Lys Pro Val Lys
            1060                1065                1070
Pro Glu Asn Ser Thr Asp Asn Gly Met Leu Asn Pro Glu Gly Asn Val
        1075                1080                1085
Gly Ser Asp Pro Met Leu Asp Pro Ala Leu Glu Glu Ala Pro Ala Val
    1090                1095                1100
Asp Pro Val Gln Glu Lys Leu Glu Lys Phe Thr Ala Ser Tyr Gly Leu
1105                1110                1115                1120
Gly Leu Asp Ser Val Ile Phe Asn Met Asp Gly Thr Ile Glu Leu Arg
                1125                1130                1135
Leu Pro Ser Gly Glu Val Ile Lys Lys Asn Leu Ser Asp Phe Ile Ala
            1140                1145                1150

<210> 380

<211> 1139
<212> PRT
<213> Unknown

<220>
<223> NEW 28

<221> VARIANT
<222> (1)...(1)
<223> Xaa = Methionine or nothing

<221> VARIANT
<222> (570)...(570)
<223> Xaa = Glycine or nothing

<221> VARIANT
<222> (571)...(571)
<223> Xaa = Proline or nothing

<400> 380
Xaa Glu Asn Ile Ser Ser Leu Leu Arg Glu Leu Tyr Ala Lys Pro Leu
1               5                   10                  15
Ser Glu Arg His Val Glu Ser Asp Gly Leu Ile Phe Asp Pro Ala Gln
            20                  25                  30
Ile Thr Ser Arg Thr Ala Arg Gly Val Ala Val Pro His Gly Asn His
        35                  40                  45
Tyr His Phe Ile Pro Tyr Glu Gln Met Ser Glu Leu Glu Lys Arg Ile
    50                  55                  60
Ala Arg Ile Ile Pro Leu Arg Tyr Arg Ser Asn His Trp Val Pro Asp
65                  70                  75                  80
Ser Arg Pro Glu Gln Pro Ser Pro Gln Ser Thr Pro Glu Pro Ser Pro
                85                  90                  95
Ser Leu Gln Pro Ala Pro Asn Pro Gln Pro Ala Pro Ser Asn Pro Ile
            100                 105                 110
Asp Glu Lys Leu Val Lys Glu Ala Val Arg Lys Val Gly Asp Gly Tyr
        115                 120                 125
Val Phe Glu Glu Asn Gly Val Ser Arg Tyr Ile Pro Ala Lys Asp Leu
    130                 135                 140
Ser Ala Glu Thr Ala Ala Gly Ile Asp Ser Lys Leu Ala Lys Gln Glu
145                 150                 155                 160
Ser Leu Ser His Lys Leu Gly Ala Lys Lys Thr Asp Leu Pro Ser Ser
                165                 170                 175
Asp Arg Glu Phe Tyr Asn Lys Ala Tyr Asp Leu Leu Ala Arg Ile His
            180                 185                 190
Gln Asp Leu Leu Asp Asn Lys Gly Arg Gln Val Asp Phe Glu Val Leu
        195                 200                 205
Asp Asn Leu Leu Glu Arg Leu Lys Asp Val Ser Ser Asp Lys Val Lys
    210                 215                 220
Leu Val Asp Asp Ile Leu Ala Phe Leu Ala Pro Ile Arg His Pro Glu
225                 230                 235                 240
Arg Leu Gly Lys Pro Asn Ala Gln Ile Thr Tyr Thr Asp Asp Glu Ile

                245                 250                 255
Gln Val Ala Lys Leu Ala Gly Lys Tyr Thr Thr Glu Asp Gly Tyr Ile
            260                 265                 270
Phe Asp Pro Arg Asp Ile Thr Ser Asp Glu Gly Asp Ala Tyr Val Thr
        275                 280                 285
Pro His Met Thr His Ser His Trp Ile Lys Lys Asp Ser Leu Ser Glu
    290                 295                 300
Ala Glu Arg Ala Ala Ala Gln Ala Tyr Ala Lys Glu Lys Gly Leu Thr
305                 310                 315                 320
Pro Pro Ser Thr Asp His Gln Asp Ser Gly Asn Thr Glu Ala Lys Gly
                325                 330                 335
Ala Glu Ala Ile Tyr Asn Arg Val Lys Ala Ala Lys Lys Val Pro Leu

            340                 345                 350
Asp Arg Met Pro Tyr Asn Leu Gln Tyr Thr Val Glu Val Lys Asn Gly
        355                 360                 365
Ser Leu Ile Ile Pro His Tyr Asp His Tyr His Asn Ile Lys Phe Glu
    370                 375                 380
Trp Phe Asp Glu Gly Leu Tyr Glu Ala Pro Lys Gly Tyr Ser Leu Glu
385                 390                 395                 400
Asp Leu Leu Ala Thr Val Lys Tyr Tyr Val Glu His Pro Asn Glu Arg
                405                 410                 415
Pro His Ser Asp Asn Gly Phe Gly Asn Ala Ser Asp His Val Arg Lys
            420                 425                 430
Asn Lys Ala Asp Gln Asp Ser Lys Pro Asp Glu Asp Lys Glu His Asp
        435                 440                 445
Glu Val Ser Glu Pro Thr His Pro Glu Ser Asp Glu Lys Glu Asn His
    450                 455                 460
Ala Gly Leu Asn Pro Ser Ala Asp Asn Leu Tyr Lys Pro Ser Thr Asp
465                 470                 475                 480
Thr Glu Glu Thr Glu Glu Glu Ala Glu Asp Thr Thr Asp Glu Ala Glu
                485                 490                 495
Ile Pro Gln Val Glu Asn Ser Val Ile Asn Ala Lys Ile Ala Asp Ala
            500                 505                 510
Glu Ala Leu Leu Glu Lys Val Thr Asp Pro Ser Ile Arg Gln Asn Ala
        515                 520                 525
Met Glu Thr Leu Thr Gly Leu Lys Ser Ser Leu Leu Leu Gly Thr Lys
    530                 535                 540
Asp Asn Asn Thr Ile Ser Ala Glu Val Asp Ser Leu Leu Ala Leu Leu
545                 550                 555                 560
Lys Glu Ser Gln Pro Ala Pro Ile Gln Xaa Xaa Asp Leu Thr Glu Glu
                565                 570                 575
Gln Ile Lys Ala Ala Gln Lys His Leu Glu Glu Val Lys Thr Ser His
            580                 585                 590
Asn Gly Leu Asp Ser Leu Ser Ser His Glu Gln Asp Tyr Pro Gly Asn
        595                 600                 605
Ala Lys Glu Met Lys Asp Leu Asp Lys Lys Ile Glu Glu Lys Ile Ala
    610                 615                 620
Gly Ile Met Lys Gln Tyr Gly Val Lys Arg Glu Ser Ile Val Val Asn
625                 630                 635                 640
Lys Glu Lys Asn Ala Ile Ile Tyr Pro His Gly Asp His His His Ala
                645                 650                 655
Asp Pro Ile Asp Glu His Lys Pro Val Gly Ile Gly His Ser His Ser
            660                 665                 670
Asn Tyr Glu Leu Phe Lys Pro Glu Glu Gly Val Ala Lys Lys Glu Gly
        675                 680                 685
Asn Lys Val Tyr Thr Gly Glu Glu Leu Thr Asn Val Val Asn Leu Leu
    690                 695                 700
Lys Asn Ser Thr Phe Asn Asn Gln Asn Phe Thr Leu Ala Asn Gly Gln
705                 710                 715                 720
Lys Arg Val Ser Phe Ser Phe Pro Pro Glu Leu Glu Lys Lys Leu Gly
                725                 730                 735
Ile Asn Met Leu Val Lys Leu Ile Thr Pro Asp Gly Lys Val Leu Glu
            740                 745                 750
Lys Val Ser Gly Lys Val Phe Gly Glu Gly Val Gly Asn Ile Ala Asn
        755                 760                 765
Phe Glu Leu Asp Gln Pro Tyr Leu Pro Gly Gln Thr Phe Lys Tyr Thr
    770                 775                 780
Ile Ala Ser Lys Asp Tyr Pro Glu Val Ser Tyr Asp Gly Thr Phe Thr
785                 790                 795                 800
Val Pro Thr Ser Leu Ala Tyr Lys Met Ala Ser Gln Thr Ile Phe Tyr
                805                 810                 815
Pro Phe His Ala Gly Asp Thr Tyr Leu Arg Val Asn Pro Gln Phe Ala
            820                 825                 830
Val Pro Lys Gly Thr Asp Ala Leu Val Arg Val Phe Asp Glu Phe His
        835                 840                 845
Gly Asn Ala Tyr Leu Glu Asn Asn Tyr Lys Val Gly Glu Ile Lys Leu
    850                 855                 860
Pro Ile Pro Lys Leu Asn Gln Gly Thr Thr Arg Thr Ala Gly Asn Lys
865                 870                 875                 880
Ile Pro Val Thr Phe Met Ala Asn Ala Tyr Leu Asp Asn Gln Ser Thr

885                 890                 895
Tyr Ile Val Glu Val Pro Ile Leu Glu Lys Glu Asn Gln Thr Asp Lys
            900                 905                 910
Pro Ser Ile Leu Pro Gln Phe Lys Arg Asn Lys Ala Gln Glu Asn Ser
        915                 920                 925
Lys Leu Asp Glu Lys Val Glu Glu Pro Lys Thr Ser Glu Lys Val Glu
    930                 935                 940
Lys Glu Lys Leu Ser Glu Thr Gly Asn Ser Thr Ser Asn Ser Thr Leu
945                 950                 955                 960
Glu Glu Val Pro Thr Val Asp Pro Val Gln Glu Lys Val Ala Lys Phe
                965                 970                 975
Ala Glu Ser Tyr Gly Met Lys Leu Glu Asn Val Leu Phe Asn Met Asp
            980                 985                 990
Gly Thr Ile Glu Leu Tyr Leu Pro Ser Gly Glu Val Ile Lys Lys Asn
        995                 1000                1005
Met Ala Asp Phe Thr Gly Glu Ala Pro Gln Gly Asn Gly Glu Asn Lys
    1010                1015                1020
Pro Ser Glu Asn Gly Lys Val Ser Thr Gly Thr Val Glu Asn Gln Pro
1025                1030                1035                1040
Thr Glu Asn Lys Pro Ala Asp Ser Leu Pro Glu Ala Pro Asn Glu Lys
                1045                1050                1055
Pro Val Lys Pro Glu Asn Ser Thr Asp Asn Gly Met Leu Asn Pro Glu
            1060                1065                1070
Gly Asn Val Gly Ser Asp Pro Met Leu Asp Pro Ala Leu Glu Glu Ala
        1075                1080                1085
Pro Ala Val Asp Pro Val Gln Glu Lys Leu Glu Lys Phe Thr Ala Ser
    1090                1095                1100
Tyr Gly Leu Gly Leu Asp Ser Val Ile Phe Asn Met Asp Gly Thr Ile
1105                1110                1115                1120
Glu Leu Arg Leu Pro Ser Gly Glu Val Ile Lys Lys Asn Leu Ser Asp
                1125                1130                1135
Phe Ile Ala

<210> 381

<211> 1238
<212> PRT
<213> Unknown

<220>
<223> NEW 29

<221> VARIANT
<222> (1)...(1)
<223> Xaa = Methionine or nothing

<221> VARIANT
<222> (430)...(430)
<223> Xaa = Glycine or nothing

<221> VARIANT
<222> (431)...(431)
<223> Xaa = Proline or nothing

<400> 381
Xaa Asp Ile Asp Ser Leu Leu Lys Gln Leu Tyr Lys Leu Pro Leu Ser
 1               5                  10                  15
Gln Arg His Val Glu Ser Asp Gly Leu Ile Phe Asp Pro Ala Gln Ile
            20                  25                  30
Thr Ser Arg Thr Ala Arg Gly Val Ala Val Pro His Gly Asn His Tyr
        35                  40                  45
His Phe Ile Pro Tyr Glu Gln Met Ser Glu Leu Glu Lys Arg Ile Ala
    50                  55                  60
Arg Ile Ile Pro Leu Arg Tyr Arg Ser Asn His Trp Val Pro Asp Ser
65                  70                  75                  80
Arg Pro Glu Glu Pro Ser Pro Gln Pro Thr Pro Glu Pro Ser Pro Ser
                85                  90                  95

Pro Gln Pro Ala Pro Asn Pro Gln Pro Ala Pro Ser Asn Pro Ile Asp
                100                 105                 110
Glu Lys Leu Val Lys Glu Ala Val Arg Lys Val Gly Asp Gly Tyr Val
        115                 120                 125
Phe Glu Glu Asn Gly Val Ser Arg Tyr Ile Pro Ala Lys Asn Leu Ser
    130                 135                 140
Ala Glu Thr Ala Ala Gly Ile Asp Ser Lys Leu Ala Lys Gln Glu Ser
145                 150                 155                 160
Leu Ser His Lys Leu Gly Ala Lys Lys Thr Asp Leu Pro Ser Ser Asp
                165                 170                 175
Arg Glu Phe Tyr Asn Lys Ala Tyr Asp Leu Leu Ala Arg Ile His Gln
            180                 185                 190
Asp Leu Leu Asp Asn Lys Gly Arg Gln Val Asp Phe Glu Ala Leu Asp
        195                 200                 205
Asn Leu Leu Glu Arg Leu Lys Asp Val Ser Ser Asp Lys Val Lys Leu
    210                 215                 220
Val Asp Asp Ile Leu Ala Phe Leu Ala Pro Ile Arg His Pro Glu Arg
225                 230                 235                 240
Leu Gly Lys Pro Asn Ala Gln Ile Thr Tyr Thr Asp Asp Glu Ile Gln
                245                 250                 255
Val Ala Lys Leu Ala Gly Lys Tyr Thr Thr Glu Asp Gly Tyr Ile Phe
            260                 265                 270
Asp Pro Arg Asp Ile Thr Ser Asp Glu Gly Asp Ala Tyr Val Thr Pro
        275                 280                 285
His Met Thr His Ser His Trp Ile Lys Lys Asp Ser Leu Ser Glu Ala
    290                 295                 300
Glu Arg Ala Ala Ala Gln Ala Tyr Ala Lys Glu Lys Gly Leu Thr Pro
305                 310                 315                 320
Pro Ser Thr Asp His Gln Asp Ser Gly Asn Thr Glu Ala Lys Gly Ala
                325                 330                 335
Glu Ala Ile Tyr Asn Arg Val Lys Ala Ala Lys Lys Val Pro Leu Asp
            340                 345                 350
Arg Met Pro Tyr Asn Leu Gln Tyr Thr Val Glu Val Lys Asn Gly Ser
        355                 360                 365
Leu Ile Ile Pro His Tyr Asp His Tyr His Asn Ile Lys Phe Glu Trp
    370                 375                 380
Phe Asp Glu Gly Leu Tyr Glu Ala Pro Lys Gly Tyr Thr Leu Glu Asp
385                 390                 395                 400
Leu Leu Ala Thr Val Lys Tyr Tyr Val Glu His Pro Asn Glu Arg Pro
                405                 410                 415
His Ser Asp Asn Gly Phe Gly Asn Ala Ser Asp His Val Xaa Xaa Asn
            420                 425                 430
Met Gln Pro Ser Gln Leu Ser Tyr Ser Ser Thr Ala Ser Asp Asn Asn
        435                 440                 445
Thr Gln Ser Val Ala Lys Gly Ser Thr Ser Lys Pro Ala Asn Lys Ser
    450                 455                 460
Glu Asn Leu Gln Ser Leu Leu Lys Glu Leu Tyr Asp Ser Pro Ser Ala
465                 470                 475                 480
Gln Arg Tyr Ser Glu Ser Asp Gly Leu Val Phe Asp Pro Ala Lys Ile
                485                 490                 495
Ile Ser Arg Thr Pro Asn Gly Val Ala Ile Pro His Gly Asp His Tyr
            500                 505                 510
His Phe Ile Pro Tyr Ser Lys Leu Ser Ala Leu Glu Glu Lys Ile Ala
        515                 520                 525
Arg Met Val Pro Ile Ser Gly Thr Gly Ser Thr Val Ser Thr Asn Ala
    530                 535                 540
Lys Pro Asn Glu Val Val Ser Ser Leu Gly Ser Leu Ser Ser Asn Pro
545                 550                 555                 560
Ser Ser Leu Thr Thr Ser Lys Glu Leu Ser Ser Ala Ser Asp Gly Tyr
                565                 570                 575
Ile Phe Asn Pro Lys Asp Ile Val Glu Glu Thr Ala Thr Ala Tyr Ile
            580                 585                 590
Val Arg His Gly Asp His Phe His Tyr Ile Pro Lys Ser Asn Gln Ile
        595                 600                 605
Gly Gln Pro Thr Leu Pro Asn Asn Ser Leu Ala Thr Pro Ser Pro Ser
    610                 615                 620
Leu Pro Ile Asn Pro Gly Thr Ser His Glu Lys His Glu Glu Asp Gly
625                 630                 635                 640

Tyr Gly Phe Asp Ala Asn Arg Ile Ile Ala Glu Asp Glu Ser Gly Phe
                645                 650                 655
Val Met Ser His Gly Asp His Asn His Tyr Phe Phe Lys Lys Asp Leu
            660                 665                 670
Thr Glu Glu Gln Ile Lys Ala Ala Gln Lys His Leu Glu Glu Val Lys
        675                 680                 685
Thr Ser His Asn Gly Leu Asp Ser Leu Ser Ser His Glu Gln Asp Tyr
    690                 695                 700
Pro Gly Asn Ala Lys Glu Met Lys Asp Leu Asp Lys Lys Ile Glu Glu
705                 710                 715                 720
Lys Ile Ala Gly Ile Met Lys Gln Tyr Gly Val Lys Arg Glu Ser Ile
                725                 730                 735
Val Val Asn Lys Glu Lys Asn Ala Ile Ile Tyr Pro His Gly Asp His
            740                 745                 750
His His Ala Asp Pro Ile Asp Glu His Lys Pro Val Gly Ile Gly His
        755                 760                 765
Ser His Ser Asn Tyr Glu Leu Phe Lys Pro Glu Glu Gly Val Ala Lys
    770                 775                 780
Lys Glu Gly Asn Lys Val Tyr Thr Gly Glu Glu Leu Thr Asn Val Val
785                 790                 795                 800
Asn Leu Leu Lys Asn Ser Thr Phe Asn Asn Gln Asn Phe Thr Leu Ala
                805                 810                 815
Asn Gly Gln Lys Arg Val Ser Phe Ser Phe Pro Pro Glu Leu Glu Lys
            820                 825                 830
Lys Leu Gly Ile Asn Met Leu Val Lys Leu Ile Thr Pro Asp Gly Lys
        835                 840                 845
Val Leu Glu Lys Val Ser Gly Lys Val Phe Gly Glu Gly Val Gly Asn
    850                 855                 860
Ile Ala Asn Phe Glu Leu Asp Gln Pro Tyr Leu Pro Gly Gln Thr Phe
865                 870                 875                 880
Lys Tyr Thr Ile Ala Ser Lys Asp Tyr Pro Glu Val Ser Tyr Asp Gly
                885                 890                 895
Thr Phe Thr Val Pro Thr Ser Leu Ala Tyr Lys Met Ala Ser Gln Thr
            900                 905                 910
Ile Phe Tyr Pro Phe His Ala Gly Asp Thr Tyr Leu Arg Val Asn Pro
        915                 920                 925
Gln Phe Ala Val Pro Lys Gly Thr Asp Ala Leu Val Arg Val Phe Asp
    930                 935                 940
Glu Phe His Gly Asn Ala Tyr Leu Glu Asn Asn Tyr Lys Val Gly Glu
945                 950                 955                 960
Ile Lys Leu Pro Ile Pro Lys Leu Asn Gln Gly Thr Thr Arg Thr Ala
                965                 970                 975
Gly Asn Lys Ile Pro Val Thr Phe Met Ala Asn Ala Tyr Leu Asp Asn
            980                 985                 990
Gln Ser Thr Tyr Ile Val Glu Val Pro Ile Leu Glu Lys Glu Asn Gln
        995                 1000                1005
Thr Asp Lys Pro Ser Ile Leu Pro Gln Phe Lys Arg Asn Lys Ala Gln
    1010                1015                1020
Glu Asn Ser Lys Leu Asp Glu Lys Val Glu Glu Pro Lys Thr Ser Glu
1025                1030                1035                1040
Lys Val Glu Lys Glu Lys Leu Ser Glu Thr Gly Asn Ser Thr Ser Asn
                1045                1050                1055
Ser Thr Leu Glu Glu Val Pro Thr Val Asp Pro Val Gln Glu Lys Val
            1060                1065                1070
Ala Lys Phe Ala Glu Ser Tyr Gly Met Lys Leu Glu Asn Val Leu Phe
        1075                1080                1085
Asn Met Asp Gly Thr Ile Glu Leu Tyr Leu Pro Ser Gly Glu Val Ile
    1090                1095                1100
Lys Lys Asn Met Ala Asp Phe Thr Gly Glu Ala Pro Gln Gly Asn Gly
1105                1110                1115                1120
Glu Asn Lys Pro Ser Glu Asn Gly Lys Val Ser Thr Gly Thr Val Glu
                1125                1130                1135
Asn Gln Pro Thr Glu Asn Lys Pro Ala Asp Ser Leu Pro Glu Ala Pro
            1140                1145                1150
Asn Glu Lys Pro Val Lys Pro Glu Asn Ser Thr Asp Asn Gly Met Leu
        1155                1160                1165
Asn Pro Glu Gly Asn Val Gly Ser Asp Pro Met Leu Asp Pro Ala Leu
    1170                1175                1180

Glu Glu Ala Pro Ala Val Asp Pro Val Gln Glu Lys Leu Glu Lys Phe
1185                1190                1195                1200
Thr Ala Ser Tyr Gly Leu Gly Leu Asp Ser Val Ile Phe Asn Met Asp
                1205                1210                1215
Gly Thr Ile Glu Leu Arg Leu Pro Ser Gly Glu Val Ile Lys Lys Asn
            1220                1225                1230
Leu Ser Asp Phe Ile Ala
        1235

<210> 382

<211> 1365
<212> PRT
<213> Unknown

<220>
<223> NEW 30

<221> VARIANT
<222> (1)...(1)
<223> Xaa = Methionine or nothing

<221> VARIANT
<222> (557)...(557)
<223> Xaa = Glycine or nothing

<221> VARIANT
<222> (558)...(558)
<223> Xaa = Proline or nothing

<400> 382
   Xaa Asp Ile Asp Ser Leu Leu Lys Gln Leu Tyr Lys Leu Pro Leu Ser
    1               5                  10                  15
   Gln Arg His Val Glu Ser Asp Gly Leu Ile Phe Asp Pro Ala Gln Ile
               20                  25                  30
   Thr Ser Arg Thr Ala Arg Gly Val Ala Val Pro His Gly Asn His Tyr
           35                  40                  45
   His Phe Ile Pro Tyr Glu Gln Met Ser Glu Leu Glu Lys Arg Ile Ala
       50                  55                  60
   Arg Ile Ile Pro Leu Arg Tyr Arg Ser Asn His Trp Val Pro Asp Ser
   65                  70                  75                  80
   Arg Pro Glu Glu Pro Ser Pro Gln Pro Thr Pro Glu Pro Ser Pro Ser
                   85                  90                  95
   Pro Gln Pro Ala Pro Asn Pro Gln Pro Ala Pro Ser Asn Pro Ile Asp
               100                 105                 110
   Glu Lys Leu Val Lys Glu Ala Val Arg Lys Val Gly Asp Gly Tyr Val
           115                 120                 125
   Phe Glu Glu Asn Gly Val Ser Arg Tyr Ile Pro Ala Lys Asn Leu Ser
       130                 135                 140
   Ala Glu Thr Ala Ala Gly Ile Asp Ser Lys Leu Ala Lys Gln Glu Ser
   145                 150                 155                 160
   Leu Ser His Lys Leu Gly Ala Lys Lys Thr Asp Leu Pro Ser Ser Asp
                   165                 170                 175
   Arg Glu Phe Tyr Asn Lys Ala Tyr Asp Leu Leu Ala Arg Ile His Gln
               180                 185                 190
   Asp Leu Leu Asp Asn Lys Gly Arg Gln Val Asp Phe Glu Ala Leu Asp
           195                 200                 205
   Asn Leu Leu Glu Arg Leu Lys Asp Val Ser Ser Asp Lys Val Lys Leu
       210                 215                 220
   Val Asp Asp Ile Leu Ala Phe Leu Ala Pro Ile Arg His Pro Glu Arg
   225                 230                 235                 240
   Leu Gly Lys Pro Asn Ala Gln Ile Thr Tyr Thr Asp Asp Glu Ile Gln
                   245                 250                 255
   Val Ala Lys Leu Ala Gly Lys Tyr Thr Thr Glu Asp Gly Tyr Ile Phe
               260                 265                 270
   Asp Pro Arg Asp Ile Thr Ser Asp Glu Gly Asp Ala Tyr Val Thr Pro
           275                 280                 285
   His Met Thr His Ser His Trp Ile Lys Lys Asp Ser Leu Ser Glu Ala
       290                 295                 300
   Glu Arg Ala Ala Ala Gln Ala Tyr Ala Lys Glu Lys Gly Leu Thr Pro
   305                 310                 315                 320
   Pro Ser Thr Asp His Gln Asp Ser Gly Asn Thr Glu Ala Lys Gly Ala
                   325                 330                 335
   Glu Ala Ile Tyr Asn Arg Val Lys Ala Ala Lys Lys Val Pro Leu Asp
               340                 345                 350
   Arg Met Pro Tyr Asn Leu Gln Tyr Thr Val Glu Val Lys Asn Gly Ser
           355                 360                 365
   Leu Ile Ile Pro His Tyr Asp His Tyr His Asn Ile Lys Phe Glu Trp
       370                 375                 380
   Phe Asp Glu Gly Leu Tyr Glu Ala Pro Lys Gly Tyr Thr Leu Glu Asp
   385                 390                 395                 400
   Leu Leu Ala Thr Val Lys Tyr Tyr Val Glu His Pro Asn Glu Arg Pro
                   405                 410                 415
   His Ser Asp Asn Gly Phe Gly Asn Ala Ser Asp His Val Gln Arg Asn
               420                 425                 430
   Lys Asn Gly Gln Ala Asp Thr Asn Gln Thr Glu Lys Pro Ser Glu Glu
           435                 440                 445
   Lys Pro Gln Thr Glu Lys Pro Glu Glu Glu Thr Pro Arg Glu Glu Lys
       450                 455                 460
   Pro Gln Ser Glu Lys Pro Glu Ser Pro Lys Pro Thr Glu Glu Pro Glu
   465                 470                 475                 480
   Glu Glu Ser Pro Glu Glu Ser Glu Glu Pro Gln Val Glu Thr Glu Lys
                   485                 490                 495
   Val Glu Glu Lys Leu Arg Glu Ala Glu Asp Leu Leu Gly Lys Ile Gln
               500                 505                 510
   Asp Pro Ile Ile Lys Ser Asn Ala Lys Glu Thr Leu Thr Gly Leu Lys
           515                 520                 525
   Asn Asn Leu Leu Phe Gly Thr Gln Asp Asn Asn Thr Ile Met Ala Glu
       530                 535                 540
   Ala Glu Lys Leu Leu Ala Leu Leu Lys Glu Ser Lys Xaa Xaa Asn Met
   545                 550                 555                 560
   Gln Pro Ser Gln Leu Ser Tyr Ser Ser Thr Ala Ser Asp Asn Asn Thr
                   565                 570                 575
   Gln Ser Val Ala Lys Gly Ser Thr Ser Lys Pro Ala Asn Lys Ser Glu
               580                 585                 590
   Asn Leu Gln Ser Leu Leu Lys Glu Leu Tyr Asp Ser Pro Ser Ala Gln
           595                 600                 605
   Arg Tyr Ser Glu Ser Asp Gly Leu Val Phe Asp Pro Ala Lys Ile Ile
       610                 615                 620
   Ser Arg Thr Pro Asn Gly Val Ala Ile Pro His Gly Asp His Tyr His
   625                 630                 635                 640
   Phe Ile Pro Tyr Ser Lys Leu Ser Ala Leu Glu Glu Lys Ile Ala Arg
                   645                 650                 655
   Met Val Pro Ile Ser Gly Thr Gly Ser Thr Val Ser Thr Asn Ala Lys
               660                 665                 670
   Pro Asn Glu Val Val Ser Ser Leu Gly Ser Leu Ser Ser Asn Pro Ser
           675                 680                 685
   Ser Leu Thr Thr Ser Lys Glu Leu Ser Ser Ala Ser Asp Gly Tyr Ile
       690                 695                 700
   Phe Asn Pro Lys Asp Ile Val Glu Glu Thr Ala Thr Ala Tyr Ile Val
   705                 710                 715                 720
   Arg His Gly Asp His Phe His Tyr Ile Pro Lys Ser Asn Gln Ile Gly
                   725                 730                 735
   Gln Pro Thr Leu Pro Asn Asn Ser Leu Ala Thr Pro Ser Pro Ser Leu
               740                 745                 750
   Pro Ile Asn Pro Gly Thr Ser His Glu Lys His Glu Glu Asp Gly Tyr
           755                 760                 765
   Gly Phe Asp Ala Asn Arg Ile Ile Ala Glu Asp Glu Ser Gly Phe Val
       770                 775                 780
   Met Ser His Gly Asp His Asn His Tyr Phe Phe Lys Lys Asp Leu Thr
   785                 790                 795                 800
   Glu Glu Gln Ile Lys Ala Ala Gln Lys His Leu Glu Glu Val Lys Thr
                   805                 810                 815
   Ser His Asn Gly Leu Asp Ser Leu Ser Ser His Glu Gln Asp Tyr Pro
               820                 825                 830
   Gly Asn Ala Lys Glu Met Lys Asp Leu Asp Lys Lys Ile Glu Glu Lys
           835                 840                 845
   Ile Ala Gly Ile Met Lys Gln Tyr Gly Val Lys Arg Glu Ser Ile Val
       850                 855                 860
   Val Asn Lys Glu Lys Asn Ala Ile Ile Tyr Pro His Gly Asp His His
   865                 870                 875                 880
   His Ala Asp Pro Ile Asp Glu His Lys Pro Val Gly Ile Gly His Ser
                   885                 890                 895
   His Ser Asn Tyr Glu Leu Phe Lys Pro Glu Glu Gly Val Ala Lys Lys
               900                 905                 910
   Glu Gly Asn Lys Val Tyr Thr Gly Glu Glu Leu Thr Asn Val Val Asn
           915                 920                 925
   Leu Leu Lys Asn Ser Thr Phe Asn Asn Gln Asn Phe Thr Leu Ala Asn
       930                 935                 940
   Gly Gln Lys Arg Val Ser Phe Ser Phe Pro Pro Glu Leu Glu Lys Lys
   945                 950                 955                 960
   Leu Gly Ile Asn Met Leu Val Lys Leu Ile Thr Pro Asp Gly Lys Val
                   965                 970                 975
   Leu Glu Lys Val Ser Gly Lys Val Phe Gly Glu Gly Val Gly Asn Ile
               980                 985                 990
   Ala Asn Phe Glu Leu Asp Gln Pro Tyr Leu Pro Gly Gln Thr Phe Lys
           995                 1000                1005
   Tyr Thr Ile Ala Ser Lys Asp Tyr Pro Glu Val Ser Tyr Asp Gly Thr
       1010                1015                1020
   Phe Thr Val Pro Thr Ser Leu Ala Tyr Lys Met Ala Ser Gln Thr Ile
   1025                1030                1035                1040
   Phe Tyr Pro Phe His Ala Gly Asp Thr Tyr Leu Arg Val Asn Pro Gln
                   1045                1050                1055
   Phe Ala Val Pro Lys Gly Thr Asp Ala Leu Val Arg Val Phe Asp Glu
               1060                1065                1070
   Phe His Gly Asn Ala Tyr Leu Glu Asn Asn Tyr Lys Val Gly Glu Ile
           1075                1080                1085
   Lys Leu Pro Ile Pro Lys Leu Asn Gln Gly Thr Thr Arg Thr Ala Gly
       1090                1095                1100
   Asn Lys Ile Pro Val Thr Phe Met Ala Asn Ala Tyr Leu Asp Asn Gln
   1105                1110                1115                1120
   Ser Thr Tyr Ile Val Glu Val Pro Ile Leu Glu Lys Glu Asn Gln Thr
                   1125                1130                1135
   Asp Lys Pro Ser Ile Leu Pro Gln Phe Lys Arg Asn Lys Ala Gln Glu
               1140                1145                1150
   Asn Ser Lys Leu Asp Glu Lys Val Glu Glu Pro Lys Thr Ser Glu Lys
           1155                1160                1165
   Val Glu Lys Glu Lys Leu Ser Glu Thr Gly Asn Ser Thr Ser Asn Ser
       1170                1175                1180
   Thr Leu Glu Glu Val Pro Thr Val Asp Pro Val Gln Glu Lys Val Ala
   1185                1190                1195                1200
   Lys Phe Ala Glu Ser Tyr Gly Met Lys Leu Glu Asn Val Leu Phe Asn
                   1205                1210                1215
   Met Asp Gly Thr Ile Glu Leu Tyr Leu Pro Ser Gly Glu Val Ile Lys
               1220                1225                1230
   Lys Asn Met Ala Asp Phe Thr Gly Glu Ala Pro Gln Gly Asn Gly Glu
           1235                1240                1245
   Asn Lys Pro Ser Glu Asn Gly Lys Val Ser Thr Gly Thr Val Glu Asn
       1250                1255                1260
   Gln Pro Thr Glu Asn Lys Pro Ala Asp Ser Leu Pro Glu Ala Pro Asn
   1265                1270                1275                1280
   Glu Lys Pro Val Lys Pro Glu Asn Ser Thr Asp Asn Gly Met Leu Asn
                   1285                1290                1295
   Pro Glu Gly Asn Val Gly Ser Asp Pro Met Leu Asp Pro Ala Leu Glu
               1300                1305                1310
   Glu Ala Pro Ala Val Asp Pro Val Gln Glu Lys Leu Glu Lys Phe Thr
           1315                1320                1325
   Ala Ser Tyr Gly Leu Gly Leu Asp Ser Val Ile Phe Asn Met Asp Gly
       1330                1335                1340
   Thr Ile Glu Leu Arg Leu Pro Ser Gly Glu Val Ile Lys Lys Asn Leu
   1345                1350                1355                1360
   Ser Asp Phe Ile Ala
                   1365

<210> 383

<211> 1126
<212> PRT
<213> Unknown

<220>
<223> NEW 31

<221> VARIANT
<222> (1)...(1)
<223> Xaa = Methionine or nothing

<221> VARIANT
<222> (557)...(557)
<223> Xaa = Glycine or nothing

<221> VARIANT
<222> (558)...(558)
<223> Xaa = Proline or nothing

<400> 383
Xaa Asp Ile Asp Ser Leu Leu Lys Gln Leu Tyr Lys Leu Pro Leu Ser
1               5                   10                  15
Gln Arg His Val Glu Ser Asp Gly Leu Ile Phe Asp Pro Ala Gln Ile
            20                  25                  30
Thr Ser Arg Thr Ala Arg Gly Val Ala Val Pro His Gly Asn His Tyr
        35                  40                  45
His Phe Ile Pro Tyr Glu Gln Met Ser Glu Leu Glu Lys Arg Ile Ala
    50                  55                  60
Arg Ile Ile Pro Leu Arg Tyr Arg Ser Asn His Trp Val Pro Asp Ser
65                  70                  75                  80
Arg Pro Glu Glu Pro Ser Pro Gln Pro Thr Pro Glu Pro Ser Pro Ser
                85                  90                  95
Pro Gln Pro Ala Pro Asn Pro Gln Pro Ala Pro Ser Asn Pro Ile Asp
            100                 105                 110
Glu Lys Leu Val Lys Glu Ala Val Arg Lys Val Gly Asp Gly Tyr Val
        115                 120                 125
Phe Glu Glu Asn Gly Val Ser Arg Tyr Ile Pro Ala Lys Asn Leu Ser
    130                 135                 140
Ala Glu Thr Ala Ala Gly Ile Asp Ser Lys Leu Ala Lys Gln Glu Ser
145                 150                 155                 160
Leu Ser His Lys Leu Gly Ala Lys Lys Thr Asp Leu Pro Ser Ser Asp
                165                 170                 175
Arg Glu Phe Tyr Asn Lys Ala Tyr Asp Leu Leu Ala Arg Ile His Gln
            180                 185                 190
Asp Leu Leu Asp Asn Lys Gly Arg Gln Val Asp Phe Glu Ala Leu Asp
        195                 200                 205
Asn Leu Leu Glu Arg Leu Lys Asp Val Ser Ser Asp Lys Val Lys Leu
    210                 215                 220
Val Asp Asp Ile Leu Ala Phe Leu Ala Pro Ile Arg His Pro Glu Arg
225                 230                 235                 240
Leu Gly Lys Pro Asn Ala Gln Ile Thr Tyr Thr Asp Asp Glu Ile Gln
                245                 250                 255
Val Ala Lys Leu Ala Gly Lys Tyr Thr Thr Glu Asp Gly Tyr Ile Phe
            260                 265                 270
Asp Pro Arg Asp Ile Thr Ser Asp Glu Gly Asp Ala Tyr Val Thr Pro
        275                 280                 285
His Met Thr His Ser His Trp Ile Lys Lys Asp Ser Leu Ser Glu Ala
    290                 295                 300
Glu Arg Ala Ala Ala Gln Ala Tyr Ala Lys Glu Lys Gly Leu Thr Pro
305                 310                 315                 320
Pro Ser Thr Asp His Gln Asp Ser Gly Asn Thr Glu Ala Lys Gly Ala
                325                 330                 335
Glu Ala Ile Tyr Asn Arg Val Lys Ala Ala Lys Lys Val Pro Leu Asp
            340                 345                 350

Arg Met Pro Tyr Asn Leu Gln Tyr Thr Val Glu Val Lys Asn Gly Ser
            355                 360                 365
Leu Ile Ile Pro His Tyr Asp His Tyr His Asn Ile Lys Phe Glu Trp
        370                 375                 380
Phe Asp Glu Gly Leu Tyr Glu Ala Pro Lys Gly Tyr Thr Leu Glu Asp
385                 390                 395                 400
Leu Leu Ala Thr Val Lys Tyr Tyr Val Glu His Pro Asn Glu Arg Pro
                405                 410                 415
His Ser Asp Asn Gly Phe Gly Asn Ala Ser Asp His Val Gln Arg Asn
            420                 425                 430
Lys Asn Gly Gln Ala Asp Thr Asn Gln Thr Glu Lys Pro Ser Glu Glu
        435                 440                 445
Lys Pro Gln Thr Glu Lys Pro Glu Glu Glu Thr Pro Arg Glu Glu Lys
    450                 455                 460
Pro Gln Ser Glu Lys Pro Glu Ser Pro Lys Pro Thr Glu Glu Pro Glu
465                 470                 475                 480
Glu Glu Ser Pro Glu Glu Ser Glu Glu Pro Gln Val Glu Thr Glu Lys
                485                 490                 495
Val Glu Glu Lys Leu Arg Glu Ala Glu Asp Leu Leu Gly Lys Ile Gln
            500                 505                 510
Asp Pro Ile Ile Lys Ser Asn Ala Lys Glu Thr Leu Thr Gly Leu Lys
        515                 520                 525
Asn Asn Leu Leu Phe Gly Thr Gln Asp Asn Asn Thr Ile Met Ala Glu
    530                 535                 540
Ala Glu Lys Leu Leu Ala Leu Leu Lys Glu Ser Lys Xaa Xaa Asp Leu
545                 550                 555                 560
Thr Glu Glu Gln Ile Lys Ala Ala Gln Lys His Leu Glu Glu Val Lys
                565                 570                 575
Thr Ser His Asn Gly Leu Asp Ser Leu Ser Ser His Glu Gln Asp Tyr
            580                 585                 590
Pro Gly Asn Ala Lys Glu Met Lys Asp Leu Asp Lys Lys Ile Glu Glu
        595                 600                 605
Lys Ile Ala Gly Ile Met Lys Gln Tyr Gly Val Lys Arg Glu Ser Ile
    610                 615                 620
Val Val Asn Lys Glu Lys Asn Ala Ile Ile Tyr Pro His Gly Asp His
625                 630                 635                 640
His His Ala Asp Pro Ile Asp Glu His Lys Pro Val Gly Ile Gly His
                645                 650                 655
Ser His Ser Asn Tyr Glu Leu Phe Lys Pro Glu Glu Gly Val Ala Lys
            660                 665                 670
Lys Glu Gly Asn Lys Val Tyr Thr Gly Glu Glu Leu Thr Asn Val Val
        675                 680                 685
Asn Leu Leu Lys Asn Ser Thr Phe Asn Asn Gln Asn Phe Thr Leu Ala
    690                 695                 700
Asn Gly Gln Lys Arg Val Ser Phe Ser Phe Pro Pro Glu Leu Glu Lys
705                 710                 715                 720
Lys Leu Gly Ile Asn Met Leu Val Lys Leu Ile Thr Pro Asp Gly Lys
                725                 730                 735
Val Leu Glu Lys Val Ser Gly Lys Val Phe Gly Glu Gly Val Gly Asn
            740                 745                 750
Ile Ala Asn Phe Glu Leu Asp Gln Pro Tyr Leu Pro Gly Gln Thr Phe
        755                 760                 765
Lys Tyr Thr Ile Ala Ser Lys Asp Tyr Pro Glu Val Ser Tyr Asp Gly
    770                 775                 780
Thr Phe Thr Val Pro Thr Ser Leu Ala Tyr Lys Met Ala Ser Gln Thr
785                 790                 795                 800
Ile Phe Tyr Pro Phe His Ala Gly Asp Thr Tyr Leu Arg Val Asn Pro
                805                 810                 815
Gln Phe Ala Val Pro Lys Gly Thr Asp Ala Leu Val Arg Val Phe Asp
            820                 825                 830
Glu Phe His Gly Asn Ala Tyr Leu Glu Asn Asn Tyr Lys Val Gly Glu
        835                 840                 845
Ile Lys Leu Pro Ile Pro Lys Leu Asn Gln Gly Thr Thr Arg Thr Ala
    850                 855                 860
Gly Asn Lys Ile Pro Val Thr Phe Met Ala Asn Ala Tyr Leu Asp Asn
865                 870                 875                 880
Gln Ser Thr Tyr Ile Val Glu Val Pro Ile Leu Glu Lys Glu Asn Gln
                885                 890                 895

Thr Asp Lys Pro Ser Ile Leu Pro Gln Phe Lys Arg Asn Lys Ala Gln
            900                 905                 910
Glu Asn Ser Lys Leu Asp Glu Lys Val Glu Glu Pro Lys Thr Ser Glu
        915                 920                 925
Lys Val Glu Lys Glu Lys Leu Ser Glu Thr Gly Asn Ser Thr Ser Asn
    930                 935                 940
Ser Thr Leu Glu Glu Val Pro Thr Val Asp Pro Val Gln Glu Lys Val
945                 950                 955                 960
Ala Lys Phe Ala Glu Ser Tyr Gly Met Lys Leu Glu Asn Val Leu Phe
                965                 970                 975
Asn Met Asp Gly Thr Ile Glu Leu Tyr Leu Pro Ser Gly Glu Val Ile
            980                 985                 990
Lys Lys Asn Met Ala Asp Phe Thr Gly Glu Ala Pro Gln Gly Asn Gly
        995                 1000                1005
Glu Asn Lys Pro Ser Glu Asn Gly Lys Val Ser Thr Gly Thr Val Glu
    1010                1015                1020
Asn Gln Pro Thr Glu Asn Lys Pro Ala Asp Ser Leu Pro Glu Ala Pro
1025                1030                1035                1040
Asn Glu Lys Pro Val Lys Pro Glu Asn Ser Thr Asp Asn Gly Met Leu
                1045                1050                1055
Asn Pro Glu Gly Asn Val Gly Ser Asp Pro Met Leu Asp Pro Ala Leu
            1060                1065                1070
Glu Glu Ala Pro Ala Val Asp Pro Val Gln Glu Lys Leu Glu Lys Phe
        1075                1080                1085
Thr Ala Ser Tyr Gly Leu Gly Leu Asp Ser Val Ile Phe Asn Met Asp
    1090                1095                1100
Gly Thr Ile Glu Leu Arg Leu Pro Ser Gly Glu Val Ile Lys Lys Asn
1105                1110                1115                1120
Leu Ser Asp Phe Ile Ala
                1125
<210> 384

<211> 913
<212> PRT
<213> Unknown

<220>
<223> NEW 32

<221> VARIANT
<222> (1)...(1)
<223> Xaa = Methionine or nothing

<221> VARIANT
<222> (344)...(344)
<223> Xaa = Glycine or nothing

<221> VARIANT
<222> (345)...(345)
<223> Xaa = Proline or nothing

<400> 384
Xaa Asp Ile Leu Ala Phe Leu Ala Pro Ile Arg His Pro Glu Arg Leu
 1               5                  10                  15
Gly Lys Pro Asn Ala Gln Ile Thr Tyr Thr Asp Asp Glu Ile Gln Val
            20                  25                  30
Ala Lys Leu Ala Gly Lys Tyr Thr Thr Glu Asp Gly Tyr Ile Phe Asp
        35                  40                  45
Pro Arg Asp Ile Thr Ser Asp Glu Gly Asp Ala Tyr Val Thr Pro His
    50                  55                  60
Met Thr His Ser His Trp Ile Lys Lys Asp Ser Leu Ser Glu Ala Glu
65                  70                  75                  80
Arg Ala Ala Ala Gln Ala Tyr Ala Lys Glu Lys Gly Leu Thr Pro Pro
                85                  90                  95
Ser Thr Asp His Gln Asp Ser Gly Asn Thr Glu Ala Lys Gly Ala Glu
            100                 105                 110
Ala Ile Tyr Asn Arg Val Lys Ala Ala Lys Lys Val Pro Leu Asp Arg

        115                 120                 125
Met Pro Tyr Asn Leu Gln Tyr Thr Val Glu Val Lys Asn Gly Ser Leu
    130                 135                 140
Ile Ile Pro His Tyr Asp His Tyr His Asn Ile Lys Phe Glu Trp Phe
145                 150                 155                 160
Asp Glu Gly Leu Tyr Glu Ala Pro Lys Gly Tyr Ser Leu Glu Asp Leu
                165                 170                 175
Leu Ala Thr Val Lys Tyr Tyr Val Glu His Pro Asn Glu Arg Pro His
            180                 185                 190
Ser Asp Asn Gly Phe Gly Asn Ala Ser Asp His Val Arg Lys Asn Lys
        195                 200                 205
Ala Asp Gln Asp Ser Lys Pro Asp Glu Asp Lys Glu His Asp Glu Val
    210                 215                 220
Ser Glu Pro Thr His Pro Glu Ser Asp Glu Lys Glu Asn His Ala Gly
225                 230                 235                 240
Leu Asn Pro Ser Ala Asp Asn Leu Tyr Lys Pro Ser Thr Asp Thr Glu
                245                 250                 255
Glu Thr Glu Glu Glu Ala Glu Asp Thr Thr Asp Glu Ala Glu Ile Pro
            260                 265                 270
Gln Val Glu Asn Ser Val Ile Asn Ala Lys Ile Ala Asp Ala Glu Ala
        275                 280                 285
Leu Leu Glu Lys Val Thr Asp Pro Ser Ile Arg Gln Asn Ala Met Glu
    290                 295                 300
Thr Leu Thr Gly Leu Lys Ser Ser Leu Leu Leu Gly Thr Lys Asp Asn
305                 310                 315                 320
Asn Thr Ile Ser Ala Glu Val Asp Ser Leu Leu Ala Leu Leu Lys Glu
                325                 330                 335
Ser Gln Pro Ala Pro Ile Gln Xaa Xaa Asp Leu Thr Glu Glu Gln Ile
            340                 345                 350
Lys Ala Ala Gln Lys His Leu Glu Glu Val Lys Thr Ser His Asn Gly
        355                 360                 365
Leu Asp Ser Leu Ser Ser His Glu Gln Asp Tyr Pro Gly Asn Ala Lys
    370                 375                 380
Glu Met Lys Asp Leu Asp Lys Lys Ile Glu Glu Lys Ile Ala Gly Ile
385                 390                 395                 400
Met Lys Gln Tyr Gly Val Lys Arg Glu Ser Ile Val Val Asn Lys Glu
                405                 410                 415
Lys Asn Ala Ile Ile Tyr Pro His Gly Asp His His His Ala Asp Pro
            420                 425                 430
Ile Asp Glu His Lys Pro Val Gly Ile Gly His Ser His Ser Asn Tyr
        435                 440                 445
Glu Leu Phe Lys Pro Glu Glu Gly Val Ala Lys Lys Glu Gly Asn Lys
    450                 455                 460
Val Tyr Thr Gly Glu Glu Leu Thr Asn Val Val Asn Leu Leu Lys Asn
465                 470                 475                 480
Ser Thr Phe Asn Asn Gln Asn Phe Thr Leu Ala Asn Gly Gln Lys Arg
                485                 490                 495
Val Ser Phe Ser Phe Pro Pro Glu Leu Glu Lys Lys Leu Gly Ile Asn
            500                 505                 510
Met Leu Val Lys Leu Ile Thr Pro Asp Gly Lys Val Leu Glu Lys Val
        515                 520                 525
Ser Gly Lys Val Phe Gly Glu Gly Val Gly Asn Ile Ala Asn Phe Glu
    530                 535                 540
Leu Asp Gln Pro Tyr Leu Pro Gly Gln Thr Phe Lys Tyr Thr Ile Ala
545                 550                 555                 560
Ser Lys Asp Tyr Pro Glu Val Ser Tyr Asp Gly Thr Phe Thr Val Pro
                565                 570                 575
Thr Ser Leu Ala Tyr Lys Met Ala Ser Gln Thr Ile Phe Tyr Pro Phe
            580                 585                 590
His Ala Gly Asp Thr Tyr Leu Arg Val Asn Pro Gln Phe Ala Val Pro
        595                 600                 605
Lys Gly Thr Asp Ala Leu Val Arg Val Phe Asp Glu Phe His Gly Asn
    610                 615                 620
Ala Tyr Leu Glu Asn Asn Tyr Lys Val Gly Glu Ile Lys Leu Pro Ile
625                 630                 635                 640
Pro Lys Leu Asn Gln Gly Thr Thr Arg Thr Ala Gly Asn Lys Ile Pro
                645                 650                 655
Val Thr Phe Met Ala Asn Ala Tyr Leu Asp Asn Gln Ser Thr Tyr Ile

660                 665                 670
Val Glu Val Pro Ile Leu Glu Lys Glu Asn Gln Thr Asp Lys Pro Ser
        675                 680                 685
Ile Leu Pro Gln Phe Lys Arg Asn Lys Ala Gln Glu Asn Ser Lys Leu
    690                 695                 700
Asp Glu Lys Val Glu Glu Pro Lys Thr Ser Glu Lys Val Glu Lys Glu
705                 710                 715                 720
Lys Leu Ser Glu Thr Gly Asn Ser Thr Ser Asn Ser Thr Leu Glu Glu
                725                 730                 735
Val Pro Thr Val Asp Pro Val Gln Glu Lys Val Ala Lys Phe Ala Glu
            740                 745                 750
Ser Tyr Gly Met Lys Leu Glu Asn Val Leu Phe Asn Met Asp Gly Thr
        755                 760                 765
Ile Glu Leu Tyr Leu Pro Ser Gly Glu Val Ile Lys Lys Asn Met Ala
    770                 775                 780
Asp Phe Thr Gly Glu Ala Pro Gln Gly Asn Gly Glu Asn Lys Pro Ser
785                 790                 795                 800
Glu Asn Gly Lys Val Ser Thr Gly Thr Val Glu Asn Gln Pro Thr Glu
                805                 810                 815
Asn Lys Pro Ala Asp Ser Leu Pro Glu Ala Pro Asn Glu Lys Pro Val
            820                 825                 830
Lys Pro Glu Asn Ser Thr Asp Asn Gly Met Leu Asn Pro Glu Gly Asn
        835                 840                 845
Val Gly Ser Asp Pro Met Leu Asp Pro Ala Leu Glu Glu Ala Pro Ala
    850                 855                 860
Val Asp Pro Val Gln Glu Lys Leu Glu Lys Phe Thr Ala Ser Tyr Gly
865                 870                 875                 880
Leu Gly Leu Asp Ser Val Ile Phe Asn Met Asp Gly Thr Ile Glu Leu
                885                 890                 895
Arg Leu Pro Ser Gly Glu Val Ile Lys Lys Asn Leu Ser Asp Phe Ile
            900                 905                 910
Ala

## Description

### FIELD OF THE INVENTION

The present invention is related to antigens, epitopes and antibodies directed to these epitopes, more particularly polypeptide antigens of streptococcus pneumoniae pathogen which may be useful for prophylaxis, diagnostic or treatment of streptococcal infection.

### BACKGROUND OF THE INVENTION

S. pneumoniae is an important agent of disease in man especially among infants, the elderly and immunocompromised persons. It is a bacterium frequently isolated from patients with invasive diseases such as bacteraemia/septicaemia, pneumonia, meningitis with high morbidity and mortality throughout the world. Even with appropriate antibiotic therapy, pneumococcal infections still result in many deaths. Although the advent of antimicrobial drugs has reduced the overall mortality from pneumococcal disease, the presence of resistant pneumococcal organisms has become a major problem in the world today. Effective pneumococcal vaccines could have a major impact on the morbidity and mortality associated with S. pneumoniae disease. Such vaccines would also potentially be useful to prevent otitis media in infants and young children.

Efforts to develop a pneumococcal vaccine have generally concentrated on generating immune responses to the pneumococcal capsular polysaccharide. More than 80 pneumococcal capsular serotypes have been identified on the basis of antigenic differences. The currently available pneumococcal vaccine, comprising 23 capsular polysaccharides that most frequently caused disease, has significant shortcomings related primarily to the poor immunogenicity of some capsular polysaccharides, the diversity of the serotypes and the differences in the distribution of serotypes over time, geographic areas and age groups. In particular, the failure of existing vaccines and capsular conjugate vaccines currently in development to protect young children against all serotypes spurres evaluation of other S. pneumoniae components. Although immunogenicity of capsular polysaccharides can be improved, serotype specificity will still represent a major limitation of polysaccharide-based vaccines. The use of a antigenically conserved immunogenic pneumococcal protein antigen, either by itself or in combination with additional components, offers the possibility of a protein-based pneumococcal vaccine.

PCT WO 98/18930 published May 7, 1998 entitled "*Streptococcus Pneumoniae* antigens and vaccines" describes certain polypeptides which are claimed to be antigenic. However, no biological activity of these polypeptides is reported. Similarly, no sequence conservation is reported, which is a necessary species common vaccine candidate.

PCT WO 00/39299 describes polypeptides and polynucleotides encoding these polypeptides. PCT WO 00/39299 demonstrates that polypeptides designated as BVH-3 and BVH-11 provide protection against fatal experimental infection with pneumococci.

Therefore there remains an unmet need for Streptococcus antigens that may be used as components for the prophylaxis, diagnostic and/or therapy of Streptococcus infection.

### SUMMARY OF THE INVENTION

An isolated polynucleotide comprising a polynucleotide chosen from;
(a) a polynucleotide encoding a polypeptide having at least 70% identity to a second polypeptide chosen from: table A, B, D, E or H;
(b) a polynucleotide encoding a polypeptide having at least 95% identity to a second polypeptide chosen from: table A, B, D, E or H;
(c) a polynucleotide encoding a polypeptide having an amino sequence chosen from table A, B, D, E or H; or fragments, analogs or derivatives thereof;
(d) a polynucleotide encoding a polypeptide chosen from: table A, B, D, E or H;
(e) a polynucleotide encoding a polypeptide capable of generating antibodies having binding specificity for a polypeptide having a sequence chosen from: table A, B, D, E or H;
(f) a polynucleotide encoding an epitope bearing portion of a polypeptide chosen from table A, B, D, E or H; and
(g) a polynucleotide complementary to a polynucleotide in (a), (b), (c), (d), (e) or (f).

In other aspects, there are provided novel polypeptides encoded by polynucleotides of the invention, pharmaceutical or vaccine composition, vectors comprising polynucleotides of the invention operably linked to an expression control region, as well as host cells transfected with said vectors and methods of producing polypeptides comprising culturing said host cells under conditions suitable for expression.

### BRIEF DESCRIPTION OF THE DRAWINGS

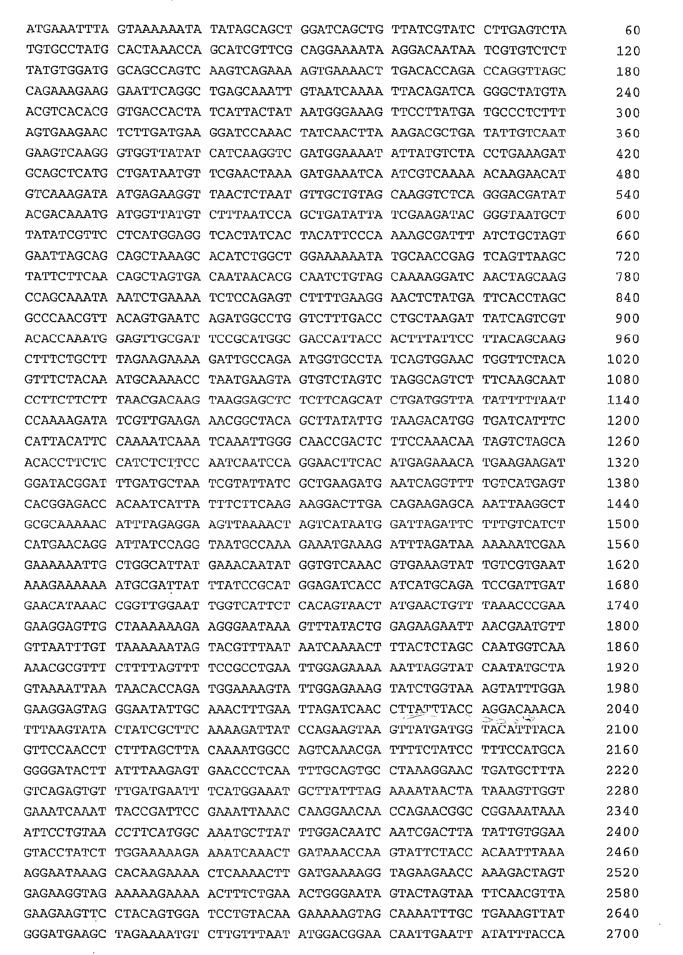

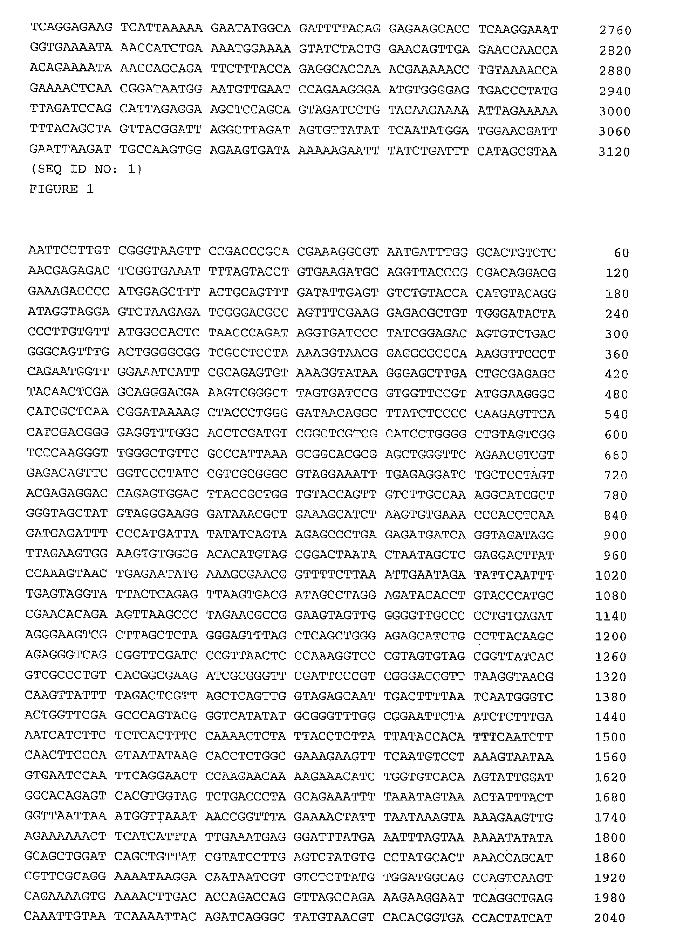

Figure 1 is the DNA sequence of SP64 BVH-3 gene; SEQ ID NO: 1

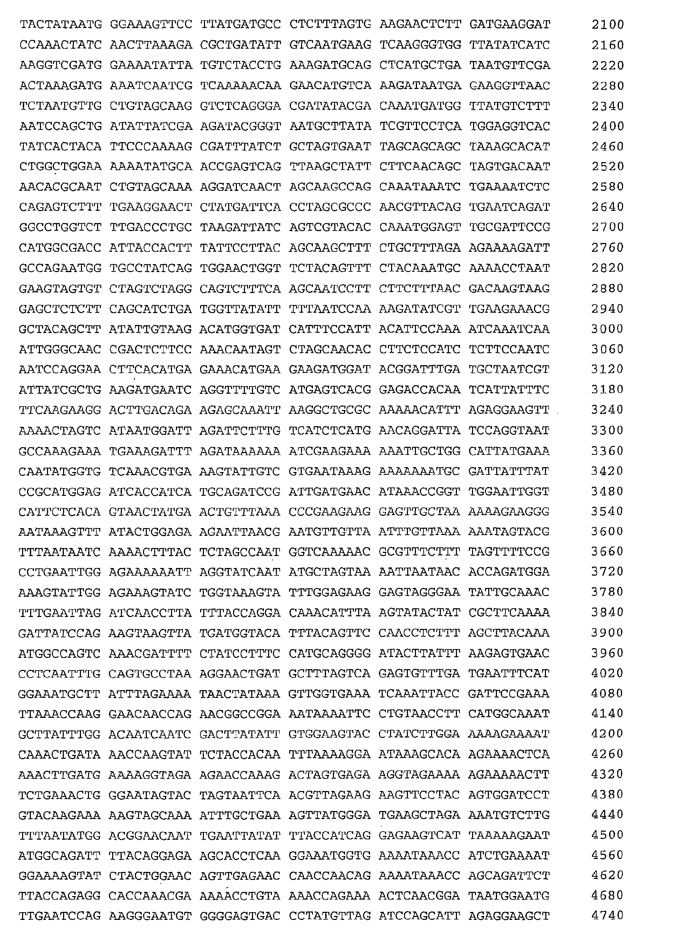

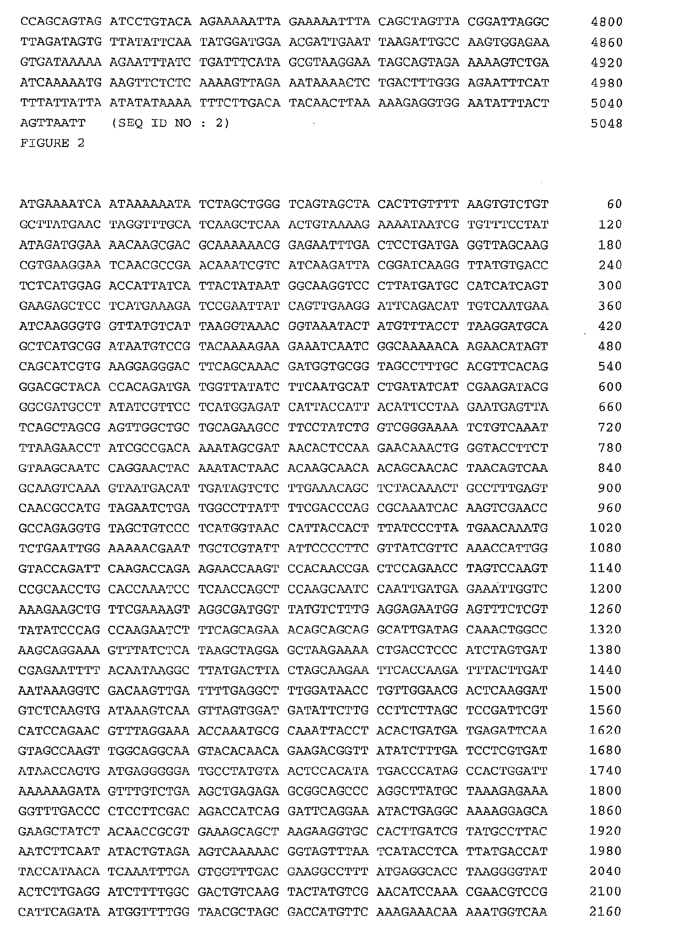

Figure 2 is a DNA sequence containing the complete SP64 BVH-3 gene at nucleotides 1777 to 4896; SEQ ID NO: 2

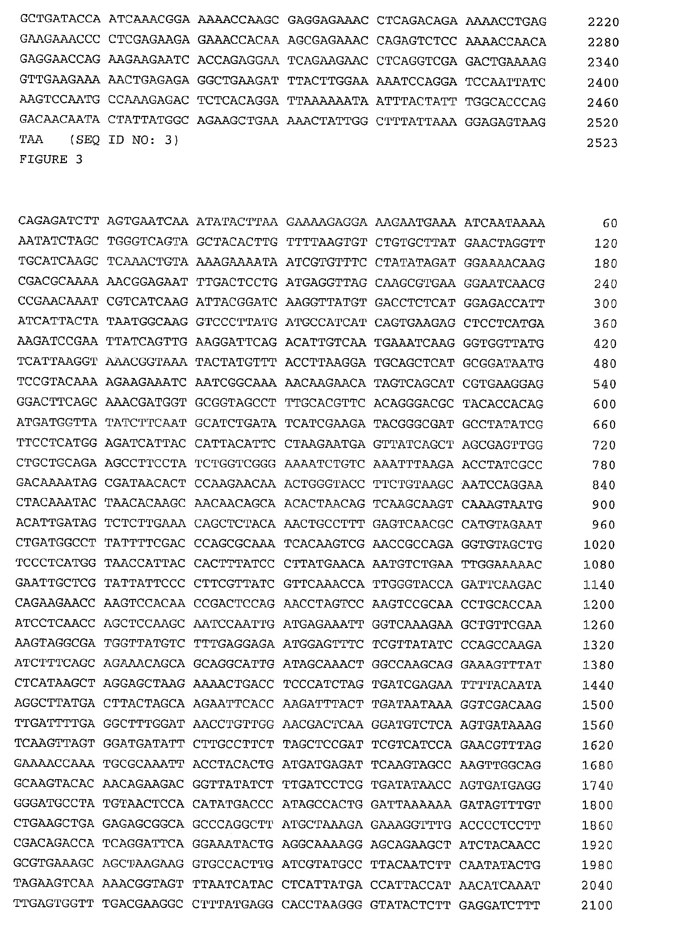

Figure 3 is the DNA sequence of SP64 BVH-11 gene; SEQ ID NO: 3

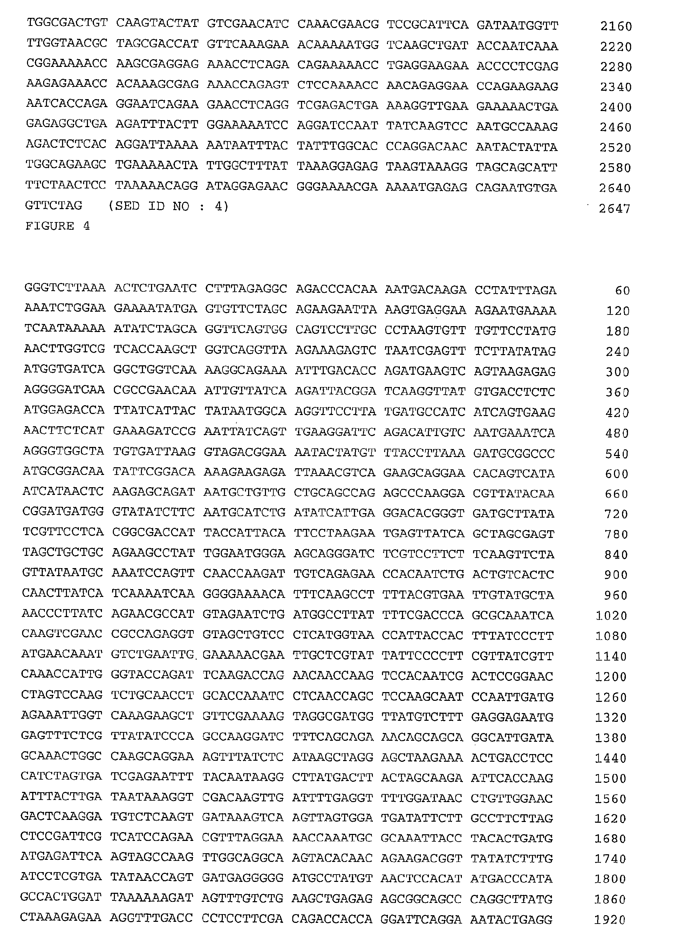

Figure 4 is a DNA sequence containing the complete SP64 BVH-11 gene at nucleotides 45 to 2567; SEQ ID NO: 4

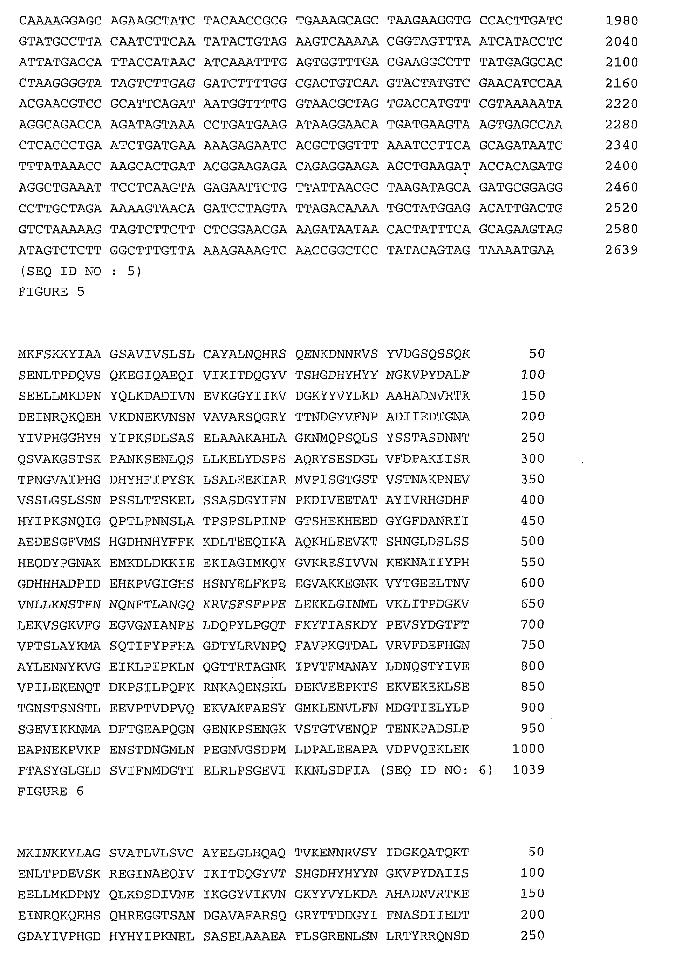

Figure 5 is a DNA sequence containing the complete SP64 BVH-11-2 gene at nucleotides 114 to 2630; SEQ ID NO: 5
Figure 6 is the amino acid sequence of SP64 BVH-3 polypeptide; SEQ ID NO: 6

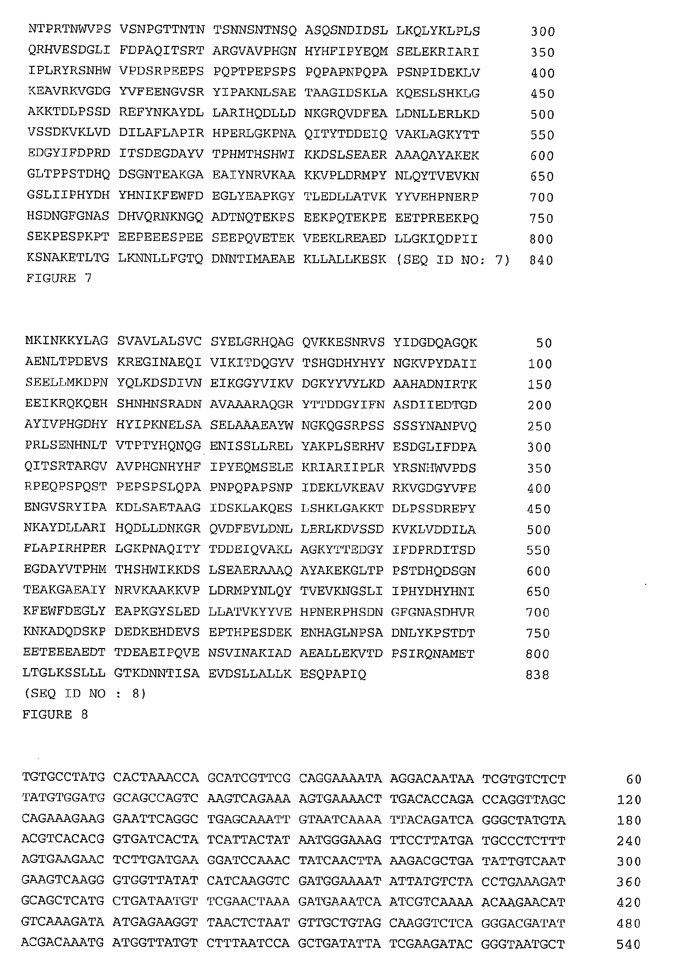

Figure 7 is the amino acid sequence of SP64 BVH-11 polypeptide; SEQ ID NO: 7
Figure 8 is the amino acid sequence of SP64 BVH-11-2 polypeptide; SEQ ID NO: 8

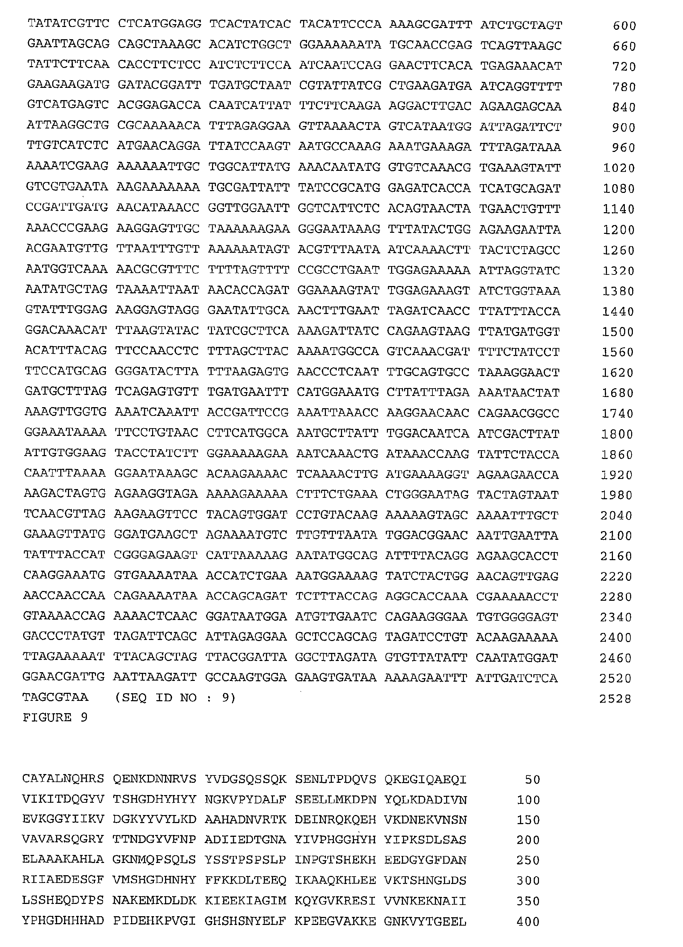

Figure 9 is the DNA sequence of SP63 BVH-3 gene; SEQ ID NO: 9

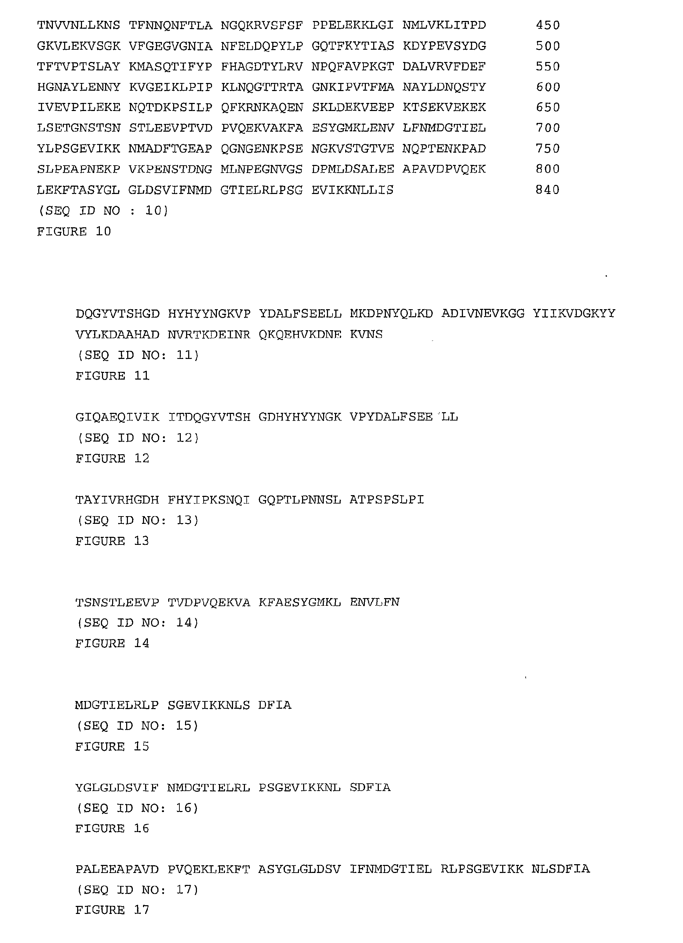

Figure 10 is the amino acid sequence of SP63 BVH-3 polypeptide; SEQ ID NO: 10
Figure 11 is the amino acid sequence of 4D4.9 polypeptide; SEQ ID NO: 11
Figure 12 is the amino acid sequence of 7G11.7 polypeptide; SEQ ID NO: 12
Figure 13 is the amino acid sequence of 7G11.9 polypeptide; SEQ ID NO: 13
Figure 14 is the amino acid sequence of 4D3.4 polypeptide; SEQ ID NO: 14
Figure 15 is the amino acid sequence of 8E3.1 polypeptide; SEQ ID NO: 15
Figure 16 is the amino acid sequence of 1 G2.2 polypeptide; SEQ ID NO: 16
Figure 17 is the amino acid sequence of 10C12.7 polypeptide; SEQ ID NO: 17

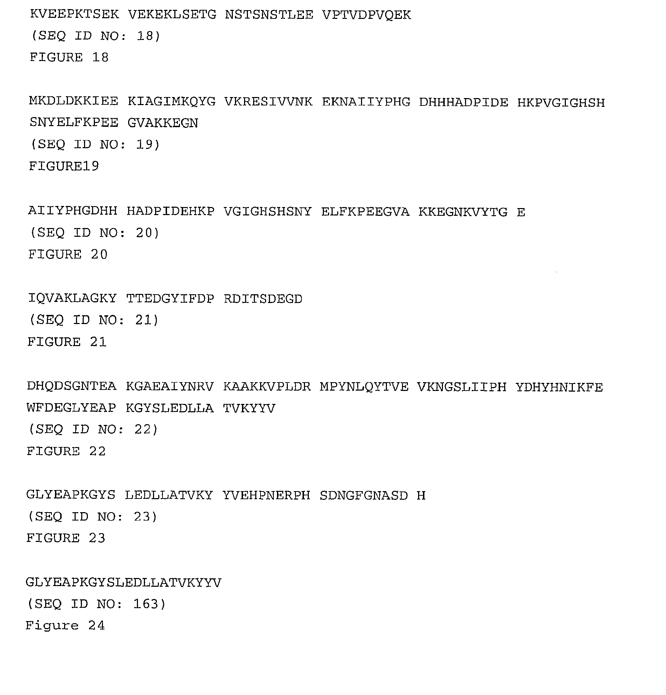

Figure 18 is the amino acid sequence of 14F6.3 polypeptide; SEQ ID NO: 18
Figure 19 is the amino acid sequence of B12D8.2 polypeptide; SEQ ID NO: 19
Figure 20 is the amino acid sequence of 7F4.1 polypeptide; SEQ ID NO: 20
Figure 21 is the amino acid sequence of 10D7.5 polypeptide; SEQ ID NO: 21
Figure 22 is the amino acid sequence of 10G9.3 polypeptide, 10A2.2 polypeptide and B11B8.1 polypeptide; SEQ ID NO: 22
Figure 23 is the amino acid sequence of 11B8.4 polypeptide; SEQ ID NO: 23
Figure 24 is the amino acid sequence of Mab H11B-11B8 target epitope; SEQ ID 163
Figure 25 is a schematic representation of the BVH-3 gene as well as location of gene sequences coding for the full length and truncated polypeptides. The relationships between DNA fragments are shown with respect to each other.
Figure 26 is a schematic representation of the BVH-11 gene as well as location of gene sequences coding for the full length and truncated polypeptides. The relationships between DNA fragments are shown with respect to each other.
Figure 27 is a schematic representation of the BVH-11-2 gene as well as location of gene sequences coding for the full length and truncated polypeptides. The relationships between DNA fragments are shown with respect to each other.
Figure 28 is a schematic representation of the BVH-3 protein and the location of internal and surface epitopes recognized by certain monoclonal antibodies.
Figure 29 is a schematic representation of the BVH-11-2 protein and the location of protective surface epitopes recognized by certain monoclonal antibodies.
Figure 30 is a map of plasmid pURV22.HIS. Kan^{R}, kanamycin-resistance coding region; cI857, bacteriophage λ cI857 temperature-sensitive repressor gene; lambda pL, bacteriophage λ transcription promotor; His-tag, 6-histidine coding region; terminator, T1 transcription terminator; ori, colE1 origin of replication.
Figure 31 depicts the comparison of the amino acid sequences of BVH-3M (sp64) and BVH-3 (Sp63) proteins by using the program Clustal W from MacVector sequence analysis software (version 6.5.3). Underneath the alignment, there is a consensus line where * and . characters indicate identical and similar amino acid residues, respectively.
Figure 32 depicts the comparison of the amino acid sequences of BVH-3, BVH-11 and BVH-11-2 proteins by using the program Clustal W from MacVector sequence analysis software (version 6.5.3). Underneath the alignment, there is a consensus line where * and . characters indicate identical and similar amino acid residues, respectively.
Figure 33 is the DNA sequence of the NEW43 gene (SEQ ID No 257).
Figure 34 is the deduced amino acid sequence of NEW43 polypeptide (SEQ ID No 258).

### DETAILED DESCRIPTION OF THE INVENTION

It was determined that portions of the BVH-3 and BVH-11 polypeptides were internal. Other portions were not present in important strains such as encapsulated s.pneumonia causing disease strains. It would be advantageous to have a polypeptide that comprises a portion that is not internal. When large portions of a polypeptide are internal, these portions are not exposed on the bacteria. However, these portions can be very immunogenic in a recombinant polypeptide and will not confer protection against infections. It would also be advantageous to have a polypeptide that comprises a portion that is present in most strains.

The present invention is concerned with polypeptides in which undesired portions have been deleted and/or modified in order to obtain a specific immune response.

In accordance with the present invention, there are also provided polypeptides or polynucleotides encoding such polypeptides comprising protective domains.

Surprisingly, when the undesired portion of the polypeptides are deleted or modified, the polypeptides have desired biological properties. This is surprising in view of the fact that some of these portions were described as being epitope bearing portion in the patent application PCT WO 98/18930. In other publications such as PCT WO 00/37105, portions identified as histidine triad and coil coiled regions were said to be of importance. The present inventors have found that variants of the polypeptide BVH-3 and BVH-11 in which certain portions were deleted and/or modified and chimeras of these polypeptides have biological properties and generate a specific immune response.

According to one aspect, the present invention provides an isolated polynucleotide encoding a polypeptide having at least 70% identity to a second polypeptide comprising a sequence as disclosed in the present application, the tables and figures.

In accordance with one aspect of the present invention, there is provided an isolated polynucleotide comprising a polynucleotide chosen from;
(a) a polynucleotide encoding a polypeptide having at least 70% identity to a second polypeptide chosen from: table B, E or H;
(b) a polynucleotide encoding a polypeptide having at least 95% identity to a second polypeptide chosen from: table B, E or H;
(c) a polynucleotide encoding a polypeptide having an amino sequence chosen from table B, E or H or fragments, analogs or derivatives thereof;
(d) a polynucleotide encoding a polypeptide chosen from: table B, E or H;
(e) a polynucleotide encoding a polypeptide capable of generating antibodies having binding specificity for a polypeptide having a sequence chosen from: table B, E or H,
(f) a polynucleotide encoding an epitope bearing portion of a polypeptide chosen from table B, E or H; and
(g) a polynycleotide complementary to a polynucleotide in (a), (b), (c), (d), (e) or (f).

According to one aspect, the present invention provides an isolated polynucleotide encoding a polypeptide having at least 70% identity to a second polypeptide comprising a sequence chosen from table A, B, D, E, G or H or fragments, analogues or derivatives thereof.

According to one aspect, the present invention provides an isolated polynucleotide encoding a polypeptide having at least 95% identity to a second polypeptide comprising a sequence chosen from table A, B, D, E, G or H or fragments, analogues or derivatives thereof.

According to one aspect, the present invention relates to polypeptides characterised by the amino acid sequence chosen from table A, B, D, E, G or H or fragments, analogues or derivatives thereof.

According to one aspect, the present invention provides an isolated polynucleotide encoding a polypeptide having at least 70% identity to a second polypeptide comprising a sequence chosen from table A, B, D, E, G or H.

According to one aspect, the present invention provides an isolated polynucleotide encoding a polypeptide having at least 95% identity to a second polypeptide comprising a sequence chosen from table A, B, D, E, G or H.

According to one aspect, the present invention relates to polypeptides characterised by the amino acid sequence chosen from table A, B, D, E, G or H.

According to one aspect, the present invention provides an isolated polynucleotide encoding a polypeptide having at least 70% identity to a second polypeptide comprising a sequence chosen from table B, E or H or fragments, analogues or derivatives thereof.

According to one aspect, the present invention provides an isolated polynucleotide encoding a polypeptide having at least 95% identity to a second polypeptide comprising a sequence chosen from B, E or H or fragments, analogues or derivatives thereof.

According to one aspect, the present invention relates to polypeptides characterised by the amino acid sequence chosen from table B, E or H or fragments, analogues or derivatives thereof.

According to one aspect, the present invention provides an isolated polynucleotide encoding a polypeptide having at least 70% identity to a second polypeptide comprising a sequence chosen from table B, E or H.

According to one aspect, the present invention provides an isolated polynucleotide encoding a polypeptide having at least 95% identity to a second polypeptide comprising a sequence chosen from B, E or H.

According to one aspect, the present invention relates to polypeptides characterised by the amino acid sequence chosen from table B, E or H.

In accordance with the present invention, all nucleotides encoding polypeptides and chimeric polypeptides are within the scope of the present invention.

In a further embodiment, the polypeptides or chimeric polypeptides in accordance with the present invention are antigenic.

In a further embodiment, the polypeptides or chimeric polypeptides in accordance with the present invention are immunogenic.

In a further embodiment, the polypeptides or chimeric polypeptides in accordance with the present invention can elicit an immune response in an individual.

In a further embodiment, the present invention also relates to polypeptides which are able to raise antibodies having binding specificity to the polypeptides or chimeric polypeptides of the present invention as defined above.

In one embodiment, the polypeptides of table A (BVH-3) or table D (BVH-11) comprise at least one epitope bearing portion.

In a further embodiment, the fragments of the polypeptides of the present invention will comprise one or more epitope bearing portion identified in Table C and F. The fragment will comprises at least 15 contiguous amino acid of the polypeptide of table C and F. The fragment will comprises at least 20 contiguous amino acid of the polypeptide of table C and F.

In a further embodiment, the epitope bearing portion of the polypeptide of table A (BVH-3) comprises at least one polypeptide listed in Table C.

In a further embodiment, the epitope bearing portion of the polypeptide of table B (BVH-11) comprises at least one polypeptide listed in Table F.

An antibody that " has binding specificity" is an antibody that recognises and binds the selected polypeptide but which does not substantially recognise and bind other molecules in a sample, such as a biological sample. Specific binding can be measured using an ELISA assay in which the selected polypeptide is used as an antigen.

Unless otherwise defined, all technical and scientific terms used herein have the same meaning as commonly understood by one of ordinary skill in the art to which this invention belongs. All publications, patent applications, patents, and other references mentioned herein are incorporated by reference in their entirety. In case of conflict, the present specification, including definitions, will control. In addition, the materials, methods, and examples are illustrative only and not intended to be limiting.

In accordance with the present invention, "protection" in the biological studies is defined by a significant increase in the survival curve, rate or period. Statistical analysis using the Log rank test to compare survival curves, and Fisher exact test to compare survival rates and numbers of days to death, respectively, might be useful to calculate P values and determine whether the difference between the two groups is statistically significant. P values of 0.05 are regarded as not significant.

As used herein, "fragments", "derivatives" or "analogues" of the polypeptides of the invention include those polypeptides in which one or more of the amino acid residues are substituted with a conserved or non-conserved amino acid residue (preferably conserved) and which may be natural or unnatural. In one embodiment, derivatives and analogues of polypeptides of the invention will have about 70% identity with those sequences illustrated in the figures or fragments thereof. That is, 70% of the residues are the same. In a further embodiment, polypeptides will have greater than 75% homology. In a further embodiment, polypeptides will have greater than 80% homology. In a further embodiment, polypeptides will have greater than 85% homology. In a further embodiment, polypeptides will have greater than 90% homology. In a further embodiment, polypeptides will have greater than 95% homology. In a further embodiment, polypeptides will have greater than 99% homology. In a further embodiment, derivatives and analogues of polypeptides of the invention will have less than about 20 amino acid residue substitutions, modifications or deletions and more preferably less than 10. Preferred substitutions are those known in the art as conserved i.e. the substituted residues share physical or chemical properties such as hydrophobicity, size, charge or functional groups.

The skilled person will appreciate that analogues or derivatives of the proteins or polypeptides of the invention will also find use in the context of the present invention, i.e. as antigenic/immunogenic material. Thus, for instance proteins or polypeptides which include one or more additions, deletions, substitutions or the like are encompassed by the present invention. In addition, it may be possible to replace one amino acid with another of similar "type". For instance replacing one hydrophobic amino acid with another hydrophilic amino acid.

One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of identity analysis are contemplated in the present invention.

In an alternative approach, the analogues or derivatives could be fusion proteins, incorporating moieties which render purification easier, for example by effectively tagging the desired protein or polypeptide, It may be necessary to remove the "tag" or it may be the case that the fusion protein itself retains sufficient antigenicity to be useful.

In an additional aspect of the invention there are provided antigenic/immunogenic fragments of the proteins or polypeptides of the invention, or of analogues or derivatives thereof.

The fragments of the present invention should include one or more such epitopic regions or be sufficiently similar to such regions to retain their antigenic/immunogenic properties. Thus, for fragments according to the present invention the degree of identity is perhaps irrelevant, since they may be 100% identical to a particular part of a protein or polypeptide, analogue or derivative as described herein. The key issue, once again, is that the fragment retains the antigenic/immunogenic properties.

Thus, what is important for analogues, derivatives and fragments is that they possess at least a degree of the antigenicity/immunogenic of the protein or polypeptide from which they are derived.

In accordance with the present invention, polypeptides of the invention include both polypeptides and chimeric polypeptides.

Also included are polypeptides which have fused thereto other compounds which alter the polypeptides biological or pharmacological properties i.e. polyethylene glycol (PEG) to increase half-life; leader or secretory amino acid sequences for ease of purification; prepro- and pro- sequences; and (poly) saccharides.

Furthermore, in those situations where amino acid regions are found to be polymorphic, it may be desirable to vary one or more particular amino acids to more effectively mimic the different epitopes of the different streptococcus strains.

Moreover, the polypeptides of the present invention can be modified by terminal -NH₂ acylation (e.g. by acetylation, or thioglycolic acid amidation, terminal carboxy amidation, e.g. with ammonia or methylamine) to provide stability, increased hydrophobicity for linking or binding to a support or other molecule.

Also contemplated are hetero and homo polypeptide multimers of the polypeptide fragments, analogues and derivatives. These polymeric forms include, for example, one or more polypeptides that have been cross-linked with cross-linkers such as avidin/biotin, gluteraldehyde or dimethylsuperimidate. Such polymeric forms also include polypeptides containing two or more tandem or inverted contiguous sequences, produced from multicistronic mRNAs generated by recombinant DNA technology.

Preferably, a fragment, analogue or derivative of a polypeptide of the invention will comprise at least one antigenic region i.e. at least one epitope.

In order to achieve the formation of antigenic polymers (i.e. synthetic multimers), polypeptides may be utilised having bishaloacetyl groups, nitroarylhalides, or the like, where the reagents being specific for thio groups. Therefore, the link between two mercapto groups of the different peptides may be a single bond or may be composed of a linking group of at least two, typically at least four, and not more than 16, but usually not more than about 14 carbon atoms.

In a particular embodiment, polypeptide fragments, analogues and derivatives of the invention do not contain a methionine (Met) starting residue. Preferably, polypeptides will not incorporate a leader or secretory sequence (signal sequence). The signal portion of a polypeptide of the invention may be determined according to established molecular biological techniques. In general, the polypeptide of interest may be isolated from a streptococcus culture and subsequently sequenced to determine the initial residue of the mature protein and therefore the sequence of the mature polypeptide.

According to another aspect, there are provided vaccine compositions comprising one or more streptococcus polypeptides of the invention in admixture with a pharmaceutically acceptable carrier diluent or adjuvant. Suitable adjuvants include oils. i.e. Freund's complete or incomplete adjuvant; salts i.e. AIK(SO₄)₂, AlNa(SO₄)₂, AlNH₄(SO₄)₂, silica, kaolin, carbon polynucleotides i.e. poly IC and poly AU. Preferred adjuvants include QuilA and Alhydrogel. Vaccines of the invention may be administered parenterally by injection, rapid infusion, nasopharyngeal absorption, dermoabsorption, or bucal or oral. Pharmaceutically acceptable carriers also include tetanus toxoid.

The term vaccine is also meant to include antibodies. In accordance with the present invention, there is also provided the use of one or more antibodies having binding specificity for the polypeptides of the present invention for the treatment or prophylaxis of streptococcus infection and/or diseases and symptoms mediated by streptococcus infection.

Vaccine compositions of the invention are used for the treatment or prophylaxis of streptococcus infection and/or diseases and symptoms mediated by streptococcus infection as described in P.R. Murray (Ed, in chief), E.J. Baron, M.A. Pfaller, F.C. Tenover and R.H. Yolken. Manual of Clinical Microbiology, ASM Press, Washington, D.C. sixth edition, 1995, 1482p which are herein incorporated by reference. In one embodiment, vaccine compositions of the present invention are used for the treatment or prophylaxis of meningitis, otitis media, bacteremia or pneumonia. In one embodiment, vaccine compositions of the invention are used for the treatment or prophylaxis of *streptococcus* infection and/or diseases and symptoms mediated by *streptococcus* infection, in particular S.pneumoniae, group A *streptococcus* (*pyogenes*), group B *streptococcus* (GBS or *agalactiae*), *dysgalactiae, uberis, nocardia* as well as *Staphylococcus aureus.* In a further embodiment, the streptococcus infection is S.pneumoniae.

In a particular embodiment, vaccines are administered to those individuals at risk of streptococcus infection such as infants, elderly and immunocompromised individuals.

As used in the present application, the term " individuals" include mammals. In a further embodiment, the mammal is human.

Vaccine compositions are preferably in unit dosage form of about 0.001 to 100 µg/kg (antigen/body weight) and more preferably 0.01 to 1 µpg/kg and most preferably 0.1 to 1 µg/kg 1 to 3 times with an interval of about 1 to 6 week intervals between immunizations.

Vaccine compositions are preferably in unit dosage form of about 0.1 µg to 10 mg and more preferably 1µg to 1 mg and most preferably 10 to 100 µg 1 to 3 times with an interval of about 1 to 6 week intervals between immunizations.

According to another aspect, there are provided polynucleotides encoding polypeptides characterised by the amino acid sequence chosen from table A, B, D, E, G or H or fragments, analogues or derivatives thereof.

According to another aspect, there are provided polynucleotides encoding polypeptides characterised by the amino acid sequence chosen from table B, E or H or fragments, analogues or derivatives thereof.

In one embodiment, polynucleotides are those illustrated in table A, B, D, E, G or H which encodes polypeptides of the invention.

In one embodiment, polynucleotides are those illustrated in table B, E or H which encodes polypeptides of the invention.

It will be appreciated that the polynucleotide sequences illustrated in the figures may be altered with degenerate codons yet still encode the polypeptides of the invention. Accordingly the present invention further provides polynucleotides which hybridise to the polynucleotide sequences herein above described (or the complement sequences thereof) having 50% identity between sequences. In one embodiment, at least 70% identity between sequences. In one embodiment, at least 75% identity between sequences. In one embodiment, at least 80% identity between sequences. In one embodiment, at least 85% identity between sequences. In one embodiment, at least 90% identity between sequences. In a further embodiment, polynucleotides are hybridizable under stringent conditions i.e. having at least 95% identity. In a further embodiment, more than 97% identity.

Suitable stringent conditions for hybridation can be readily determined by one of skilled in the art (see for example Sambrook et al., (1989) Molecular cloning : A Laboratory Manual, 2nd ed, Cold Spring Harbor, N.Y.; Current Protocols in Molecular Biology, (1999) Edited by Ausubel F.M. et al., John Wiley & Sons, Inc., N. Y.).

In a further embodiment, the present invention provides polynucleotides that hybridise under stringent conditions to either
(a) a DNA sequence encoding a polypeptide or
(b) the complement of a DNA sequence encoding a polypeptide;
wherein said polypeptide comprising a sequence chosen from table A, B, D, E, G or H or fragments or analogues thereof.

In a further embodiment, the present invention provides polynucleotides that hybridise under stringent conditions to either
(c) a DNA sequence encoding a polypeptide or
(d) the complement of a DNA sequence encoding a polypeptide;
wherein said polypeptide comprising a sequence chosen from table B, E or H or fragments or analogues thereof.

In a further embodiment, the present invention provides polynucleotides that hybridise under stringent conditions to either
(a) a DNA sequence encoding a polypeptide or
(b) the complement of a DNA sequence encoding a polypeptide;
wherein said polypeptide comprises at least 10 contiguous amino acid residues from a polypeptide comprising a sequence chosen from table A, B, D, E, G or H or fragments or analogues thereof.

In a further embodiment, the present invention provides polynucleotides that hybridise under stringent conditions to either
(c) a DNA sequence encoding a polypeptide or
(d) the complement of a DNA sequence encoding a polypeptide;
wherein said polypeptide comprises at least 10 contiguous amino acid residues from a polypeptide comprising a sequence chosen from table B, E or H or fragments or analogues thereof.

In a further embodiment, polynucleotides are those encoding polypeptides of the invention illustrated in table A, B, D, E, G or H.

As will be readily appreciated by one skilled in the art, polynucleotides include both DNA and RNA.

The present invention also includes polynucleotides complementary to the polynucleotides described in the present application.

In a further aspect, polynucleotides encoding polypeptides of the invention, or fragments, analogues or derivatives thereof, may be used in a DNA immunization method. That is, they can be incorporated into a vector which is replicable and expressible upon injection thereby producing the antigenic polypeptide in vivo. For example polynucleotides may be incorporated into a plasmid vector under the control of the CMV promoter which is functional in eukaryotic cells. Preferably the vector is injected intramuscularly.

According to another aspect, there is provided a process for producing polypeptides of the invention by recombinant techniques by expressing a polynucleotide encoding said polypeptide in a host cell and recovering the expressed polypeptide product. Alternatively, the polypeptides can be produced according to established synthetic chemical techniques i.e. solution phase or solid phase synthesis of oligopeptides which are ligated to produce the full polypeptide (block ligation).

General methods for obtention and evaluation of polynucleotides and polypeptides are described in the following references: Sambrook et al, Molecular Cloning: A Laboratory Manual, 2nd ed, Cold Spring Harbor, N. Y., 1989; Current Protocols in Molecular Biology, Edited by Ausubel F.M. et al., John Wiley and Sons, Inc. New York; PCR Cloning Protocols, from Molecular Cloning to Genetic Engineering, Edited by White B.A., Humana Press, Totowa, New Jersey, 1997, 490 pages; Protein Purification, Principles and Practices, Scopes R.K., Springer-Verlag, New York, 3rd Edition, 1993, 380 pages; Current Protocols in Immunology, Edited by Coligan J.E. et al., John Wiley & Sons Inc., New York which are herein incorporated by reference.

For recombinant production, host cells are transfected with vectors which encode the polypeptide, and then cultured in a nutrient media modified as appropriate for activating promoters, selecting transformants or amplifying the genes. Suitable vectors are those that are viable and replicable in the chosen host and include chromosomal, non-chromosomal and synthetic DNA sequences e.g. bacterial plasmids, phage DNA, baculovirus, yeast plasmids, vectors derived from combinations of plasmids and phage DNA. The polypeptide sequence may be incorporated in the vector at the appropriate site using restriction enzymes such that it is operably linked to an expression control region comprising a promoter, ribosome binding site (consensus region or Shine-Dalgarno sequence), and optionally an operator (control element). One can select individual components of the expression control region that are appropriate for a given host and vector according to established molecular biology principles (Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York incorporated herein by reference). Suitable promoters include but are not limited to LTR or SV40 promoter, E. coli lac, tac or trp promoters and the phage lambda P_{L} promoter. Vectors will preferably incorporate an origin of replication as well as selection markers i.e. ampicilin resistance gene. Suitable bacterial vectors include pET, pQE70, pQE60, pQE-9, pbs, pD10 phagescript, psiX174, pbluescript SK, pbsks, pNH8A, pNH16a, pNH18A, pNH46A, ptrc99a, pKK223-3, pKK233-3, pDR540, pRIT5 and eukaryotic vectors pBlueBacIII, pWLNEO, pSV2CAT, pOG44, pXT1, pSG, pSVK3, pBPV, pMSG and pSVL. Host cells may be bacterial i.e. E. coli, Bacillus subtilis, Streptomyces; fungal i.e. Aspergillus niger, Aspergillus nidulins; yeast i.e. Saccharomyces or eukaryotic i.e. CHO, COS.

Upon expression of the polypeptide in culture, cells are typically harvested by centrifugation then disrupted by physical or chemical means (if the expressed polypeptide is not secreted into the media) and the resulting crude extract retained to isolate the polypeptide of interest. Purification of the polypeptide from culture media or lysate may be achieved by established techniques depending on the properties of the polypeptide i.e. using ammonium sulfate or ethanol precipitation , acid extraction, anion or cation exchange chromatography, phosphocellulose chromatography, hydrophobic interaction chromatography, hydroxylapatite chromatography and lectin chromatography. Final purification may be achieved using HPLC.

The polypeptide may be expressed with or without a leader or secretion sequence. In the former case the leader may be removed using post-translational processing (see US 4,431,739; US 4,425,437; and US 4,338,397 incorporated herein by reference) or be chemically removed subsequent to purifying the expressed polypeptide.

According to a further aspect, the streptococcus polypeptides of the invention may be used in a diagnostic test for streptococcus infection, in particular S. pneumoniae infection. Several diagnostic methods are possible, for example detecting streptococcus organism in a biological sample, the following procedure may be followed:
a) obtaining a biological sample from a patient;
b) incubating an antibody or fragment thereof reactive with a streptococcus polypeptide of the invention with the biological sample to form a mixture; and
c) detecting specifically bound antibody or bound fragment in the mixture which indicates the presence of streptococcus.

Alternatively, a method for the detection of antibody specific to a streptococcus antigen in a biological sample containing or suspected of containing said antibody may be performed as follows:
a) obtaining a biological sample from a patient;
b) incubating one or more streptococcus polypeptides of the invention or fragments thereof with the biological sample to form a mixture; and
c) detecting specifically bound antigen or bound fragment in the mixture which indicates the presence of antibody specific to streptococcus.

One of skill in the art will recognize that this diagnostic test may take several forms, including an immunological test such as an enzyme-linked immunosorbent assay (ELISA), a radioimmunoassay or a latex agglutination assay, essentially to determine whether antibodies specific for the polypeptide are present in an organism.

The DNA sequences encoding polypeptides of the invention may also be used to design DNA probes for use in detecting the presence of streptococcus in a biological sample suspected of containing such bacteria. The detection method of this invention comprises:
a) obtaining the biological sample from a patient;
b) incubating one or more DNA probes having a DNA sequence encoding a polypeptide of the invention or fragments thereof with the biological sample to form a mixture; and
c) detecting specifically bound DNA probe in the mixture which indicates the presence of streptococcus bacteria.

The DNA probes of this invention may also be used for detecting circulating streptococcus i.e. S.pneumoniae nucleic acids in a sample, for example using a polymerase chain reaction, as a method of diagnosing streptococcus infections. The probe may be synthesized using conventional techniques and may be immobilized on a solid phase, or may be labelled with a detectable label. A preferred DNA probe for this application is an oligomer having a sequence complementary to at least about 6 contiguous nucleotides of the streptococcus pneumoniae polypeptides of the invention.

Another diagnostic method for the detection of streptococcus in a patient comprises:
a) labelling an antibody reactive with a polypeptide of the invention or fragment thereof with a detectable label;
b) administering the labelled antibody or labelled fragment to the patient; and
c) detecting specifically bound labelled antibody or labelled fragment in the patient which indicates the presence of streptococcus.

A further aspect of the invention is the use of the streptococcus polypeptides of the invention as immunogens for the production of specific antibodies for the diagnosis and in particular the treatment of streptococcus infection. Suitable antibodies may be determined using appropriate screening methods, for example by measuring the ability of a particular antibody to passively protect against streptococcus infection in a test model. One example of an animal model is the mouse model described in the examples herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal, or preferably monoclonal. It may be specific for a number of epitopes associated with the streptococcus pneumoniae polypeptides but is preferably specific for one.

A further aspect of the invention is the use of the antibodies directed to the streptococcus polypeptides of the invention for passive immunization. One could use the antibodies described in the present application. Suitable antibodies may be determined using appropriate screening methods, for example by measuring the ability of a particular antibody to passively protect against streptococcus infection in a test model. One example of an animal model is the mouse model described in the examples herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal, or preferably monoclonal. It may be specific for a number of epitopes associated with the streptococcus pneumoniae polypeptides but is preferably specific for one.

The following are reference tables summarizing the sequences disclosed in the present application:

### TABLE A, B and C Variants and Epitope of BVH-3

**Table A**

| Family | Polypeptide SEQ ID NO |
|---|---|
| BVH-3 | |
| New21 | aa 396-1039 of SEQ ID. 6 |
| New25 | aa 233-1039 of SEQ ID. 6 |
| New40 | aa 408-1039 of SEQ ID. 6 |

**TABLE B -**

| Family | Polypeptide SEQ ID NO |
|---|---|
| BVH-3 | |
| NEW1-mutl** | 235 |
| NEW35A | 236 |
| NEW42 | 237 |
| NEW49 | 238 |
| NEW50 | 239 |
| NEW51 | 240 |
| NEW52 | 241 |
| NEW53 | 242 |
| NEW54 | 243 |
| NEW55 | 244 |
| NEW56 | 245 |
| NEW56-mut2** | 245 |
| NEW56-mut3** | 245 |
| NEW57 | 246 |
| NEW63 | 247 |
| NEW64 | 248 |
| NEW65 | 249 |
| NEW66 | 250 |
| NEW76 | 251 |
| NEW105 | 252 |
| NEW106 | 253 |
| NEW107 | 254 |

| | |
|---|---|
| ** silent mutation, i.e. the polypeptide is the same as New1 or New 56 | |

**TABLE C- Epitopes of BVH-3**

| | |
|---|---|
| 7G11.7 | 12 |
| 7G11.9 | 13 |
| B12D8.2 | 19 |
| 7F4.1 | 20 |
| 14F6.3 | 18 |
| 4D3.4 | 14 |
| 10C12.7 | 17 |
| 8E3.1 | 15 |
| 1G2.2 | 16 |

### TABLE D, E and F Variants and Epitope of BVH-11-

**TABLE D-**

| Family | Polypeptide SEQ ID NO |
|---|---|
| BVH-11 | |
| Ncw19 | aa 497-838 of Seq. ID 8 |
| New24 | aa 227-838 of Seq. ID 8 |

**TABLE E-**

| Family | Polypeptide SEQ ID NO |
|---|---|
| BVH-11 | |
| New 43 | 258 |
| NEW60 | 293 |
| NEW61 | 294 |
| NEW62 | 295 |
| NEW80 | 296 |
| NEW81 | 297 |
| NEW82 | 298 |
| NEW83 | 299 |
| NEW84 | 300 |
| NEW85 | 301 |
| NEW88D1 | 302 |
| NEW88D2 | 303 |
| NEW88 | 304 |

**TABLE F-epitopes of BVH-11**

| | |
|---|---|
| 10D7.5 | 21 |
| 10G9.3 | 22 |
| B11B8.1 | 22 |
| 10A2.2 | 22 |
| 11 b8.4 | 23 |
| 3A4.1 | 24 |

### TABLE G and H Chimeras-

**TABLE G**

| Family | Polypeptide SEQ ID NO |
|---|---|
| Chimeras with BVH-11 and BVH-3 | |
| New17 | **M*-NEW5-G*P*-NEW1** |
| New20 | **M*-NEW1-G*P*-NEW5** |
| New26 | **M*-NEW10-G*P*-NEW25** |
| New27 | **M*-NEW19-G*P*-NEW25** |
| New28 | **M*-NEW10-G*P*-NEW1** |
| New29 | **M*-NEWS-G*P*-NEW25** |
| New30 | **M*-NEW4-G*P*-NEW25** |
| New31 | **M*-NEW4-G*P*-NEW1** |
| NEW32 | **M*-NE19-G*P*-NEW1** |

| | |
|---|---|
| * OPTIONAL AMINO ACID | |

**TABLE H**

| Family | Polypeptide SEQ ID NO |
|---|---|
| Chimeras with BVH-11 and BVH-3 | |
| VP 89 | 305 |
| VP 90 | 306 |
| VP 91 | 307 |
| VP 92 | 308 |
| VP 93 | 309 |
| VP 94 | 310 |
| VP108 | 311 |
| VP109 | 312 |
| VP110 | 313 |
| VP111 | 314 |
| VP112 | 315 |
| VP113 | 316 |
| VP114 | 317 |
| VP115 | 318 |
| VP116 | 319 |
| VP117 | 320 |
| VP119 | 321 |
| VP120 | 322 |
| VP121 | 323 |
| VP122 | 324 |
| VP123 | 325 |
| VP124 | 326 |

The invention comprises the following embodiments:
1. An isolated polynucleotide comprising a polynucleotide chosen from;
   (a) a polynucleotide encoding a polypeptide having at least 70% identity to a second polypeptide chosen from: table B, E or H;
   (b) a polynucleotide encoding a polypeptide having at least 95% identity to a second polypeptide chosen from: table B, E or H;
   (c) a polynucleotide encoding a polypeptide having an amino sequence chosen from table B, E or H or fragments, analogs or derivatives thereof;
   (d) a polynucleotide encoding a polypeptide chosen from: table B, E or H;
   (e) a polynucleotide encoding a polypeptide capable of generating antibodies having binding specificity for a polypeptide having a sequence chosen from: table B, E or H,
   (f) a polynucleotide encoding an epitope bearing portion of a polypeptide chosen from table B, E or H; and
   (g) a polynycleotide complementary to a polynucleotide in (a), (b), (c), (d), (e) or (f).
2. The isolated polynucleotide of embodiment 1 wherein said polynucleotide is (a).
3. The isolated polynucleotide of embodiment 1 wherein said polynucleotide is (b).
4. The isolated polynucleotide of embodiment 1 wherein said polynucleotide is (c).
5. The isolated polynucleotide of embodiment 1 wherein said polynucleotide is (d).
6. The isolated polynucleotide of embodiment 1 wherein said polynucleotide is (e).
7. The isolated polynucleotide of embodiment 1 wherein said polynucleotide is (f).
8. The isolated polynucleotide of embodiment 1 wherein said polynucleotide is (g).
9. The isolated polynucleotide of embodiment 7 wherein said polynucleotide is chosen from table B.
10. The isolated polynucleotide of embodiment 9 wherein said epitope bearing portion is chosen from table C.
11. The isolated polynucleotide of embodiment 7 wherein said polynucleotide is chosen from table E.
12. The isolated polynucleotide of embodiment 11 wherein said epitope bearing portion is chosen from table F.
13. The polynucleotide of anyone of embodiments 1 to 12, wherein said polynucleotide is DNA.
14. The polynucleotide of anyone of embodiments 1 to 12, wherein said polynucleotide is RNA.
15. A vector comprising the polynucleotide of embodiment 13, wherein said DNA is operably linked to an expression control region.
16. A host cell transfected with the vector of embodiment 15.
17. A process for producing a polypeptide comprising culturing a host cell according to embodiment 16 under conditions suitable for expression of said polypeptide.
18. An isolated polypeptide comprising a member chosen from:
   (a) a polypeptide having at least 70% identity to a second polypeptide having an amino acid sequence chosen from: table B, E or H;
   (b) a polypeptide having at least 95% identity to a second polypeptide having an amino acid sequence chosen from: table B, E or H;
   (c) a polypeptide having an amino acid sequence chosen from table B, E or H;
   (d) a polypeptide having amino acid sequence chosen from: table B, E or H or fragments, analogs or derivatives thereof;
   (e) a polypeptide capable of generating antibodies having binding specificity for a second polypeptide having a sequence chosen from table B, E or H;
   (f) an epitope bearing portion of a polypeptide having an amino acid sequence chosen from: table B, E or H;
   (g) the polypeptide of (a), (b), (c), (d), (e), or (f) wherein wherein the. N-terminal Met residue is deleted; or
   (h) the polypeptide of (a), (b), (c), (d), (e), or (f) wherein the secretory amino acid sequence is deleted.
19. The polypeptide of embodiment 18 wherein said polypeptide is (f).
20. The polypeptide of embodiment 19 wherein said is chosen from table B.
21. The polypeptide of embodiment 20 wherein said epitope bearing portion is chosen from table C.
22. The polypeptide of embodiment 19 wherein said is chosen from table E.
23. The polypeptide of embodiment 22 wherein said epitope bearing portion is chosen from table F.
24. A chimeric polypeptide comprising two or more polypeptides chosen from table B, E or H thereof; provided that the polypeptides are linked as to form a chimeric polypeptide.
25. A vaccine composition comprising a polypeptide according to any one of embodiments 18 to 24 and a pharmaceutically acceptable carrier, diluent or adjuvant.
26. A method for therapeutic or prophylactic treatment of meningitis, otitis media, bacteremia or pneumonia infection in an individual susceptible to meningitis, otitis media, bacteremia or pneumonia infection comprising administering to said individual a therapeutic or prophylactic amount of a composition according to embodiment 25.
27. A method for therapeutic or prophylactic treatment of streptococcal bacterial infection in an individual susceptible to streptococcal infection comprising administering to said individual a therapeutic or prophylactic amount of a composition according to embodiment 25.
28. A method according to embodiment 26, wherein said individual is a mammal.
29. A method according to embodiment 27, wherein said individual is a mammal.
30. A method according to embodiment 26, wherein said individual is a human.
31. A method according to embodiment 27, wherein said individual is a human
32. A method according to embodiment 27, wherein said bacterial infection is S.pneumoniae, group A *streptococcus* (*pyogenes*), group B *streptococcus* (GBS or *agalactiae*), *dysgalactiae, uberis, nocardia* or *Staphylococcus aureus.*
33. A method according to embodiment 27, wherein said bacterial infection is S.pneumoniae.
34. Use of a vaccine composition according to embodiment 25 for the prophylactic or therapeutic treatment of Streptococcal infection in an animal susceptible to or infected with streptococcal infection comprising administering to said animal a prophylactic or therapeutic amount of the composition.

### EXAMPLE 1

This example describes the bacterial strains, plasmids, PCR primers, recombinant proteins and hybridoma antibodies used herein.

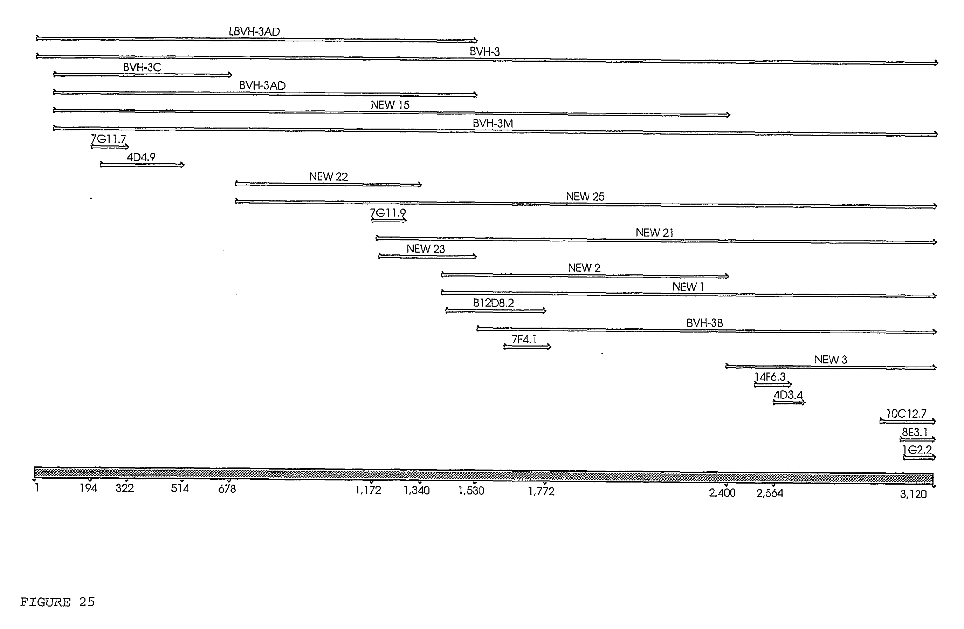

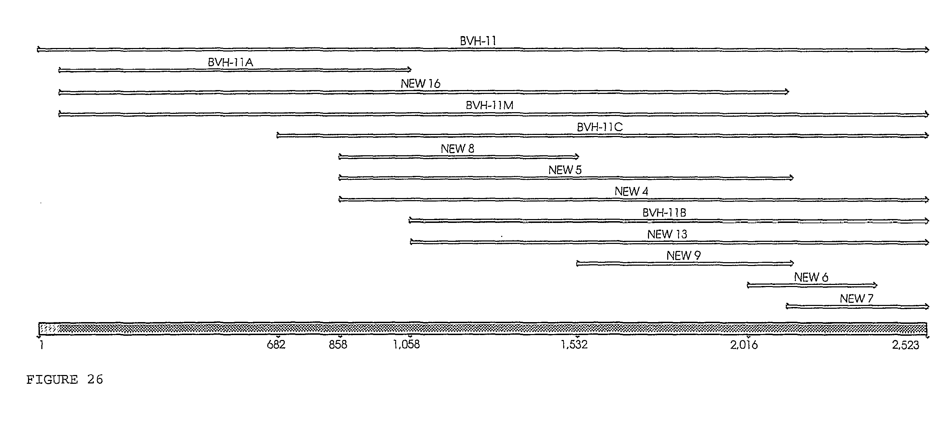

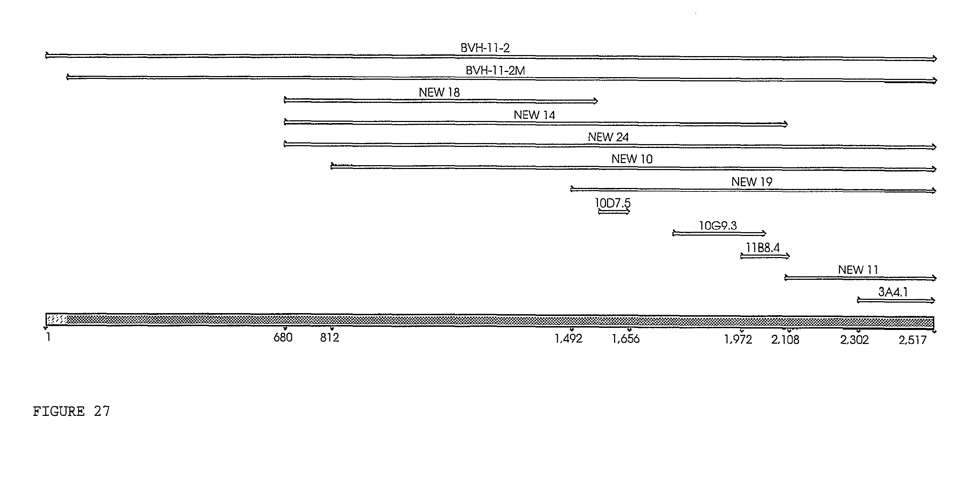

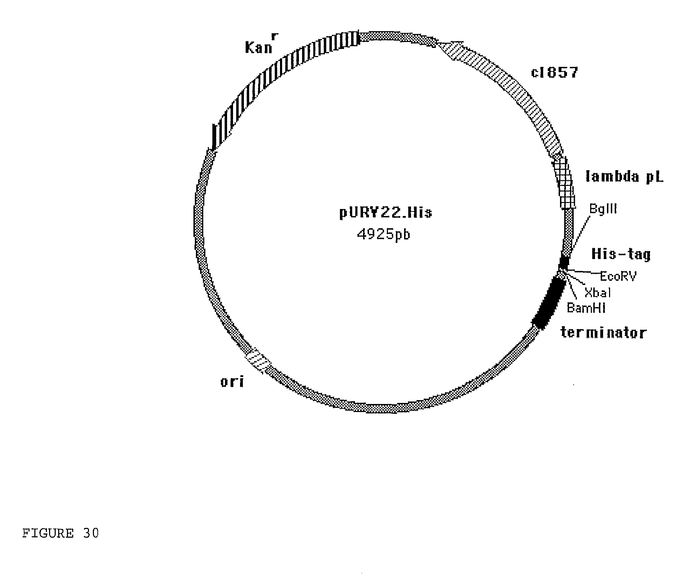

S. pneumoniae SP64 (serogroup 6) and SP63 (serogroup 9) clinical isolates were provided by the Laboratoire de la Santé Publique du Québec, Sainte-Anne-de-Bellevue; Rxl strain, a nonencapsulated derivative of the type 2 strain D39 and the type 3 strain WU2 were provided by David E. Briles from University of Alabama, Birmingham and the type 3 clinical isolate P4241 was provided by the Centre de Recherche en Infectiologie du Centre Hospitalier de l'Université Laval, Sainte-Foy. E. coli strains DH5α (Gibco BRL, Gaithesburg, MD); AD494 (λDE3) (Novagen, Madison, WI) and BL21 (λDE3) (Novagen) as well as plasmid superlinker pSL301 vector (Invitrogen, San Diego, CA); pCMV-GH vector (gift from Dr. Stephen A. Johnston, Department for Biochemistry, University of Texas, Dallas, Texas); pET32 and pET21 (Novagen) and pURV22.HIS expression vectors (Figure 30) were used in this study. The pURV22.HIS vector contains a cassette of the bacteriophage λ cI857 temperature-sensitive repressor gene from which the functional P_{R} promoter has been deleted. The inactivation of the cI857 repressor by a temperature increase from the range of 30-37°C to 37-42°C results in the induction of the gene under the control of promoter λPL. The PCR primers used for the generation of the recombinant plasmids had a restriction endonuclease site at the 5' end, thereby allowing directional cloning of the amplified product into the digested plasmid vector. The PCR oligonucleotide primers used are listed in the following Table 1. The location of the gene sequences coding for BVH-3, BVH-11 and BVH-11-2 gene products is summarized in the Figure 25, Figure 26 and Figure 27, respectively.

**Table 1. List of PCR oligonucleotide primers**

| Primer | SEQ ID NO | Sequence 5' - 3' | Nucleotide position | Restriction sites |
|---|---|---|---|---|
| OCRR 479 | 25 | cagtagatctgtgcctatgcactaaac | SEQ ID 1: 61-78 SEQ ID 9: 1-18 | Bg1II |
| OCRR 480 | 26 | gatctctagactactgctattccttacgctatg | SEQ ID 2: 4909-4887 SEQ ID 9: 2528-2519 | XbaI |
| OCRR 497 | 27 | atcactcgagcattacctggataatcctgt | SEQ ID 1: 1525-1506 | XhoI |
| OCRR 498 | 28 | ctgctaagcttatgaaagatttagat | SEQ ID 1: 1534-1548 | HindIII |
| OCRR 499 | 29 | gatactcgagctgctattccttac | SEQ ID 2: 4906-4893 | XhoI |
| HAMJ 172 | 30 | gaatctcgagttaagctgctgctaattc | SEQ ID 1: 675-661 | XhoI |
| HAMJ 247 | 31 | gacgctcgagcgctatgaaatcagataaattc | SEQ ID 1: 3117-3096 | XhoI |
| HAMJ 248 | 32 | gacgctcgagggcattacctggataatcctgttcatg | SEQ ID 1: 1527-1501 | Xhol |
| HAMJ 249 | 33 | cagtagatctcttcatcatttattgaaaagagg | SEQ ID 2: 1749-1771 | BglII |
| HAMJ 278 | 34 | ttatttcttccatatggacttgacagaagagcaaattaag | SEQ ID 1: 1414-1437 | NdeI |
| HAMJ 279 | 35 | cgccaagcttcgctatgaaatcagataaattc | SEQ ID 1: 3117-3096 | HindIII |
| HAMJ 280 | 36 | cgccaagcttttccacaatataagtcgattgatt | SEQ ID 1: 2400-2377 | HindIII |
| HAMJ 281 | 37 | ttatttcttccatatggaagtacctatcttggaaaaagaa | SEQ ID 1: 2398-2421 | NdeI |
| HAMJ 300 | 38 | ttatttcttccatatggtgcctatgcactaaaccagc | SEQ ID 1: 62-82 | NdeI |
| HAMJ 313 | 39 | ataagaatgcggccgcttccacaatataagtcgattgatt | SEQ ID 1: 2400-2377 | NotI |
| OCRR 487 | 40 | cagtagatctgtgcttatgaactaggtttgc | SEQ ID 3: 58-79 | BglII |
| OCRR 488 | 41 | gatcaagcttgctgctacctttacttactctc | SEQ ID 4: 2577-2556 | HindIII |
| HAMJ 171 | 42 | ctgagatatccgttatcgttcaaacc | SEQ ID 3: 1060-1075 | EcoRV |
| HAMJ 251 | 43 | ctgcaagcttttaaaggggaataatacg | SEQ ID 3: 1059-1045 | HindIII |
| HAMJ 264 | 44 | cagtagatctgcagaagccttcctatctg | SEQ ID 3: 682-700 | BglII |
| HAMJ 282 | 45 | tcgccaagcttcgttatcgttcaaaccattggg | SEQ ID 3: 1060-1081 | HindIII |
| HAMJ 283 | 46 | ataagaatgcggccgccttactctcctttaataaagccaat agtt | SEQ ID 3: 2520-2492 | NotI |
| HAMJ 284 | 47 | catgccatggacattgatagtctcttgaaacagc | SEQ ID 3: 856-880 | NcoI |
| HAMJ 285 | 48 | cgccaagcttcttactctcctttaataaagccaatag | SEQ ID 3: 2520-2494 | HindIII |
| HAMJ 286 | 49 | cgacaagcttaacatggtcgctagcgttacc | SEQ ID 3: 2139-2119 SEQ ID 5: 2210-2190 | HindIII |
| HAMJ 287 | 50 | cataccatgggcctttatgaggcacctaag | SEQ ID 3: 2014-2034 | NcoI |
| HAMJ 288 | 51 | cgacaagcttaagtaaatcttcagcctctctcag | SEQ ID 3: 2376-2353 | HindIII |
| HAMJ 289 | 52 | gataccatggctagcgaccatgttcaaagaa | SEQ ID 3: 2125-2146 | NcoI |
| HAMJ 290 | 53 | cgccaagcttatcatccactaacttgactttatcac | SEQ ID 3: 1533-1508 | HindIII |
| HAMJ 291 | 54 | cataccatggatattcttgccttcttagctccg | SEQ ID 3: 1531-1554 | NcoI |
| HAMJ 301 | 55 | catgccatggtgcttatgaactaggtttgc | SEQ ID 3: 59-79 | NcoI |
| HAMJ 302 | 56 | cgccaagctttagcgttaccaaaaccattatc | SEQ ID 3: 2128-2107 | HindIII |
| HAMJ 160 | 57 | gtattagatctgttcctatgaacttggtcgtcacca | SEQ ID 5: 172-196 | BglII |
| HAMJ 186 | 58 | cgcctctagactactgtataggagccgg | SEQ ID 5: 2613-2630 | XbaI |
| HAMJ 292 | 59 | catgccatggaaaacatttcaagccttttacgtg | SEQ ID 5: 925-948 | NcoI |
| HAMJ 293 | 60 | cgacaagcttctgtataggagccggttgactttc | SEQ ID 5: 2627-2604 | HindIII |
| HAMJ 294 | 61 | catgccatggttcgtaaaaataaggcagaccaag | SEQ ID 5: 2209-2232 | NcoI |
| HAMJ 297 | 62 | catgccatggaagcctattggaatgggaag | SEQ ID 5: 793-812 | NcoI |
| HAMJ 352 | 63 | catgccatggaagcctattggaatgggaagc | SEQ ID 5: 793-813 | NcoI |
| HAMJ 353 | 64 | cgccaagcttgtaggtaatttgcgcatttgg | SEQ ID 5: 1673-1653 | HindIII |
| HAMJ 354 | 65 | cgccaagcttctgtataggagccggttgac | SEQ ID 5: 2627-2608 | HindIII |
| HAMJ 355 | 66 | catgccatggatattcttgccttcttagctcc | SEQ ID 5: 1603-1624 | NcoI |
| HAMJ 404 | 67 | ttatttcttccatatgcatggtgatcatttccattaca | SEQ ID 1: 1186-1207 | NdeI |
| HAMJ 464 | 68 | gatgcatatgaatatgcaaccgagtcagttaagc | SEQ ID 1: 697-720 | NdeI |
| HAMJ 465 | 69 | gatgctcgagagcatcaaatccgtatccatc | SEQ ID 1: 1338-1318 | XhoI |
| HAMJ 466 | 70 | gatgcatatggatcatttccattacattcca | SEQ ID 1: 1192-1212 | NdeI |
| HAMJ 467 | 71 | gacaagcttggcattacctggataatcctg | SEQ ID 1: 1527-1507 | HindIII |
| HAMJ 352 | 72 | catgccatggaagcctattggaatgggaagc | SEQ ID 5: 793-813 | NcoI |
| HAMJ 470 | 73 | ataagaatgcggccgccgctatgaaatcagataaattc | SEQ ID 1: 3096-3117 | NotI |
| HAMJ 471 | 168 | atatgggcccctgtataggagccggttgactttc | SEQ ID 5: 2626-2604 | Apa I |
| HAMJ 472 | 169 | atatgggcccaatatgcaaccgagtcagttaagc | SEQ ID 1: 720-697 | Apa I |
| HAMJ 350 | 170 | atatgggcccaacatggtcgctagcgttacc | SEQ ID 3: 2139-2119 | Apa I |
| HAMJ 351 | 171 | tcccgggcccgacttgacagaagagcaaattaag | SEQ ID 1: 1414-1437 | Apa I |
| HAMJ 358 | 172 | catgccatgggacttgacagaagagcaaattaag | SEQ ID 1: 1415-1437 | NcoI |
| HAMJ 359 | 173 | tcccgggccccgctatgaaatcagataaattc | SEQ ID 1: 3116-3096 | Apa I |
| HAMJ 403 | 174 | atatgggcccgacattgatagtctcttgaaacagc | SEQ ID 3: 856-880 | Apa I |
| HAMJ 361 | 175 | cgccaagcttaacatggtcgctagcgttacc | SEQ ID 3: 2139-2119 | Hind III |
| HAMJ 483 | 176 | atatgggccccttactctcctttaataaagccaatag | SEQ ID 3: 2520-2494 | Apa I |

Molecular biology techniques were performed according to standard methods. See for example, Sambrook, J., Fritsch, E.F., Maniatis, T., "Molecular cloning. A laboratory manual" Vol.1-2-3 (second edition) Cold Spring Harbour Laboratory Press, 1989, New York, which is herein incorporated by reference. PCR-amplified products were digested with restriction endonucleases and ligated to either linearized plasmid pSL301, pCMV-GH, pET or pURV22.HIS expression vector digested likewise or digested with enzymes that produce compatible cohesive ends. Recombinant pSL301 and recombinant pCMV-GH plasmids were digested with restriction enzymes for the in-frame cloning in pET expression vector. When pET vectors were used, clones were first stabilized in E. coli DH5α before introduction into E. coli BL21 (λDE3) or AD494 (λDE3) for expression of full-length or truncated BVH-3, BVH-11 or BVH-11-2 molecules. Each of the resultant plasmid constructs was confirmed by nucleotide sequence analysis. The recombinant proteins were expressed as N-terminal fusions with the thioredoxin and His-tag (pET32 expression system); as C-terminal fusions with an His-tag (pET21 expression system); or as N-terminal fusions with an His-tag (pURV22.HIS expression system). The expressed recombinant proteins were purified from supernatant fractions obtained after centrifugation of sonicated IPIG- (pET systems) or heat-(pURV22.HIS) induced E. coli using a His-Bind metal chelation resin (QIAgen, Chatsworth, CA). The gene products generated from S. pneumoniae SP64 are listed in the following Table 2. The gene fragment encoding BVH-3-Sp63 protein (amino acid residues 21 to 840 on SEQ ID NO: 10) was generated from S. pneumoniae SP63 using the PCR-primer sets OCRR479-OCRR480 and the cloning vector pSL301. The recombinant pSL301-BVH-3Sp63 was digested for the in-frame cloning in pET32 vector for the expression of the BVH-3-Sp63 molecule.

**Table 2. Lists of truncated BVH-3, BVH-11, BVH-11-2 and Chimeric gene products generated from S. pneumoniae SP64**

| PCR-primer sets | Protein designation | Identification | Encoded amino acids (SEQ ID No6) | Cloning vector |
|---|---|---|---|---|
| OCRR479-OCRR480 | BVH-3M | BVH-3 w/oss | 21-1039 | pSL301 |
| OCRR479-OCRR497 | BVH-3AD | BVH-3 N'end w/oss | 21-509 | pSL301 |
| HAMJ248-HAMJ249 | L-BVH-3AD | BVH-3 N'end | 1-509 | pET-21(+) |
| OCRR498-OCRR499 | BVH-3B | BVH-3 C'end | 512-1039 | pSL301 |
| OCRR479-HAMJ172 | BVH-3C | BVH-3 N'end w/ oss | 21-225 | pET-32 c(+) |
| OCRR487-OCRR488 | BVH-11M | BVH-11 w/oss | 20-840 | pCMV-GH |
| HAMJ251-OCRR487 | BVH-11A | BVH-11 N'end w/ oss | 20-353 | pET-32c(+) |
| HAMJ171-OCRR488 | BVH- | BVH-11 C'end | 354-840 | pET-32 a (+) |
| HAMJ264-OCRR488 | BVH- | BVH-11 C'end | 228-840 | pET-32 a (+) |
| HAMJ278-HAMJ279 | NEW1 | BVH-3 C'end | 472-1039 | pET-21b(+) |
| HAMJ278-HAMJ280 | NEW2 | BVH-3 C'end | 472-800 | pET-21b(+) |
| HAMJ281-HAMJ279 | NEW3 | BVH-3 C'end | 800-1039 | pET-21b(+) |
| HAMJ284-HAMJ285 | NEW4 | BVH-11 C' end | 286-840 | pET-21d(+) |
| HAMJ284-HAMJ286 | NEW5 | BVH-11 | 286-713 | pET-21d(+) |
| HAMJ287-HAMJ288 | NEW6 | BVH-11 | 672-792 | pET-21d(+) |
| HAMJ285-HAMJ289 | NEW7 | BVH- I C'end | 709-840 | pET-21d(+) |
| HAMJ284-HAMJ290 | NEW8 | BVH-11 | 286-511 | pET-21d(+) |
| HAMJ286-HAMJ291 | NEW9 | BVH-11 | 511-713 | pET-21d(+) |
| HAMJ160-AMJ186 | BVH- | BVH-11-2 w/o | 20-838 | pSL301 |
| HAMJ292-HAMJ293 | NEW10 | BVH-11-2 | 271-838 | pET-21d(+) |
| HAMJ293-HAMJ294 | NEW1 | BVH-11-2 | 699-838 | pET-21d(+) |
| HAMJ282-HAMJ283 | NEW13 3 | BVH-11 C'end | 354-840 | pET-21b(+) |
| HAMJ286-HAMJ297 | NEW14 | BVH-11-2 | 227-699 | pET-21d(+) |
| HAMJ300-HAMJ313 | NEW15 | BVH-3 N'end | 21-800 | pET-21b(+) |
| HAMJ301-HAMJ302 | NEW16 | BVH-11 N'end w/ oss | 20-709 | pET-21d(+) |
| HAMJ352-HAMJ353 | NEW18 | BVH-11-2 internal | 227-520 | pET21d (+) |
| HAMJ354-HAMJ355 | NEW19 | BVH-11-2 C'end | 497-838 | pET21d(+) |
| HAMJ404-HAMJ279 | NEW21 | BVH-3 C'end | 396-1039 | pET21b(+) |
| HAMJ464-HAMJ465 | NEW22 | BVH-3 internal | 233-446 | pET-21a (+) |
| HAMJ466-HAMJ467 | NEW23 | BVH-3 internal | 398-509 | pET-21b (+) |
| HAMJ352-HAMJ293 | NEW24 | BVH-11-2 C'end | 227-838 | pET-21d (+) |
| HAMJ464-HAMJ470 | NEW25 | BVH-3 C'end | 233-1039 | pET-21b (+) |
| HAMJ278-HAMJ279 (NEW1) HAMJ282-HAMJ283 (NEW13) | NEW12 | Chimera* | M-NEW1-KL - NEW 13 | pET 21 b (+) |
| HAMJ284-HAMJ350 (NEW5) HAMJ351-HAMJ279 (NEW1) | NEW17 | Chimera* | M- NEW 5 -GP - NEW 1 | pET 21 d (+) |
| HAMJ358-HAMJ359 (NEW1) HAMJ403-HAM361 (NEW5) | NEW20 | Chimera* | M-NEW 1-GP-NEW 5 | pET 21 d (+) |
| HAMJ292-HAMJ471 (NEW10) HAMJ472-HAMJ470 (NEW25) | NEW26 | Chimera* | M- NEW 10 - GP - NEW 25 | pET21d(+) |
| HAMJ355-HAMJ471 (NEW19) HAMJ472-HAMJ470 (NEW25) | NEW27 | Chimera* | M- NEW19 -GP - NEW25 | pET21d(+) |
| HAMJ292-HAMJ47 (NE W 10) HAMJ351 - HAMJ279 (NEW1) | NEW28 | Chimera* | M- NEW 10 - GP - NEW1 | pET21d(+) |
| HAMJ284-HAMJ350 (NEW5) HAMJ472-HAMJ470 (NEW25) | NEW29 | Chimera* | M- NEW 5 -GP - NEW25 | pET21d(+) |
| HAMJ284-HAMJ483 (NEW4) HAMJ472-HAMJ470 (NEW25) | NEW30 | Chimera* | M- NEW 4 - GP-NEW25 | pET21d(+) |
| HAMJ284-HAMJ483 (NEW4) HAMJ351-HAMJ279 (NEW1) | NEW31 | Chimera* | M- NEW 4 -GP - NEW1 | pET21d(+) |
| HAMJ355-HAMJ47 (NEW19) HAMJ51-HAMJ279 (NEW1) | NEW32 | Chimera* | M- NEW 19 - GP - NEW1 | pET21d(+) |

| | | | | |
|---|---|---|---|---|
| w/oss: without signal sequence. Analysis of the BVH-3, BVH-11 and BVH-11-2 protein sequences suggested the presence of putative hydrophobic leader sequences. * encoded amino acids for the chimeras are expressed as the gene product, additional non essential amino acids residue were added M is methionine, K is lysine, L is leucine, G is glycine and P is proline. | | | | |

Monoclonal antibody (Mab)-secreting hybridomas were obtained by fusions of spleen cells from immunized mice and non-secreting, HGPRT-deficient mouse myeloma SP2/0 cells by the methods of Fazekas De St-Groth and Scheidegger (J Immunol Methods 35 : 1-21, 1980) with modifications (J. Hamel et al. J Med Microbiol 23 : 163-170, 1987). Female BALB/c mice (Charles River, St-Constant, Quebec, Canada) were immunized with either BVH-3M (thioredoxin-His•Tag-BVH-3M fusion protein/ pET32 system), BVH-11M (thioredoxin-His•Tag-BVH-11M fusion protein/ pET32 system), BVH-11-2M (thioredoxin-His•Tag-BVH-11-2M fusion protein/ pET32 system), BVH-11B (thioredoxin-His•Tag-BVH-11B fusion protein/ pET32 system), BVH-3M (His•Tag-BVH-3 fusion protein/ pURV22.HIS system) or NEW1 (NEW1-His•Tag fusion protein/ pET21 system) gene products from S. pneumoniae strain SP64 to generate the Mab series H3-, H11-, H112-, H11B-, H3V-, and HN1-, respectively. Culture supernatants of hybridomas were initially screened by enzyme-linked-immunoassay (ELISA) according to the procedure described by Hamel et al. (Supra) using plates coated with preparations of purified recombinant BVH-3, BVH-11 and/or BVH-11-2 proteins or suspensions of heat-killed S. pneumoniae cells. The Mab-secreting hybridomas selected for further characterization are listed in Table 3 and Table 4 from the following Example 2. The class and subclass of Mab immunoglobulins were determined by ELISA using commercially available reagents (Southern Biotechnology Associates, Birmingham, AL).

Furthermore, the cloning and expression of chimeric gene(s) encoding for chimeric polypeptides and the protection observed after vaccination with these chimeric polypeptides are described.

BVH-3 and BVH-11 gene fragments corresponding to the 3'end of the genes were amplified by PCR using pairs of oligonucleotides engineered to amplify gene fragments to be included in the chimeric genes. The primers used had a restriction endonuclease site at the 5' end, thereby allowing directional in-frame cloning of the amplified product into digested plasmid vectors (Table 1 and Table 2). PCR-amplified products were digested with restriction endonucleases and ligated to linearized plasmid pET21 or pSL301 vector. The resultant plasmid constructs were confirmed by nucleotide sequence analysis. The recombinant pET21 plasmids containing a PCR product were linearized by digestion with restriction enzymes for the in-frame cloning of a second DNA fragment and the generation of a chimeric gene encoding for a chimeric pneumococcal protein molecule. Recombinant pSL301 plasmids containing a PCR product were digested with restriction enzymes for the obtention of the DNA inserts. The resulting insert DNA fragments were purified and inserts corresponding to a given chimeric gene were ligated into pET21 vector for the generation of a chimeric gene. The recombinant chimeric polypeptides listed in Table 2 were as C-terminal fusion with an His-tag. The expressed recombinant proteins were purified from supernatant fractions obtained from centrifugation of sonicated IPTG-induced E. coli cultures using a His-Bind metal chelation resin (QIAgen, Chatsworth, CA).

Groups of 8 female BALB/c mice (Charles River) were immunized subcutaneously two times at three-week intervals with 25 µg of either affinity purified His•Tag-fusion protein identified in presence of 15-20 µg of QuilA adjuvant. Ten to 14 days following the last immunization, the mice were challenged challenged intravenously with 10E5-10E6 CFU of S. pneumoniae type 3 strain WU2. The polypeptides and fragments are capable of eliciting a protective immune response.

**Table 2A**

| Experiment | Immunogen | Alive: Dead | Days to death post-infection |
|---|---|---|---|
| 1 | none | 0 : 8 | 1, 1, 1, 1, 1, 1, 1, 1 |
| | NEW 1 | 2 : 6 | 1, 2, 2, 2, 2, 2, >14, >14 |
| | NEW 13 | 1 : 7 | 1, 1, 3, 3, 4, 5, 5, >14 |
| | NEW 12 | 6 : 2 | 3, 11, 6X >14 |
| | BVH-3M | 1 : 7 | 3, 3, 3, 3, 3, 3, 3, >14 |
| | | | |
| 2 | none | 0 : 8 | 1, 1, 1, 1, 1, 1, 1, 1 |
| | NEW 17 | 7 : 1 | 4, 7 X >14 |
| | NEW 12 | 3 : 5 | 3, 3, 3, 4, 5, > 14, > 14, > 14 |
| | | | |
| 3 | none | 0 : 8 | 2, 2, 2, 2, 2, 2, 2, 2 |
| | NEW 18 | 1 : 7 | 2, 2, 2, 2, 3, 3, 3, 3 |
| | NEW 19 | 8 : 0 | 8 X >14 |
| | NEW 10 | 8 : 0 | 8 X >14 |
| | BVH-11-2 | 8 : 0 | 8 X >14 |

### EXAMPLE 2

This example describes the identification of peptide domains carrying target epitopes using Mabs and recombinant truncated proteins described in example 1.

Hybridomas were tested by ELISA against truncated BVH-3, BVH-11 or BVH-11-2 gene products in order to characterize the epitopes recognized by the Mabs. The truncated gene products were generated from S. pneumoniae SP64 strain except for BVH-3-Sp63 which was generated from S. pneumoniae SP63 strain. As a positive control, the reactivity of each antibody was examined with full-length BVH-3, BVH-11 or BVH-11-2 recombinant proteins. In some cases, the Mab reactivity was evaluated by Western immunoblotting after separation of the gene product by SDS-PAGE and transfer on nitrocellulose paper. The reactivities observed is set forth in the following Table 3 and Table 4.

**Table 3. ELISA reactivity of BVH-3-reactive Mabs with a panel of eleven BVH-3 gene products and the BVH-11M molecule**

| | Gene products tested | | | | | | | | | | | |
|---|---|---|---|---|---|---|---|---|---|---|---|---|
| Mabs (IgG isotype) | BVH-3M | BVH-3AD | BVH-3B | BVH-3C | NEW 1 | NEW 2 | NEW 3 | NEW 21 | NEW 22 | NEW 23 | BVH-3 SP63 | BVH-11M |
| H3-4F9 (1) | + | + | - | + | - | - | - | - | - | - | + | + |
| H3-4D4 (1) | + | + | - | + | - | - | - | - | - | - | + | + |
| H3-9H12 (1) | + | + | - | + | - | - | - | - | - | - | + | + |
| H3-7G2 (1) | + | + | - | - | - | - | - | - | + | - | - | - |
| H3-10A1 (1) | + | + | - | - | - | - | - | + | - | + | + | - |
| H3-4D3 (1) | + | - | + | - | + | - | + | + | - | - | + | - |
| H11-6E7 (1) | + | + | - | + | - | - | - | NT | NT | NT | + | + |
| H11-10H10 (2a) | + | + | - | + | - | - | - | NT | NT | NT | + | + |
| H11-7G11 (2b) | + | + | + | + | + | + | - | NT | NT | NT | + | + |
| H3V-4F3 (1) | + | - | + | - | + | - | - | + | - | - | + | - |
| H3V-2F2 (1) | + | - | + | - | + | + | - | + | - | - | + | - |
| H3V-7F4 (1) | + | - | + | - | + | + | - | + | - | - | + | - |
| H3V-7H3 (1) | + | - | + | - | + | - | + | + | - | - | + | - |
| H3V-13B8 (1) | + | - | + | - | + | - | + | + | - | - | + | - |
| H3V-9C2 (1) | + | + | - | +/- | - | - | - | - | + | - | +/- | +/- |
| H3V-9C6 (1) | + | + | - | - | - | - | - | - | + | - | - | - |
| H3V-16A7 (1) | + | + | - | - | - | - | - | + | - | + | - | - |
| H3V-15A10 (1) | + | + | + | +/- | + | + | - | + | + | + | + | +/- |
| H3V-6B3 (1/2) | + | + | NT | NT | + | + | - | + | + | - | NT | - |
| HN1-SH3 (2b) | + | - | + | NT | + | - | - | + | - | - | + | - |
| HN1-8E3 (2a) | + | - | + | NT | + | - | - | + | - | - | + | - |
| HN1-14F6 (2a) | + | - | + | NT | + | - | - | + | - | - | + | - |
| HN1-2G2 (1) | + | - | + | NT | + | + | - | + | - | - | + | - |
| HNl-12D8(2a) | + | - | + | NT | + | + | - | + | - | - | + | - |
| HN1-14B2 (2a) | + | - | + | NT | + | + | - | + | - | - | + | - |
| HN1-1G2 (2a) | + | - | + | NT | + | - | + | + | - | - | + | - |
| HN1-10C12 (1) | + | - | + | NT | + | - | + | + | - | - | + | - |
| HN1-3E5 (1) | + | + | - | - | + | + | - | + | - | + | + | - |

| | | | | | | | | | | | | |
|---|---|---|---|---|---|---|---|---|---|---|---|---|
| NT: not tested +/- : very low reactivity but higher than background, possible non-specific Mab binding | | | | | | | | | | | | |

**Table 4. ELISA reactivity of BVH-11 and/or BVH-11-2-reactive Mabs with a panel of fourteen BVH-11 and BVH-11-2 gene products and the BVH-3M molecule**

| | Gene products tested | | | | | | | | | | | | | | | |
|---|---|---|---|---|---|---|---|---|---|---|---|---|---|---|---|---|
| Mabs (IgG isotype) | BVH-11M | BVH-11A | BVH11B | BVH-11C | NEW 5 | NEW 6 | NEW 7 | NEW 8 | NEW 9 | NEW 10 | NEW 11 | NEW 14 | NEW 18 | NEW 19 | BVH-11-2-M | BVH-3M |
| H3-4F9 (1) | + | + | - | - | - | - | - | - | - | - | - | - | - | - | + | + |
| H3-4D4 (1) | + | + | - | - | - | - | - | - | - | - | - | - | - | - | + | + |
| H3-9H12 (1) | + | + | - | - | - | - | - | - | - | - | - | - | - | - | + | + |
| H11-6E7 (1) | + | + | - | - | - | - | - | - | - | - | - | - | - | - | + | + |
| H11-10H10 (2a) | + | + | - | - | - | - | - | - | - | - | - | - | - | - | + | + |
| H11-7G11 (2b) | + | + | - | - | - | - | - | - | - | - | - | - | - | - | + | + |
| H11-1B12 (1) | + | + | - | - | - | - | - | - | - | - | - | - | - | - | + | - |
| H11-7B9 (2a) | + | + | - | - | - | - | - | - | - | - | - | - | - | - | + | - |
| H11-3H5 (1) | + | - | + | + | + | - | - | -* | - | + | - | + | + | - | + | - |
| H11-10B8(1) | + | - | + | + | + | - | - | -* | - | + | - | + | + | - | + | - |
| H11-1A2 (1) | + | - | + | + | + | - | - | -* | - | + | - | + | + | - | + | - |
| H112-3A1 (1) | + | - | + | NT | + | - | - | + | - | + | - | + | + | - | + | - |
| H112-13C11 (1) | + | +/- | + | NT | + | - | - | + | - | + | - | + | + | - | + | - |
| H112-10H10 (1) | + | + | - | NT | + | - | - | + | - | + | - | + | + | - | + | - |
| H112-1D8 (2a) | + | + | - | NT | + | - | - | + | - | + | - | + | + | - | + | - |
| H112-10G9 92b) | + | - | + | NT | + | - | - | - | + | + | - | + | - | + | + | - |
| H112-10A2(1) | + | - | + | NT | + | - | - | +/- | + | + | - | + | - | + | + | - |
| H112-3E8 (2a) | + | - | + | NT | + | - | - | +/- | - | + | - | + | - | + | + | - |
| H112-10D7 (2a) | + | - | + | NT | + | - | - | - | - | + | - | + | - | - | + | - |
| H112-2H7 (2a) | + | + | - | NT | - | - | - | - | - | - | - | - | - | - | + | - |
| H112-6H7 (1) | + | + | - | NT | - | - | - | - | - | - | - | - | - | - | + | - |
| H112-3A4 (2a) | - | - | - | NT | - | - | - | - | - | + | + | - | - | + | + | - |
| H112-10C5(1) | - | - | - | NT | - | - | - | - | - | + | + | - | - | + | + | - |
| H112-14H6 (1) | - | - | - | NT | - | - | - | - | - | + | + | - | - | + | + | - |
| H112-7G2 (1) | - | - | - | NT | - | - | - | - | - | + | - | + | + | - | + | - |
| H112-13H10 (2a) | - | - | - | NT | - | - | - | - | - | - | - | + | + | - | + | - |
| H112-7E8 (2B) | +/- | - | - | NT | - | - | - | - | - | - | - | - | +/- | - | + | - |
| H112-7H6 (1) | +/- | - | - | NT | - | - | - | - | - | +/- | - | - | - | - | + | - |
| H11B-5F10(1) | + | - | + | + | + | - | - | + | - | + | - | + | + | - | + | - |
| H11B-15G2 (1) | + | - | + | + | + | - | - | + | - | + | - | + | + | - | + | - |
| H11B-13D5 (2) | + | - | + | + | + | - | - | - | + | + | - | + | - | + | + | - |
| H11B-11B8 (1) | + | - | + | + | + | - | - | - | + | + | - | + | - | + | + | - |
| H11B-7E11 (1) | + | - | + | + | + | - | - | - | - | + | - | + | - | - | + | - |
| H11B-1C9 (1) | + | - | + | + | + | - | - | - | - | + | - | + | - | - | + | - |
| H11B-5E3 (2) | + | - | + | + | - | - | + | - | - | - | - | - | - | - | - | - |
| H11B-6E8 (1) | + | - | + | + | - | - | + | - | - | - | - | - | - | - | - | - |

| | | | | | | | | | | | | | | | | |
|---|---|---|---|---|---|---|---|---|---|---|---|---|---|---|---|---|
| NT : not tested +/- : very low reactivity but higher than background, possible non-specific Mab binding * : a strong signal was detected when tested by Western immunoblotting | | | | | | | | | | | | | | | | |

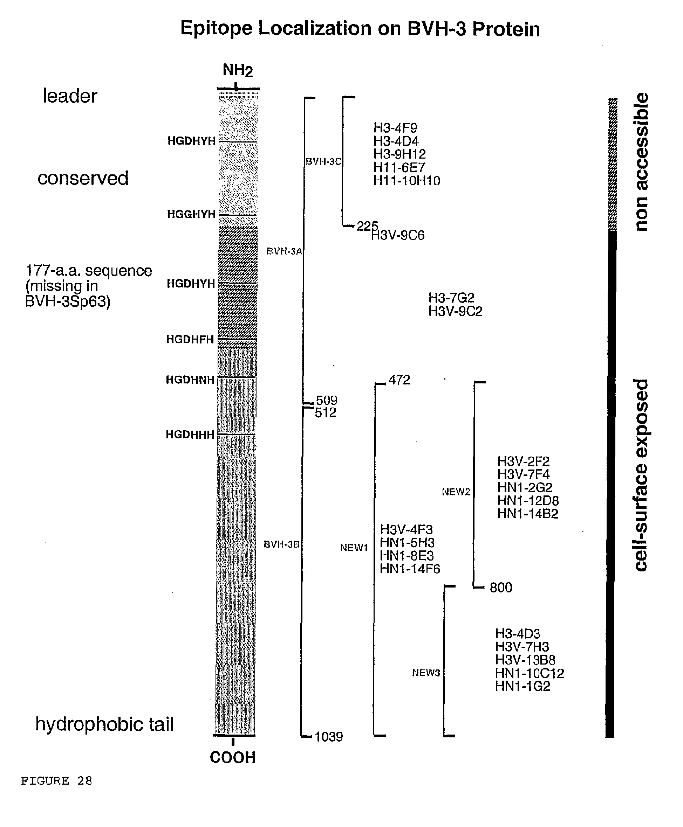

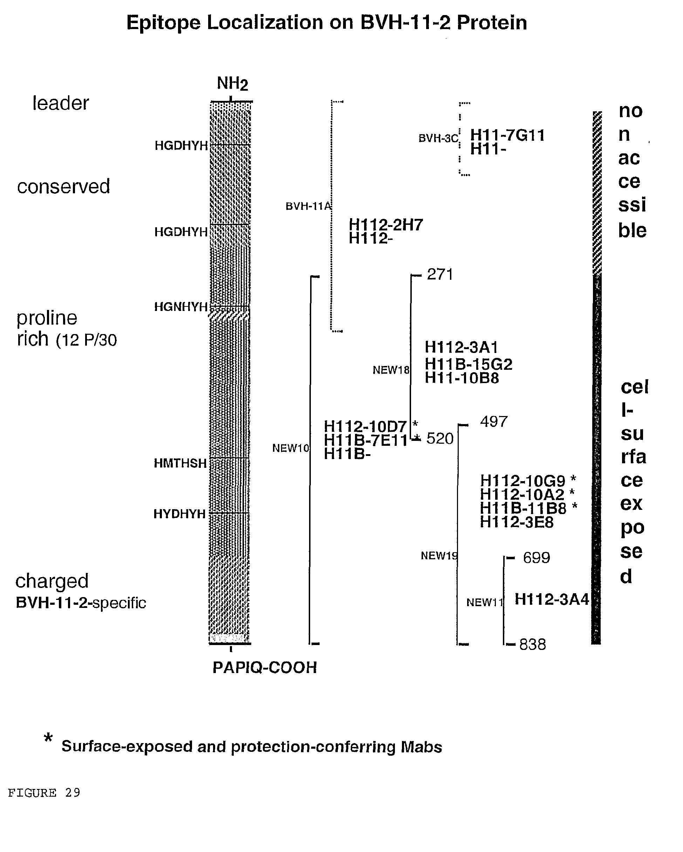

The deduced locations of the epitopes are summarized in Figure 28 and Figure 29. As can be seen from the data in Table 3, BVH-3-reactive Mabs can be divided into two groups : BVH-3A and BVH-3B-reactive Mabs with the exception of Mabs H11-7G11 and H3V-15A10 which reacted with both, BVH-3A and BVH-3B molecules. The BVH-3A-reactive Mabs can be subdivided in two subgroups of antibodies depending of their reactivity or lack of reactivity with BVH-3C recombinant protein. Mab reactive with BVH-3C protein recognized epitopes shared by both, BVH-3 and BVH-11 proteins. As can be seen in Table 4, these BVH-3 and BVH-11-cross-reactive Mabs were also reactive with BVH-11A and BVH-11-2M recombinant proteins. BVH-3B-reactive Mabs can be subdivided into three subgroups according to their reactivity with NEW1, NEW2 and NEW3 recombinant proteins. Some Mabs were only reactive with the NEW1 protein while other Mabs were reactive with either, NEW1 and NEW2 or NEW1 and NEW3 recombinant proteins.

Mabs H11-7G11 and H3V-15A10 react with epitopes in more than one position on BVH-3. The reactivity of H11-7G11 with BVH-3AD, BVH-3B, BVH-3C, BVH-11A and BVH-11-2M molecules suggests that H11-7G11 epitope might comprised HXXHXH sequence. This sequence is repeated, respectively, 6 and 5 times in BVH-3 and BVH-11/BVH-11-2 protein sequences. The lack of reactivity of Mab H11-7G11 with NEW 10 molecule suggests that the epitope includes the HGDHXH sequence. Multiple-position mapping of H3V-15A10 epitope on BVH-3 is suggested by the reactivity of the Mab with two BVH-3 fragments that do not overlap.

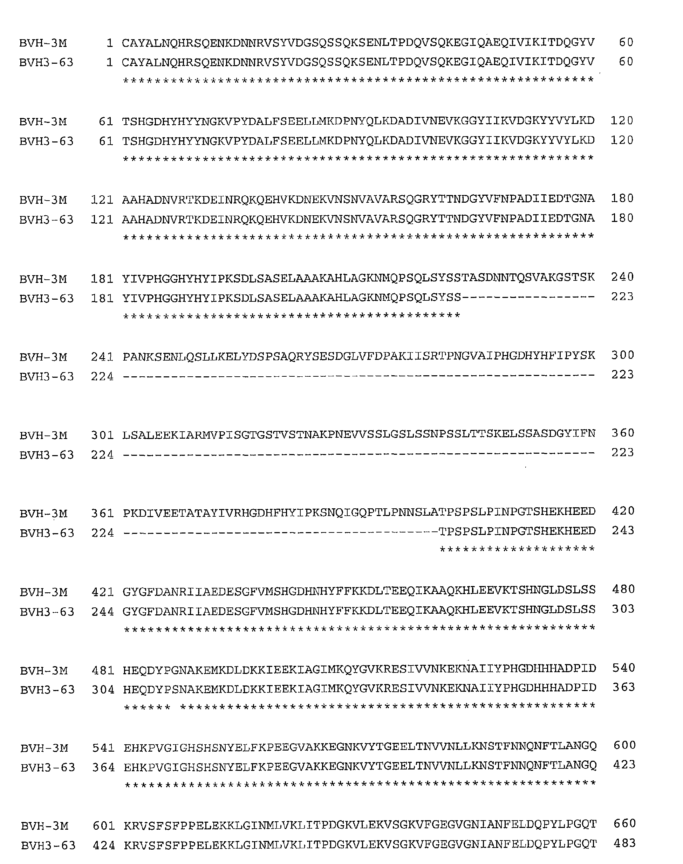

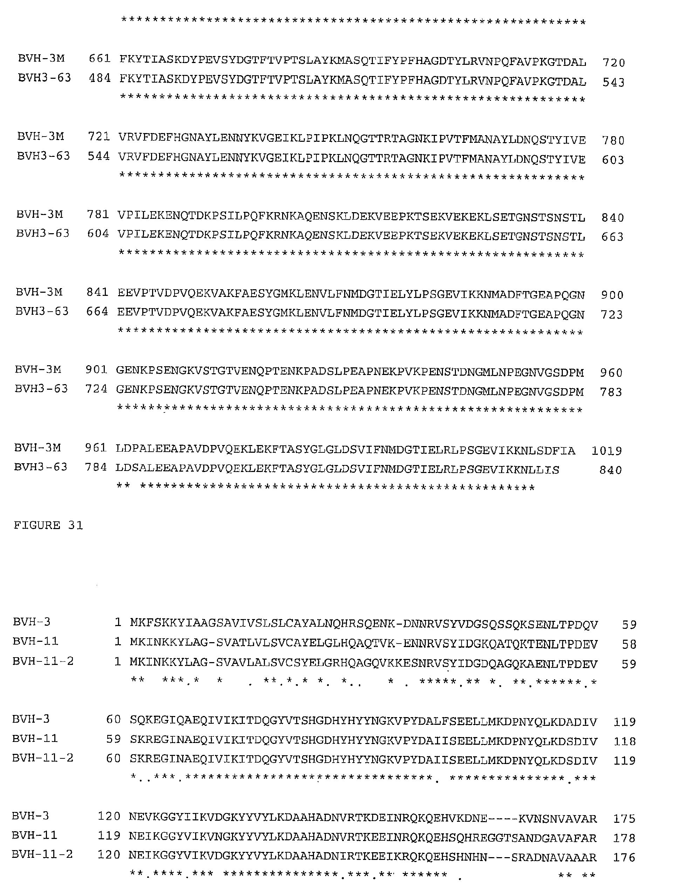

Interestingly, Mabs H3-7G2, H3V-9C6 and H3V-16A7 were not reactive with BVH-3 Sp63 thus allowing the location of their corresponding epitopes on a 177-amino acid fragment comprised between amino acids 244 and 420 on BVH-3 molecule of S. pneumoniae SP64 (Figure 31).

As can be seen from the data in Table 4, the Mabs that are reactive with BVH-11 and/or BVH-11-2 and that do not recognize BVH-3 molecules can be divided into three groups according to their reactivities with BVH-11A and NEW10 recombinant proteins. Some Mabs reacted exclusively with either BVH-11A or NEW10 protein while other Mabs were reactive with both, BVH-11A and NEW10 recombinant proteins.

### EXAMPLE 3

This example describes the construction of BVH-3 and BVH-11-2 gene libraries for the mapping of epitopes.

BVH-3 and BVH-11-2 gene libraries were constructed using recombinant pCMV-GH and PSL301 plasmid DNA containing respectively, BVH-3 gene sequence spanning nucleotides 1837 to 4909 (SEQ ID NO: 2) or BVH-11-2 gene sequence spanning nucleotides 172 to 2630 (SEQ ID NO: 5) and the Novatope^{®} library construction and screening system (Novagen). The recombinant plasmids containing BVH-3 or BVH-11-2 gene fragment were purified using QIAgen kit (Chatsworth, CA) and digested with the restriction enzymes BgIII and XbaI respectively. The resulting BgIII-XbaI DNA fragments were purified using the QIAquick gel extraction kit from QIAgen and digested with Dnase I for the generation of randomly cleaved DNA. DNA fragments of 50 to 200 bp were purified, treated with T4 DNA polymerase to blunt the target DNA ends and add a single 3' dA residue, and ligated into pSCREEN-T-Vector (Novagen) following the procedures suggested by the manufacturer (Novatope^{®} System, Novagen). The gene libraries of E. coli clones, each of which expressing a small peptide derived from BVH-3 or BVH-11-2 genes were screened by standard colony lift methods using Mabs as immunoprobes. The colony screening was not successful with Mabs producing very high backgrounds on colony lifts. Moreover, in some cases, Mabs failed to detect epitope-expressing-colonies. The lack of reactivity can possibly be explained by the small amount of recombinant proteins produced or the recognition of conformation-dependent epitopes consisting of different protein domains. Sequencing of DNA inserts from positive clones determined the location of the segment that encodes the target epitope. The data are presented in Table 5. The peptides encoded by DNA inserts into the recombinant pSCREEN-T vector can be purified and used as immunogens as described below in Example 6.

The peptide sequences obtained from the screening of BVH-3 and BVH-11-2 gene libraries with the Mabs are in agreement with the Mab ELISA reactivities against the truncated gene products. As expected, the amino acid sequences obtained from H11-7G11 contained the sequence HGDHXH. These findings provide additional evidence for the location of epitopes recognized with the Mabs. Interestingly, although the Mabs H112-10G9, H112-10A2 and H11B-11B8 were reactive against the same peptide sequence (amino acid residues 594 to 679 on BVH-11-2 protein sequence), clones corresponding to the sequence spanning from amino acid residues 658 to 698 were only picked up by Mab H11B-11B8 thus revealing the location of H11B-11B8 epitope between amino acid residues 658 to 679 (SEQ ID NO: 163). Mabs H112-10G9, H112-10A2, and H11B-11B8 are directed against 3 distinct non overlapping epitopes located closely on the peptide sequence corresponding to amino acid residues 594 to 679 (SEQ ID NO: 22).

**Table 5. Peptide sequences obtained from the screening of BVH-3 and BVH-11-2 gene libraries with Mabs**

| Mab | Clone/ Protein designation | Nucleotide position | Amino acid position | Amino acid sequence | SEQ ID NO |
|---|---|---|---|---|---|
| H3-4D4 | 4D4.9 | SEQ ID 1: 226-509 | SEQ ID 6: 76-169 | DQGYVTSHGDHYHYYNGKVPYDALFSEELLMKDPNYQLKDA DIVNEVKGGYIIKVDGKYYVYLKDAAHADNVRTKDEINRQK QEHVKDNEKVNS | 11 |
| H11-7G11 | 7G11.7 | SEQ ID 1:193-316 | SEQ ID 6: 64-105 | GIQAEQIVIKITDQGYVTSHGDHYHYYNGKVPYDALFSEELL | 12 |
| H11-7G11 | 7G11.9 | SEQ ID 1: 1171-1284 | SEQ ID 6: 390-428 | TAYIVRHGDHFHYIPKSNQIGQPTLPNNSLATPSPSLPI | 13 |
| H3-4D3 | 4D3.4 | SEQ ID 1: 2565-2670 | SEQ ID 6: 855-890 | TSNSTLEEVPTVDPVQEKVAKFAESYGMKLENVLFN | 14 |
| HN1-8E3 | 8E3.1 | SEQ ID 1: 3004-3120 | SEQ ID 6: 1016-1039 | MDGTIELRLPSGEVIKKNLSDFIA | 15 |
| HN1-1G2 | 1G2.2 | sEQ ID 1: 3017-3120 | sEQ ID 6: 1005-1039 | YGLGLDSVIFNMDGTIELRLPSGEVIKKNLSDFIA | 16 |
| HN1-10C12 | 10C12.7 | sEQ ID 1: 2936-3120 | sEQ ID 6: 983-1039 | PALEEAPAVDPVQEKLEKFTASYGLGLDSVIFNMDGTIELR LPSGEVIKKNLSDFIA | 17 |
| HN1-14F6 | 14F6.3 | SEQ ID 1: 2501-2618 | SEQ ID 6: 833-872 | KVEEPKTSEKVEKEKLSETGNSTSNSTLEEVPTVDPVQEK | 18 |
| HN1-12D8 | B12D8.2 | sEQ ID 1: 1433-1767 | sEQ ID 6: 512-589 | MKDLDKKIEEKIAGIMKQYGVKRESIVVNKEKNAIIYPHGD HHHADPIDEHKPVGIGHSHSNYELFKPEEGVAKKEGN | 19 |
| H3V-7F4 | 7F4.1 | SEQ ID 1: 1633-1785 | SEQ ID 6: 545-595 | AIIYPHGDHHHADPIDEHKPVGIGHSHSNYELFKPEEGVAK KEGNKVYTGE | 20 |
| H112-10D7 | 10D7.5 | SEQ ID 5: 1685-1765 | SEQ ID 8: 525-553 | IQVAKLAGKYTTEDGYIFDPRDITSDEGD | 21 |
| H112-10G9 | 10G9.3 | SEQ ID 5: 1893-2150 | SEQ ID 8: 594-679 | DHQDSGNTEAKGAEAIYNRVKAAKKVPLDRMPYNLQYTVEV KNGSLIIPHYDHYHNIKFEWFDEGLYEAPKGYSLEDLLATV KYYV | 22 |
| H112-10A2 | 10A2.2 | SEQ ID 5: 1893-2150 | SEQ ID 8: 594-679 | DHQDSGNTEAKGAEAIYNRVKAAKKVPLDRMPYNLQYTVEV KNGSLIIPHYDHYHNIKFEWFDEGLYEAPKGYSLEDLLATV KYYV | 22 |
| H11-11B8 | B11B8.1 | SEQ ID 5: 1893-2150 | SEQ ID 8: 594-679 | DHQDSGNTEAKGAEAIYNRVKAAKKVPLDRMPYNLQYTVEV KNGSLIIPHYDHYHNIKFEWFDEGLYEAPKGYSLEDLLATV KYYV | 22 |
| H11B-11B8 | 11B8.4 | SEQ ID 5: 2085-2217 | SEQ ID 8: 658-698 | GLYEAPKGYSLEDLLATVKYYVEHPNERPHSDNGFGNASDH | 23 |
| H112-3A4 | 3A4.1 | SEQ ID 5: 2421-2626 | SEQ ID 8: 769-837 | VENSVINAKIADAEALLEKVTDPSIRQNAMETLTGLKSSLL LGTKDNNTISAEVDSLLALLKESQPAPI | 24 |

### EXAMPLE 4

This example describes the immunization of animals with recombinant proteins for the generation of antibody reactive with BVH-3, BVH-11 and/or BVH-11-2.

NZW rabbits (Charles River Laboratories, St-Constant, Québec, Canada) were immunized subcutaneously at multiple sites with 50 µg or 100 µg of the purified BVH-3M, L-BVH-3AD, NEW1, NEW13, or L-BVH-11 recombinant protein in presence of 80 µg of QuilA adjuvant (Cedarlane Laboratoratories Ltd, Hornby, Canada). The rabbits were boosted two times at three-week intervals with the same antigen and blood samples were collected before each immunization and 6 to 28 days following the last immunization. The sera samples were designated preimmune, post 1^{st}, post 2^{nd} or post 3^{rd} injection. The rabbit immune response to immunization was evaluated by ELISA using recombinant BVH-3M (BVH-3M-His•Tag fusion protein/ pET21 system) or BVH-11M (BVH-11M-His•Tag fusion protein/ pET21 system) proteins or suspensions of heat-killed S. pneumoniae Rx-1 cells as coating antigens. ELISA titer was defined as the reciprocal of the highest sera dilution at which absorbance A₄₁₀ value was 0.1 above the background value. Antibodies reactive with BVH-3 and/or BVH-11 epitopes were elicited following immunization in all animals as shown in the following Table 6. Antibody reactive with recombinant or pneumococcal antigens was not present in the preimmune sera. The immune response to immunization was detectable in the sera of each rabbit after a single injection of recombinant antigen. The antibody response following the second injection with either antigen tested was characterized by a strong increase in antibody titer. Interestingly, good titers of antibody reactive with S. pneumoniae cells, with an average titer of 52,000 after the third immunization, were obtained, thus establishing that native pneumococcal epitopes are expressed on the recombinant E. coli gene products. These data support the potential use of BVH-3, BVH-11 and/or BVH-11-2 gene products and the antibody raised to BVH-3, BVH-11 and/or BVH-11-2 gene products as vaccines for the prevention and the treatment of pneumococcal disease, respectively.

**Table 6. Rabbit Antibody response to immunization with BVH-3 and BVH-11 gene products**

| | | | ELISA Titer with coating antigen | | |
|---|---|---|---|---|---|
| Rabbit | Immunogen | Sera sample | BVH-3M | BVH-11M | S. pneumoniae |
| #15 | BVH-3M (50µg) | Preimmune | NT | NT | 200 |
| | | Post-1^{st} | NT | NT | 1,600 |
| | | Post-2^{nd} | NT | NT | 20,000 |
| | | Post-3^{rd} | 512,000 | NT | 40,000 |
| #16 | BVH-3M (100µg) | Preimmune | NT | NT | 200 |
| | | post 1^{st} | NT | NT | 1,600 |
| | | post 2^{nd} | NT | NT | 40,000 |
| | | post 3^{rd} | 10⁶ | NT | 80,000 |
| #112 | L-BVH-3AD (50 µg) | Preimmune | <100 | NT | NT |
| | | post 1^{st} | 16,000 | NT | NT |
| | | post 2^{nd} | 512,000 | NT | NT |
| | | post 3^{rd} | 2x10⁶ | NT | 32,000 |
| #113 | New 1 (50 µg) | Preimmune | <100 | NT | NT |
| | | post 1^{st} | 16,000 | NT | NT |
| | | post 2^{nd} | 512,000 | NT | NT |
| | | post 3^{rd} | 10⁶ | NT | 64,000 |
| #114 | New 13 (50 µg) | Preimmune | NT | <100 | NT |
| | | post 1^{st} | NT | 16,000 | NT |
| | | post 2^{nd} | NT | 64,000 | NT |
| | | post 3^{rd} | NT | 256,000 | 32,000 |
| #116 | L-BVH-11 (50 µg) | Preimmune | NT | <100 | NT |
| | | post 1^{st} | NT | 64,000 | NT |
| | | post 2^{nd} | NT | 10⁶ | NT |
| | | post 3^{rd} | NT | 2x10⁶ | 64,000 |

| | | | | | |
|---|---|---|---|---|---|
| NT: not tested | | | | | |

### EXAMPLE 5

This example describes the protection of animals against fatal experimental pneumococcal infection by administration of antibody raised to BVH-3, BVH-11 or BVH-11-2 gene products.

High-titer Mab preparations were obtained from ascites fluid of mice inoculated intraperitoneally with Mab-secreting hybridoma cells according to the method described by Brodeur et al (J Immunol Methods 71: 265-272,1984). Sera samples were collected from rabbits immunized with BVH-3M as described in Example 4. The rabbit sera collected after the third immunization and ascites fluid were used for the purification of antibodies by precipitation using 45 to 50% saturated ammonium sulfate. The antibody preparations were dissolved and dialyzed against phosphate-buffered saline (PBS).

CBA/N (xid) mice (National Cancer Institute, Frederick, MA) were injected intraperitoneally with either 0.1 ml of purified rabbit antibodies or 0.2 ml of ascites fluid before intravenous challenge with approximately 200 CFU of the type 3 S. pneumoniae strain WU2. Control mice received sterile PBS or antibodies purified from preimmune rabbit sera or sera from rabbits immunized with an unrelated N. meningitidis recombinant protein antigen. One group of mice was challenged with S. pneumoniae before the administration of anti-BVH-3 antibody. Samples of the S. pneumoniae challenge inoculum were plated on chocolate agar plates to determine the number of CFU and verify the challenge dose. The CBA/N mice were chosen because of their high susceptibility to S. pneumoniae infection. The LD₅₀ of WU2 injected intravenously to CBA/N mice is estimated to be ≤10 CFU. Deaths were recorded at 24-h intervals for a period of at least 7 days.

The protection data obtained from mice injected with rabbit anti-BVH-3 antibody are set forth in the following Table 7. Nine out of 10 mice receiving the anti-BVH-3 antibody survived the challenge in contrast to none of 10 mice injected with control antibody or PBS buffer. The observation that antibody raised to the BVH-3-M molecule passively protected even when administered after the challenge demonstrated the ability of anti-BVH-3 antibody to prevent death even from an already established infection.

**Table 7. Protective effects of rabbit antibody to BVH-3-M gene in CBA/N mice challenged i.v. with WU2 pneumococci**

| Antibody preparation | Time of antibody administration | Alive : Dead | Days to death post-infection |
|---|---|---|---|
| Anti-BVH3M | 1 h before infection | 5 : 0 | >14, >14, >14, >14, >14 |
| Anti-N. meningitidis | 1 h before infection | 0 : 5 | 2, 2, 2, 2, 2 |
| Anti-BVH-3M | 0. 5 h post-infection | 4 : 1 | 2, >14, >14, >14, >14 |
| None (PBS) | 1 h before infection | 0 : 5 | 1, 2, 2, 2, 2 |

CBA/N mice were infected with 1000 CFU of WU2 S. pneumoniae before or after intraperitoneal administration of 0.1 ml of rabbit antibody.

In an other experiment, 0.1 ml of rabbit antibody prepared from preimmune and immune sera were administered intraperitoneally to CBA/N mice four hours before intranasal challenge with 280 CFU of S. pneumoniae P4241 type 3 strain. As seen in the following Table 8, all immunized mice survived the challenge while none of 9 mice receiving preimmune sera antibody or buffer alone were alive on day 6 post-infection. S. pneumoniae hemocultures on day 11 post-challenge were negative for all surviving mice. Furthermore, 100% protection was observed in mice receiving monoclonal antibodies H112-10G9 or a mixture of H112-10G9 and H11B-7E11 which are directed against BVH-11/BVH-11-2.

**Table 8. Protective effects of passive transfer of rabbit antibody to BVH-3-M gene product or anti-BVH-11/BVH-11-2 specific Mabs in CBA/N mice challenged i.n. with P4241 pneumococci**

| Antibody preparation | Alive : Dead | Days to death post-infection |
|---|---|---|
| Anti-BVH-3M | 5 : 0 | >11, >11, >11, >11, >11 |
| Antibody from preimmune sera | 0 : 5 | 3, 3, 3, 6, 6 |
| H112-10G9 | 4 : 0 | >11, >11,>11,>11 |
| H112-10G9+H11B-7E11 | 5 : 0 | >11, >11, >11, >11, >11 |
| None (PBS) | 0 : 4 | 3, 3, 3, 3 |

Altogether, the results from Table 7 and Table 8 clearly establish that immunization of animals with a BVH-3 gene product such as BVH-3M elicited protective antibodies capable of preventing experimental bacteremia and pneumonia infections.

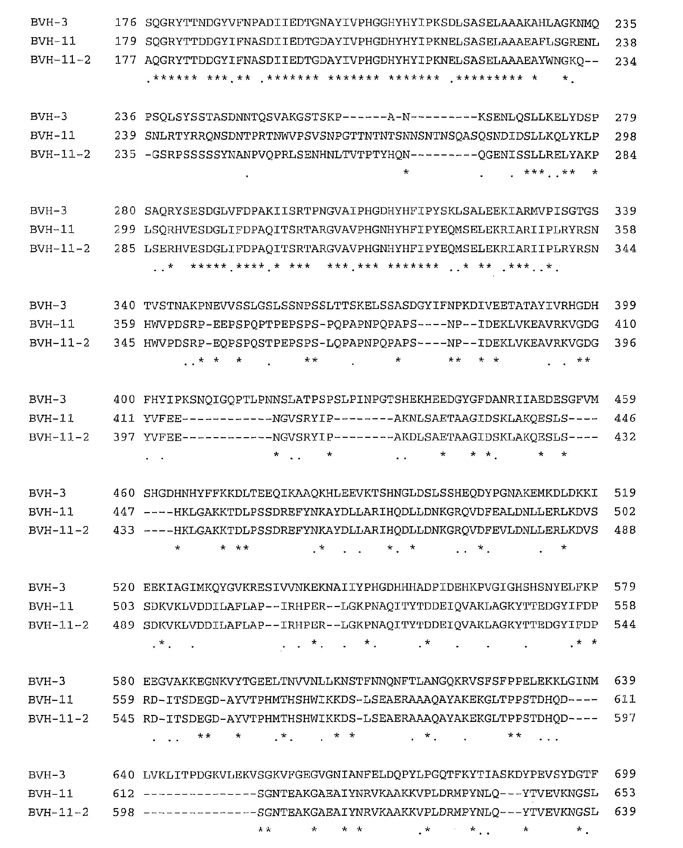

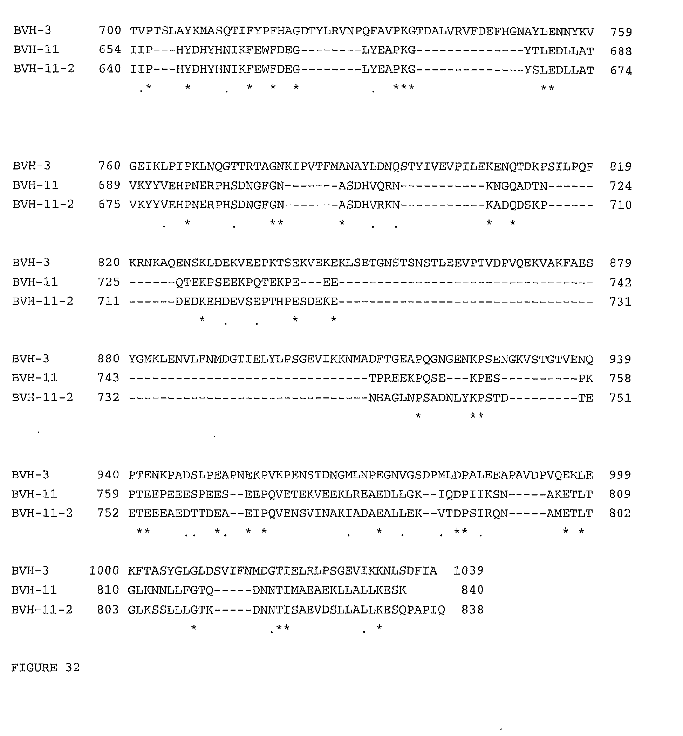

The protection data obtained for mice injected with ascites fluid are set forth in the following Table 9. Administration of a volume of 0.2 ml of ascites fluid of 0.2 ml of some sets of ascites fluid prevented death from experimental infection. For example, H112-3A4 + H112-10G9 and H112-10G2 + H112-10D7 sets of 2 Mabs conferred complete protection against experimental infection. These data indicated that antibody targetting BVH-11 and/or BVH-11-2 epitopes gave efficient protection. The Mabs H112-3A4, H112-10G9, H112-10D7, H112-10A2, H112-3E8, H112-10C5, H11B-11B8, H11B-15G2, H11B-1C9, H11B-7E11, H11B-13D5 and H11-10B8 were present in at least one protective pair of Mabs and were said to be protective and reactive against protective epitopes. The locations of protection-conferring epitopes on BVH-11-2 molecules are summarized in Table 10 and Figure 29. Protective Mabs H112-3A4, H112-10G9, H112-10D7, H112-10A2, H112-3E8, H112-10C5, H11B-11B8, H11B-15G2, H11B-1C9, H11B-7E11, H11B-13D5 and H11-10B8 were all reactive with New 10 protein corresponding to amino acid residues 271 to 838 on the BVH-11-2 molecule. Six out of these 12 Mabs were directed against epitopes present in the NEW 19 protein and 3 protective Mabs recognized NEW 14. Interestingly, Mab H112-3A4 and H112-10C5 reacted with distinct epitopes exclusive to BVH-11-2 located at the carboxyl end comprised between amino acid residues 769 and 837. Also, Mabs H11-7G11, H11-6E7 and H3-4F9 reactive with epitopes shared by pneumococcal BVH-3, BVH-11 and BVH-11-2 molecules did not succeed to protect even if given in combination with protective H112-10G9 or H112-11B8 Mab. These Mabs recognized epitopes located at the amino end of the BVH-3, BVH-11 and BVH-11-2 molecules comprising, respectively, the first 225, 228 and 226 amino acid residues. The comparison of the BVH-3, BVH-11 and BVH-11-2 protein sequences revealed that a large number of amino acids were conserved in the amino end portion comprising these 225-228 residues with a global 72.8% identity (Figure 32).

Altogether the data set forth in Table 9 and Table 10 suggest that the protection eliciting BVH-11- and BVH-11-2-epitopes is comprised in the carboxy terminal product containing amino acids 229 to 840 and 227 to 838, on BVH-11 and BVH-11-2 proteins, respectively.

**Table 9. Passive immunization with BVH-11- and/or BVH-11-2-specific Mabs can protect mice from lethal experimental pneumococcal infection.**

| Experiment | Mab | Alive : Dead | Days to death post-infection |
|---|---|---|---|
| 1 | H112 3A4 + H112-10G9 | 6 : 0 | 6 X >10 |
| | H112-3A4 + H112-10D7 | 5 : 1 | 4, 5X >10 |
| | None | 0 : 6 | 2, 2, 2, 2, 2, 6 |
| 2 | H112-10 A2 + H112-10D7 | 5 : 1 | 3, 5X >10 |
| | H112-3E8+H112-10G9 | 6 : 0 | 6 X >10 |
| | None | 0 : 6 | 2, 2, 2, 2, 2, 2 |
| 3 | H112-10D7+H11B-11B8 | 6 : 0 | 6 X >10 |
| | H112-10G9 + H11B-15G2 | 3 : 3 | 2, 6, 6, 3 X >10 |
| | None | 0 : 6 | 2, 2, 2, 2, 2, 2 |
| 4 | H112-10G9+H112-10D7 | 5 : 0 | 5 X >11 |
| | None | 0 : 5 | 2, 2, 2, 2, 2 |
| 5 | H112-10G9 + H11-10B8 | 4 : 1 | 8, 4 X >14 |
| | H112-10G9 + H11B-7E11 | 5 : 0 | 5 X >4 |
| | None | 0 : 3 | 1, 2, 2 |
| 6 | H112-10G9 + H11B-1C9 | 4 : 1 | 4, 4 X >14 |
| | None | 0 : 3 | 2, 2, 2 |
| 7 | H112-10C5 + H11B-13D5 | 5 : 0 | 5 X >14 |
| | None | 3 : 3 | 2, 2, 2 |

| | | | |
|---|---|---|---|
| CBA/N mice were injected intraperitoneally with a total of 0.2 ml of ascites fluid 4 hours before intravenous challenge with S. pneumoniae WU2. | | | |

**Table 10. Deduced locations of protection-conferring epitopes on BVH-11-2 molecules.**

| Mabs | Protection | Gene products carrying Mab-epitope |
|---|---|---|
| H112-3A4 | + | NEW 19 and NEW 11 |
| H112-10G9 | + | NEW 19 |
| H112-10D7 | + | NEW 14 and NEW 10 |
| H112-10A2 | + | NEW 19 |
| H112-3E8 | + | NEW 19 |
| H11B-11B8 | + | NEW 19 |
| H11B-15G2 | + | NEW 18 |
| H11B-7E11 | + | NEW 14 and NEW 10 |
| H11-10B8 | + | NEW 18 |
| H11B-1C9 | + | NEW 14 and NEW 10 |
| H112-3A1 | - | NEW 18 and NEW 8 |
| H112-10H10 | - | NEW 18 and NEW 8 |
| H112-2H7 | - | BVH-11-2M |
| H112-6H7 | - | BVH-11-2M |
| H11-7G11 | - | BVH-11A and BVH-3C |
| H11-6E7 | - | BVH-11A and BVH-3C |
| H112-10C5 | + | NEW 19, NEW 11 and 3A4.1 |
| H11B-13D5 | + | NEW 19 |
| H112-7G2 | - | NEW 18 |
| H112-7E8 | - | BVH-11-2M |
| H3-4F9 | - | BVH-11A and BVH-3C |

Altogether the data presented in this example substantiate the potential use of antibodies raised to BVH-3, BVH-11 or BVH-11-2 molecules as therapeutic means to prevent, diagnose or treat S. pneumoniae diseases.

### EXAMPLE 6

This example describes the localization of surface-exposed peptide domains using Mabs described in Example 1.

S. pneumoniae type 3 strain WU2 was grown in Todd Hewitt (TH) broth (Difco Laboratories, Detroit MI) enriched with 0.5% Yeast extract (Difco Laboratories) at 37°C in a 8% CO₂ atmosphere to give an OD₆₀₀ of 0.260 (∼10⁸ CFU/ml). The bacterial suspension was then aliquoted in 1 ml samples and the S . pneumoniae cells were pelletted by centrifugation and resuspended in hybridoma culture supernatants. The bacterial suspensions were then incubated for 2 h at 4°C. Samples were washed twice in blocking buffer [PBS containing 2% bovine serum albumin (BSA)], and then 1 ml of goat fluorescein (FITC)-conjugated anti-mouse IgG + IgM diluted in blocking buffer was added. After an additional incubation of 60 min at room temperature, samples were washed twice in blocking buffer and fixed with 0.25 % formaldehyde in PBS buffer for 18-24 h at 4°C. Cells were washed once in PBS buffer and resuspended in 500 µl of PBS buffer. Cells were kept in the dark at 4°C until analyzed by flow cytometry(Epics^{®} XL; Beckman Coulter, Inc.). Ten thousands (10,000) cells were analyzed per sample and the results were expressed as % Fluorescence and Fluorescence index (FI) values. The % Fluorescence is the number of fluorescein-labelled S. pneumoniae cells divided by 100 and the FI value is the median fluorescence value of pneumococci treated with Mab supernatant divided by the fluorescence value of pneumococci treated with the conjugate alone or with a control unrelated Mab. A FI value of 1 indicated that the Mab has not been detected at the surface of the bacteria whereas a FI value higher than 2 was considered positive when at least 10 % of the pneumococcal cells were labelled and indicated that the Mab was reactive with cell-surface exposed epitopes. The following Table 11 summarized the data obtained with the Mabs tested by flow cytometry.

Flow cytometric analysis revealed that the Mabs reactive with BVH-3C and/or BVH-11A molecules did not bind to the cell surface. In contrast, with the exception of Mabs H3V-9C6 and H3V-16A7, the Mabs reactive with NEW 1, NEW 2, NEW 3, NEW 22 or NEW 23 BVH-3 gene products were detected at the surface of pneumococci. These data indicated that the first 225 amino acid residues located at the amino end of BVH-3 are internal. The lack of binding of Mabs H3V-9C6 and H3V-16A7 suggest some portions of the sequence corresponding to the 177-amino acids absent from the BVH-3 molecule of S. pneumoniae SP63 appears not to be accessible to antibodies.

Results from BVH-11- and/or BVH-11-2-reactive Mabs revealed that there is a good correlation between surface-exposure and protection. All Mabs reactive with internal epitopes as determined by the flow cytometry assay were not protective whereas all the protective Mabs described in Example 5 gave a positive signal in flow cytometry. Although an FI value of 9.0 and a % Fluorescence of 81.2 were obtained with Mab H11-7G11, this Mab was not shown to protect. Additional assays can be used to further evaluate whether this Mab and its corresponding epitope might participate in anti-infectious immunity.

**Table 11. Results from the binding of Mabs at the surface of S. pneumoniae by flow cytometry analysis**

| Mab | % Fluorescence | FI | Binding | Gene products carrying Mab-epitope |
|---|---|---|---|---|
| H3-4F9 | 3.4 | 1.2 | - | BVH-3C and BVH-11A |
| H3-4D4 | 3.4 | 1.2 | - | BVH-3C and BVH-11A |
| H3-9HI2 | 2.5 | 1.1 | - | BVH-3C and BVH-11A |
| H3-7G2 | 66.2 | 6.3 | + | NEW 22 |
| H3-10A1 | 58.8 | 5.6 | + | NEW 23 |
| H3-4D3 | 33.2 | 3.5 | + | NEW 3 |
| H3V-4F3 | 24.4 | 2.9 | + | NEW 1 |
| H3V-2F2 | 15.6 | 2.4 | + | NEW 2 |
| H3V-7F4 | 58.7 | 5.6 | + | NEW 2 |
| H3V-7H3 | 68.8 | 6.9 | + | NEW 3 |
| H3V-13B8 | 75.0 | 7.7 | + | NEW 3 |
| H3V-9C2 | 66.4 | 6.2 | + | NEW 22 |
| H3V-9C6 | 2.9 | 1.0 | - | NEW 22 |
| H3V-16A7 | 6.6 | 1.7 | - | NEW 23 |
| H3V-15A10 | 58.7 | 5.7 | + | NEW 22 and NEW 23 |
| HN1-5H3 | 43.4 | 5.3 | + | NEW 1 |
| HN1-8E3 | 57.4 | 6.6 | + | NEW 1 |
| HN1-14F6 | 57.8 | 6.7 | + | NEW 1 |
| HN1-2G2 | 54.8 | 6.3 | + | NEW 2 |
| HN1-12D8 | 14.3 | 3.0 | + | NEW 2 |
| HN1-14B2 | 11.5 | 2.7 | + | NEW 2 |
| HN1-1G2 | 59.9 | 7.0 | + | NEW 3 |
| HN1-10C12 | 13.6 | 2.8 | + | NEW 3 |
| H11-6E7 | 4.9 | 1.2 | - | BVH-3C and BVH-11A |
| H11-10H10 | 6.5 | 1.6 | - | BVH-3C and BVH-11A |
| H11-7G11 | 81.2 | 9.0 | + | BVH-3C and NEW 2 |
| H11-1B12 | 3.1 | 1.2 | - | BVH-11A |
| H11-7B9 | 2.4 | 1.1 | - | BVH-11A |
| H11-10B8 | 81.1 | 9.1 | + | NEW 18 and NEW 8 |
| H11-1A2 | 84.4 | 10 | + | NEW 18 and NEW 8 |
| H11-3H5 | 84.0 | 9.8 | + | NEW 18 and NEW 8 |
| H112-13C11 | 49.3 | 5.9 | + | NEW 18 and NEW 8 |
| H112-10H10 | 0.4 | 1.0 | - | BVH-11A and NEW 18 |
| H112-1D8 | 0.4 | 1.0 | - | BVH-11A and NEW 18 |
| H112-10G9 | 78.9 | 10.4 | + | NEW 19 |
| H112-10A2 | 75.5 | 9.6 | + | NEW 19 |
| H112-3E8 | 62.5 | 7.5 | + | NEW 19 |
| H112-10D7 | 64.5 | 7.7 | + | NEW 14 |
| H112-2H7 | 0.7 | 1.1 | - | BVH-11A |
| H112-6H7 | 0.3 | 1.0 | - | BVH-11A |
| H112-3A4 | 70.1 | 8.9 | + | NEW 11 |
| H112-10C5 | 86.3 | 9.2 | + | NEW 11 AND 3A4.1 |
| H112-14H6 | 89.6 | 11 | + | NEW 11 |
| H112-14H6 | 0.8 | 1.4 | - | NEW 11 |
| H112-7G2 | 4.7 | 2.0 | - | NEW 18 |
| H112-13H10 | 0.5 | 1.0 | - | NEW 18 |
| H112-7E8 | 0.4 | 1.0 | - | BVH-11-2M |
| H112-7H6 | 0.2 | 1.0 | - | BVH-11-2M |
| H11B-5F10 | 3.1 | 1.1 | - | NEW 18 |
| H11B-15G2 | 60.2 | 5.7 | + | NEW 18 and NEW 8 |
| H11B-13D5 | 75.7 | 8.3 | + | NEW 19 |
| H11B-11B8 | 78.4 | 8.3 | + | NEW 19 |
| H11B-7E11 | 32.3 | 3.5 | + | NEW 14 |
| H11B-1C9 | 57.3 | 5.5 | + | NEW 14 |
| H11B-5E3 | 1.8 | 1.0 | - | NEW 7 |
| H11B-6E8 | 2.4 | 1.0 | - | NEW 7 |

### EXAMPLE 7

This example describes the immunization of animals with peptide epitopes of BVH-3 and BVH-11-2.

The recombinant pSCREEN-T vector (Novagen, Madison, WI) containing DNA fragment (nucleotides 2421 to 2626 on SEQ ID NO : 5) encoding the Mab 3A4-epitope (SEQ ID NO: 24) was transformed by electroporation (Gene Pulser II apparatus, BIO-RAD Labs, Mississauga, Canada) into E. coli Tuner (λDE3) pLysS [BL21 (F'ompT hsdSB (rB⁻mB⁻) gal dcm lacYI pLysS (Cm^{r})] (Novagen). In this strain, the expression of the fusion protein is controlled by the T7 promoter which is recognized by the T7 RNA polymerase (present on the λDE3 prophage, itself under the control of the lac promoter inducible by isopropy β-D-thiogalactopyranoside (IPTG). The pLysS plasmid reduces the basal fusion protein expression level by coding for a T7 lysozyme, which is a natural inhibitor of the T7 RNA polymerase.

The transformants were grown at 37°C with 250 RPM agitation in LB broth (peptone 10g/l, yeast extract 5g/l, NaCl 5g/l) supplemented with 50mM glucose, 100µg/ml carbenicillin and 34 µg/ml chloramphenicol, until the absorbance at 600nm reached a value of 0,7. The overexpression of T7gene 10 protein-His•Tag-3A4.1 fusion protein was then induced by the addition of IPTG to a final concentration of 1mM and further incubation at 25°C with 250 RPM agitation for 3 hours. Induced cells from a 800-ml culture were pelleted by centrifugation and frozen at -70°C. The fusion protein was purified from the soluble cell fraction by affinity chromatography based on the binding of a six histidine residues sequence (His-Tag) to divalent cations (Ni²⁺) immobilized on a metal chelation Ni-NTA resin (Qiagen, Mississauga, Canada). Briefly, the pelleted cells were thawed and resuspended in Tris buffered sucrose solution (50mM Tris, 25% (w/v) sucrose) and frozen at -70°C for 15 minutes. Cells were incubated 15 minutes on ice in the presence of 2mg/ml lysozyme before disruption by sonication. The lysate was centrifuged at 12000 RPM for 30 minutes and Nickel charged Ni-NTA resin (QIAgen) was added to the supernatant for an overnight incubation at 4°C, with 100 RPM agitation. After washing the resin with a buffer consisting of 20mM Tris, 500mM NaCl, 20mM imidazole pH 7,9, the fusion 3A4.1 protein was eluted with the same buffer supplemented with 250mM imidazole. The removal of the salt and imidazole was done by dialysis against PBS at 4°C. The protein concentration was determined with BCA protein assay reagent kit (Perce, Rockford, IL) and adjusted to 760 µg/ml.

To evaluate whether immunization with an epitope peptide sequence could confer protection against disease, groups of 6 female CBA/N (xid) mice (National Cancer Institute) are immunized subcutaneously three times at three-week intervals with affinity purified T7gene10 protein-His•Tag-3A4.1 fusion protein or, as control, with QuilA adjuvant alone in PBS. Twelve to fourteen days following the third immunization, the mice are challenged intravenously with S. pneumoniae WU2 strain or intranasally with P4241 strain. Samples of the S. pneumoniae challenge inoculum are plated on chocolate agar plates to determine the number of CFU and to verify the challenge dose. The challenge dose are approximately 300 CFU. Deaths are recorded daily for a period of 14 days and on day 14 post-challenge, the surviving mice are sacrificed and blood samples tested for the presence of S. pneumoniae organisms. The 3A4.1 protein or other tested protein is said protective when the number of mice surviving the infection or the median number of days to death is significantly greater in the 3A4.1-immunized group compared to the control mock-immunized group.

### EXAMPLE 8

This example illustrates the improvement of the antibody response to pneumococci using BVH-3 fragments and variants thereof.

The combined results obtained from studies of Mab reactivity with truncated gene products, epitope-expressing colonies and live intact pneumococci presented in examples 2, 3 and 6, allowed to delineate between surface-exposed and internal epitopes. The epitopes detected by Mabs that efficiently bound to pneumococci cells mapped to a region comprised between amino acid residues 223 to 1039 of BVH-3 described in SEQ ID NO 6. The existence of protective epitopes in the BVH-3-carboxyl half was confirmed by demonstrating that mice immunized with NEW1 molecule were protected from fatal infection with P4241 strain.

Gene sequence comparison revealed that in some strains, the region of BVH-3 encoding for amino acids 244 to 420 as described in SEQ ID NO6 is absent thus suggesting the lack of utility of this sequence in vaccine to prevent disease caused by such strains (SEQ ID NO: 9 versus SEQ ID NO: 1). Further BVH-3 fragments or variants thereof were designed in the purpose to develop a universal highly effective vaccine that would target the immune response to ubiquitous surface-exposed protective epitopes. BVH-3 gene fragments designated NEW1 (encoding amino acid residues 472 to 1039 from SEQ ID NO: 6) and NEW40 (encoding amino acid residues 408 to 1039 from SEQ ID NO: 6) were amplified from the S. pneumoniae strain SP64 by PCR using pairs of oligonucleotides engineered for the amplification of the appropriate gene fragment. Each of the primers had a restriction endonuclease site at the 5' end, thereby allowing directional in-frame cloning of the amplified product into the digested plasmid vector. PCR-amplified products were digested with restriction endonucleases and ligated to linearized plasmid pET21 (Novagen) expression vector digested likewise. Oligonucleotide primers HAMJ489(ccgaattccatatgcaaattgggcaaccgactc; NdeI) and HAMJ279 (cgccaagcttcgctatgaaatcagataaattc; HindIII) were used for the NEW 40 construction. Clones were first stabilized in E. coli DH5α before introduction into E. coli BL21(λDE3) for expression of the truncated gene products. Variants from NEW1 and NEW40 were generated by mutagenesis using the Quickchange Site-Directed Mutagenesis kit from Stratagene and the oligonucleotides designed to incorporate the appropriate mutation. The presence of 6 histidine tag residues on the C-terminus of the recombinant molecules simplified the purification of the proteins by nickel chromatography. The following tables 12 and 13 describe the sequences of the primers used for the mutagenesis experiments and the variant gene products generated, respectively. Mutagenesis experiments using primer sets 39, 40, 46, 47 or 48 resulted in silent changes and were performed in the purpose of improving the expression of the desired gene or gene fragment since it was observed that during the course of expression, BVH-3 gene and fragments of, shorter secondary translation initiation products were coexpressed.

**Table 12. List of PCR oligonucleotide primer sets used for site-directed mutagenesis on BVH-3 gene truncates**

| Primer set | Primer identification | SEQ ID No | Primer SEQUENCE 5' --- > 3' |
|---|---|---|---|
| 9 | HAMJ513 | 177 | GAATCAGGTTTTGTCATGAGTTCCGGAGACCACAATCATTATTTC GAAATAATGATTGTGGTCTCCGGAACTCATGACAAAACCTGATTC |
| | HAMJ514 | 178 | |
| 10 | HAMJ515 | 179 | GTCATGAGTTCCGGAGACTCCAATCATTATTTCTTCAAGAAGG CCTTCTTGAAGAAATAATGATTGGAGTCTCCGGAACTCATGAC |
| | HAMJ516 | 180 | |
| 11 | HAMJ517 | 181 | ATGAGTTCGGAGACTCCAATTCTTATTTCTTCAAGAAGGACTTG CAAGTCCTTCTTGAAGAAATAAGAATTGGAGTCTCCGGAACTCAT |
| | HAMJ518 | 182 | |
| 14 | CHAN51 | 183 | GCGATTATTTATCCGTCTGGAGATCACCATCATGC GCATGATGGTGATCTCCAGACGGATAAATAATCGC |
| | CHAN52 | 184 | |
| 17 | CHAN53 | 185 | CCGTCTGGAGATGGCCATCATGCAGATCCG CGGATCTGCATGATGGCCATCTCCAGACGG |
| | CHAN54 | 186 | |
| 19 | CHAN47 | 187 | CCGCAGGGAGATAAGCGTCATGCAGATCCGATTG CAATCGGATCTGCATGACGCTTATCTCCCTGCGG |
| | CHAN48 | 188 | |
| 20 | CHAN55 | 189 | CCGTCTGGAGATGGCACTCATGCAGATCCGATTG CAATCGGATCTGCATGAGTGCCATCTCCAGACGG |
| | CHAN56 | 190 | |
| 22 | CHAN57 | 191 | CCGTCTGGAGATGGCACTTCTGCAGATCCGATTGATG CATCAATCGGATCTGCAGAAGTGCCATCTCCAGACGG |
| | CHAN58 | 192 | |
| 23 | HAMJ523 | 193 | CCGCATGGAGATGGCCATCATGCAGATCCG CGGATCTGCATGATGGCCATCTCCATGCGG |
| | HAMJ524 | 194 | |
| 24 | HAMJ526 | 195 | GTCATGAGTCACGGAGACTCCAATCATTATTTCTTCAAGAAGG CCTTCTTGAAGAAATAATGATTGGAGTCTCCGTGACTCATGAC |
| | HAMJ527 | 196 | |
| 25 | HAMJ528 | 197 | ATGAGTCACGGAGACCACAATTCTTATTTCTTCAAGAAGGACTTG CAAGTCCTTCTTGAAGAAATAAGAATTGTGGTCTCCGTGACTCAT |
| | HAMJ529 | 198 | |
| 29 | HAMJ569 | 199 | TACCTCATTATGACTCTTACTCTAACATCAAATTTGAGTGGTTTG CAAACCACTCAAATTTGATGTTAGAGTAAGAGTCATAATGAGGTA |
| | HAMJ570 | 200 | |
| 30 | HAMJ571 | 201 | TACCTTCTTATGACCATTACTCTAACATCAAATTTGAGTGGTTTG AAACCACTCAAATTTGATGTTAGAGTAATGGTCATAAGAAGGTA |
| | HAMJ572 | 202 | |
| 31 | HAMJ573 | 203 | AACGGTAGTTTAATCATACCTTCTAAAGACCATTACCATAACATC GATGTTATGGTAATGGTCTTTAGAAGGTATGATTAAACTACCGTT |
| | HAMJ574 | 204 | |
| 32 | HAMJ575 | 205 | CGGTAGTTTAATCATACCTCATAAGGACTCTTACCATAACATCAAA TTTGATGTTATGGTAAGAGTCCTTATGAGGTATGATTAAACTACCG |
| | HAMJ576 | 206 | |
| 33 | HAMJ577 | 207 | AACGGTAGTTTAATCATACCTGACCATTACCATAACATCAAATTTG CAAATTTGATGTTATGGTAATGGTCAGGTATGATTAAACTACCGTT |
| | HAMJ578 | 208 | |
| 34 | HAMJ579 | 209 | AACGGTAGTTTAATCATACCTTACCATAACATCAAATTTGAGTGG CCACTCAAATTTGATGTTATGGTAAGGTATGATTAAACTACCGTT |
| | HAMJ580 | 210 | |
| 3 5 | HAMJ581 | 211 | ACCGGTAGTTTAATCATACCTAACATCAAATTTGAGTGGTTTGAC GTCAAACCACTCAAATTTGATGTTAGGTATGATTAAACTACCGTT |
| | HAMJ582 | 212 | |
| 37 | HAMJ536 | 213 | CCTATGTAACTCCACATATAACCCATAGCCACTGG CCAGTGGCTATGGGTTATATGTGGAGTTACATAGG |
| | HAMJ537 | 214 | |
| 39 | HAMJ550 | 215 | CGTGAAAGTATTGTCGTAAATAAAGAAAAAAATGCG CGCATTTTTTTCTTTATTTACGACAATACTTTCACG |
| | HAMJ551 | 216 | |
| 40 | HAMJ586 | 217 | CATGAAGAAGATGGTTACGGTTTCGATGCTAACCGTATTATCGCTGAAG CTTCAGCGATAATACGGTTAGCATCGAAACCGTAACCATCTTCTTCTG |
| | HAMJ587 | 218 | |
| 41 | HAMJ588 | 219 | GAATCAGGTTTTGTCATGAGTGACCACAATCATTATTTCTTC GAAGAAATAATGATTGTGGTCACTCATGACAAAACCTGATTC |
| | HAMJ589 | 220 | |
| 42 | HAMJ590 | 221 | GAAGATGAATCAGGTTTTGTCATGAGTAATCATTATTTCTTCAAG CTTGAAGAAATAATGATTACTCATGACAAAACCTGATTCATCTTC |
| | HAMJ591 | 222 | |
| 43 | HAMJ592 | 223 | GAAGATGAATCAGGTTTTGTCATGAGTATTTCTTCAAGAAGGAC GTCCTTCTTGAAGAAATAACTCATGACAAAACCTGATTCATCTTC |
| | HAMJ593 | 224 | |
| 44 | HAMJ594 | 225 | AAAATGCGATTATTTATCCGCACCATCATGCAGATCCGATTG CAATCGGATCTGCATGATGGTGCGGATAAATAATCGCATTTT |
| | HAMJ595 | 226 | |
| 45 | HAMJ600 | 227 | AAAATGCGATTATTTATCCGGCAGATCCGATTGATGAACATAAAC GTTTATGTTCATCAATCGGATCTGCCGGATAAATAATCGCATTTT |
| | HAMJ601 | 228 | |
| 46 | HAMJ604 | 229 | GATGCTAACCGTATAATCGCCGAAGACGAATCAGGTTTTGTCATG CATGACAAAACCTGATTCGTCTTCGGCGATTATACGGTTAGCATC |
| | HAMJ605 | 230 | |
| 47 | HAMJ606 | 231 | CGCCGAAGACGAATCCGGCTTTGTAATGAGTCACGGAGACTCC GGAGTCTCCGTGACTCATTACAAAGCCGGATTCGTCTTCGGCG |
| | HAMJ607 | 232 | |
| 48 | HAMJ608 | 233 | CATCTCATGAACAGGATTATCCCGGCAACGCCAAAGAAATGAAAG CTTTCATTTCTTTGGCGTTGCCGGGATAATCCTGTTCATGAGATG |
| | HAMJ609 | 234 | |

**Table 13. Lists of truncated variant BVH-3 gene products generated from S. pneumoniae SP64**

| Protein designation | Gene/ Protein SEQ ID NO | Protein Identification* | PCR primer set (ref. table 12) | Gene used for mutagenesis |
|---|---|---|---|---|
| NEW1-mutl** | 255 | NEW1 | 39 | NEW1 |
| NEW35A | 256 | NEW1 550-SGDGTS-555 | 14, 17, 20, 22 | NEW1 |
| NEW42 | 257 | NEW40 55-SGDSNS-60 144-SGDGTS-149 | 9, 10, 11, 14, 17, 20, 22 | NEW40 |
| NEW49 | 258 | NEW40 55-SGDHNH-60 | 9 | NEW40 |
| NEW50 | 259 | NEW40 55-SGDSNH-60 | 10 | NEW49 |
| NEW51 | 260 | NEW40 55-SGDHNH-60 144-SGDHHH-149 | 14 | NEW49 |
| NEW52 | 261 | NEW40 55-SGDSNH-60 144-SGDGHN-149 10, | 10, 17 | NEW51 |
| NEW53 | 262 | NEW40 55-HDGHNH-60 144-SGDHHH-149 | 14 | NEW40 |
| NEW54 | 263 | NEW40 55-SGDHNH-60 144-SGDGHH-149 | 17 | NEW53 |
| NEW55 | 264 | NEW1 550-HGDGHH-555 | 23 | NEW1 |
| NEW56 | 265 | NEW40 55-HGDSNH-60 144-SGDHHH-149 | 24 | NEW53 |
| NEW56-mut2** | 266 | NEW56 | 40 | NEW56 |
| NEW56-mut3** | 267 | NEW56 | 46, 47, 48 | NEW56 |
| NEW57 | 268 | NEW40 55-HGDHNS-60 144-SGDHHH-149 | 25 | NEW53 |
| NEW63 | 269 | NEW40 55-HGDSNH-60 144-HGDHHH-149 | 24 | NEW40 |
| NEW64 | 270 | NEW40 55-HGDHNS-60 144-HGDHHH-149 | 25 | NEW40 |
| NEW65 | 271 | NEW40 55-HGDSNH-60 144-HGDGHH-149 | 23 | NEW63 |
| NEW66 | 272 | NEW40 55-HGDHNS-60 144-HGDGHH-149 | 23 | NEW64 |
| NEW76 | 273 | NEW40 55-HGDHNS-60 144-SGDGHH-149 | 17 | NEW64 |
| NEW105 | 274 | NEW40 55- -60 | 41,42,43 | NEW40 |
| NEW106 | 275 | NEW40 144- -149 | 44,45 | NEW40 |
| NEW107 | 276 | NEW40 55- -60 144- -149 | 44,45 | NEW105 |

| | | | | |
|---|---|---|---|---|
| * The underlined amino acid residues represent the modification in protein sequence. Nucleotides/amino acid residues are deleted in NEW105, NEW106 and NEW107 constructs. ** silent mutation, i.e. the polypeptide is the same as New1. | | | | |

Groups of 7 or 8 female BALB/c mice (Charles River) immunized as described earlier in example 1 were used for protection experiments against intranasal challenge with virulent S. pneumoniae P4241 strain. The mice were observed for 10 to 14 days post-infection. Data from Table 15 clearly indicate that the NEW35A molecule was equivalent to the parental NEW1 in term of protection. Interestingly, high survival rates where obtained for NEW 40- and NEW56-immunized groups with 7 and 8 survivors out of 8 animals, respectively. Similarly, NEW25 comprising amino acid residues 233 to 1039 protected 7 out of 8 animals from lethal infection.

**Table 14. Protection mediated by BVH-3 fragments or variants thereof in experimental pneumonia**

| Experiment | Immunogen | Alive: Dead | Days to death post-infection |
|---|---|---|---|
| 1 | Quil A | 0 : 8 | 4, 4, 4, 4, 4, 4, 4, 4 |
| | NEW 1 | 5 : 3 | 5, 7, 7, >14, >14, >14, >14, >14 |
| | NEW 35A | 5 : 2 | 9, 10, >14, >14, >14, >14, >14 |
| | NEW 40 | 7 : 1 | 13, >14, >14, >14, >14, >14, >14, >14 |
| | BVH-3M | 4 : 4 | 7, 8, 10, 12, >14, >14, >14, >14 |
| 2 | Quil A | 0 : 8 | 3, 3, 4, 4, 4, 4, 4, 4 |
| | NEW 52 | 4 : 4 | 7, 7, 8, 9, >10, >10, >10, >10 |
| | NEW56 | 8 : 0 | 8 X >10 |
| | NEW 40 | 7 : 1 | 6, >10, >10, >10, >10, >10, >10, >10 |
| 3 | QuilA | 0 : 8 | 3, 3, 4, 4, 4, 4, 4, 4 |
| | NEW 25 | 7 : 1 | 6, >13, >13, >13, >13, >13, >13, >13 |

Additionally, flow cytometry analyses of the binding capacity of the sera antibodies from the vaccinated animals revealed that NEW 40 and NEW56 antibodies labelled live intact pneumococci more efficiently than antibodies raised to BVH-3M (Table 15).

**Table 15. Binding of mouse sera antibodies at the surface of S. pneumoniae type 3 strain WU2 as measured by flow cytometry.**

| Antisera | | Fluorescence index | | |
|---|---|---|---|---|
| | Experiment 1 | Experiment 2 | Experiment 3 | Mean ± SE |
| BVH-3M | 9.2 | 11.4 | 14.5 | 11.7 ± 1.5 |
| NEW1 | 11.5 | 10.1 | nd* | 10.8 ± 0.7 |
| NEW35A | 14.3 | 12.9 | nd | 13.6 ± 0.7 |
| NEW40 | 20.4 | 19.1 | 20.2 | 19.9 ± 0.4 |
| NEW56 | nd | 16.7 | 20.2 | 18.5 ± 1.8 |
| NEW52 | nd | 16.6 | 19.3 | 18.0 ± 1.4 |
| Adjuvant alone | 1.9 | 1.6 | 1.2 | 1.6 ± 0.2 |

| | | | | |
|---|---|---|---|---|
| * nd:not done | | | | |

Cytometry results are expressed as fluorescence index value where the fluorescence index is the median fluorescence value of pneumococci treated with test sera divided by the background fluorescence value of pneumococci treated with the fluorescein conjugate alone. In these flow cytometric assays, all sera were used at a dilution of 1: 50 and the sera from mice immunized with BVH-3C fragment or QuilA adjuvant alone gave a value similar to the background value.

Altogether the protection and pneumococci antibody binding data indicate that vaccination using NEW1 or NEW40 molecules and variants thereof, directs the immune response to conserved protective surface-exposed epitopes.

### EXAMPLE 9

This example describes the cloning and expression of a chimeric deletant BVH-11-2 gene encoding for a chimeric polypeptide corresponding to BVH-11-2 conserved protective surface-exposed epitopes present in most if not all S. pneumoniae strains.

BVH-11-2 gene fragments corresponding to 4 gene regions, were amplified by PCR using pairs of oligonucleotides engineered to amplify fragments originating from SEQ ID NO:5 spanning nucleotides 1662 to 1742, 1806 to 2153, 2193 to 2414 and 2484 to 2627 from S. pneumoniae strain Sp64 BVH-11-2 gene.

The primers used, HAMJ490-491, HAMJ492-HAMJ493, HAMJ494-HAMJ495, HAMJ496-HAMJ354 had a restriction endonuclease site at the 5' end, thereby allowing directional in-frame cloning of the amplified product into the digested pET21b (+) plasmid vector (Table 16). PCR-amplified products were digested with restriction endonucleases and ligated to linearized plasmid pSL301 vector digested likewise except for the PCR-amplified fragment obtained with the primer pair HAMJ490-HAMJ491. The HAMJ490-HAMJ491 PCR-amplified product was purified from agarose gel using a QIAquick gel extraction kit from QIAgen (Chatsworth, CA) and ligated into pGEM-T plasmid vector without any prior restriction endonuclease digestion. The resultant plasmid constructs were confirmed by nucleotide sequence analysis. The recombinant plasmids containing each of the four were digested with restriction endonucleases corresponding with the 5' end of each primer pair used for the PCR-amplification. The fragments were purified from agarose gel like described earlier and were all ligated to linearized plasmid pET21b (+) digested with the restriction enzymes NdeI and HindIII for the in-frame cloning of, the four different regions of the BVH11-2 gene. Clones were first stabilized in E. coli DH5α before introduction into E. coli BL21(λDE3) for expression of a chimeric pneumococcal protein molecule.

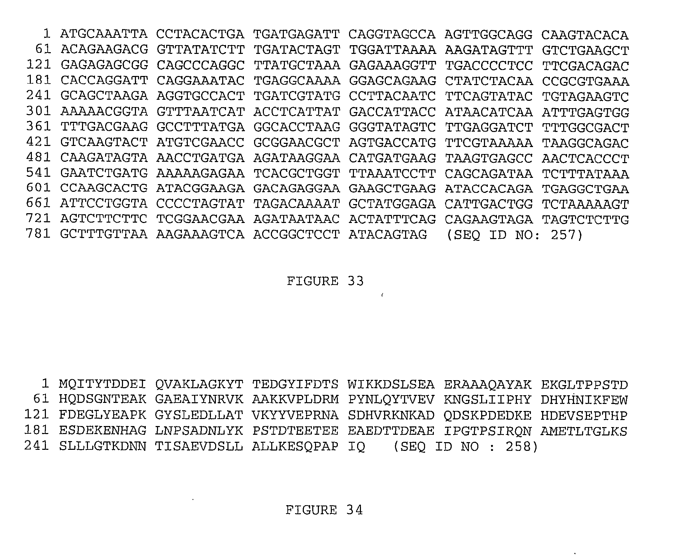

The resulting NEW43 gene sequence (SEQ ID No 257) is described in Figure 33.

The deduced amino acid sequence of NEW43 protein (SEQ ID No 258) is described in Figure 34.

**Table 16. List of PCR oligonucleotide primers used to construct the NEW43 , VP43S and NEW86**

| Primer | SEQ ID NO | Sequence 5'-3' | Nucleotide position | Restriction sites |
|---|---|---|---|---|
| HAMJ490 | 259 | ccgaattccatatgcaaattacctacactgatgatg | SEQ ID 5: 1662-1683 | NdeI |
| HAMJ491 | 260 | ggactagtatcaaagatataaccgtcttc | SEQ ID 5: 1742-1722 | SpeI |
| HAMJ492 | 261 | ggactagttggattaaaaaagatagtttgtctg | SEQ ID 5: 1806-1830 | SpeI |
| HAMJ493 | 262 | ttcccgcggttcgacatagtacttgacagtcg | SEQ ID 5: 2153-2131 | SacII |
| HAMJ494 | 263 | ttcccgcggaacgctagtgaccatgttcg | SEQ ID 5: 2193-2212 | SacII |
| HAMJ495 | 264 | cggggtaccaggaatttcagcctcatctgtg | SEQ ID 5: 2414-2393 | KpnI |
| HAMJ496 | 265 | cccggtacccctagtattagacaaaatgctatggag | SEQ ID 5 : 2484-2510 | KpnI |
| HAMJ354 | 65 | cgccaagcttctgtataggagccggttgac | SEQ ID 5: 2627-2608 | HindIII |
| HAMJ583 | 266 | ggatcccgggaggtatgattaaactaccg | SEQ ID 5 : 2039-2021 | SmaI |
| HAMJ584 | 267 | catgcccgggaacatcaaatttgagtggtttgac | SEQ ID 5: 2058-2081 | SmaI |
| HAMJ610 | 268 | cttgatcgacatatgttggcaggcaagtacacaacag | SEQ ID. 5: 1701-1722 | NdeI |

**Table 17. List of truncated BVH-11-2 gene fragments generated from S. pneumoniae SP64 for the construction of NEW43**

| PCR-primer sets | Gene fragment designation | Corresponding amino acid residues on SEQ ID NO : 8 | Cloning vector |
|---|---|---|---|
| HAMJ490-HAMJ491 | NEW43a | 517-543 | pGEM-T |
| HAMJ492-HAMJ493 | NEW43b | 565-680 | pSL301 |
| HAMJ494-HAMJ495 | NEW43c | 694-767 | pSL301 |
| HAMJ496-HAMJ354 | NEW43d | 791-838 | pSL301 |

**Table 18. Properties of NEW86 and VP43S genes generated from NEW43 gene**

| PCR-primer sets | Gene/Protein designation | Identification |
|---|---|---|
| HAMJ610-HAMJ354 | VP43S | NEW43 C' end corresponding to residues 15-272) |
| HAMJ490-HAMJ583 HAMJ584-HAMJ354 | NEW 86 | NEW43 109- PG -114 |

NEW43-derived molecules designated VP43S and NEW 86 were generated from gene amplification and cloning experiments using PCR primers described in Tables 16 and 18 and pET21 expression plasmid vector. Variants from NEW43 were generated by mutagenesis using the Quickchange Site-Directed Mutagenesis kit from Stratagene and the oligonucleotides designed to incorporate the appropriate mutation. The presence of 6 histidine tag residues on the C-terminus of the recombinant molecules simplified the purification of the proteins by nickel chromatography. The following tables 19 and 20 describe the sequences of the primers used for the mutagenesis experiments and the NEW43 variant gene products generated, respectively.

**Table 19. List of PCR oligonucleotide primer sets used for site-directed mutagenesis on NEW43 gene**

| Primer set | Primer identification | SEQ ID NO | Primer SEQUENCE 5' --- > 3' |
|---|---|---|---|
| 1 | HAMJ 497 | 269 | AACGGTAGTTTAATCATACCTTCTTATGACCATTACCATAACATC GATGTTATGGTAATGGTCATAAGAAGGTATGATTAAACTACCGTT |
| | HAMJ 498 | 270 | |
| 2 | HAMJ499 | 271 | AATCATACCTTCTTATGACTCTTACCATAACATCAAATTTGAGTG CACTCAAATTTGATGTTATGGTAAGAGTCATAAGAAGGTATGATT |
| | HAMJ500 | 272 | |
| 3 | HAMJ501 | 273 | TACCTTCTTATGACTCTTACTCTAACATCAAATTTGAGTGGTTTG CAAACCACTCAAATTTGATGTTAGAGTAAGAGTCATAAGAAGGTA |
| | HAMJ502 | 274 | |
| 26 | HAMJ530 | 275 | AATCATACCTCATTATGACTCTTACCATAACATCAAATTTGAGTG CACTCAAATTTGATGTTATGGTAAGAGTCATAATGAGGTATGATT |
| | HAMJ531 | 276 | |
| 27 | HAMJ532 | 277 | TACCTCATTATGACCATTACTCTAACATCAAATTTGAGTGGTTTG CAAACCACTCAAATTTGATGTTAGAGTAATGGTCATAATGAGGTA |
| | HAMJ533 | 278 | |
| 29 | HAMJ569 | 279 | TACCTCATTATGACTCTTACTCTAACATCAAATTTGAGTGGTTTG CAAACCACTCAAATTTGATGTTAGAGTAAGAGTCATAATGAGGTA |
| | HAMJ570 | 280 | |
| 30 | HAMJ571 | 281 | TACCTTCTTATGACCATTACTCTAACATCAAATTTGAGTGGTTTG AAACCACTCAAATTTGATGTTAGAGTAATGGTCATAAGAAGGTA |
| | HAMJ572 | 282 | |
| 31 | HAMJ573 | 283 | AACGGTAGTTTAATCATACCTTCTAAAGACCATTACCATAACATC GATGTTATGGTAATGGTCTTTAGAAGGTATGATTAAACTACCGTT |
| | HAMJ574 | 284 | |
| 32 | HAMJ575 | 285 | CGGTAGTTTAATCATACCTCATAAGGACTCTTACCATAACATCAAA TTTGATGTTATGGTAAGAGTCCTTATGAGGTATGATTAAACTACCG |
| | HAMJ576 | 286 | |
| 33 | HAMJ577 | 287 | AACGGTAGTTTAATCATACCTGACCATTACCATAACATCAAATTTG CAAATTTGATGTTATGGTAATGGTCAGGTATGATTAAACTACCGTT |
| | HAMJ578 | 288 | |
| 34 | HAMJ579 | 289 | AACGGTAGTTTAATCATACCTTACCATAACATCAAATTTGAGTGG CCACTCAAATTTGATGTTATGGTAAGGTATGATTAAACTACCGTT |
| | HAMJ580 | 290 | |
| 35 | HAMJ581 | 291 | ACCGGTAGTTTAATCATACCTAACATCAAATTTGAGTGGTTTGAC GTCAAACCACTCAAATTTGATGTTAGGTATGATTAAACTACCGTT |
| | HAMJ582 | 292 | |

**Table 20. List of NEW43 variant gene products generated from S. pneumoniae SP64**

| Polypeptide designation | Polypeptide SEQ ID NO | Polypeptide identification* | PCR primer set (ref. table 22) | Gene used for mutagenesis |
|---|---|---|---|---|
| NEW60 | 293 | NEW43 109-SYDHYH-114 | 1 | NEW43 |
| NEW61 | 294 | NEW43 109-HYDSYH-114 | 26 | NEW43 |
| NEW62 | 295 | NEW43 109-HYDHYS-114 | 27 | NEW43 |
| NEW80 | 296 | NEW43 109-SYDSYH-114 | 2 | NEW60 |
| NEW81 | 297 | NEW43 109-SYDSYS-114 | 3 | NEW80 |
| NEW82 | 298 | NEW43 109-HYDSYS-114 | 29 | NEW61 |
| NEW83 | 299 | NEW43 109-SYDHYS-114 | 30 | NEW60 |
| NEW84 | 300 | NEW43 109-SKDHYH-114 | 31 | NEW60 |
| NEW85 | 301 | NEW43 109-HKDSYH-114 | 32 | NEW61 |
| NEW88D1 | 302 | NEW43 109- DHYH-114 | 33 | NEW43 |
| NEW88D2 | 303 | NEW43 109- YH-114 | 34 | NEW88D1 |
| NEW88 | 304 | NEW43 109- -114 | 35 | NEW88D2 |

| | | | | |
|---|---|---|---|---|
| * The underlined amino acid residues represent the modification in protein sequence. Nucleotides/amino acid residues are deleted in NEW88D1, NEW88D2 and NEW88 constructs. | | | | |

Groups of 7 or 8 female BALB/c mice (Charles River) immunized as described earlier in example 1 were used for protection experiments against intranasal challenge with virulent S. pneumoniae P4241 strain. Data from Table 21 clearly indicate that NEW 19, NEW43 and variants thereof provided protection against experimental pneumonia.

**Table 21. Protection mediated by NEW19 and NEW43 fragments or variants thereof in experimental pneumonia**

| Experiment | Immunogen | Alive : Dead | Median day alive |
|---|---|---|---|
| 1 | Quil A | 0 : 8 | 4, 4, 4, 4, 4, 4, 4, 5 |
| | NEW 19 | 7 : 1 | 5, 7X >14 |
| | NEW 43 | 8 : 0 | 8X >14 |
| 2 | Quil A | 0 : 8 | 4, 4, 4, 4, 4, 5, 5, 5 |
| | NEW 43 | 7 : 1 | 8, 7X >14 |
| | NEW 80 | 6 : 2 | 5, 6, 6X >14 |
| | NEW 83 | 6 : 2 | 8, 10, 6X >14 |
| 3 | Quil A | 0 : 8 | 4, 4, 4, 4, 5, 5, 5, 5 |
| | NEW 43 | 7 : 1 | 5, 7X >8 |
| | NEW 88D1 | 5 : 3 | 5, 6, 6, 6X >8 |
| | NEW 88D2 | 5 : 3 | 6, 6, 6, 6 X >8 |
| | NEW 88 | 7 : 1 | 6, 7X >8 |
| 3 | Quil A | 0 : 8 | 4, 4, 4, 5, 5, 5, 5, 6 |
| | NEW 60 | 8 : 0 | 8 X >8 |
| | NEW 84 | 8 : 0 | 8 X >8 |
| | NEW 85 | 5 : 3 | 5, 7, 7, 5 X >8 |
| | NEW 86 | 5 : 3 | 5, 6, 6, 5 X >8 |

### EXAMPLE 10

This example describes the cloning and expression of chimeric genes encoding for a chimeric protein corresponding to the carboxy-terminal region of BVH-3 or variants thereof in fusion, at either the carboxyl end or the amino end, to NEW43 or variants thereof.

The chimeric genes comprising a BVH-3 truncate variant gene and a NEW43 or NEW43 variant gene have been designed following the procedure described in example 1. The polypeptides encoded by these chimeric genes are listed in the table 22. Briefly, gene fragments to be included in a chimeric gene were amplified by PCR using pairs of oligonucleotides engineered so that the primers had a restriction endonuclease site at the 5' end, thereby allowing directional in-frame cloning of the amplified product into digested plasmid vectors (Table 23 and Table 24). PCR-amplified products were digested with restriction endonucleases and ligated to linearized plasmid pSL301 vector. The resultant plasmid construct were confirmed by nucleotide sequence analysis. The recombinant pSL301 plasmids containing a PCR product were redigested with the same endonuclease restriction enzyme for the obtention of the DNA inserts. The resulting insert DNA fragments were purified and inserts corresponding to a given chimeric gene were ligated into pURV22-NdeI vector for the generation of a chimeric gene. The expressed recombinant proteins were purified from supernatant fractions obtained from centrifugation of sonicated heat-induced E. coli cultures using multiple chromatographic purification steps.

**Table 22. List of polypeptides encoded by chimeric genes comprising a BVH-3 truncate variant gene and a NEW43 or NEW43 variant gene**

| Polypeptide designation | SEQ ID NO | Identification |
|---|---|---|
| VP 89 | 327 | M-New56 -GP- New43* |
| VP 90 | 328 | M-New43 -GP- New56 |
| VP 91 | 329 | M-New52 -GP- New43 |
| VP 92 | 330 | M-New43 -GP- New52 |
| VP 93 | 331 | M-New56-GP-New60 |
| VP 94 | 332 | M-New60 -GP- New56 |
| VP 108 | 333 | M-New56-GP-New88 |
| VP109 | 334 | M-New88 -GP-New56 |
| VP110 | 335 | M-New60 -GP- New105 |
| VP111 | 336 | M-New60 -GP- New107 |
| VP112 | 337 | M-New88 -GP-New105 |
| VP113 | 338 | M-New88 -GP-New107 |
| VP114 | 339 | M-New80 -GP- New105 |
| VP115 | 340 | M-New80 -GP- New107 |
| VP116 | 341 | M-New83 -GP-New105 |
| VP117 | 342 | M-New83 -GP-New107 |
| VP119 | 343 | M-New43S- GP-New105 |
| VP120 | 344 | M-New43S- GP-New107 |
| VP121 | 345 | M-New80S-GP-Newl05 |
| VP122 | 346 | M-New80S- GP-New107 |
| VP123 | 347 | M-New88S- GP-New105 |
| VP124 | 348 | M-New88S- GP-New107 |

| | | |
|---|---|---|
| * Encoded amino acids for the chimeras are expressed as the gene product, additional amino acid residues were added M is methionine, G is glycine and P is proline. | | |

**Table 23. List of PCR oligonucleotide primer pairs designed for the generation of the chimeric genes encoding the polypeptides listed in Table 22.**

| Primer set | PCR-primer identification | Gene used for PCR amplification | Corresponding position of the gene fragment on |
|---|---|---|---|
| 49 | HAMJ490-HAMJ471 | Variant New43 | N-terminal |
| 50 | HAMJ564-HAMJ556 | Variant New43 | C-terminal |
| 51 | HAMJ489-HAMJ359 | Variant New40 | N-terminal |
| 52 | HAMJ559-HAMJ557 | Variant New40 | C-terminal |
| 53 | HAMJ610-HAMJ471 | Variant New43S | N-terminal |

**Table 24. List of PCR oligonucleotide primers designed for the generation of the chimeric genes encoding the polypeptides listed in Table 22.**

| Primer | SEQ ID NO | Sequence 5'-3' | Restriction site |
|---|---|---|---|
| HAMJ490 | 259 | ccgaattccatatgcaaattacctacactgatgatg | NdeI |
| HAMJ471 | 168 | atatgggcccctgtataggagccggttgactttc | ApaI |
| HAMJ564 | 327 | atatgggccccaaattacctacactgatgatgagattcagg | ApaI |
| HAMJ556 | 328 | ataagaatgcggccgcctactgtataggagccggttgactttc | NotI |
| HAMJ489 | 329 | ccgaattccatatgcaaattgggcaaccgactc | NdeI |
| HAMJ359 | 173 | tcccgggccccgctatgaaatcagataaattc | ApaI |
| HAMJ559 | 330 | atatgggccccaaattgggcaaccgactc | ApaI |
| HAMJ354 | 65 | cgccaagcttctgtataggagccggttgac | HindIII |
| HAMJ610 | 268 | cttgatcgacatatgttggcaggcaagtacacaacag | NdeI |
| HAMJ557 | 331 | ataagaatgcggccgcttacgctatgaaatcagataaattc | NotI |
| HAMJ279 | 35 | cgccaagcttcgctatgaaatcagataaattc | HindIII |

## Claims

1. An isolated polypeptide fragment of a BVH-11-2 polypeptide, said BVH-11-2 polypeptide comprising the amino acid sequence set forth in SEQ ID NO:8, wherein the polypeptide fragment comprises an amino acid sequence at least 95% identical to the amino acid sequence set forth at positions 227-838 of SEQ ID NO:8, and wherein the isolated polypeptide is capable of inducing an immune response to *Streptococcus pneumoniae.*

2. The isolated polypeptide according to claim 1 wherein the polypeptide fragment consists of an amino acid sequence at least 95% identical to the amino acid sequence set forth at positions 227-838 of SEQ ID NO:8.

3. An isolated polypeptide consisting of an amino acid sequence at least 95% identical to the amino acid sequence set forth at positions 497-838 of SEQ ID NO:8, wherein the polypeptide is capable of inducing an immune response to *Streptococcus pneumoniae.*

4. The isolated polypeptide according to claim 1 wherein the polypeptide consists of the amino acid sequence set forth at positions 497-838 of SEQ ID NO:8.

5. An isolated polypeptide consisting of an amino acid sequence at least 95% identical to the amino acid sequence set forth in SEQ ID NO:21, SEQ ID NO:22, or SEQ ID NO:23, wherein the polypeptide is capable of inducing an immune response to *Streptococcus pneumoniae.*

6. The isolated polypeptide of any one of claims 1-5, wherein the polypeptide is fused to a saccharide.

7. The isolated polypeptide of any one of claims 1-5, wherein the polypeptide is fused to a moiety, wherein the moiety is a purification tag.

8. An isolated polynucleotide that encodes the polypeptide according to any one of claims 1 to 5.

9. An expression vector comprising the polynucleotide according to claim 8, wherein the polynucleotide is operably linked to an expression control region.

10. A host cell comprising the expression vector according to claim 9.

11. A process for producing the polypeptide of any one of claim 1 to 5, said process comprising
(a) culturing a host cell according to claim 10 under conditions suitable for expression of the polypeptide; and
(b) isolating the polypeptide.

12. A vaccine composition comprising
(a) the polypeptide according to any one of claims 1 to 5; and
(b) a pharmaceutically acceptable carrier or diluent.

13. The vaccine composition according to claim 12, further comprising a pharmaceutically acceptable adjuvant.

14. Use of the polypeptide according to any one of claims 1 to 5 for the manufacture of a medicament for treatment or prophylaxis of a streptococcal infection.

15. Use of a polypeptide comprising an amino acid sequence at least 95% identical to the amino acid sequence set forth in SEQ ID NO:8 for the manufacture of a medicament for treatment or prophylaxis of a streptococcal infection in a human infant, wherein the unit dose of the polypeptide is 10 µg to 100 µg.
